(19) Europäisches Patentamt
European Patent Office
Office européen des brevets

(11) EP 1 070 711 B1

(12) EUROPEAN PATENT SPECIFICATION

(45) Date of publication and mention of the grant of the patent:
**14.04.2004 Bulletin 2004/16**

(21) Application number: **00306196.7**

(22) Date of filing: **20.07.2000**

(51) Int Cl.[7]: **C07D 401/04**, C07D 401/14, C07D 453/02, C07D 451/02, C07D 471/04, C07D 455/02, A61P 11/06, A61P 9/10, A61P 17/06, A61P 29/00, A61K 31/4427, A61K 31/4375

# (54) Heteroaryl-substituted pyrrole derivatives, their preparation and their therapeutic uses

Heteroaryl-substituierte Pyrrole, Verfahren zu ihrer Herstellung und ihre therapeutische Anwendung

Dérivés de pyrrole hétéroaryl substitués, leur préparation et leur utilisation thérapeutique

(84) Designated Contracting States:
**AT BE CH CY DE DK ES FI FR GB GR IE IT LI LU MC NL PT SE**

(30) Priority: **21.07.1999 JP 20549199**
**27.12.1999 JP 36967899**

(43) Date of publication of application:
**24.01.2001 Bulletin 2001/04**

(60) Divisional application:
**02011912.9 / 1 243 589**

(73) Proprietor: **Sankyo Company Limited**
**Chuo-ku, Tokyo 103-8426 (JP)**

(72) Inventors:
- **Kimura, Tomio**
**Shinagawa-ku, Tokyo 140-8710 (JP)**
- **Aoki, Kazumasa**
**Shinagawa-ku, Tokyo 140-8710 (JP)**
- **Nakao, Akira**
**Shinagawa-ku, Tokyo 140-8710 (JP)**
- **Ushiyama, Shigeru**
**Shinagawa-ku, Tokyo 140-8710 (JP)**
- **Shimozato, Takaichi**
**Shinagawa-ku, Tokyo 140-8710 (JP)**
- **Ohkawa, Nobuyuki**
**Shinagawa-ku, Tokyo 140-8710 (JP)**

(74) Representative: **Gibson, Christian John Robert**
**MARKS & CLERK,**
**57/60 Lincoln's Inn Fields**
**London WC2A 3LS (GB)**

(56) References cited:
**WO-A-96/21452**
**WO-A-97/05877**
**US-A- 5 792 778**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 070 711 B1

## Description

The present invention relates to a series of heteroaryl-substituted pyrrole derivatives which have excellent inhibitory activity against the production of inflammatory cytokines such as interleukin (IL)-1, IL-6 and IL-8 and tumor necrosis factor (TNF), particularly IL-1 and TNF. As a consequence, the compounds of the present invention have valuable anti-pyretic, analgesic, anti-viral and anti-inflammatory activity and are useful in the prophylaxis and treatment of autoimmune diseases such as chronic rheumatism, bone diseases such as osteoporosis and the many other diseases in which the above-described inflammatory cytokines take part. The invention also provides compositions containing these novel compounds, uses of said novel compounds and processes for their preparation.

Non-steroidal anti-inflammatory drugs (NSAIDs) have been widely used for the treatment and prophylaxis of various inflammatory diseases and in pain relief because they have, as their main pharmacological activity, anti-pyretic, analgesic, and anti-inflammatory activity which is based on their ability to inhibit the biosynthesis of prostaglandin (PG) through the inhibition of cyclooxygenase activity. Another class of compounds commonly used for the treatment of rheumatoid arthritis is the disease-modifying anti-rheumatic drugs (DMARDs), examples of which include methotrexate and sulphasalazine. This is a wide class of drugs in which the compounds have no common mechanism of action. For the treatment of chronic rheumatism, NSAIDs are used nosotropically and DMARDs are used etiotropically. There are a number of problems associated with these classes of drugs. Conventional NSAIDs can induce undesirable side effects including gastrointestinal disorders such as gastric ulcers and renal disorders, resulting in difficulties for any patient who has to take such a drug for an extended period of time. DMARDs can also induce undesirable side effects including nausea and diarrhoea and, furthermore, they have not yet been clearly shown to exhibit a stable, long-lasting effect.

A class of active substances generally called cytokines, which are produced in the body by immunocytes, has recently been found. One group of cytokines is known as the inflammatory cytokines and it includes interleukin (IL)-1, IL-6 and IL-8 and tumor necrosis factor (TNF). The inflammatory cytokines have been demonstrated to play a major role in a number of biological processes. These include action as an inflammatory mediator through the stimulation of the arachidonic acid metabolic pathway leading to the production of PG, the migration of leukocytes, the production of acute phase protein, and activation of osteoclasts.

It is believed that the inflammatory cytokines are associated with many diseases including inflammatory diseases and the induction of bone resorption. Due to their mechanism of action, which is different from that of conventional drugs such as those described above, compounds which are able to inhibit the production of inflammatory cytokines are expected to provide an improved new generation of anti-pyretic, analgesic and anti-inflammatory drugs and medicaments for the treatment of autoimmune diseases such as chronic rheumatism, bone diseases such as osteoporosis and the many other diseases in which the above-described inflammatory cytokines are believed to take part.

Compounds which are said to demonstrate inhibitory activity against the production of inflammatory cytokines include various heteroaryl compounds [see, for example, WO 96/21452, WO 97/5877, US-A-5792778, WO 97/23479, US 5 792 778 and J. Med. Chem., 39, 3929-3937 (1996)]. Examples of compounds of this type include the following:

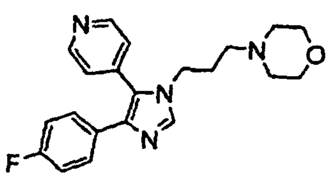

SB210313

J.Med.Chem., 39, 3929-3937(1996)

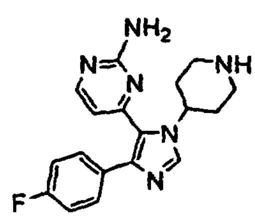

WO97/23479 Compound of Example 6

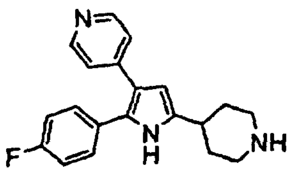

WO97/5877
Compound of
Example 4

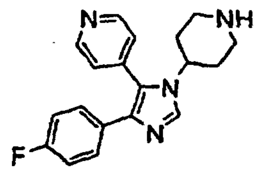

WO96/21452
Compound of
Example 23

There is, however, a need for further compounds having improved activity, pharmacokinetics and safety.

The compounds of the present invention are compounds of the following formula (I), and pharmacologically acceptable salts, esters or other derivatives thereof:

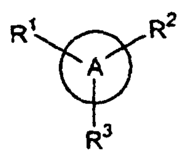

(I)

wherein:

A represents a pyrrole ring;

$R^1$ is a phenyl or naphthyl group which may optionally be substituted with at least one substituent selected from the group consisting of Substituent group α defined below, Substituent group β defined below, Substituent group γ defined below and Substituent group δ defined below;

$R^2$ is a pyridyl or pyrimidinyl group which may optionally be substituted with at least one substituent selected from the group consisting of Substituent group α defined below, Substituent group β defined below, Substituent group γ defined below and Substituent group δ defined below; and

$R^3$ is a group of the following formula (II):

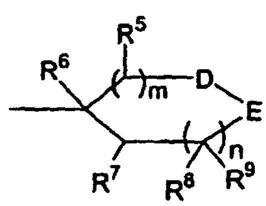

(II)

wherein

m represents 0 or 1,
n represents 1 or 2,
one of D and E represents a group of formula $>NR^{10}$ and the other represents a group of formula $>CR^{11}R^{12}$,
one of $R^5$ and $R^7$ together with $R^6$ represents a single bond and the other represents a substituent selected from the group consisting of hydrogen atoms, Substituent group α defined below, Substituent group γ defined below and Substituent group δ defined below,

$R^8$, $R^9$ and $R^{12}$ may be the same or different from one another and each represents a substituent selected from the group consisting of hydrogen atoms, Substituent group α defined below, Substituent group γ defined below and Substituent group δ defined below, and $R^{10}$ and $R^{11}$ may be the same or different from one another and each represents a substituent selected from the group consisting of hydrogen atoms, Substituent group α defined below, Substituent group γ defined below and Substituent group δ defined below or $R^{10}$ and $R^{11}$ together form an alkylene group having from 1 to 6 carbon atoms which is unsubstituted or is substituted with at least one substituent selected from the group consisting of Substituent group α defined below, Substituent group β defined below, Substituent group y defined below and Substituent group δ defined below;

PROVIDED THAT said substituents $R^1$ and $R^3$ are bonded to the two atoms of said pyrrole ring which are adjacent to the atom of the pyrrole ring to which said substituent $R^2$ is bonded;

Substituent group α comprises hydroxyl groups, nitro groups, cyano groups, halogen atoms, lower alkoxy groups defined below, halogeno lower alkoxy groups defined below, lower alkylthio groups defined below, and halogeno lower alkylthio groups defined below;

Substituent group β comprises groups of formula $-NR^cR^d$, wherein $R^c$ and $R^d$ are the same or different from each other and each is independently selected from the group consisting of hydrogen atoms, lower alkyl groups defined below, lower alkenyl groups defined below, lower alkynyl groups defined below, aralkyl groups defined below and lower alkylsulfonyl groups, or
$R^c$ and $R^d$, together with the nitrogen atom to which $R^c$ and $R^d$ are bonded, form a heterocyclyl group defined below;

Substituent group γ comprises lower alkyl groups defined below which are substituted with a group of formula $-NR^cR^d$, wherein $R^c$ and $R^d$ are as defined above; and

Substituent group δ comprises lower alkyl groups defined below which may optionally be substituted with at least one substituent selected from Substituent group α defined above, lower alkenyl groups defined below which may optionally be substituted with at least one substituent selected from Substituent group α defined above, lower alkynyl groups defined below which may optionally be substituted with at least one substituent selected from Substituent group α defined above, aralkyl groups defined below and cycloalkyl groups defined below;

said lower alkyl groups in the definition of substituents $R^c$ and $R^d$ above, said lower alkyl groups which may optionally be substituted with at least one substituent selected from Substituent group α in the definition of Substituent group δ above and said lower alkyl moiety of the lower alkyl groups substituted with a group of formula $-NR^cR^d$ in the definition of Substituent group γ above are straight or branched alkyl groups having from 1 to 6 carbon atoms;

said lower alkenyl groups in the definition of substituents $R^c$ and $R^d$ above and said lower alkenyl groups which may optionally be substituted with at least one substituent selected from Substituent group α in the definition of Substituent group 8 above are straight or branched alkenyl groups having from 2 to 6 carbon atoms;

said lower alkynyl groups in the definition of substituents $R^c$ and $R^d$ above and said lower alkynyl groups which may optionally be substituted with at least one substituent selected from Substituent group α in the definition of Substituent group δ above are straight or branched alkynyl groups having from 2 to 6 carbon atoms;

said aralkyl groups in the definition of $R^c$ and $R^d$ above and in the definition of Substituent group δ above are lower alkyl groups as defined above which are substituted with at least one aryl group selected from aromatic hydrocarbon groups having from 6 to 14 carbon atoms in one or more rings, said aromatic hydrocarbon groups optionally being fused with a cycloalkyl group having from 3 to 10 carbon atoms;

said cycloalkyl groups in Substituent group δ above are cycloalkyl groups having from 3 to 7 carbon atoms;

said lower alkylsulfonyl groups in the definition of substituents $R^c$ and $R^d$ are lower alkyl groups as defined above which are bonded to a sulfonyl group;

said heterocyclyl groups formed by substitutents $R^c$ and $R^d$ together with the nitrogen atom to which they are bonded are 4- to 12- membered non-aromatic heterocyclyl groups containing one nitrogen atom and optionally further containing one further heteroatom selected from the group consisting of oxygen, sulphur and nitrogen

atoms, said heterocyclyl groups optionally being fused with another cyclic group selected from the group consisting of aryl groups as defined in the definition of aralkyl groups above and heteroaryl groups selected from 5- to 7-membered aromatic heterocyclic groups containing from 1 to 3 heteroatoms selected from the group consisting of sulphur, oxygen and nitrogen atoms;

said lower alkoxy groups in the definition of Substituent group $\alpha$ above are lower alkyl groups as defined above which are bonded to an oxygen atom;

said halogeno lower alkoxy groups in the definition of Substituent group $\alpha$ above are lower alkoxy groups as defined above which are substituted with at least one halogen atom;

said lower alkylthio groups in the definition of Substituent group $\alpha$ above are lower alkyl groups as defined above which are bonded to a sulfur atom; and

said halogeno lower alkylthio groups in the definition of Substituent group $\alpha$ above are lower alkylthio groups as defmed above which are substituted with at least one halogen atom.

The present invention also provides a pharmaceutical composition comprising an effective amount of a pharmacologically active compound together with a carrier or diluent therefor, wherein said pharmacologically active compound is a compound of formula (I) or a pharmacologically acceptable salt, ester or amide thereof.

The present invention also provides a compound of formula (I) or a pharmacologically acceptable salt, ester or amide thereof for use as a medicament.

The present invention also provides the use of at least one compound of formula (I) or a pharmacologically acceptable salt, ester or amide thereof in the manufacture of a medicament for inhibiting the production of inflammatory cytokines in a mammal, which may be human.

The present invention also provides the use of at least one compound of formula (I) or a pharmacologically acceptable salt, ester or amide thereof in the manufacture of a medicament for inhibiting bone resorption in a mammal, which may be human.

The present invention also provides the use of at least one compound of formula (I) or a pharmacologically acceptable salt, ester or amide thereof in the manufacture of a medicament for the treatment or prophylaxis of inflammatory diseases in a mammal, which may be human.

The present invention also provides the use of at least one compound of formula (I) or a pharmacologically acceptable salt, ester or amide thereof in the manufacture of a medicament for the treatment or prophylaxis of viral diseases in a mammal, which may be human.

The present invention also provides the use of at least one compound of formula (I) or a pharmacologically acceptable salt, ester or amide thereof in the manufacture of a medicament for relieving pain or pyrexia in a mammal, which may be human.

The present invention also provides the use of at least one compound of formula (I) or a pharmacologically acceptable salt, ester or amide in the manufacture of a medicament for the treatment or prophylaxis of chronic rheumatoid arthritis in a mammal, which may be human.

The present invention also provides the use of at least one compound of formula (I) or a pharmacologically acceptable salt, ester or amide thereof in the manufacture of a medicament for the treatment or prophylaxis of osteoarthritis in a mammal, which may be human.

The present invention also provides the use of at least one compound of formula (I) or a pharmacologically acceptable salt, ester or amide thereof in the manufacture of a medicament for the treatment or prophylaxis of cancer in a mammal, which may be human.

The present invention also provides the use of at least one compound of formula (I) or a pharmacologically acceptable salt, ester or amide thereof in the manufacture of a medicament for the treatment or prophylaxis of hepatitis in a mammal, which may be human.

The present invention also provides the use of at least one compound of formula (I) or a pharmacologically acceptable salt, ester or amide thereof in the manufacture of a medicament for the treatment or prophylaxis of a disease selected from the group consisting of allergic diseases, septicaemia, psoriasis, osteoporosis, ulcerative colitis, diabetes, nephritis, ischemic heart disease, Alzheimer's disease and arteriosclerosis in a mammal, which may be human.

The requirement that the substituents $R^1$ and $R^3$ must be bonded to the two atoms of the pyrrole ring which are adjacent to the atom of the pyrrole ring to which the substituent $R^2$ is bonded in the compounds of the above formula (I) means that the compounds of formula (I) are selected from compounds of the following formulae (I-1) to (I-5):

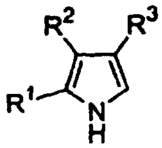
(I-1)

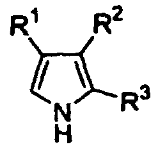
(I-2)

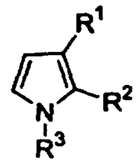
(I-3)

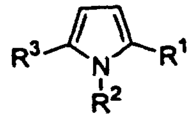
(I-4)

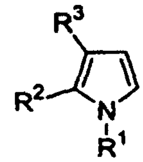
(I-5)

wherein $R^1$, $R^2$ and $R^3$ are as defined above.

Where $R^1$ represents a phenyl or naphtyl group which may optionally be substituted with at least one substituent selected from the group consisting of Substituent group α defined above, Substituent group β defined above, Substituent group γ defined above and Substituent group δ defined above; said groups are most preferably phenyl groups.

Where $R^1$ represents phenyl or naphthyl group which is substituted with at least one substituent selected from the group consisting of Substituent group α defined above, Substituent group β defined above, Substituent group γ defined above and Substituent group δ defined above, it is preferably a phenyl or naphtyl group substituted with 1 to 4 substituents selected from the group consisting of Substituent group α, Substituent group β, Substituent group γ and Substituent group δ, and more preferably it is a phenyl or naphthyl group substituted with 1 to 3 substituents selected from Substituent group α, Substituent group β, Substituent group γ and Substituent group δ. Examples of such substituted phenyl or naphthyl groups include 4-fluorophenyl, 3-fluorophenyl, 4-chlorophenyl, 3-chlorophenyl, 3,4-difluorophenyl, 3,4-dichlorophenyl, 3,4,5-trifluorophenyl, 3-chloro-4-fluorophenyl, 3-difluoromethoxyphenyl, 3-trifluoromethoxyphenyl and 3-trifluoromethylphenyl groups.

Where $R^2$ represents a pyridyl or primidinyl group, 4-pyridyl and 4-pyrimidinyl groups are most preferred.

Where $R^2$ represents a pyridyl or primidinyl group, said groups optionally being substituted with at least one substituent selected from the group consisting of Substituent group a, Substituent group β, Substituent group γ and Substituent group δ, said pyridyl or primidinyl group is preferably a group substituted with 1 to 3 substituents selected from the group consisting of Substituent group α, Substituent group β, Substituent group γ and Substituent group δ, more preferably it is a pyridyl or primidinyl group substituted with one or two substituents selected from the group consisting of Substituent group α, Substituent group β, Substituent group γ and Substituent group δ, still more preferably it is a pyridyl or primidinyl group substituted with one substituent selected from the group consisting of Substituent group α, Substituent group β, Substituent group γ and Substituent group δ, and particularly preferably it is a 4-pyridyl or 4-pyrimidinyl group which is substituted with one substituent selected from the group consisting of Substituent group α, Substituent group β, Substituent group γ and Substituent group δ at the 2-position of said group. Most preferably, said group is a 4-pyridyl or 4-pyrimidinyl group which is substituted at the 2-position with one substituent selected from the group consisting of Substituent group β and Substituent group γ. Examples of such substituted groups include 2-amino-4-pyridyl, 2-amino-4-pyrimidinyl, 2-methylamino-4-pyridyl, 2-methylamino-4-pyrimidinyl, 2-methoxy-4-pyridyl, 2-methoxy-4-pyrimidinyl, 2-benzylamino-4-pyridyl, 2-benzylamino-4-pyrimidinyl, 2-(α-methylbenzylamino)-4-pyridyl and 2-(α-methylbenzylamino)-4-pyrimidinyl groups.

The lower alkyl groups in the definition of substituents $R^c$ and $R^d$, the lower alkyl groups which may optionally be substituted with at least one substituent selected from Substituent group α in the definition of Substituent group δ and the lower alkyl moiety of the lower alkyl groups substituted with a group of formula $-NR^cR^d$ in the definition of Substituent group y are straight or branched alkyl groups having from 1 to 6 carbon atoms. Examples of said lower alkyl groups include methyl, ethyl, propyl, isopropyl, n-butyl, isobutyl, s-butyl, tert-butyl, n-pentyl, isopentyl, 2-methylbutyl, neopentyl, 1-ethylpropyl, n-hexyl, isohexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl and 2-ethylbutyl groups. Alkyl groups having from 1 to 4 carbon atoms are preferred, methyl, ethyl and propyl groups are more preferred, and methyl and ethyl groups are most preferred.

The lower alkenyl groups in the definition of substituents $R^c$ and $R^d$ and the lower alkenyl groups which may optionally be substituted with at least one substituent selected from Substituent group α in the definition of Substituent group δ are straight or branched alkenyl groups having from 2 to 6 carbon atoms. Examples of said lower alkenyl groups include vinyl, 2-propenyl, 1-methyl-2-propenyl, 2-methyl-2-propenyl, 2-ethyl-2-propenyl, 2-butenyl, 1-methyl-2-butenyl, 2-methyl-2-butenyl, 1-ethyl-2-butenyl, 3-butenyl, 1-methyl-3-butenyl, 2-methyl-3-butenyl, 1-ethyl-3-butenyl, 2-pentenyl, 1-methyl-2-pentenyl, 2-methyl-2-pentenyl, 3-pentenyl, 1-methyl-3-pentenyl, 2-methyl-3-pentenyl, 4-pentenyl, 1-methyl-4-pentenyl, 2-methyl-4-pentenyl, 2-hexenyl, 3-hexenyl, 4-hexenyl and 5-hexenyl groups. Alkenyl groups having from 2 to 4 carbon atoms are preferred, and alkenyl groups having 2 or 3 carbon atoms are most preferred.

The lower alkynyl groups in the definition ofsubstituents $R^c$ and $R^d$ and the lower alkynyl groups which may

optionally be substituted with at least one substituent selected from Substituent group α in the definition of Substituent group δ are straight or branched alkynyl groups having from 2 to 6 carbon atoms. Examples of said lower alkynyl groups include ethynyl, 2-propynyl, 1-methyl-2-propynyl, 2-butynyl, 1-methyl-2-butynyl, 1-ethyl-2-butynyl, 3-butynyl, 1-methyl-3-butynyl, 2-methyl-3-butynyl, 1-ethyl-3-butynyl, 2-pentynyl, 1-methyl-2-pentynyl, 3-pentynyl, 1-methyl-3-pentynyl, 2-methyl-3-pentynyl, 4-pentynyl, 1-methyl-4-pentynyl, 2-methyl-4-pentynyl, 2-hexynyl, 3-hexynyl, 4-hexynyl and 5-hexynyl groups. Alkynyl groups having from 2 to 4 carbon atoms are preferred, and alkynyl groups having 2 or 3 carbon atoms are most preferred.

The aralkyl group in the definition of $R^c$, $R^d$ and Substituent group δ is a lower alkyl group as defined above which is substituted with at least one aryl group having from 6 to 14 carbon atoms in one or more rings, preferably from 6 to 10 carbon atoms, said groups optionally being fused with a cycloalkyl group having from 3 to 10 carbon atoms. Examples of said aralkyl group include benzyl, phenanthrenyl-methyl, anthracenylmethyl, α-naphthylmethyl, β-naphthylmethyl, diphenylmethyl, triphenylmethyl, α-naphthyldiphenylmethyl, 9-anthrylmethyl, piperonyl, 1-phenethyl, 2-phenethyl, 1-naphthylethyl, 2-naphthylethyl, 1-phenylpropyl, 2-phenylpropyl, 3-phenylpropyl, 1-naphthylpropyl, 2-naphthylpropyl, 3-naphthylpropyl, 1-phenylbutyl, 2-phenylbutyl, 3-phenylbutyl, 4-phenylbutyl, 1-naphthylbutyl, 2-naphthylbutyl, 3-naphthylbutyl, 4-naphthylbutyl, 1-phenylpentyl, 2-phenylpentyl, 3-phenylpentyl, 4-phenylpentyl, 5-phenylpentyl, 1-naphthylpentyl, 2-naphthylpentyl, 3-naphthylpentyl, 4-naphthylpentyl, 5-naphthylpentyl, 1-phenylhexyl, 2-phenylhexyl, 3-phenylhexyl, 4-phenylhexyl, 5-phenylhexyl, 6-phenylhexyl, 1-naphthylhexyl, 2-naphthylhexyl, 3-naphthylhexyl, 4-naphthylhexyl, 5-naphthylhexyl and 6-naphthylhexyl groups, of which benzyl, phenanthrenylmethyl, anthracenylmethyl, α-naphthylmethyl, β-naphthylmethyl, diphenylmethyl, triphenylmethyl, 9-anthrylmethyl, piperonyl, 1-phenethyl, 2-phenethyl, 1-phenylpropyl, 2-phenylpropyl, 3-phenylpropyl, 1-phenylbutyl, 2-phenylbutyl, 3-phenylbutyl and 4-phenylbutyl are preferred.

The aryl moiety of the aralkyl groups defined and exemplified above may be substituted with from 1 to 3 groups selected from the group consisting of Substituent group α defined above, Substituent group β defined above, Substituent group γ defined above and Substituent group δ defined above. Examples of said substituted aralkyl groups include aralkyl groups substituted with at least one halogen atom, examples of which include 2-fluorobenzyl, 3-fluorobenzyl, 4-fluorobenzyl, 2-chlorobenzyl, 3-chlorobenzyl, 4-chlorobenzyl, 2-bromobenzyl, 3-bromobenzyl, 4-bromobenzyl, 3,5-difluorobenzyl, 2,5-difluorophenethyl, 2,6-difluorobenzyl, 2,4-difluorophenethyl, 3,5-dibromobenzyl, 2,5-dibromophenethyl, 2,6-dichlorobenzyl, 2,4-dichlorophenethyl, 2,3,6-trifluorobenzyl, 2,3,4-trifluorophenethyl, 3,4,5-trifluorobenzyl, 2,5,6-trifluorophenethyl, 2,4,6-trifluorobenzyl, 2,3,6-tribromophenethyl, 2,3,4-tribromobenzyl, 3,4,5-tribromophenethyl, 2,5,6-trichlorobenzyl, 2,4,6-trichlorophenethyl, 1-fluoro-2-naphthylmethyl, 2-fluoro-1-naphthylethyl, 3-fluoro-1-naphthylmethyl, 1-chloro-2-naphthylethyl, 2-chloro-1-naphthylmethyl, 3-bromo-1-naphthylethyl, 3,8-difluoro-1-naphthylmethyl, 2,3-difluoro-1-naphthylethyl, 4,8-difluoro-1-naphthylmethyl, 5,6-difluoro-1-naphthylethyl, 3,8-dichloro-1-naphthylmethyl, 2,3-dichloro-1-naphthylethyl, 4,8-dibromo-1-naphthylmethyl, 5,6-dibromo-1-naphthylethyl, 2,3,6-trifluoro-1-naphthylmethyl, 2,3,4-trifluoro-1-naphthylethyl, 3,4,5-trifluoro-1-naphthylmethyl, 4,5,6-trifluoro-1-naphthylethyl, 2,4,8-trifluoro-1-naphthylmethyl, bis(2-fluorophenyl)-methyl, 3-fluorophenylphenylmethyl, bis(4-fluorophenyl)methyl, 4-fluorophenylphenylmethyl, bis(2-chlorophenyl)methyl, bis(3-chlorophenyl)methyl, bis(4-chlorophenyl)methyl, 4-chlorophenylphenylmethyl, 2-bromophenylphenylmethyl, 3-bromophenylphenylmethyl, bis(4-bromophenyl)methyl, bis(3,5-difluorophenyl)methyl, bis(2,5-difluorophenyl)methyl, bis(2,6-difluorophenyl)methyl, 2,4-difluorophenylphenylmethyl, bis(3,5-dibromophenyl)methyl, 2,5-dibromophenylphenylmethyl, 2,6-dichlorophenylphenylmethyl, bis(2,4-dichlorophenyl)methyl and bis(2,3,6-trifluorophenyl)methyl groups; aralkyl groups substituted with at least one halogeno lower alkyl group (as defined below), examples of which include 2-trifluoromethylbenzyl, 3-trifluoromethylphenethyl, 4-trifluoromethylbenzyl, 2-trichloromethylphenethyl, 3-dichloromethylbenzyl, 4-trichloromethylphenethyl, 2-tribromomethylbenzyl, 3-dibromomethylphenethyl, 4-dibromomethylbenzyl, 3,5-bistrifluoromethylphenethyl, 2,5-bistrifluoromethylbenzyl, 2,6-bistrifluoromethylphenethyl, 2,4-bistrifluoromethylbenzyl, 3,5-bistribromomethylphenethyl, 2,5-bisdibromomethylbenzyl, 2,6-bisdichloromethylphenethyl, 2,4-bisdichloromethylbenzyl, 2,3,6-tristrifluoromethylphenethyl, 2,3,4-tristrifluoromethylbenzyl, 3,4,5-tristrifluoromethylphenethyl, 2,5,6-tristrifluoromethylbenzyl, 2,4,6-tristrifluoromethylphenethyl, 2,3,6-tristribromomethylbenzyl, 2,3,4-trisdibromomethylphenethyl, 3,4,5-tristribromomethylbenzyl, 2,5,6-trisdichloromethylphenethyl, 2,4,6-trisdichloromethylbenzyl, 1-trifluromethyl-2-naphthylethyl, 2-trifluoromethyl-1-naphthylmethyl, 3-trifluoromethyl-1-naphthylethyl, 1-trichloromethyl-2-naphthylmethyl, 2-dichloromethyl-1-naphthylethyl, 3-tribromomethyl-1-naphthylmethyl, 3,8-bistrifluoromethyl-1-naphthylethyl, 2,3-bistrifluoromethyl-1-naphthylmethyl, 4,8-bistrifluoromethyl-1-naphthylethyl, 5,6-bistrifluoromethyl-1-naphthylmethyl, 3,8-bistrichloromethyl-1-naphthylethyl, 2,3-bisdichloromethyl-1-naphthylmethyl, 4,8-bisdibromomethyl-1-naphthylethyl, 5,6-bistribromomethyl-1-naphthylmethyl, 2,3,6-tristrifluoromethyl-1-naphthylethyl, 2,3,4-tristrifluoromethyl-1-naphthylmethyl, 3,4,5-tristrifluoromethyl-1-naphthylethyl, 4,5,6-tristrifluoromethyl-1-naphthylmethyl, 2,4,8-tristrifluoromethyl-1-naphthylmethyl, bis(4-trifluoromethylphenyl)methyl, 4-trifluoromethylphenylphenylmethyl, bis(2-trichloromethylphenyl)methyl, bis(3-trichloromethylphenyl)-methyl, bis(4-trifluoromethylphenyl)methyl, 2-tribromomethylphenylphenylmethyl, 3-tribromomethylphenylphenylmethyl, bis(2-tribromomethylphenyl)methyl, bis(3,5-bistrifluoromethylphenyl)methyl, bis(2,5-bistrifluoromethylphenyl)methyl, bis(2,6-bistrifluoromethylphenyl)methyl, 2,4-bistrifluoromethylphenylphenylmethyl, bis(3,5-bistribromomethylphenyl)methyl, 2,5-bistribromomethylphenylphenylmethyl, 2,6-bistrichloromethylphenylphenylmethyl, bis(2,4-bistrichloromethylphenyl)methyl and bis(2,3,6-tristrifluoromethylphenyl)methyl groups; aralkyl groups substituted with at least one lower alkyl group (as defined above), examples of which include 2-methylbenzyl, 3-methylbenzyl, 4-methylbenzyl, 2-methylphenethyl, 4-methylphenethyl, 2-ethylbenzyl, 3-propylphenethyl, 4-ethylbenzyl, 2-butylphenethyl, 3-pentylbenzyl, 4-pentylphenethyl, 3,5-dimethylbenzyl, 2,5-dimethylphenethyl, 2,6-dimethylbenzyl, 2,4-dimethylphenethyl, 3,5-dibutylbenzyl, 2,5-dipentylphenethyl, 2,6-dipropylbenzyl, 2,4-dipropylphenethyl, 2,3,6-trimethylbenzyl, 2,3,4-trimethylphenethyl, 3,4,5-trimethylbenzyl, 2,4,6-trimethylbenzyl, 2,5,6-trimethylphenethyl, 2,3,6-tributylphenethyl, 2,3,4-tripentylbenzyl, 3,4,5-tributylphenethyl, 2,5,6-tripropylbenzyl, 2,4,6-tripropylphenethyl, 1-methyl-2-naphthylmethyl, 2-methyl-1-naphthylethyl, 3-methyl-1-naphthylmethyl, 1-ethyl-2-naphthylethyl, 2-propyl-1-naphthylmethyl, 3-butyl-1-naphthylethyl, 3,8-dimethyl-1-naphthylmethyl, 2,3-dimethyl-1-naphthylethyl, 4,8-dimethyl-1-naphthylmethyl, 5,6-dimethyl-1-naphthylethyl, 3,8-diethyl-1-naphthylmethyl, 2,3-dipropyl-1-naphthylmethyl, 4,8-dipentyl-1-naphthylethyl, 5,6-dibutyl-1-naphthylmethyl, 2,3,6-trimethyl-1-naphthylmethyl, 2,3,4-trimethyl-1-naphthylethyl, 3,4,5-trimethyl-1-naphthylmethyl, 4,5,6-trimethyl-1-naphthylmethyl, 2,4,8-trimethyl-1-naphthylmethyl, bis(2-methylphenyl)methyl, 3-methylphenylphenylmethyl, bis(4-methylphenyl)methyl, 4-methylphenylphenylmethyl, bis(2-ethylphenyl)methyl, bis(3-ethylphenyl)methyl, bis(4-ethylphenyl)methyl, 2-propylphenylphenylmethyl, 3-propylphenylphenylmethyl, bis(4-propylphenyl)methyl, bis(3,5-dimethylphenyl)methyl, bis(2,5-dimethylphenyl)-methyl, bis (2,6-dimethylphenyl)methyl, 2,4-dimethylphenylphenylmethyl, bis(3,5-dipropylphenyl)methyl, 2,5-dipropylphenylphenylmethyl, 2,6-diethylphenylphenylmethyl, bis(2,4-diethylphenyl)methyl and bis(2,3,6-trimethylphenyl)methyl groups; aralkyl groups substituted with at least one lower alkoxy group (as defined below), examples of which include 2-methoxybenzyl, 3-methoxybenzyl, 4-methoxybenzyl, 3-methoxyphenethyl, 2-ethoxyphenethyl, 3-propoxybenzyl, 4-ethoxyphenethyl, 2-butoxybenzyl, 3-pentoxyphenethyl, 4-pentoxybenzyl, 3,5-dimethoxyphenethyl, 2,5-dimethoxybenzyl, 2,6-dimethoxyphenethyl, 2,4-dimethoxybenzyl, 3,5-dibutoxyphenethyl, 2,5-dipentoxybenzyl, 2,6-dipropoxyphenethyl, 2,4-dipropoxybenzyl, 2,3,6-trimethoxyphenethyl, 2,3,4-trimethoxybenzyl, 3,4,5-trimethoxyphenethyl, 2,5,6-trimethoxybenzyl, 2,4,6-trimethoxyphenethyl, 2,3,6-tributoxybenzyl, 2,3,4-tripentoxyphenethyl, 3,4,5-tributoxybenzyl, 2,5,6-tripropoxyphenethyl, 2,4,6-tripropoxybenzyl, 1-methoxy-2-naphthylmethyl, 2-methoxy-1-naphthylmethyl, 3-methyl-1-naphthylmethyl, 1-ethoxy-2-naphthylmethyl, 2-propoxy-1-naphthylmethyl, 3-butoxy-1-naphthylethyl, 3,8-dimethoxy-1-naphthylmethyl, 2,3-dimethoxy-1-naphthylmethyl, 4,8-dimethoxy-1-naphthylethyl, 5,6-dimethoxy-1-naphthylmethyl, 3,8-dimethyl-1-naphthylmethyl, 2,3-dipropoxy-1-naphthylethyl, 4,8-dipentoxy-1-naphthylmethyl, 5,6-dibutoxy-1-naphthylmethyl, 2,3,6-trimethoxy-1-naphthylethyl, 2,3,4-trimethoxy-1-naphthylmethyl, 3,4,5-trimethoxy-1-naphthylmethyl, 4,5,6-trimethoxy-1-naphthylethyl, 2,4,8-trimethoxy-1-naphthylmethyl, bis(2-methoxyphenyl) methyl, 3-methoxyphenylphenylmethyl, bis(4-methoxyphenyl)-methyl, 4-methoxyphenylphenylmethyl, bis(2-ethoxyphenyl)methyl, bis(3-ethoxyphenyl)methyl, bis(4-ethoxyphenyl)methyl, 2-propoxyphenylphenylmethyl, 3-propoxyphenylphenylmethyl, bis(4-propoxyphenyl)methyl, bis(3,5-dimethoxyphenyl)methyl, bis(2,5-dimethoxyphenyl)methyl, bis (2,6-dimethoxyphenyl)methyl, 2,4-dimethoxyphenylphenylmethyl, bis(3,5-dipropoxyphenyl)methyl, 2,5-dipropoxyphenylphenylmethyl, 2,6-diethoxyphenylphenylmethyl, bis(2,4-diethoxyphenyl)-methyl and bis(2,3,6-trimethoxyphenyl) methyl groups; aralkyl groups substituted with at least one amino group, examples of which include 2-aminophenethyl, 3-aminobenzyl, 4-aminophenethyl, 3,5-diaminobenzyl, 2,5-diaminophenethyl, 2,6-diaminobenzyl, 2,4-diaminophenethyl, 2,3,6-triaminobenzyl, 2,3,4-triaminophenethyl, 3,4,5-triaminobenzyl, 2,5,6-triaminophenethyl, 2,4,6-triaminobenzyl, 1-amino-2-naphthylmethyl, 2-amino-1-naphthylethyl, 3-amino-1-naphthylmethyl, 3,8-diamino-1-naphthylmethyl, 2,3-diamino-1-naphthylethyl, 4,8-diamino-1-naphthylmethyl, 5,6-diamino-1-naphthylmethyl, 2,3,6-triamino-1-naphthylethyl, 2,3,4-triamino-1-naphthylmethyl, 3,4,5-triamino-1-naphthylmethyl, 4,5,6-triamino-1-naphthylethyl, 2,4,8-triamino-1-naphthylmethyl, bis(2-aminophenyl)methyl, 3-aminophenylphenylmethyl, bis(4-aminophenyl)methyl, 4-aminophenylphenylmethyl, bis(3,5-diaminophenyl)methyl, bis(2,5-diaminophenyl)methyl, bis(2,6-diaminophenyl)methyl, 2,4-diaminophenylphenylmethyl and bis(2,3,6-triaminophenyl)-methyl groups; aralkyl groups substituted with at least one nitro group, examples of which include 2-nitrophenethyl, 3-nitrobenzyl, 4-nitrobenzyl, 4-nitrophenethyl, 3,5-dinitrobenzyl, 2,5-dinitrophenethyl, 2,6-dinitrobenzyl, 2,4-dinitrophenethyl, 2,3,6-trinitrobenzyl, 2,3,4-trinitrophenethyl, 3,4,5-trinitrobenzyl, 2,5,6-trinitrophenethyl, 2,4,6-trinitrobenzyl, 1-nitro-2-naphthylmethyl, 2-nitro-1-naphthylethyl, 3-nitro-1-naphthylmethyl, 3,8-dinitro-1-naphthylmethyl, 2,3-dinitro-1-naphthylethyl, 4,8-dinitro-1-naphthylmethyl, 5,6-dinitro-1-naphthylmethyl, 2,3,6-trinitro-1-naphthylethyl, 2,3,4-trinitro-1-naphthylmethyl, 3,4,5-trinitro-1-naphthylmethyl, 4,5,6-trinitro-1-naphthylethyl, 2,4,8-trinitro-1-naphthylmethyl, bis(2-nitrophenyl)methyl, 3-nitrophenylphenylmethyl, bis (4-nitrophenyl)methyl, 4-nitrophenylphenylmethyl, bis(3,5-dinitrophenyl)methyl, bis(2,5-dinitrophenyl)methyl, bis (2,6-dinitrophenyl)methyl, 2,4-dinitrophenylphenylmethyl and bis(2,3,6-trinitrophenyl)methyl groups; and aralkyl groups substituted with at least one cyano group, examples of which include 2-cyanophenethyl, 3-cyanobenzyl, 4-cyanobenzyl, 4-cyanobenzyldiphenylmethyl, 4-cyanophenethyl, 3,5-dicyanobenzyl, 2,5-dicyanophenethyl, 2,6-dicyanobenzyl, 2,4-dicyanophenethyl, 2,3,6-tricyanobenzyl, 2,3,4-tricyanophenethyl, 3,4,5-tricyanobenzyl, 2,5,6-tricyanophenethyl, 2,4,6-tricyanobenzyl, 1-cyano-2-naphthylmethyl, 3-cyano-1-naphthylmethyl, 3,8-dicyano-1-naphthylmethyl, 2,3-dicyano-1-naphthylethyl, 4,8-dicyano-1-naphthylmethyl, 5,6-dicyano-1-naphthylmethyl, 2,3,6-tricyano-1-naphthylethyl, 2,3,4-tricyano-1-naphthylmethyl, 3,4,5-tricyano-1-naphthylmethyl, 4,5,6-tricyano-1-naphthylethyl,

2,4,8-tricyano-1-naphthylmethyl, bis(2-cyanophenyl)-methyl, 3-cyanophenylphenylmethyl, bis(4-cyanophenyl)methyl, 4-cyanophenylphenylmethyl, bis(3,5-dicyanophenyl)methyl, bis(2,5-dicyanophenyl)methyl, bis(2,6-dicyanophenyl) methyl, 2,4-dicyanophenylphenylmethyl and bis(2,3,6-tricyanophenyl)methyl groups.

Of the above, unsubstituted aralkyl groups and aralkyl groups substituted with at least one substituent selected from the group consisting of halogen atoms, lower alkyl groups and lower alkoxy groups are preferred, unsubstituted aralkyl groups and aralkyl groups substituted with at least one substituent selected from the group consisting of halogen atoms and lower alkyl groups are more preferred, and unsubstituted aralkyl groups are most preferred.

Where substituents $R^c$ or $R^d$ represent a lower alkylsulfonyl group, this is a group in which a lower alkyl group, defined and exemplified above, is bonded to a sulfonyl group ($-SO_2-$). The lower alkylsulfonyl group is preferably a straight or branched alkylsulfonyl group having from 1 to 4 carbon atoms, more preferably a methylsulfonyl, ethylsulfonyl, propylsulfonyl, isopropylsulfonyl or butylsulfonyl group, and most preferably a methylsulfonyl, ethylsulfonyl or propylsulfonyl group.

Where substituents $R^c$ and $R^d$ together with the nitrogen atom to which they are bonded form a heterocyclyl group, said heterocyclyl group is a 4- to 12-membered non-aromatic heterocyclic group containing one nitrogen atom and optionally containing one further heteroatom selected from the group consisting of oxygen atoms, sulfur atoms and nitrogen atoms. Preferably, it is a 4- to 10-membered non-aromatic heterocyclic group containing one nitrogen atom and optionally containing one further heteroatom selected from the group consisting of oxygen atoms, sulfur atoms and nitrogen atoms. Examples of said heterocyclyl group include 1-azetidinyl, 1-pyrrolidinyl, 1-pyrrolinyl, 1-imidazolidinyl, 1-imidazolinyl, I-pyrazolidinyl, 1-pyrazolinyl, 3-oxazolidinyl, 3-thiazolidinyl, 1-piperidyl, tetrahydropyridin-1-yl, dihydropyridin-1-yl, 1-piperazinyl, 4-morpholinyl, 4-thiomorpholinyl, 1-homopiperidyl, 8-azabicyclo-[3.2.1]octan-8-yl, 8-azabicyclo[3.2.1]octen-8-yl, 9-azabicyclo[3.3.1]nonan-9-yl and 9-azabicyclo[3.3.1]nonen-9-yl groups.

Where substituents $R^c$ and $R^d$ together with the nitrogen atom to which they are bonded form a heterocyclyl group as defined and exemplified above, said heterocyclyl groups may be fused with another cyclic group selected from the group consisting of aryl groups defined above and heteroaryl groups defined above. Examples of such fused heterocyclyl groups include tetrahydroquinolin-1-yl and tetrahydroisoquinolin-2-yl.

The halogen atoms in the definition of Substituent group $\alpha$ include fluorine, chlorine, bromine and iodine atoms, of which fluorine and chlorine atoms are preferred.

Where the substituent in the definition of Substituent group $\alpha$ is a lower alkoxy group, this is a group in which an oxygen atom is bonded to a lower alkyl group as defined and exemplified above. The alkoxy groups are preferably straight or branched alkoxy groups having 1 to 4 carbon atoms, more preferably methoxy, ethoxy, propoxy, isopropoxy or butoxy groups, and particularly preferably methoxy, ethoxy or propoxy groups.

Where the substituent in the definition of Substituent group $\alpha$ is a halogeno lower alkoxy group this is a group in which a lower alkoxy group as defined above is substituted with at least one halogen atom as exemplified above. The halogeno lower alkoxy groups preferably have from 1 to 4 carbon atoms, and are more preferably selected from the group consisting of difluoromethoxy, trifluoromethoxy and 2,2,2-trifluoromethoxy groups. Difluoromethoxy groups are most preferred.

Where the substituent in the definition of Substituent group $\alpha$ is a lower alkylthio group this is a group in which a sulfur atom is bonded to a lower alkyl group as defined and exemplified above. The lower alkylthio groups are preferably straight or branched alkylthio groups having 1 to 4 carbon atoms, more preferably methylthio, ethylthio, propylthio, isopropylthio or butylthio groups, and particularly preferably methylthio, ethylthio or propylthio groups.

Where the substituent in the definition of Substituent group $\alpha$ is a halogeno lower alkylthio group this is a group in which a lower alkylthio group as defined above is substituted with at least one halogen atom as exemplified above. The halogeno lower alkylthio groups preferably have from 1 to 4 carbon atoms, and are more preferably selected from the group consisting of difluoromethylthio, trifluoromethylthio and 2,2,2-trifluoroethylthio groups.

A preferred group of substituents of Substituent group $\alpha$ is Substituent group $\alpha^1$ which comprises halogen atoms, lower alkoxy groups as defined above and halogeno lower alkoxy groups as defined above.

A preferred group of substituents of Substituent group $\beta$ is Substituent group $\beta^1$ which comprises a group of formula $-NR^cR^d$ wherein one of $R^c$ and $R^d$ is selected from the group consisting of hydrogen atoms and lower alkyl groups as defined above and the other is selected from the group consisting of hydrogen atoms, lower alkyl groups as defined above and aralkyl groups as defined above.

A preferred group of substituents of Substituent group $\gamma$ is Substituent group $\gamma^1$ which comprises lower alkyl groups as defined above which are substituted with a substituent selected from the group consisting of amino groups, amino groups substituted with one or two lower alkyl groups as defined above and amino groups substituted with an aralkyl group as defined above. Of these, substituents in which the alkyl moiety which is substituted with a substituent selected from the group consisting of amino groups, amino groups substituted with one or two lower alkyl groups and amino groups substituted with an aralkyl is an alkyl group having from 1 to 4 carbon atoms are preferred. Aminomethyl, 2-aminoethyl, 3-aminopropyl, methylaminomethyl, 2-(methylamino)ethyl, 3-(methylamino)propyl, ethylaminomethyl, 2-(ethylamino)ethyl, 3-(ethylamino)propyl, dimethylaminomethyl, 2-(dimethylamino)ethyl, 3-(dimethylamino)propyl, diethylaminomethyl, 2-(diethylamino)ethyl, 3-(diethylamino)propyl, benzylaminomethyl, 2-(benzylamino)-ethyl and 3-(benzylamino)propyl groups are more preferred.

A preferred group of substituents of Substituent group δ is Substituent group $\delta^1$ which comprises lower alkyl groups as defined above, halogeno lower alkyl groups, hydroxy lower alkyl groups and nitro lower alkyl groups.

The halogeno lower alkyl groups in the definition of Substituent group $\delta^1$ above are lower alkyl groups as defined above which are substituted with at least one halogen atom. Halogeno alkyl groups having from 1 to 4 carbon atoms are preferred, trifluoromethyl, trichloromethyl, difluoromethyl, dichloromethyl, dibromomethyl, fluoromethyl, 2,2,2-trichloroethyl, 2,2,2-trifluoroethyl, 2-bromoethyl, 2-chloroethyl, 2-fluoroethyl and 2,2-dibromoethyl groups are more preferred, trifluoromethyl, trichloromethyl, difluoromethyl and fluoromethyl groups are still more preferred, and trifluoromethyl groups are most preferred.

The hydroxy lower alkyl groups in the definition of Substituent group $\delta^1$ above are lower alkyl groups as defined above which are substituted with at least one hydroxy group. Hydroxyalkyl groups having from 1 to 4 carbon atoms are preferred, and hydroxymethyl, 2-hydroxyethyl and 3-hydroxypropyl groups are most preferred.

The nitro lower alkyl groups in the definition of Substituent group $\delta^1$ above are lower alkyl groups as defined above which are substituted with at least one nitro group. Nitroalkyl groups having from 1 to 4 carbon atoms are preferred, and nitromethyl, 2-nitroethyl and 3-nitropropyl are most preferred.

The group $R^3$ is a group of formula (II):

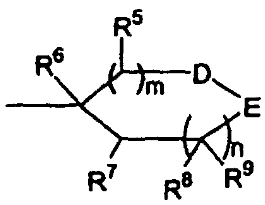

wherein:

m represents 0 or 1;
n represents 1 or 2;
$R^5$, $R^6$, $R^7$, $R^8$ and $R^9$ may be the same or different from one another and each is selected from the group consisting of hydrogen atoms, Substituent group α defined above, Substituent group β defined above, Substituent group γ defined above and Substituent group δ defined above;
one of D and E represents a group of formula >$NR^{10}$ wherein $R^{10}$ is selected from the group consisting of hydrogen atoms, Substituent group γ defined above and Substituent group δ defined above, and the other represents a group of formula >$CR^{11}R^{12}$ wherein $R^{11}$ and $R^{12}$ are the same or different from one another and each is selected from the group consisting of hydrogen atoms, Substituent group α defined above, Substituent group β defined above, Substituent group γ defined above and Substituent group δ defined above, or
$R^6$ may form, together with $R^5$ or $R^7$, a single bond, and/or $R^{10}$ and $R^{11}$ together may form a lower alkylene group defined below which is unsubstituted or is substituted with at least one substituent selected from the group consisting of Substituent group a defined above, Substituent group β defined above, Substituent group γ defined above and Substituent group δ defined above.

In the definition of $R^3$, one of $R^5$ and $R^7$ forms a single bond together with $R^6$, and the other is selected from the group consisting of hydrogen atoms, Substituent group α, Substituent group γ and Substituent group δ, more preferably selected from the group consisting of hydrogen atoms, lower alkyl groups as defined above and aralkyl groups as defined above, and most preferably selected from the group consisting of hydrogen atoms, alkyl groups having from 1 to 4 carbon atoms, benzyl groups and phenethyl groups.

Each of $R^8$, $R^9$, $R^{10}$, $R^{11}$ and $R^{12}$ is preferably selected from the group consisting of hydrogen atoms, Substituent group a, Substituent group γ and Substituent group δ, more preferably selected from the group consisting of hydrogen atoms, lower alkyl groups as defined above and aralkyl groups as defined above, and most preferably selected from the group consisting of hydrogen atoms, alkyl groups having from 1 to 4 carbon atoms, benzyl groups and phenethyl groups.

Where $R^{10}$ and $R^{11}$ together form a lower alkylene group, this is a straight or branched alkylene group having from 1 to 6 carbon atoms, examples of which include methylene, ethylene, trimethylene, propylene, tetramethylene, 1-methyltrimethylene, 2-methyltrimethylene, 1,1-dimethylethylene, pentamethylene, 1,1-dimethyltrimethylene, 2,2-dimethyltrimethylene, 1,2-dimethyltrimethylene and hexamethylene groups. Straight or branched alkylene groups

having from 3 to 6 carbon atoms are preferred, straight or branched alkylene group having 3 or 4 carbon atoms are more preferred, and straight chain alkylene groups having 3 or 4 carbon atoms are most preferred.

Preferred examples of the above group of formula (II) include:

(a) a group of formula (II) wherein m is 1 and n is 1;

(b) a group of formula (II) wherein one of $R^5$ and $R^7$ together with $R^6$ represents a single bond and the other is a hydrogen atom;

(c) a group of formula (II) wherein $R^7$, $R^8$ and $R^9$ may be the same or different from one another and each is selected from the group consisting of hydrogen atoms, Substituent group α defined above, Substituent group γ defined above and Substituent group δ defined above;

(d) a group of formula (II) wherein $R^{10}$, $R^{11}$ and $R^{12}$ may be the same or different from one another and each is selected from the group consisting of hydrogen atoms, Substituent group α defined above, Substituent group γ defined above and Substituent group δ defined above;

(e) a group of formula (II) wherein $R^{10}$ and $R^{11}$ together form a straight chain alkylene group having 3 or 4 carbon atoms which is unsubstituted or is substituted with at least one substituent selected from the group consisting of Substituent group α defined above, Substituent group β defined above, Substituent group γ defined above and Substituent group δ defined above; and

(f) a group of formula (II) wherein each of $R^7$, $R^8$ and $R^9$ is a hydrogen atom, and $R^{10}$ and $R^{11}$ together form a straight chain alkylene group having 3 or 4 carbon atoms which is unsubstituted or is substituted with at least one substituent selected from the group consisting of Substituent group α defined above, Substituent group β defined above, Substituent group γ defined above and Substituent group δ defined above.

Of these, more preferred examples of the above group of formula (II) include:

(g) a group of formula (II) wherein m is 1, n is 1, D represents a group of formula $>CR^{11}R^{12}$ and E represents a group of formula $>NR^{10}$; and

(h) a group of formula (II) wherein m is 1, n is 1, D represents a group of formula $>CR^{11}R^{12}$, E represents a group of formula $>NR^{10}$, and at least one of the subsitituents $R^8$, $R^9$, $R^{11}$ and $R^{12}$ is a substituent selected from the group consisting of Substituent group α defined above, Substituent group γ defined above and Substituent group δ defined above.

The present invention encompasses esters and other derivatives of the compounds of formula (I). These esters and other derivatives are compounds of formula (I) in which a functional group (for example, a hydroxyl group, an amino group, an imino group or a sulfonamide group) of said compound of formula (I) is modified by the addition of a protecting group using conventional techniques well-known in the art (see, for example, "Protective Groups in Organic Synthesis, Second Edition, Theodora W. Greene and Peter G.M. Wuts, 1991, John Wiley & Sons, Inc.).

There is no particular restriction on the nature of this protecting group, provided that, where the ester or other derivative is intended for therapeutic purposes, it must be pharmacologically acceptable, i.e. the protecting group must be capable of being removed by a metabolic process (e.g. hydrolysis) on administration of said compound to the body of a live mammal to give a compound of formula (I) or a salt thereof. In other words, the pharmacologically acceptable esters or other derivatives are pro-drugs of the compounds of formula (I) of the present invention. Where, however, the ester or other derivative of the compound of formula (I) of the present invention is intended for non-therapeutic purposes (e.g. as an intermediate in the preparation of other compounds), then the requirement that said ester or other derivative is pharmacologically acceptable does not apply.

Whether an ester or other derivative of a compound of formula (I) of the present invention is pharmacologically acceptable can be easily determined. The compound under investigation is intravenously administered to an experimental animal such as a rat or mouse and the body fluids of the animal are thereafter studied. If a compound of formula (I) or a pharmacologically acceptable salt thereof can be detected in the body fluids, the compound under investigation is judged to be a pharmacologically acceptable ester or other derivative.

The compounds of formula (I) of the present invention can be converted to an ester, examples of which include a compound of formula (I) in which a hydroxyl group present therein is esterified. The ester residue may be a general protecting group where the esterified compound is to be used as an intermediate or a protecting group which is capable of being removed by a metabolic process (e.g. hydrolysis) *in vivo* where the esterified compound is one which is pharmacologically acceptable.

The general protecting group referred to above is a protecting group which is removable by a chemical process such as hydrolysis, hydrogenolysis, electrolysis or photolysis. Preferred examples of such a general protecting group used to synthesise a compound of formula (I) in which a hydroxyl residue therein is esterified include the following:

(i) aliphatic acyl groups, examples of which include

alkylcarbonyl groups having from 1 to 25 carbon atoms, examples of which include formyl, acetyl, propionyl, butyryl, isobutyryl, pentanoyl, pivaloyl, valeryl, isovaleryl, octanoyl, nonanoyl, decanoyl, 3-methylnonanoyl, 8-methylnonanoyl, 3-ethyloctanoyl, 3,7-dimethyloctanoyl, undecanoyl, dodecanoyl, tridecanoyl, tetradecanoyl, pentadecanoyl, hexadecanoyl, 1-methylpentadecanoyl, 14-methylpentadecanoyl, 13,13-dimethyltetradecanoyl, heptadecanoyl, 15-methylhexadecanoyl, octadecanoyl, 1-methylheptadecanoyl, nonadecanoyl, eicosanoyl and heneicosanoyl groups,
halogenated alkylcarbonyl groups having from 1 to 25 carbons in which the alkyl moiety thereof is substituted by at least one halogen atom, examples of which include chloroacetyl, dichloroacetyl, trichloroacetyl and trifluoroacetyl groups,
lower alkoxyalkylcarbonyl groups which comprise an alkylcarbonyl group having from 1 to 25 carbon atoms in which the alkyl moiety thereof is substituted with at least one lower alkoxy group as defined above, examples of said lower alkoxyalkylcarbonyl groups including methoxyacetyl groups, and
unsaturated alkylcarbonyl groups having from 1 to 25 carbon atoms, examples of which include acryloyl, propioloyl, methacryloyl, crotonoyl, isocrotonoyl and (E)-2-methyl-2-butenoyl groups;
of these, alkylcarbonyl groups having from 1 to 6 carbon atoms are preferred;

(ii) aromatic acyl groups, examples of which include

arylcarbonyl groups which comprise a carbonyl group which is substituted with an aryl group as defined above, examples of which include benzoyl, α-naphthoyl and β-naphthoyl groups,
halogenated arylcarbonyl groups which comprise an arylcarbonyl group as defined above which is substituted with at least one halogen atom, examples of which include 2-bromobenzoyl and 4-chlorobenzoyl groups,
lower alkylated arylcarbonyl groups which comprise an arylcarbonyl group as defined above which is substituted with at least one lower alkyl group as defined above, examples of which include 2,4,6-trimethyl-benzoyl and 4-toluoyl groups,
lower alkoxylated arylcarbonyl groups which comprise an arylcarbonyl group as defined above which is substituted with at least one lower alkoxy group as defined above, examples of which include 4-anisoyl groups,
nitrated arylcarbonyl groups which comprise an arylcarbonyl group as defined above which is substituted with at least one nitro group, examples of which include 4-nitrobenzoyl and 2-nitrobenzoyl groups,
lower alkoxycarbonylated arylcarbonyl groups which comprise an arylcarbonyl group as defined above which is substituted with a carbonyl group which is itself substituted with a lower alkoxy group as defined above, examples of which include 2-(methoxycarbonyl)benzoyl groups, and
arylated arylcarbonyl groups which comprise an arylcarbonyl group as defined above which is substituted with at least one aryl group as defined above, examples of which include 4-phenylbenzoyl groups;

(iii) alkoxycarbonyl groups, examples of which include

lower alkoxycarbonyl groups which comprise a carbonyl group substituted with a lower alkoxy group as defined above, examples of which include methoxycarbonyl, ethoxycarbonyl, propoxycarbonyl, butoxycarbonyl, s-butoxycarbonyl, t-butoxycarbonyl and isobutoxycarbonyl groups, and
lower alkoxycarbonyl groups as defined above which are substituted with at least one substituent selected from the group consisting of halogen atoms and tri(lower alkyl)silyl groups (wherein said lower alkyl groups are as defined above), examples of which include 2,2,2-trichloroethoxycarbonyl and 2-trimethylsilylethoxycarbonyl groups;

(iv) tetrahydropyranyl or tetrahydrothiopyranyl groups which may optionally be substituted with at least one substituent selected from lower alkyl groups as defined above, halogen atoms and lower alkoxy groups as defined above, examples of which include tetrahydropyran-2-yl, 3-bromotetrahydropyran-2-yl, 4-methoxytetrahydropyran-4-yl, tetrahydrothiopyran-2-yl and 4-methoxytetrahydrothiopyran-4-yl groups;

(v) tetrahydrofuranyl or tetrahydrothiofuranyl groups which may optionally be substituted with at least one substituent selected from lower alkyl groups as defined above, halogen atoms and lower alkoxy groups as defined above, examples of which include tetrahydrofuran-2-yl and tetrahydrothiofuran-2-yl groups;

(vi) silyl groups, examples of which include

tri(lower alkyl)silyl groups (wherein said lower alkyl groups are as defined above), examples of which include trimethylsilyl, triethylsilyl, isopropyldimethylsilyl, t-butyldimethylsilyl, methyldiisopropylsilyl, methyl-di-t-butylsilyl and triisopropylsilyl groups, and
tri(lower alkyl)silyl groups in which at least one of said lower alkyl groups is substituted with 1 or 2 aryl groups as defined above, examples of which include diphenylmethylsilyl, diphenylbutylsilyl, diphenylisopropylsilyl and phenyldiisopropylsilyl groups;

(vii) alkoxymethyl groups, examples of which include

lower alkoxymethyl groups which comprise a methyl group which is substituted with a lower alkoxy group as defined above, examples of which include methoxymethyl, 1,1-dimethyl-1-methoxymethyl, ethoxymethyl, propoxymethyl, isopropoxymethyl, butoxymethyl and t-butoxymethyl groups,
lower alkoxylated lower alkoxymethyl groups which comprise a lower alkoxymethyl group as defined above in which the alkoxy moiety thereof is substituted with a lower alkoxy group as defined above, examples of which include 2-methoxyethoxymethyl groups, and
lower halogeno alkoxymethyl groups which comprise a lower alkoxymethyl group as defined above in which the alkoxy moiety thereof is substituted with at least one halogen atom, examples of which include 2,2,2-trichloroethoxymethyl and bis(2-chloroethoxy)methyl groups;

(viii) substituted ethyl groups, examples of which include

lower alkoxylated ethyl groups which comprise an ethyl group which is substituted with a lower alkoxy group as defined above, examples of which include 1-ethoxyethyl and 1-(isopropoxy)ethyl groups, and
halogenated ethyl groups such as 2,2,2-trichloroethyl groups;

(ix) aralkyl groups as define above, examples of which include

lower alkyl groups as defined above which are substituted with from 1 to 3 aryl groups as defined above, examples of which include benzyl, α-naphthylmethyl, β-naphthylmethyl, diphenylmethyl, triphenylmethyl, α-naphthyldiphenylmethyl and 9-anthrylmethyl groups, and
lower alkyl groups as defined above which are substituted with from 1 to 3 aryl groups as defined above in which said aryl moiety is substituted with at least one substituent selected from the group consisting of lower alkyl groups as defined above, lower alkoxy groups as defined above, nitro groups, halogen atoms and cyano groups, examples of which include 4-methylbenzyl, 2,4,6-trimethylbenzyl, 3,4,5-trimethylbenzyl, 4-methoxybenzyl, 4-methoxyphenydiphenylmethyl, 2-nitrobenzyl, 4-nitrobenzyl, 4-chlorobenzyl, 4-bromobenzyl and 4-cyanobenzyl groups;

(x) "alkenyloxycarbonyl groups" which comprise a carbonyl group which is substituted with an alkenyloxy group having from 2 to 6 carbon atoms, examples of which include vinyloxycarbonyl and allyloxycarbonyl groups; and

(xi) aralkyloxycarbonyl groups which comprise a carbonyl group which is substituted with an aralkyloxy group (which is an oxygen atom substituted with an aralkyl group as defined above), in which the aryl moiety thereof may optionally be substituted with one or two substituents selected from lower alkoxy groups as defined above and nitro groups, examples of which include benzyloxycarbonyl, 4-methoxybenzyloxycarbonyl, 3,4-dimethoxybenzyloxycarbonyl, 2-nitrobenzyloxycarbonyl and 4-nitrobenzyloxycarbonyl groups.

The protecting group which is capable of being removed by a metabolic process (e.g. hydrolysis) *in vivo* is one, which on administration to the body of a live mammal is removable by a metabolic process (e.g. hydrolysis) to give a compound of formula (I) or a salt thereof. Preferred examples of such a protecting group which is used to synthesise a compound of formula (I) in which a hydroxyl residue therein is esterified include the following:

(i) 1-(acyloxy)lower alkyl groups, examples of which include

1-(aliphatic acyloxy)lower alkyl groups which comprise a lower alkyl group as defined above which is substituted with an alkylcarbonyloxy group having from 1 to 6 carbon atoms, examples of which include formyloxymethyl, acetoxymethyl, dimethylaminoacetoxymethyl, propionyloxymethyl, butyryloxymethyl, pivaloyloxymethyl, valeryloxymethyl, isovaleryloxymethyl, hexanoyloxymethyl, 1-formyloxyethyl, 1-acetoxyethyl, 1-propionyloxyethyl, 1-butyryloxyethyl, 1-pivaloyloxyethyl, 1-valeryloxyethyl, 1-isovaleryloxyethyl, 1-hexanoyloxyethyl,

1-formyloxypropyl, 1-acetoxypropyl, 1-propionyloxypropyl, 1-butyryloxypropyl, 1-pivaloyloxypropyl, 1-valeryloxypropyl, 1-isovaleryloxypropyl, 1-hexanoyloxypropyl, 1-acetoxybutyl, 1-propionyloxybutyl, 1-butyrytoxybutyl, 1-pivaloyloxybutyl, 1-acetoxypentyl, 1-propionyloxypentyl, 1-butyryloxypentyl, 1-pivaloyloxypentyl and 1-pivaloyloxyhexyl groups,

1-(cycloalkylcarbonyloxy)lower alkyl groups which comprise a lower alkyl group as defined above which is substituted with a cycloalkylcarbonyloxy group in which a carbonyloxy group is substituted with a cyclohexyl group as defined above, examples of which include cyclopentylcarbonyloxymethyl, cyclohexylcarbonyloxymethyl, 1-cyclopentylcarbonyloxyethyl, 1-cyclohexylcarbonyloxyethyl, 1-cyclopentylcarbonyloxypropyl, 1-cyclohexylcarbonyloxypropyl, 1-cyclopentylcarbonyloxybutyl and 1-cyclohexylcarbonyloxybutyl groups, and

1-(aromatic acyloxy)lower alkyl groups which comprise a lower alkyl group as defined above which is substituted with an aralkyloxycarbonyl group which comprises a carbonyl group which is substituted with an aralkyloxy group (said aralkyloxy group comprising an oxygen atom which is substituted with an aralkyl group as defined above), examples of which include benzoyloxymethyl groups;

(ii) substituted carbonyloxyalkyl groups, examples of which include

(lower alkoxycarbonyloxy)alkyl groups which comprise a lower alkyl group as defined above which is substituted with a lower alkoxycarbonyloxy group which comprises a carbonyloxy group substituted with a lower alkoxy group as defined above, examples of which include methoxycarbonyloxymethyl, ethoxycarbonyloxymethyl, propoxycarbonyloxymethyl, isopropoxycarbonyloxymethyl, butoxycarbonyloxymethyl, isobutoxycarbonyloxymethyl, pentyloxycarbonyloxymethyl, hexyloxycarbonyloxymethyl, cyclohexyloxycarbonyloxymethyl, cyclohexyloxycarbonyloxy(cyclohexyl)methyl, 1-(methoxycarbonyloxy)ethyl, 1-(ethoxycarbonyloxy)ethyl, 1-(propoxycarbonyloxy)ethyl, 1-(isopropoxycarbonyloxy)ethyl, 1-(butoxycarbonyloxy)ethyl, 1-(isobutoxycarbonyloxy)ethyl, 1-(t-butoxycarbonyloxy)ethyl, 1-(pentyloxycarbonyloxy)ethyl, 1-(hexyloxycarbonyloxy)ethyl, 1-(cyclopentyloxycarbonyloxy)ethyl, 1-(cyclopentyloxycarbonyloxy)propyl, 1-(cyclohexyloxycarbonyloxy)propyl, 1-(cyclopentyloxycarbonyloxy)butyl, 1-(cyclohexyloxycarbonyloxy)butyl, 1-(cyclohexyloxycarbonyloxy)ethyl, 1-(ethoxycarbonyloxy)propyl, 1-(methoxycarbonyloxy)propyl, 1-(ethoxycarbonyloxy)propyl, 1-(propoxycarbonyloxy)propyl, 1-(isopropoxycarbonyloxy)propyl, 1-(butoxycarbonyloxy)propyl, 1-(isobutoxycarbonyloxy)propyl, 1-(pentyloxycarbonyloxy)propyl, 1-(hexyloxycarbonyloxy)propyl, 1-(methoxycarbonyloxy)butyl, 1-(ethoxycarbonyloxy)butyl, 1-(propoxycarbonyloxy)butyl, 1-(isopropoxycarbonyloxy)butyl, 1-(butoxycarbonyloxy) butyl, 1-(isobutoxycarbonyloxy)butyl, 1-(methoxycarbonyloxy)pentyl, 1-(ethoxycarbonyloxy)pentyl, 1-(methoxycarbonyloxy)hexyl and 1-(ethoxycarbonyloxy)hexyl groups, and oxodioxolenylmethyl groups, which comprise a methyl group which is substituted with an oxodioxolenyl group which itself may optionally be substituted with a group selected from the group consisting of lower alkyl groups as defined above and aryl groups as defined above which may optionally be substituted with at least one lower alkyl group as defined above, lower alkoxy group as defined above or halogen atom, examples of which include (5-phenyl-2-oxo-1,3-dioxolen-4-yl)methyl, [5-(4-methylphenyl)-2-oxo-1,3-dioxolen-4-yl]methyl, [5-(4-methoxyphenyl)-2-oxo-1,3-dioxolen-4-yl]methyl, [5-(4-fluorophenyl)-2-oxo-1,3-dioxolen-4-yl]methyl, [5-(4-chlorophenyl)-2-oxo-1,3-dioxolen-4-yl]methyl, (2-oxo-1,3-dioxolen-4-yl)-methyl, (5-methyl-2-oxo-1,3-dioxolen-4-yl)methyl, (5-ethyl-2-oxo-1,3-dioxolen-4-yl) methyl, (5-propyl-2-oxo-1,3-dioxolen-4-yl)methyl, (5-isopropyl-2-oxo-1,3-dioxolen-4-yl)methyl and (5-butyl-2-oxo-1,3-dioxolen-4-yl)methyl groups;

(iii) phthalidyl groups which comprise a phthalidyl group which may optionally be substituted with a substituent selected from the group consisting of lower alkyl groups as defined above and lower alkoxy groups as defined above, examples of which include phthalidyl, dimethylphthalidyl and dimethoxyphthalidyl groups;

(iv) aliphatic acyl groups as defined and exemplified above in relation to the general protecting group for a hydroxyl group;

(v) aromatic acyl groups as defined and exemplified above in relation to the general protecting group for a hydroxyl group;

(vi) half-ester salt residues of succinic acid;

(vii) phosphate ester salt residues;

(viii) ester-forming residues of an amino acid;

(ix) carbamoyl groups which may optionally be substituted with 1 or 2 lower alkyl groups as defined above; and

(x) 1-(acyloxy)alkoxycarbonyl groups which comprise a lower alkoxycarbonyl group as defined above in which the lower alkoxy moiety is substituted with an aliphatic acyloxy group as defined above or an aromatic acyloxy group as defined above, examples of which include pivaloyloxymethyloxycarbonyl groups.

Of the above protecting groups which are capable of being removed by a metabolic process (e.g. hydrolysis) *in vivo* which are used to synthesise a compound of formula (I) in which a hydroxyl residue therein is esterified, the substituted carbonyloxyalkyl groups are preferred.

In the case where the compound of formula (I) of the present invention has an amino group, an imino group and/or a sulfonamide group, the compound can be converted to a derivative other than the esters described above and the pharmacologically acceptable salts described below. The "other derivatives" of the compounds of formula (I) include such derivatives. Example of such derivatives include an amide derivative in which an aliphatic acyl group defined and exemplified above or an aromatic acyl group defined and exemplified above is bonded to a nitrogen atom of an amino group, imino group and/or sulfonamide group present in said compound of formula (I). Where said derivative is a pharmacologically acceptable derivative of a compound of formula (I) it must be capable of being removed by a metabolic process (e.g. hydrolysis) on administration of said compound to the body of a live mammal to give a compound of formula (I) or a salt thereof.

Where the compound of formula (I) of the present invention or a pharmacologically acceptable ester or other derivative thereof has a basic group, such as an amino group, the compound can be converted to a salt by reacting it with an acid, and in the case where the compound of formula (I) of the present invention or a pharmacologically acceptable ester or other derivative thereof has an acidic group, such as a sulfonamide group, the compound can be converted to a salt by reacting it with a base. The compounds of the present invention encompass such salts. Where said salts are to be used for a therapeutic use, they must be pharmacologically acceptable.

Preferred examples of the salts formed with a basic group present in the compound of formula (I) of the present invention include inorganic acid salts such as hydrohalogenated acid salts (e.g. hydrochlorides, hydrobromides and hydroiodides), nitrates, perchlorates, sulfates and phosphates; organic acid salts such as lower alkanesulfonates in which the lower alkyl moiety thereof is as defined above (e.g. methanesulfonates, trifluoromethanesulfonates and ethanesulfonates), arylsulfonates in which the aryl moiety thereof is as defined above (e.g. benzenesulfonate or p-toluenesulfonate), acetates, malates, fumarates, succinates, citrates, ascprbates, tartrates, oxalates and maleates; and amino acid salts such as glycine salts, lysine salts, arginine salts, ornithine salts, glutamates and aspartates.

Preferred example of the salts formed with an acidic group present in the compound of formula (I) of the present invention include metal salts such as alkali metal salts (e.g. sodium salts, potassium salts and lithium salts), alkali earth metal salts (e.g. calcium salts and magnesium salts), aluminum salts and iron salts; amine salts such as inorganic amine salts (e.g. ammonium salts) and organic amine salts (e.g. t-octylamine salts, dibenzylamine salts, morpholine salts, glucosamine salts, phenylglycinealkyl ester salts, ethylenediamine salts, N-methylglucamine salts, guanidine salts, diethylamine salts, triethylamine salts, dicyclohexylamine salts, N,N'-dibenzylethylenediamine salts, chloroprocaine salts, procaine salts, diethanolamine salts, N-benzylphenethylamine salts, piperazine salts, tetramethylammonium salts and tris(hydroxymethyl)aminomethane salts; and amino acid salts such as glycine salts, lysine salts, arginine salts, ornithine salts, glutamates and aspartates.

The compound of formula (I) of the present invention can sometimes take up water upon exposure to the atmosphere or when recrystallized to absorb water or to form a hydrate and such hydrates are also included within the scope of the present invention. Additionally, certain other solvents may be taken up by the compounds of the present invention to produce solvates, which also form a part of the present invention.

The compounds of formula (I) of the present invention can sometimes exist in the form of geometrical isomers (cis and trans isomers) and, where said compounds contain one or more asymmetric centres, optical isomers. For the compounds of the present invention, each of said isomers and mixture of said isomers are depicted by a single formula, i.e. the formula (I). Accordingly, the present invention covers both the individual isomers and mixtures thereof in any proportion, including racemic mixtures.

Preferred classes of compounds of the present invention are those compounds of formula (I) and pharmacologically acceptable salts, esters and amides thereof wherein:

(A) $R^1$ is a phenyl group which may optionally be substituted with at least one substituent selected from the group consisting of Substituent group $\alpha^1$ defined above, Substituent group $\beta^1$ defined above, Substituent group $\gamma^1$ defined above and Substituent group $\delta^1$ defined above;

(B) $R^1$ is a phenyl group which may optionally be substituted with at least one substituent selected from the group consisting of halogen atoms, halogeno lower alkyl groups as defined above and a halogeno lower alkoxy groups

as defined above;

(C) $R^1$ is a phenyl, 4-fluorophenyl, 3-fluorophenyl, 3-chlorophenyl, 3,4-difluorophenyl, 3,4,5-trifluorophenyl, 3-chloro-4-fluorophenyl, 3-difluoromethoxyphenyl or 3-trifluoromethylphenyl group;

(D) $R^2$ is a 4-pyridyl or 4-pyrimidinyl group which may optionally be substituted with at least one substituent selected from the group consisting of Substituent group $\alpha$ defined above, Substituent group $\beta$ defined above, Substituent group $\gamma$ defined above and Substituent group $\delta$ defined above;

(E) $R^2$ is a 4-pyridyl or 4-pyrimidinyl group which may optionally be substituted at the 2-position thereof with a substituent selected from the group consisting of Substituent group $\alpha$ defined above, Substituent group $\beta$ defined above, Substituent group $\gamma$ defined above and Substituent group $\delta$ defined above;

(F) $R^2$ is a 4-pyridyl or 4-pyrimidinyl group which may optionally be substituted at the 2-position thereof with a substituent selected from the group consisting of methoxy, amino, methylamino, benzylamino, and $\alpha$-methylbenzylamino groups;

(G) $R^3$ is a group of formula (II) as defined above wherein m is 1 and n is 1;

(H) $R^3$ is a group of formula (II) as defined above wherein E represents a group of formula $>NR^{10}$ and D represents a group of formula $>CR^{11}R^{12}$;

(I) $R^3$ is a group of formula (II) as defined above wherein $R^{10}$ and $R^{11}$ together form a straight chain alkylene group having 3 or 4 carbon atoms which is unsubstituted or is substituted with at least one substituent selected from the group consisting of Substituent group $\alpha$ defined above, Substituent group $\beta$ defined above, Substituent group $\gamma$ defined above and Substituent group $\delta$ defined above;

(J) $R^3$ is a group of formula (II) as defined above wherein each of $R^7$, $R^8$ and $R^9$ is a hydrogen atom, and $R^{10}$ and $R^{11}$ together form a straight chain alkylene group having 3 or 4 carbon atoms which is unsubstituted or is substituted with at least one substituent selected from the group consisting of Substituent group $\alpha$ defined above, Substituent group $\beta$ defined above, Substituent group $\gamma$ defined above and Substituent group $\delta$ defined above; and

(K) said compound of formula (I) is a compound of formula (I-1) or (I-3) below wherein $R^1$, $R^2$ and $R^3$ are as defined above:

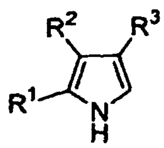

(I-1)

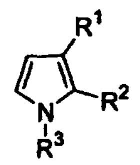

(I-3)

Compounds of formula (I) which comprise any combination of the factors selected freely from the four groups consisting of (A) to (C) above; (D) to (F) above; (G) to (J) above; and (K) above are preferred.

More preferred compounds of the present invention are compounds of formula (I) and pharmacologically acceptable salts, esters and other derivatives thereof, wherein:

(i) $R^1$ is as defined in (A) above, $R^2$ is as defined in (D) above and $R^3$ is as defined in (G) above;

(ii) $R^1$ is as defined in (A) above, $R^2$ is as defined in (D) above and $R^3$ is as defined in (H) above;

(iii) $R^1$ is as defined in (C) above, $R^2$ is as defined in (F) above and $R^3$ is as defined in (I) above;

(iv) $R^1$ is as defined in (C) above, $R^2$ is as defined in (F) above and $R^3$ is as defined in (J) above; and

(v) any of the above compounds (i) to (iv) wherein the compound of formula (I) is a compound of formula (I-1) or (I-3) as defined in (K) above.

Particularly preferred compounds of the present invention are compounds of formula (I) selected from the following group of compounds, and pharmacologically acceptable salts, esters and amides thereof:

4-(1-acetyl-1,2,3,6-tetrahydropyridin-4-yl)-2-(4-fluorophenyl)-3-(pyridin-4-yl)-1*H*-pyrrole,
2-(4-fluorophenyl)-3-(pyridin-4-yl)-4-(1,2,3,6-tetrahydropyridin-4-yl)-1*H*-pyrrole, 2-(4-fluorophenyl)-4-(1-methyl-1,2,3,6-tetrahydropyridin-4-yl)-3-(pyridin-4-yl)-1*H*-pyrrole,
2-(4-fluorophenyl)-3-(pyridin-4-yl)-4-(1,2,3,6-tetrahydro-2,2,6,6-tetramethylpyridin-4-yl)-1*H*-pyrrole,
2-(4-fluorophenyl)-4-(2-methyl-1,2,3,6-tetrahydropyridin-4-yl)-3-(pyridin-4-yl)-1*H*-pyrrole,
2-(4-fluorophenyl)-4-(6-methyl-1,2,3,6-tetrahydropyridin-4-yl)-3-(pyridin-4-yl)-1*H*-pyrrole,
4-(1-ethyl-1,2,3,6-tetrahydropyridin-4-yl)-2-(4-fluorophenyl)-3-(pyridin-4-yl)-1*H*-pyrrole,
2-(4-fluorophenyl)-4-(1-isopropyl-1,2,3,6-tetrahydropyridin-4-yl)-3-(pyridin-4-yl)-1*H*-pyrrole,
2-(4-fluorophenyl)-4-(1-propyl-1,2,3,6-tetrahydropyridin-4-yl)-3-(pyridin-4-yl)-1*H*-pyrrole,
2-(4-fluorophenyl)-4-(1-phenethyl-1,2,3,6-tetrahydropyridin-4-yl)-3-(pyridin-4-yl)-1*H*-pyrrole,
2-(4-fluorophenyl)-4-(1,2,3,5,8,8a-hexahydroindolizin-7-yl)-3-(pyridin-4-yl)-1*H*-pyrrole,
2-(4-fluorophenyl)-4-(1,2,3,5,6,8a-hexahydroindolizin-7-yl)-3-(pyridin-4-yl)-1*H*-pyrrole,
4-(1,6-dimethyl-1,2,3,6-tetrahydropyridin-4-yl)-2-(4-fluorophenyl)-3-(pyridin-4-yl)-1*H*-pyrrole,
4-(1,2-dimethyl-1,2,3,6-tetrahydropyridin-4-yl)-2-(4-fluorophenyl)-3-(pyridin-4-yl)-1*H*-pyrrole,
2-(4-fluorophenyl)-4-(1,2,2,6,6-pentamethyl-1,2,3,6-tetrahydropyridin-4-yl)-3-(pyridin-4-yl)-1*H*-pyrrole,
4-(1-ethyl-6-methyl-1,2,3,6-tetrahydropyridin-4-yl)-2-(4-fluorophenyl)-3-(pyridin-4-yl)-1*H*-pyrrole,
4-(1-ethyl-2-methyl-1,2,3,6-tetrahydropyridin-4-yl)-2-(4-fluorophenyl)-3-(pyridin-4-yl)-1*H*-pyrrole,
4-(6-allyl-1-methyl-1,2,3,6-tetrahydropyridin-4-yl)-2-(4-fluorophenyl)-3-(pyridin-4-yl)-1*H*-pyrrole,
4-(2-allyl-1-methyl-1,2,3,6-tetrahydropyridin-4-yl)-2-(4-fluorophenyl)-3-(pyridin-4-yl)-1*H*-pyrrole,
4-(6-benzyl-1-methyl-1,2,3,6-tetrahydropyridin-4-yl)-2-(4-fluorophenyl)-3-(pyridin-4-yl)-1*H*-pyrrole,
4-(2-benzyl-1-methyl-1,2,3,6-tetrahydropyridin-4-yl)-2-(4-fluorophenyl)-3-(pyridin-4-yl)-1*H*-pyrrole,
2-(4-fluorophenyl)-4-(6-methyl-1-propyl-1,2,3,6-tetrahydropyridin-4-yl)-3-(pyridin-4-yl)-1*H*-pyrrole,
2-(4-fluorophenyl)-4-(2-methyl-1-propyl-1,2,3,6-tetrahydropyridin-4-yl)-3-(pyridin-4-yl)-1*H*-pyrrole,
2-(4-fluorophenyl)-4-(1,3,4,6,7,9a-hexahydro-2*H*-quinolizin-8-yl)-3-(pyridin-4-yl)-1*H*-pyrrole,
2-(4-fluorophenyl)-4-(1,3,4,6,9,9a-hexahydro-2*H*-quinolizin-8-yl)-3-(pyridin-4-yl)-1*H*-pyrrole,
2-(4-fluorophenyl)-4-(1,2,3,5,8,8a-hexahydroindolizin-7-yl)-3-(2-methylaminopyrimidin-4-yl)-1*H*-pyrrole,
2-(4-fluorophenyl)-4-(1,2,3,5,6,8a-hexahydroindolizin-7-yl)-3-(2-methylaminopyrimidin-4-yl)-1*H*-pyrrole,
2-(3,4-difluorophenyl)-4-(1,2,3,5,8,8a-hexahydroindolizin-7-yl)-3-(pyridin-4-yl)-1*H*-pyrrole,
2-(3,4-difluorophenyl)-4-(1,2,3,5,6,8a-hexahydroindolizin-7-yl)-3-(pyridin-4-yl)-1*H*-pyrrole,
4-(1,2,3,5,8,8a-hexahydroindolizin-7-yl)-3-(pyridin-4-yl)-2-(3-trifluoromethylphenyl)-1*H*-pyrrole,
4-(1,2,3,5,6,8a-hexahydroindolizin-7-yl)-3-(pyridin-4-yl)-2-(3-trifluoromethylphenyl)-1*H*-pyrrole,
2-(3-fluorophenyl)-4-(1,2,3,5,8,8a-hexahydroindolizin-7-yl)-3-(pyridin-4-yl)-1*H*-pyrrole,
2-(3-fluorophenyl)-4-(1,2,3,5,6,8a-hexahydroindolizin-7-yl)-3-(pyridin-4-yl)-1*H*-pyrrole,
2-(3-chlorophenyl)-4-(1,2,3,5,8,8a-hexahydroindolizin-7-yl)-3-(pyridin-4-yl)-1*H*-pyrrole,
2-(3-chlorophenyl)-4-(1,2,3,5,6,8a-hexahydroindolizin-7-yl)-3-(pyridin-4-yl)-1*H*-pyrrole,
4-(1,2,3,5,8,8a-hexahydroindolizin-7-yl)-2-phenyl-3-(pyridin-4-yl)-1*H*-pyrrole, and 4-(1,2,3,5,6,8a-hexahydroindolizin-7-yl)-2-phenyl-3-(pyridin-4-yl)-1*H*-pyrrole.

The most preferred compounds of the present invention are compounds of formula (I) selected from the following group of compounds, and pharmacologically acceptable salts, esters and amides thereof:

2-(4-fluorophenyl)-3-(pyridin-4-yl)-4-(1,2,3,6-tetrahydropyridin-4-yl)-1*H*-pyrrole, 2-(4-fluorophenyl)-4-(6-methyl-1,2,3,6-tetrahydropyridin-4-yl)-3-(pyridin-4-yl)-1*H*-pyrrole,
4-(1-ethyl-1,2,3,6-tetrahydropyridin-4-yl)-2-(4-fluorophenyl)-3-(pyridin-4-yl)-1*H*-pyrrole,
2-(4-fluorophenyl)-4-(1-isopropyl-1,2,3,6-tetrahydropyridin-4-yl)-3-(pyridin-4-yl)-1*H*-pyrrole,
2-(4-fluorophenyl)-4-(1-propyl-1,2,3,6-tetrahydropyridin-4-yl)-3-(pyridin-4-yl)-1*H*-pyrrole,
2-(4-fluorophenyl)-4-(1-phenethyl-1,2,3,6-tetrahydropyridin-4-yl)-3-(pyridin-4-yl)-1*H*-pyrrole,
2-(4-fluorophenyl)-4-(1,2,3,5,6,8a-hexahydroindolizin-7-yl)-3-(pyridin-4-yl)-1*H*-pyrrole,
4-(1,6-dimethyl-1,2,3,6-tetrahydropyridin-4-yl)-2-(4-fluorophenyl)-3-(pyridin-4-yl)-1*H*-pyrrole,
4-(1,2-dimethyl-1,2,3,6-tetrahydropyridin-4-yl)-2-(4-fluorophenyl)-3-(pyridin-4-yl)-1*H*-pyrrole,
4-(1-ethyl-6-methyl-1,2,3,6-tetrahydropyridin-4-yl)-2-(4-fluorophenyl)-3-(pyridin-4-yl)-1*H*-pyrrole,
4-(1-ethyl-2-methyl-1,2,3,6-tetrahydropyridin-4-yl)-2-(4-fluorophenyl)-3-(pyridin-4-yl)-1*H*-pyrrole,

2-(4-fluorophenyl)-4-(6-methyl-1-propyl-1,2,3,6-tetrahydropyridin-4-yl)-3-(pyridin-4-yl)-1*H*-pyrrole,
2-(4-fluorophenyl)-4-(2-methyl-1-propyl-1,2,3,6-tetrahydropyridin-4-yl)-3-(pyridin-4-yl)-1*H*-pyrrole,
2-(4-fluorophenyl)-4-(1,3,4,6,7,9a-hexahydro-2*H*-quinolizin-8-yl)-3-(pyridin-4-yl)-1*H*-pyrrole,
2-(3,4-difluorophenyl)-4-(1,2,3,5,6,8a-hexahydroindolizin-7-yl)-3-(pyridin-4-yl)-1*H*-pyrrole, and
4-(1,2,3,5,6,8a-hexahydroindolizin-7-yl)-3-(pyridin-4-yl)-2-(3-trifluoromethylphenyl)-1*H*-pyrrole.

Specific examples of compounds of formula (I) of the present invention include the following compounds of formula (I-1) (Table 1) and formula (I-3) (Table 2).

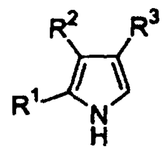

(I-1)

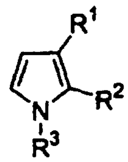

(I-3)

In these Tables, the following abbreviations are used:

| | |
|---|---|
| Ac | acetyl |
| Bn | benzyl |
| Bu | butyl |
| t-Bu | t-butyl |
| Et | ethyl |
| Hp | heptyl |
| Hx | hexyl |
| cHx | cyclohexyl |
| Ind | indolizinyl |
| Me | methyl |
| Nn | nonyl |
| Oc | octyl |
| Ph | phenyl |
| Phet | phenethyl |
| deH-Pip | dehydropiperidyl (i.e. tetrahydropyridyl) |
| Pn | pentyl |
| cPn | cyclopentyl |
| Pr | propyl |
| iPr | isopropyl |
| cPr | cyclopropyl |
| Pym | pyrimidinyl |
| Pyr | pyridyl |
| Qui | quinolizinyl |

**Table 1**

| Compound No. | $R^1$ | $R^2$ | $R^3$ |
|---|---|---|---|
| | | | |
| | | | |
| | | | |
| | | | |
| | | | |
| | | | |
| | | | |
| | | | |
| | | | |
| | | | |
| | | | |
| | | | |
| | | | |
| | | | |
| | | | |
| | | | |
| | | | |
| | | | |
| | | | |
| | | | |
| | | | |
| | | | |
| | | | |
| | | | |
| | | | |
| | | | |
| | | | |
| | | | |

| Compound No. | $R^1$ | $R^2$ | $R^3$ |
|---|---|---|---|
| 1-49 | Ph | 4-Pyr | 4-(3,4-deH-Pip) |
| 1-51 | Ph | 4-Pyr | 1-Me-4-(3,4-deH-Pip) |

| Compound No. | $R^1$ | $R^2$ | $R^3$ |
|---|---|---|---|
| 1-143 | 4-F-Ph | 4-Pyr | 4-(3,4-deH-Pip) |
| 1-145 | 4-F-Ph | 4-Pyr | 1-Me-4-(3,4-deH-Pip) |

| Compound No. | $R^1$ | $R^2$ | $R^3$ |
|---|---|---|---|
| 1-236 | 3-F-Ph | 4-Pyr | 4-(3,4-deH-Pip) |
| 1-238 | 3-F-Ph | 4-Pyr | 1-Me-4-(3,4-deH-Pip) |

| Compound No. | $R^1$ | $R^2$ | $R^3$ |
|---|---|---|---|
| 1-329 | 3,4-diF-Ph | 4-Pyr | 4-(3,4-deH-Pip) |
| 1-331 | 3,4-diF-Ph | 4-Pyr | 1-Me-4-(3,4-deH-Pip) |

| Compound No. | $R^1$ | $R^2$ | $R^3$ |
|---|---|---|---|
| 1-422 | 3-Cl-Ph | 4-Pyr | 4-(3,4-deH-Pip) |
| 1-424 | 3-Cl-Ph | 4-Pyr | 1-Me-4-(3,4-deH-Pip) |

| Compound No. | $R^1$ | $R^2$ | $R^3$ |
|---|---|---|---|
| 1-515 | Ph | 2-$NH_2$-4-Pym | 4-(3,4-deH-Pip) |
| 1-517 | Ph | 2-$NH_2$-4-Pym | 1-Me-4-(3,4-deH-Pip) |

| Compound No. | $R^1$ | $R^2$ | $R^3$ |
|---|---|---|---|
| 1-608 | 4-F-Ph | 2-$NH_2$-4-Pym | 4-(3,4-deH-Pip) |
| 1-610 | 4-F-Ph | 2-$NH_2$-4-Pym | 1-Me-4-(3,4-deH-Pip) |

| Compound No. | $R^1$ | $R^2$ | $R^3$ |
|---|---|---|---|
| 1-701 | 3-F-Ph | 2-$NH_2$-4-Pym | 4-(3,4-deH-Pip) |
| 1-703 | 3-F-Ph | 2-$NH_2$-4-Pym | 1-Me-4-(3,4-deH-Pip) |

| Compound No. | $R^1$ | $R^2$ | $R^3$ |
|---|---|---|---|
| 1-794 | 3,4-diF-Ph | 2-$NH_2$-4-Pym | 4-(3,4-deH-Pip) |
| 1-796 | 3,4-diF-Ph | 2-$NH_2$-4-Pym | 1-Me-4-(3,4-deH-Pip) |

| Compound No. | $R^1$ | $R^2$ | $R^3$ |
|---|---|---|---|
| 1-887 | 3-Cl-Ph | 2-$NH_2$-4-Pym | 4-(3,4-deH-Pip) |
| 1-889 | 3-Cl-Ph | 2-$NH_2$-4-Pym | 1-Me-4-(3,4-deH-Pip) |

| Compound No. | $R^1$ | $R^2$ | $R^3$ |
|---|---|---|---|
| 1-980 | Ph | 2-MeNH-4-Pym | 4-(3,4-deH-Pip) |
| 1-982 | Ph | 2-MeNH-4-Pym | 1-Me-4-(3,4-deH-Pip) |

| Compound No. | $R^1$ | $R^2$ | $R^3$ |
|---|---|---|---|
| 1-1073 | 4-F-Ph | 2-MeNH-4-Pym | 4-(3,4-deH-Pip) |
| 1-1075 | 4-F-Ph | 2-MeNH-4-Pym | 1-Me-4-(3,4-deH-Pip) |

| Compound No. | $R^1$ | $R^2$ | $R^3$ |
|---|---|---|---|
| 1-1166 | 3-F-Ph | 2-MeNH-4-Pym | 4-(3,4-deH-Pip) |
| 1-1168 | 3-F-Ph | 2-MeNH-4-Pym | 1-Me-4-(3,4-deH-Pip) |

| Compound No. | $R^1$ | $R^2$ | $R^3$ |
|---|---|---|---|
| 1-1259 | 3,4-diF-Ph | 2-MeNH-4-Pym | 4-(3,4-deH-Pip) |
| 1-1261 | 3,4-diF-Ph | 2-MeNH-4-Pym | 1-Me-4-(3,4-deH-Pip) |

| Compound No. | $R^1$ | $R^2$ | $R^3$ |
|---|---|---|---|
| | | | |
| 1-1352 | 3-Cl-Ph | 2-MeNH-4-Pym | 4-(3,4-deH-Pip) |
| 1-1354 | 3-Cl-Ph | 2-MeNH-4-Pym | 1-Me-4-(3,4-deH-Pip) |

| Compound No. | $R^1$ | $R^2$ | $R^3$ |
|---|---|---|---|
| 1-1413 | 3-Cl-4-F-Ph | 4-Pyr | 4-(3,4-deH-Pip) |
| 1-1415 | 3-Cl-4-F-Ph | 4-Pyr | 1-Me-4-(3,4-deH-Pip) |

| Compound No. | $R^1$ | $R^2$ | $R^3$ |
|---|---|---|---|
| 1-1448 | 3,4,5-triF-Ph | 4-Pyr | 4-(3,4-deH-Pip) |
| 1-1450 | 3,4,5-triF-Ph | 4-Pyr | 1-Me-4-(3,4-deH-Pip) |

| Compound No. | $R^1$ | $R^2$ | $R^3$ |
|---|---|---|---|
| 1-1483 | 3-$CF_3$-Ph | 4-Pyr | 4-(3,4-deH-Pip) |
| 1-1485 | 3-$CF_3$-Ph | 4-Pyr | 1-Me-4-(3,4-deH-Pip) |

| Compound No. | $R^1$ | $R^2$ | $R^3$ |
|---|---|---|---|
| 1-1518 | 3-$CHF_2O$-Ph | 4-Pyr | 4-(3,4-deH-Pip) |
| 1-1520 | 3-$CHF_2O$-Ph | 4-Pyr | 1-Me-4-(3,4-deH-Pip) |

| Compound No. | $R^1$ | $R^2$ | $R^3$ |
|---|---|---|---|
| 1-1553 | 3-Cl-4-F-Ph | 2-$NH_2$-4-Pym | 4-(3,4-deH-Pip) |
| 1-1555 | 3-Cl-4-F-Ph | 2-$NH_2$-4-Pym | 1-Me-4-(3,4-deH-Pip) |

| Compound No. | $R^1$ | $R^2$ | $R^3$ |
|---|---|---|---|
| 1-1588 | 3,4,5-triF-Ph | 2-$NH_2$-4-Pym | 4-(3,4-deH-Pip) |

| Compound No. | $R^1$ | $R^2$ | $R^3$ |
|---|---|---|---|
| 1-1590 | 3,4,5-triF-Ph | 2-$NH_2$-4-Pym | 1-Me-4-(3,4-deH-Pip) |

| Compound No. | $R^1$ | $R^2$ | $R^3$ |
|---|---|---|---|
| 1-1623 | 3-$CF_3$-Ph | 2-$NH_2$-4-Pym | 4-(3,4-deH-Pip) |
| 1-1625 | 3-$CF_3$-Ph | 2-$NH_2$-4-Pym | 1-Me-4-(3,4-deH-Pip) |

| Compound No. | $R^1$ | $R^2$ | $R^3$ |
|---|---|---|---|
| 1-1658 | 3-$CHF_2$O-Ph | 2-$NH_2$-4-Pym | 4-(3,4-deH-Pip) |
| 1-1660 | 3-$CHF_2$O-Ph | 2-$NH_2$-4-Pym | 1-Me-4-(3,4-deH-Pip) |

| Compound No. | $R^1$ | $R^2$ | $R^3$ |
|---|---|---|---|
| 1-1693 | 3-Cl-4-F-Ph | 2-MeNH-4-Pym | 4-(3,4-deH-Pip) |
| 1-1695 | 3-Cl-4-F-Ph | 2-MeNH-4-Pym | 1-Me-4-(3,4-deH-Pip) |

| Compound No. | $R^1$ | $R^2$ | $R^3$ |
|---|---|---|---|
| 1-1728 | 3,4,5-triF-Ph | 2-MeNH-4-Pym | 4-(3,4-deH-Pip) |
| 1-1730 | 3,4,5-triF-Ph | 2-MeNH-4-Pym | 1-Me-4-(3,4-deH-Pip) |

| Compound No. | $R^1$ | $R^2$ | $R^3$ |
|---|---|---|---|
| 1-1763 | 3-$CF_3$-Ph | 2-MeNH-4-Pym | 4-(3,4-deH-Pip) |
| 1-1765 | 3-$CF_3$-Ph | 2-MeNH-4-Pym | 1-Me-4-(3,4-deH-Pip) |

| Compound No. | $R^1$ | $R^2$ | $R^3$ |
|---|---|---|---|
| 1-1798 | 3-$CHF_2$O-Ph | 2-MeNH-4-Pym | 4-(3,4-deH-Pip) |

| Compound No. | $R^1$ | $R^2$ | $R^3$ |
|---|---|---|---|
| 1-1800 | 3-$CHF_2O$-Ph | 2-MeNH-4-Pym | 1-Me-4-(3,4-deH-Pip) |

| Compound No. | $R^1$ | $R^2$ | $R^3$ |
|---|---|---|---|
| 1-1833 | Ph | 2-$NH_2$-4-Pyr | 4-(3,4-deH-Pip) |
| 1-1835 | Ph | 2-$NH_2$-4-Pyr | 1-Me-4-(3,4-deH-Pip) |

| Compound No. | $R^1$ | $R^2$ | $R^3$ |
|---|---|---|---|
| 1-1868 | 4-F-Ph | 2-$NH_2$-4-Pyr | 4-(3,4-deH-Pip) |
| 1-1870 | 4-F-Ph | 2-$NH_2$-4-Pyr | 1-Me-4-(3,4-deH-Pip) |

| Compound No. | $R^1$ | $R^2$ | $R^3$ |
|---|---|---|---|
| 1-1903 | 3-F-Ph | 2-$NH_2$-4-Pyr | 4-(3,4-deH-Pip) |
| 1-1905 | 3-F-Ph | 2-$NH_2$-4-Pyr | 1-Me-4-(3,4-deH-Pip) |

| Compound No. | $R^1$ | $R^2$ | $R^3$ |
|---|---|---|---|
| 1-1938 | 3,4-diF-Ph | 2-$NH_2$-4-Pyr | 4-(3,4-deH-Pip) |
| 1-1940 | 3,4-diF-Ph | 2-$NH_2$-4-Pyr | 1-Me-4-(3,4-deH-Pip) |

| Compound No. | $R^1$ | $R^2$ | $R^3$ |
|---|---|---|---|
| 1-1973 | 3-Cl-Ph | 2-$NH_2$-4-Pyr | 4-(3,4-deH-Pip) |
| 1-1975 | 3-Cl-Ph | 2-$NH_2$-4-Pyr | 1-Me-4-(3,4-deH-Pip) |

| Compound No. | $R^1$ | $R^2$ | $R^3$ |
|---|---|---|---|
| 1-2008 | Ph | 2-MeNH-4-Pyr | 4-(3,4-deH-Pip) |

| Compound No. | $R^1$ | $R^2$ | $R^3$ |
|---|---|---|---|
| 1-2010 | Ph | 2-MeNH-4-Pyr | 1-Me-4-(3,4-deH-Pip) |

| Compound No. | $R^1$ | $R^2$ | $R^3$ |
|---|---|---|---|
| 1-2043 | 4-F-Ph | 2-MeNH-4-Pyr | 4-(3,4-deH-Pip) |
| 1-2045 | 4-F-Ph | 2-MeNH-4-Pyr | 1-Me-4-(3,4-deH-Pip) |

| Compound No. | $R^1$ | $R^2$ | $R^3$ |
|---|---|---|---|
| 1-2078 | 3-F-Ph | 2-MeNH-4-Pyr | 4-(3,4-deH-Pip) |
| 1-2080 | 3-F-Ph | 2-MeNH-4-Pyr | 1-Me-4-(3,4-deH-Pip) |

| Compound No. | $R^1$ | $R^2$ | $R^3$ |
|---|---|---|---|
| 1-2113 | 3,4-diF-Ph | 2-MeNH-4-Pyr | 4-(3,4-deH-Pip) |
| 1-2115 | 3,4-diF-Ph | 2-MeNH-4-Pyr | 1-Me-4-(3,4-deH-Pip) |

| Compound No. | $R^1$ | $R^2$ | $R^3$ |
|---|---|---|---|
| 1-2148 | 3-Cl-Ph | 2-MeNH-4-Pyr | 4-(3,4-deH-Pip) |
| 1-2150 | 3-Cl-Ph | 2-MeNH-4-Pyr | 1-Me-4-(3,4-deH-Pip) |

| Compound No. | $R^1$ | $R^2$ | $R^3$ |
|---|---|---|---|
| 1-2183 | Ph | 4-Pym | 4-(3,4-deH-Pip) |
| 1-2185 | Ph | 4-Pym | 1-Me-4-(3,4-deH-Pip) |

| Compound No. | $R^1$ | $R^2$ | $R^3$ |
|---|---|---|---|
| 1-2218 | 4-F-Ph | 4-Pym | 4-(3,4-deH-Pip) |

| Compound No. | $R^1$ | $R^2$ | $R^3$ |
|---|---|---|---|
| 1-2220 | 4-F-Ph | 4-Pym | 1-Me-4-(3,4-deH-Pip) |

| Compound No. | $R^1$ | $R^2$ | $R^3$ |
|---|---|---|---|
| 1-2253 | 3-F-Ph | 4-Pym | 4-(3,4-deH-Pip) |
| 1-2255 | 3-F-Ph | 4-Pym | 1-Me-4-(3,4-deH-Pip) |

| Compound No. | $R^1$ | $R^2$ | $R^3$ |
|---|---|---|---|
| 1-2288 | 3,4-diF-Ph | 4-Pym | 4-(3,4-deH-Pip) |
| 1-2290 | 3,4-diF-Ph | 4-Pym | 1-Me-4-(3,4-deH-Pip) |

| Compound No. | $R^1$ | $R^2$ | $R^3$ |
|---|---|---|---|
| 1-2323 | 3-Cl-Ph | 4-Pym | 4-(3,4-deH-Pip) |
| 1-2325 | 3-Cl-Ph | 4-Pym | 1-Me-4-(3,4-deH-Pip) |

| Compound No. | $R^1$ | $R^2$ | $R^3$ |
|---|---|---|---|
| 1-2358 | Ph | 2-MeO-4-Pym | 4-(3,4-deH-Pip) |
| 1-2360 | Ph | 2-MeO-4-Pym | 1-Me-4-(3,4-deH-Pip) |

| Compound No. | $R^1$ | $R^2$ | $R^3$ |
|---|---|---|---|
| 1-2393 | 4-F-Ph | 2-MeO-4-Pym | 4-(3,4-deH-Pip) |
| 1-2395 | 4-F-Ph | 2-MeO-4-Pym | 1-Me-4-(3,4-deH-Pip) |

| Compound No. | $R^1$ | $R^2$ | $R^3$ |
|---|---|---|---|
| 1-2428 | 3-F-Ph | 2-MeO-4-Pym | 4-(3,4-deH-Pip) |

| Compound No. | $R^1$ | $R^2$ | $R^3$ |
|---|---|---|---|
| 1-2430 | 3-F-Ph | 2-MeO-4-Pym | 1-Me-4-(3,4-deH-Pip) |

| Compound No. | $R^1$ | $R^2$ | $R^3$ |
|---|---|---|---|
| 1-2463 | 3,4-diF-Ph | 2-MeO-4-Pym | 4-(3,4-deH-Pip) |
| 1-2465 | 3,4-diF-Ph | 2-MeO-4-Pym | 1-Me-4-(3,4-deH-Pip) |

| Compound No. | $R^1$ | $R^2$ | $R^3$ |
|---|---|---|---|
| 1-2498 | 3-Cl-Ph | 2-MeO-4-Pym | 4-(3,4-deH-Pip) |
| 1-2500 | 3-Cl-Ph | 2-MeO-4-Pym | 1-Me-4-(3,4-deH-Pip) |

| Compound No. | $R^1$ | $R^2$ | $R^3$ |
|---|---|---|---|
| 1-2525 | 4-F-Ph | 4-Pyr | 1-Ac-4-(3,4-deH-Pip) |

| Compound No. | $R^1$ | $R^2$ | $R^3$ |
|---|---|---|---|
| 1-2557 | 4-F-Ph | 2-$NH_2$-4-Pym | 1-Ac-4-(3,4-deH-Pip) |

| Compound No. | $R^1$ | $R^2$ | $R^3$ |
|---|---|---|---|
| 1-2580 | 4-F-Ph | 2-MeNH-4-Pym | 1-Ac-4-(3,4-deH-Pip) |
| 1-2596 | 4-F-Ph | 2-BnNH-4-Pyr | 4-(3,4-deH-Pip) |
| 1-2598 | 4-F-Ph | 2-BnNH-4-Pym | 4-(3,4-deH-Pip) |
| 1-2600 | 4-F-Ph | 2-(α-Me-BnNH)-4-Pyr | 4-(3,4-deH-Pip) |
| 1-2602 | 4-F-Ph | 2-(α-Me-BnNH)-4-Pym | 4-(3,4-deH-Pip) |
| 1-2604 | 3-Cl-Ph | 2-BnNH-4-Pyr | 4-(3,4-deH-Pip) |
| 1-2606 | 3-Cl-Ph | 2-BnNH-4-Pym | 4-(3,4-deH-Pip) |
| 1-2608 | 3-Cl-Ph | 2-(α-Me-BnNH)-4-Pyr | 4-(3,4-deH-Pip) |

| Compound No. | $R^1$ | $R^2$ | $R^3$ |
|---|---|---|---|
| 1-2610 | 3-Cl-Ph | 2-(α-Me-BnNH)-4-Pym | 4-(3,4-deH-Pip) |
| 1-2612 | $3-CF_3-Ph$ | 2-BnNH-4-Pyr | 4-(3,4-deH-Pip) |
| 1-2614 | $3-CF_3-Ph$ | 2-BnNH-4-Pym | 4-(3,4-deH-Pip) |
| 1-2616 | $3-CF_3-Ph$ | 2-(α-Me-BnNH)-4-Pyr | 4-(3,4-deH-Pip) |
| 1-2618 | $3-CF_3-Ph$ | 2-(α-Me-BnNH)-4-Pym | 4-(3,4-deH-Pip) |
| 1-2627 | 4-F-Ph | 4-Pyr | 3-(3,4-deH-Pip) |
| 1-2628 | 4-F-Ph | 4-Pyr | 1-Me-3-(3,4-deH-Pip) |
| 1-2629 | 4-F-Ph | 4-Pyr | 1-Et-4-(3,4-deH-Pip) |
| 1-2630 | 4-F-Ph | 4-Pyr | 1-Pr-4-(3,4-deH-Pip) |
| 1-2632 | 4-F-Ph | 4-Pyr | 1-iPr-4-(3,4-deH-Pip) |
| 1-2634 | 4-F-Ph | 4-Pyr | 1-Bu-4-(3,4-deH-Pip) |
| 1-2635 | 4-F-Ph | 4-Pyr | 1-tBu-4-(3,4-deH-Pip) |
| 1-2636 | 4-F-Ph | 4-Pyr | 1-Pn-4-(3,4-deH-Pip) |
| 1-2637 | 4-F-Ph | 4-Pyr | 1-Hx-4-(3,4-deH-Pip) |
| 1-2638 | 4-F-Ph | 4-Pyr | 1-Hp-4-(3,4-deH-Pip) |

| Compound No. | $R^1$ | $R^2$ | $R^3$ |
|---|---|---|---|
| 1-2639 | 4-F-Ph | 4-Pyr | 1-Oc-4-(3,4-deH-Pip) |
| 1-2640 | 4-F-Ph | 4-Pyr | 1-Nn-4-(3,4-deH-Pip) |
| 1-2641 | 4-F-Ph | 4-Pyr | 1-cPr-4-(3,4-deH-Pip) |
| 1-2642 | 4-F-Ph | 4-Pyr | 1-cPn-4-(3,4-deH-Pip) |
| 1-2643 | 4-F-Ph | 4-Pyr | 1-cHx-4-(3,4-deH-Pip) |
| 1-2644 | 4-F-Ph | 4-Pyr | 1-Bn-4-(3,4-deH-Pip) |
| 1-2645 | 4-F-Ph | 4-Pyr | 1-Phet-4-(3,4-deH-Pip) |
| 1-2646 | 4-F-Ph | 4-Pyr | 1-(3-Ph-Pr)-4-(3,4-deH-Pip) |
| 1-2647 | 4-F-Ph | 4-Pyr | 1-(4-Ph-Bu)-4-(3,4-deH-Pip) |
| 1-2648 | 4-F-Ph | 4-Pyr | 1-Allyl-4-(3,4-deH-Pip) |
| 1-2649 | 4-F-Ph | 4-Pyr | 1-Propargyl-4-(3,4-deH-Pip) |
| 1-2650 | 4-F-Ph | 4-Pyr | 2,2,6,6-tetraMe-4-(3,4-deH-Pip) |
| 1-2651 | 4-F-Ph | 4-Pyr | 1,2,2,6,6-pentaMe-4-(3,4-deH-Pip) |
| 1-2653 | 4-F-Ph | 4-Pyr | 7-(1,2,3,5,6,8a-hexaH-Ind) |
| 1-2654 | 4-F-Ph | 4-Pyr | 7-(1,2,3,5,8,8a-hexaH-Ind) |
| 1-2656 | 4-F-Ph | 4-Pyr | 8-(1,3,4,6,7,9a-hexaH-2H-Qui) |
| 1-2657 | 4-F-Ph | 4-Pyr | 8-(1,3,4,6,9,9a-hexaH-2H-Qui) |
| 1-2659 | 4-F-Ph | 4-Pyr | 2,2-diMe-4-(3,4-deH-Pip) |
| 1-2660 | 4-F-Ph | 4-Pyr | 1,2,2-triMe-4-(3,4-deH-Pip) |
| 1-2661 | 4-F-Ph | 4-Pyr | 2,2-diMe-4-(4,5-deH-Pip) |
| 1-2662 | 4-F-Ph | 4-Pyr | 1,2,2-triMe-4-(4,5-deH-Pip) |
| 1-2663 | 4-F-Ph | 4-Pyr | 2,6-diMe-4-(3,4-deH-Pip) |
| 1-2664 | 4-F-Ph | 4-Pyr | 1,2,6-triMe-4-(3,4-deH-Pip) |
| 1-2665 | 4-F-Ph | 4-Pyr | 2-Me-4-(3,4-deH-Pip) |
| 1-2666 | 4-F-Ph | 4-Pyr | 1,2-diMe-4-(3,4-deH-Pip) |

| Compound No. | $R^1$ | $R^2$ | $R^3$ |
|---|---|---|---|
| 1-2667 | 4-F-Ph | 4-Pyr | 1-Et-2-Me-4-(3,4-deH-Pip) |
| 1-2668 | 4-F-Ph | 4-Pyr | 1-Pr-2-Me-4-(3,4-deH-Pip) |
| 1-2669 | 4-F-Ph | 4-Pyr | 1-Bu-2-Me-4-(3,4-deH-Pip) |
| 1-2670 | 4-F-Ph | 4-Pyr | 1-Phet-2-Me-4-(3,4-deH-Pip) |
| 1-2671 | 4-F-Ph | 4-Pyr | 2-Et-4-(3,4-deH-Pip) |
| 1-2672 | 4-F-Ph | 4-Pyr | 1-Me-2-Et-4-(3,4-deH-Pip) |
| 1-2673 | 4-F-Ph | 4-Pyr | 1,2-diEt-4-(3,4-deH-Pip) |
| 1-2674 | 4-F-Ph | 4-Pyr | 1-Pr-2-Et-4-(3,4-deH-Pip) |
| 1-2675 | 4-F-Ph | 4-Pyr | 1-Bu-2-Et-4-(3,4-deH-Pip) |
| 1-2676 | 4-F-Ph | 4-Pyr | 1-Phet-2-Et-4-(3,4-deH-Pip) |
| 1-2677 | 4-F-Ph | 4-Pyr | 2-Pr-4-(3,4-deH-Pip) |
| 1-2678 | 4-F-Ph | 4-Pyr | 1-Me-2-Pr-4-(3,4-deH-Pip) |
| 1-2679 | 4-F-Ph | 4-Pyr | 1-Et-2-Pr-4-(3,4-deH-Pip) |
| 1-2680 | 4-F-Ph | 4-Pyr | 1,2-diPr-4-(3,4-deH-Pip) |
| 1-2681 | 4-F-Ph | 4-Pyr | 1-Bu-2-Pr-4-(3,4-deH-Pip) |
| 1-2682 | 4-F-Ph | 4-Pyr | 1-Phet-2-Pr-4-(3,4-deH-Pip) |
| 1-2683 | 4-F-Ph | 4-Pyr | 2-Bu-4-(3,4-deH-Pip) |
| 1-2684 | 4-F-Ph | 4-Pyr | 1-Me-2-Bu-4-(3,4-deH-Pip) |
| 1-2685 | 4-F-Ph | 4-Pyr | 1-Et-2-Bu-4-(3,4-deH-Pip) |
| 1-2686 | 4-F-Ph | 4-Pyr | 1-Pr-2-Bu-4-(3,4-deH-Pip) |
| 1-2687 | 4-F-Ph | 4-Pyr | 1,2-diBu-4-(3,4-deH-Pip) |
| 1-2688 | 4-F-Ph | 4-Pyr | 1-Phet-2-Bu-4-(3,4-deH-Pip) |
| 1-2689 | 4-F-Ph | 4-Pyr | 2-Allyl-4-(3,4-deH-Pip) |
| 1-2690 | 4-F-Ph | 4-Pyr | 1-Me-2-Allyl-4-(3,4-deH-Pip) |
| 1-2691 | 4-F-Ph | 4-Pyr | 1-Et-2-Allyl-4-(3,4-deH-Pip) |
| 1-2692 | 4-F-Ph | 4-Pyr | 1-Pr-2-Allyl-4-(3,4-deH-Pip) |
| 1-2693 | 4-F-Ph | 4-Pyr | 1-Bu-2-Allyl-4-(3,4-deH-Pip) |
| 1-2694 | 4-F-Ph | 4-Pyr | 1-Phet-2-Allyl-4-(3,4-deH-Pip) |
| 1-2695 | 4-F-Ph | 4-Pyr | 2-Bn-4-(3,4-deH-Pip) |
| 1-2696 | 4-F-Ph | 4-Pyr | 1-Me-2-Bn-4-(3,4-deH-Pip) |

| Compound No. | $R^1$ | $R^2$ | $R^3$ |
|---|---|---|---|
| 1-2697 | 4-F-Ph | 4-Pyr | 1-Et-2-Bn-4-(3,4-deH-Pip) |
| 1-2698 | 4-F-Ph | 4-Pyr | 1-Pr-2-Bn-4-(3,4-deH-Pip) |
| 1-2699 | 4-F-Ph | 4-Pyr | 1-Bu-2-Bn-4-(3,4-deH-Pip) |
| 1-2700 | 4-F-Ph | 4-Pyr | 1-Phet-2-Bn-4-(3,4-deH-Pip) |
| 1-2701 | 4-F-Ph | 4-Pyr | 2-Phet-4-(3,4-deH-Pip) |
| 1-2702 | 4-F-Ph | 4-Pyr | 1-Me-2-Phet-4-(3,4-deH-Pip) |
| 1-2703 | 4-F-Ph | 4-Pyr | 1-Et-2-Phet-4-(3,4-deH-Pip) |
| 1-2704 | 4-F-Ph | 4-Pyr | 1-Pr-2-Phet-4-(3,4-deH-Pip) |
| 1-2705 | 4-F-Ph | 4-Pyr | 1-Bu-2-Phet-4-(3,4-deH-Pip) |
| 1-2706 | 4-F-Ph | 4-Pyr | 1,2-diPhet-4-(3,4-deH-Pip) |
| 1-2707 | 4-F-Ph | 4-Pyr | 2-Me-4-(4,5-deH-Pip) |
| 1-2708 | 4-F-Ph | 4-Pyr | 1,2-diMe-4-(4,5-deH-Pip) |
| 1-2709 | 4-F-Ph | 4-Pyr | 1-Et-2-Me-4-(4,5-deH-Pip) |
| 1-2710 | 4-F-Ph | 4-Pyr | 1-Pr-2-Me-4-(4,5-deH-Pip) |
| 1-2711 | 4-F-Ph | 4-Pyr | 1-Bu-2-Me-4-(4,5-deH-Pip) |
| 1-2712 | 4-F-Ph | 4-Pyr | 1-Phet-2-Me-4-(4,5-deH-Pip) |
| 1-2713 | 4-F-Ph | 4-Pyr | 2-Et-4-(4,5-deH-Pip) |
| 1-2714 | 4-F-Ph | 4-Pyr | 1-Me-2-Et-4-(4,5-deH-Pip) |
| 1-2715 | 4-F-Ph | 4-Pyr | 1,2-diEt-4-(4,5-deH-Pip) |
| 1-2716 | 4-F-Ph | 4-Pyr | 1-Pr-2-Et-4-(4,5-deH-Pip) |
| 1-2717 | 4-F-Ph | 4-Pyr | 1-Bu-2-Et-4-(4,5-deH-Pip) |
| 1-2718 | 4-F-Ph | 4-Pyr | 1-Phet-2-Et-4-(4,5-deH-Pip) |
| 1-2719 | 4-F-Ph | 4-Pyr | 2-Pr-4-(4,5-deH-Pip) |
| 1-2720 | 4-F-Ph | 4-Pyr | 1-Me-2-Pr-4-(4,5-deH-Pip) |
| 1-2721 | 4-F-Ph | 4-Pyr | 1-Et-2-Pr-4-(4,5-deH-Pip) |
| 1-2722 | 4-F-Ph | 4-Pyr | 1,2-diPr-4-(4,5-deH-Pip) |
| 1-2723 | 4-F-Ph | 4-Pyr | 1-Bu-2-Pr-4-(4,5-deH-Pip) |
| 1-2724 | 4-F-Ph | 4-Pyr | 1-Phet-2-Pr-4-(4,5-deH-Pip) |
| 1-2725 | 4-F-Ph | 4-Pyr | 2-Bu-4-(4,5-deH-Pip) |
| 1-2726 | 4-F-Ph | 4-Pyr | 1-Me-2-Bu-4-(4,5-deH-Pip) |

| Compound No. | $R^1$ | $R^2$ | $R^3$ |
|---|---|---|---|
| 1-2727 | 4-F-Ph | 4-Pyr | 1-Et-2-Bu-4-(4,5-deH-Pip) |
| 1-2728 | 4-F-Ph | 4-Pyr | 1-Pr-2-Bu-4-(4,5-deH-Pip) |
| 1-2729 | 4-F-Ph | 4-Pyr | 1,2-diBu-4-(4,5-deH-Pip) |
| 1-2730 | 4-F-Ph | 4-Pyr | 1-Phet-2-Bu-4-(4,5-deH-Pip) |
| 1-2731 | 4-F-Ph | 4-Pyr | 2-Allyl-4-(4,5-deH-Pip) |
| 1-2732 | 4-F-Ph | 4-Pyr | 1-Me-2-Allyl-4-(4,5-deH-Pip) |
| 1-2733 | 4-F-Ph | 4-Pyr | 1-Et-2-Allyl-4-(4,5-deH-Pip) |
| 1-2734 | 4-F-Ph | 4-Pyr | 1-Pr-2-Allyl-4-(4,5-deH-Pip) |
| 1-2735 | 4-F-Ph | 4-Pyr | 1-Bu-2-Allyl-4-(4,5-deH-Pip) |
| 1-2736 | 4-F-Ph | 4-Pyr | 1-Phet-2-Allyl-4-(4,5-deH-Pip) |
| 1-2737 | 4-F-Ph | 4-Pyr | 2-Bn-4-(4,5-deH-Pip) |
| 1-2738 | 4-F-Ph | 4-Pyr | 1-Me-2-Bn-4-(4,5-deH-Pip) |
| 1-2739 | 4-F-Ph | 4-Pyr | 1-Et-2-Bn-4-(4,5-deH-Pip) |
| 1-2740 | 4-F-Ph | 4-Pyr | 1-Pr-2-Bn-4-(4,5-deH-Pip) |
| 1-2741 | 4-F-Ph | 4-Pyr | 1-Bu-2-Bn-4-(4,5-deH-Pip) |
| 1-2742 | 4-F-Ph | 4-Pyr | 1-Phet-2-Bn-4-(4,5-deH-Pip) |
| 1-2743 | 4-F-Ph | 4-Pyr | 2-Phet-4-(4,5-deH-Pip) |
| 1-2744 | 4-F-Ph | 4-Pyr | 1-Me-2-Phet-4-(4,5-deH-Pip) |
| 1-2745 | 4-F-Ph | 4-Pyr | 1-Et-2-Phet-4-(4,5-deH-Pip) |
| 1-2746 | 4-F-Ph | 4-Pyr | 1-Pr-2-Phet-4-(4,5-deH-Pip) |
| 1-2747 | 4-F-Ph | 4-Pyr | 1-Bu-2-Phet-4-(4,5-deH-Pip) |
| 1-2748 | 4-F-Ph | 4-Pyr | 1,2-diPhet-4-(4,5-deH-Pip) |
| 1-2753 | 4-F-Ph | 2-$NH_2$-4-Pym | 3-(3,4-deH-Pip) |
| 1-2754 | 4-F-Ph | 2-$NH_2$-4-Pym | 1-Me-3-(3,4-deH-Pip) |
| 1-2755 | 4-F-Ph | 2-$NH_2$-4-Pym | 1-Et-4-(3,4-deH-Pip) |
| 1-2756 | 4-F-Ph | 2-$NH_2$-4-Pym | 1-Pr-4-(3,4-deH-Pip) |

| Compound No. | $R^1$ | $R^2$ | $R^3$ |
|---|---|---|---|
| 1-2758 | 4-F-Ph | 2-$NH_2$-4-Pym | 1-iPr-4-(3,4-deH-Pip) |
| 1-2760 | 4-F-Ph | 2-$NH_2$-4-Pym | 1-Bu-4-(3,4-deH-Pip) |
| 1-2761 | 4-F-Ph | 2-$NH_2$-4-Pym | 1-tBu-4-(3,4-deH-Pip) |
| 1-2762 | 4-F-Ph | 2-$NH_2$-4-Pym | 1-Pn-4-(3,4-deH-Pip) |
| 1-2763 | 4-F-Ph | 2-$NH_2$-4-Pym | 1-Hx-4-(3,4-deH-Pip) |
| 1-2764 | 4-F-Ph | 2-$NH_2$-4-Pym | 1-Hp-4-(3,4-deH-Pip) |
| 1-2765 | 4-F-Ph | 2-$NH_2$-4-Pym | 1-Oc-4-(3,4-deH-Pip) |
| 1-2766 | 4-F-Ph | 2-$NH_2$-4-Pym | 1-Nn-4-(3,4-deH-Pip) |
| 1-2767 | 4-F-Ph | 2-$NH_2$-4-Pym | 1-cPr-4-(3,4-deH-Pip) |
| 1-2768 | 4-F-Ph | 2-$NH_2$-4-Pym | 1-cPn-4-(3,4-deH-Pip) |
| 1-2769 | 4-F-Ph | 2-$NH_2$-4-Pym | 1-cHx-4-(3,4-deH-Pip) |
| 1-2770 | 4-F-Ph | 2-$NH_2$-4-Pym | 1-Bn-4-(3,4-deH-Pip) |
| 1-2771 | 4-F-Ph | 2-$NH_2$-4-Pym | 1-Phet-4-(3,4-deH-Pip) |
| 1-2772 | 4-F-Ph | 2-$NH_2$-4-Pym | 1-(3-Ph-Pr)-4-(3,4-deH-Pip) |
| 1-2773 | 4-F-Ph | 2-$NH_2$-4-Pym | 1-(4-Ph-Bu)-4-(3,4-deH-Pip) |
| 1-2774 | 4-F-Ph | 2-$NH_2$-4-Pym | 1-Allyl-4-(3,4-deH-Pip) |
| 1-2775 | 4-F-Ph | 2-$NH_2$-4-Pym | 1-Propargyl-4-(3,4-deH-Pip) |
| 1-2776 | 4-F-Ph | 2-$NH_2$-4-Pym | 2,2,6,6-tetraMe-4-(3,4-deH-Pip) |
| 1-2777 | 4-F-Ph | 2-$NH_2$-4-Pym | 1,2,2,6,6-pentaMe-4-(3,4-deH-Pip) |
| 1-2779 | 4-F-Ph | 2-$NH_2$-4-Pym | 7-(1,2,3,5,6,8a-hexaH-Ind) |
| 1-2780 | 4-F-Ph | 2-$NH_2$-4-Pym | 7-(1,2,3,5,8,8a-hexaH-Ind) |
| 1-2782 | 4-F-Ph | 2-$NH_2$-4-Pym | 8-(1,3,4,6,7,9a-hexaH-2H-Qui) |
| 1-2783 | 4-F-Ph | 2-$NH_2$-4-Pym | 8-(1,3,4,6,9,9a-hexaH-2H-Qui) |

| Compound No. | $R^1$ | $R^2$ | $R^3$ |
|---|---|---|---|
| 1-2785 | 4-F-Ph | $2-NH_2-4-Pym$ | 2,2-diMe-4-(3,4-deH-Pip) |
| 1-2786 | 4-F-Ph | $2-NH_2-4-Pym$ | 1,2,2-triMe-4-(3,4-deH-Pip) |
| 1-2787 | 4-F-Ph | $2-NH_2-4-Pym$ | 2,2-diMe-4-(4,5-deH-Pip) |
| 1-2788 | 4-F-Ph | $2-NH_2-4-Pym$ | 1,2,2-triMe-4-(4,5-deH-Pip) |
| 1-2789 | 4-F-Ph | $2-NH_2-4-Pym$ | 2,6-diMe-4-(3,4-deH-Pip) |
| 1-2790 | 4-F-Ph | $2-NH_2-4-Pym$ | 1,2,6-triMe-4-(3,4-deH-Pip) |
| 1-2791 | 4-F-Ph | $2-NH_2-4-Pym$ | 2-Me-4-(3,4-deH-Pip) |
| 1-2792 | 4-F-Ph | $2-NH_2-4-Pym$ | 1,2-diMe-4-(3,4-deH-Pip) |
| 1-2793 | 4-F-Ph | $2-NH_2-4-Pym$ | 1-Et-2-Me-4-(3,4-deH-Pip) |
| 1-2794 | 4-F-Ph | $2-NH_2-4-Pym$ | 1-Pr-2-Me-4-(3,4-deH-Pip) |
| 1-2795 | 4-F-Ph | $2-NH_2-4-Pym$ | 1-Bu-2-Me-4-(3,4-deH-Pip) |
| 1-2796 | 4-F-Ph | $2-NH_2-4-Pym$ | 1-Phet-2-Me-4-(3,4-deH-Pip) |
| 1-2797 | 4-F-Ph | $2-NH_2-4-Pym$ | 2-Et-4-(3,4-deH-Pip) |
| 1-2798 | 4-F-Ph | $2-NH_2-4-Pym$ | 1-Me-2-Et-4-(3,4-deH-Pip) |
| 1-2799 | 4-F-Ph | $2-NH_2-4-Pym$ | 1,2-diEt-4-(3,4-deH-Pip) |
| 1-2800 | 4-F-Ph | $2-NH_2-4-Pym$ | 1-Pr-2-Et-4-(3,4-deH-Pip) |
| 1-2801 | 4-F-Ph | $2-NH_2-4-Pym$ | 1-Bu-2-Et-4-(3,4-deH-Pip) |
| 1-2802 | 4-F-Ph | $2-NH_2-4-Pym$ | 1-Phet-2-Et-4-(3,4-deH-Pip) |
| 1-2803 | 4-F-Ph | $2-NH_2-4-Pym$ | 2-Pr-4-(3,4-deH-Pip) |
| 1-2804 | 4-F-Ph | $2-NH_2-4-Pym$ | 1-Me-2-Pr-4-(3,4-deH-Pip) |
| 1-2805 | 4-F-Ph | $2-NH_2-4-Pym$ | 1-Et-2-Pr-4-(3,4-deH-Pip) |
| 1-2806 | 4-F-Ph | $2-NH_2-4-Pym$ | 1,2-diPr-4-(3,4-deH-Pip) |
| 1-2807 | 4-F-Ph | $2-NH_2-4-Pym$ | 1-Bu-2-Pr-4-(3,4-deH-Pip) |
| 1-2808 | 4-F-Ph | $2-NH_2-4-Pym$ | 1-Phet-2-Pr-4-(3,4-deH-Pip) |
| 1-2809 | 4-F-Ph | $2-NH_2-4-Pym$ | 2-Bu-4-(3,4-deH-Pip) |
| 1-2810 | 4-F-Ph | $2-NH_2-4-Pym$ | 1-Me-2-Bu-4-(3,4-deH-Pip) |
| 1-2811 | 4-F-Ph | $2-NH_2-4-Pym$ | 1-Et-2-Bu-4-(3,4-deH-Pip) |
| 1-2812 | 4-F-Ph | $2-NH_2-4-Pym$ | 1-Pr-2-Bu-4-(3,4-deH-Pip) |
| 1-2813 | 4-F-Ph | $2-NH_2-4-Pym$ | 1,2-diBu-4-(3,4-deH-Pip) |
| 1-2814 | 4-F-Ph | $2-NH_2-4-Pym$ | 1-Phet-2-Bu-4-(3,4-deH-Pip) |

| Compound No. | $R^1$ | $R^2$ | $R^3$ |
|---|---|---|---|
| 1-2815 | 4-F-Ph | 2-$NH_2$-4-Pym | 2-Allyl-4-(3,4-deH-Pip) |
| 1-2816 | 4-F-Ph | 2-$NH_2$-4-Pym | 1-Me-2-Allyl-4-(3,4-deH-Pip) |
| 1-2817 | 4-F-Ph | 2-$NH_2$-4-Pym | 1-Et-2-Allyl-4-(3,4-deH-Pip) |
| 1-2818 | 4-F-Ph | 2-$NH_2$-4-Pym | 1-Pr-2-Allyl-4-(3,4-deH-Pip) |
| 1-2819 | 4-F-Ph | 2-$NH_2$-4-Pym | 1-Bu-2-Allyl-4-(3,4-deH-Pip) |
| 1-2820 | 4-F-Ph | 2-$NH_2$-4-Pym | 1-Phet-2-Allyl-4-(3,4-deH-Pip) |
| 1-2821 | 4-F-Ph | 2-$NH_2$-4-Pym | 2-Bn-4-(3,4-deH-Pip) |
| 1-2822 | 4-F-Ph | 2-$NH_2$-4-Pym | 1-Me-2-Bn-4-(3,4-deH-Pip) |
| 1-2823 | 4-F-Ph | 2-$NH_2$-4-Pym | 1-Et-2-Bn-4-(3,4-deH-Pip) |
| 1-2824 | 4-F-Ph | 2-$NH_2$-4-Pym | 1-Pr-2-Bn-4-(3,4-deH-Pip) |
| 1-2825 | 4-F-Ph | 2-$NH_2$-4-Pym | 1-Bu-2-Bn-4-(3,4-deH-Pip) |
| 1-2826 | 4-F-Ph | 2-$NH_2$-4-Pym | 1-Phet-2-Bn-4-(3,4-deH-Pip) |
| 1-2827 | 4-F-Ph | 2-$NH_2$-4-Pym | 2-Phet-4-(3,4-deH-Pip) |
| 1-2828 | 4-F-Ph | 2-$NH_2$-4-Pym | 1-Me-2-Phet-4-(3,4-deH-Pip) |
| 1-2829 | 4-F-Ph | 2-$NH_2$-4-Pym | 1-Et-2-Phet-4-(3,4-deH-Pip) |
| 1-2830 | 4-F-Ph | 2-$NH_2$-4-Pym | 1-Pr-2-Phet-4-(3,4-deH-Pip) |
| 1-2831 | 4-F-Ph | 2-$NH_2$-4-Pym | 1-Bu-2-Phet-4-(3,4-deH-Pip) |
| 1-2832 | 4-F-Ph | 2-$NH_2$-4-Pym | 1,2-diPhet-4-(3,4-deH-Pip) |
| 1-2833 | 4-F-Ph | 2-$NH_2$-4-Pym | 2-Me-4-(4,5-deH-Pip) |
| 1-2834 | 4-F-Ph | 2-$NH_2$-4-Pym | 1,2-diMe-4-(4,5-deH-Pip) |
| 1-2835 | 4-F-Ph | 2-$NH_2$-4-Pym | 1-Et-2-Me-4-(4,5-deH-Pip) |
| 1-2836 | 4-F-Ph | 2-$NH_2$-4-Pym | 1-Pr-2-Me-4-(4,5-deH-Pip) |
| 1-2837 | 4-F-Ph | 2-$NH_2$-4-Pym | 1-Bu-2-Me-4-(4,5-deH-Pip) |
| 1-2838 | 4-F-Ph | 2-$NH_2$-4-Pym | 1-Phet-2-Me-4-(4,5-deH-Pip) |
| 1-2839 | 4-F-Ph | 2-$NH_2$-4-Pym | 2-Et-4-(4,5-deH-Pip) |
| 1-2840 | 4-F-Ph | 2-$NH_2$-4-Pym | 1-Me-2-Et-4-(4,5-deH-Pip) |
| 1-2841 | 4-F-Ph | 2-$NH_2$-4-Pym | 1,2-diEt-4-(4,5-deH-Pip) |
| 1-2842 | 4-F-Ph | 2-$NH_2$-4-Pym | 1-Pr-2-Et-4-(4,5-deH-Pip) |
| 1-2843 | 4-F-Ph | 2-$NH_2$-4-Pym | 1-Bu-2-Et-4-(4,5-deH-Pip) |
| 1-2844 | 4-F-Ph | 2-$NH_2$-4-Pym | 1-Phet-2-Et-4-(4,5-deH-Pip) |

| Compound No. | $R^1$ | $R^2$ | $R^3$ |
|---|---|---|---|
| 1-2845 | 4-F-Ph | $2-NH_2-4-Pym$ | 2-Pr-4-(4,5-deH-Pip) |
| 1-2846 | 4-F-Ph | $2-NH_2-4-Pym$ | 1-Me-2-Pr-4-(4,5-deH-Pip) |
| 1-2847 | 4-F-Ph | $2-NH_2-4-Pym$ | 1-Et-2-Pr-4-(4,5-deH-Pip) |
| 1-2848 | 4-F-Ph | $2-NH_2-4-Pym$ | 1,2-diPr-4-(4,5-deH-Pip) |
| 1-2849 | 4-F-Ph | $2-NH_2-4-Pym$ | 1-Bu-2-Pr-4-(4,5-deH-Pip) |
| 1-2850 | 4-F-Ph | $2-NH_2-4-Pym$ | 1-Phet-2-Pr-4-(4,5-deH-Pip) |
| 1-2851 | 4-F-Ph | $2-NH_2-4-Pym$ | 2-Bu-4-(4,5-deH-Pip) |
| 1-2852 | 4-F-Ph | $2-NH_2-4-Pym$ | 1-Me-2-Bu-4-(4,5-deH-Pip) |
| 1-2853 | 4-F-Ph | $2-NH_2-4-Pym$ | 1-Et-2-Bu-4-(4,5-deH-Pip) |
| 1-2854 | 4-F-Ph | $2-NH_2-4-Pym$ | 1-Pr-2-Bu-4-(4,5-deH-Pip) |
| 1-2855 | 4-F-Ph | $2-NH_2-4-Pym$ | 1,2-diBu-4-(4,5-deH-Pip) |
| 1-2856 | 4-F-Ph | $2-NH_2-4-Pym$ | 1-Phet-2-Bu-4-(4,5-deH-Pip) |
| 1-2857 | 4-F-Ph | $2-NH_2-4-Pym$ | 2-Allyl-4-(4,5-deH-Pip) |
| 1-2858 | 4-F-Ph | $2-NH_2-4-Pym$ | 1-Me-2-Allyl-4-(4,5-deH-Pip) |
| 1-2859 | 4-F-Ph | $2-NH_2-4-Pym$ | 1-Et-2-Allyl-4-(4,5-deH-Pip) |
| 1-2860 | 4-F-Ph | $2-NH_2-4-Pym$ | 1-Pr-2-Allyl-4-(4,5-deH-Pip) |
| 1-2861 | 4-F-Ph | $2-NH_2-4-Pym$ | 1-Bu-2-Allyl-4-(4,5-deH-Pip) |
| 1-2862 | 4-F-Ph | $2-NH_2-4-Pym$ | 1-Phet-2-Allyl-4-(4,5-deH-Pip) |
| 1-2863 | 4-F-Ph | $2-NH_2-4-Pym$ | 2-Bn-4-(4,5-deH-Pip) |
| 1-2864 | 4-F-Ph | $2-NH_2-4-Pym$ | 1-Me-2-Bn-4-(4,5-deH-Pip) |
| 1-2865 | 4-F-Ph | $2-NH_2-4-Pym$ | 1-Et-2-Bn-4-(4,5-deH-Pip) |
| 1-2866 | 4-F-Ph | $2-NH_2-4-Pym$ | 1-Pr-2-Bn-4-(4,5-deH-Pip) |
| 1-2867 | 4-F-Ph | $2-NH_2-4-Pym$ | 1-Bu-2-Bn-4-(4,5-deH-Pip) |
| 1-2868 | 4-F-Ph | $2-NH_2-4-Pym$ | 1-Phet-2-Bn-4-(4,5-deH-Pip) |
| 1-2869 | 4-F-Ph | $2-NH_2-4-Pym$ | 2-Phet-4-(4,5-deH-Pip) |
| 1-2870 | 4-F-Ph | $2-NH_2-4-Pym$ | 1-Me-2-Phet-4-(4,5-deH-Pip) |
| 1-2871 | 4-F-Ph | $2-NH_2-4-Pym$ | 1-Et-2-Phet-4-(4,5-deH-Pip) |
| 1-2872 | 4-F-Ph | $2-NH_2-4-Pym$ | 1-Pr-2-Phet-4-(4,5-deH-Pip) |
| 1-2873 | 4-F-Ph | $2-NH_2-4-Pym$ | 1-Bu-2-Phet-4-(4,5-deH-Pip) |
| 1-2874 | 4-F-Ph | $2-NH_2-4-Pym$ | 1,2-diPhet-4-(4,5-deH-Pip) |

| Compound No. | $R^1$ | $R^2$ | $R^3$ |
|---|---|---|---|
| 1-2879 | 4-F-Ph | 2-MeNH-4-Pym | 3-(3,4-deH-Pip) |
| 1-2880 | 4-F-Ph | 2-MeNH-4-Pym | 1-Me-3-(3,4-deH-Pip) |
| 1-2881 | 4-F-Ph | 2-MeNH-4-Pym | 1-Et-4-(3,4-deH-Pip) |
| 1-2882 | 4-F-Ph | 2-MeNH-4-Pym | 1-Pr-4-(3,4-deH-Pip) |
| 1-2884 | 4-F-Ph | 2-MeNH-4-Pym | 1-iPr-4-(3,4-deH-Pip) |
| 1-2886 | 4-F-Ph | 2-MeNH-4-Pym | 1-Bu-4-(3,4-deH-Pip) |
| 1-2887 | 4-F-Ph | 2-MeNH-4-Pym | 1-tBu-4-(3,4-deH-Pip) |
| 1-2888 | 4-F-Ph | 2-MeNH-4-Pym | 1-Pn-4-(3,4-deH-Pip) |
| 1-2889 | 4-F-Ph | 2-MeNH-4-Pym | 1-Hx-4-(3,4-deH-Pip) |
| 1-2890 | 4-F-Ph | 2-MeNH-4-Pym | 1-Hp-4-(3,4-deH-Pip) |
| 1-2891 | 4-F-Ph | 2-MeNH-4-Pym | 1-Oc-4-(3,4-deH-Pip) |
| 1-2892 | 4-F-Ph | 2-MeNH-4-Pym | 1-Nn-4-(3,4-deH-Pip) |
| 1-2893 | 4-F-Ph | 2-MeNH-4-Pym | 1-cPr-4-(3,4-deH-Pip) |
| 1-2894 | 4-F-Ph | 2-MeNH-4-Pym | 1-cPn-4-(3,4-deH-Pip) |
| 1-2895 | 4-F-Ph | 2-MeNH-4-Pym | 1-cHx-4-(3,4-deH-Pip) |
| 1-2896 | 4-F-Ph | 2-MeNH-4-Pym | 1-Bn-4-(3,4-deH-Pip) |
| 1-2897 | 4-F-Ph | 2-MeNH-4-Pym | 1-Phet-4-(3,4-deH-Pip) |
| 1-2898 | 4-F-Ph | 2-MeNH-4-Pym | 1-(3-Ph-Pr)-4-(3,4-deH-Pip) |
| 1-2899 | 4-F-Ph | 2-MeNH-4-Pym | 1-(4-Ph-Bu)-4-(3,4-deH-Pip) |
| 1-2900 | 4-F-Ph | 2-MeNH-4-Pym | 1-Allyl-4-(3,4-deH-Pip) |
| 1-2901 | 4-F-Ph | 2-MeNH-4-Pym | 1-Propargyl-4-(3,4-deH-Pip) |
| 1-2902 | 4-F-Ph | 2-MeNH-4-Pym | 2,2,6,6-tetraMe-4-(3,4-deH-Pip) |

| Compound No. | $R^1$ | $R^2$ | $R^3$ |
|---|---|---|---|
| 1-2903 | 4-F-Ph | 2-MeNH-4-Pym | 1,2,2,6,6-pentaMe-4-(3,4-deH-Pip) |
| 1-2905 | 4-F-Ph | 2-MeNH-4-Pym | 7-(1,2,3,5,6,8a-hexaH-Ind) |
| 1-2906 | 4-F-Ph | 2-MeNH-4-Pym | 7-(1,2,3,5,8,8a-hexaH-Ind) |
| 1-2908 | 4-F-Ph | 2-MeNH-4-Pym | 8-(1,3,4,6,7,9a-hexaH-2H-Qui) |
| 1-2909 | 4-F-Ph | 2-MeNH-4-Pym | 8-(1,3,4,6,9,9a-hexaH-2H-Qui) |
| 1-2911 | 4-F-Ph | 2-MeNH-4-Pym | 2,2-diMe-4-(3,4-deH-Pip) |
| 1-2912 | 4-F-Ph | 2-MeNH-4-Pym | 1,2,2-triMe-4-(3,4-deH-Pip) |
| 1-2913 | 4-F-Ph | 2-MeNH-4-Pym | 2,2-diMe-4-(4,5-deH-Pip) |
| 1-2914 | 4-F-Ph | 2-MeNH-4-Pym | 1,2,2-triMe-4-(4,5-deH-Pip) |
| 1-2915 | 4-F-Ph | 2-MeNH-4-Pym | 2,6-diMe-4-(3,4-deH-Pip) |
| 1-2916 | 4-F-Ph | 2-MeNH-4-Pym | 1,2,6-triMe-4-(3,4-deH-Pip) |
| 1-2917 | 4-F-Ph | 2-MeNH-4-Pym | 2-Me-4-(3,4-deH-Pip) |
| 1-2918 | 4-F-Ph | 2-MeNH-4-Pym | 1,2-diMe-4-(3,4-deH-Pip) |
| 1-2919 | 4-F-Ph | 2-MeNH-4-Pym | 1-Et-2-Me-4-(3,4-deH-Pip) |
| 1-2920 | 4-F-Ph | 2-MeNH-4-Pym | 1-Pr-2-Me-4-(3,4-deH-Pip) |
| 1-2921 | 4-F-Ph | 2-MeNH-4-Pym | 1-Bu-2-Me-4-(3,4-deH-Pip) |
| 1-2922 | 4-F-Ph | 2-MeNH-4-Pym | 1-Phet-2-Me-4-(3,4-deH-Pip) |
| 1-2923 | 4-F-Ph | 2-MeNH-4-Pym | 2-Et-4-(3,4-deH-Pip) |
| 1-2924 | 4-F-Ph | 2-MeNH-4-Pym | 1-Me-2-Et-4-(3,4-deH-Pip) |
| 1-2925 | 4-F-Ph | 2-MeNH-4-Pym | 1,2-diEt-4-(3,4-deH-Pip) |
| 1-2926 | 4-F-Ph | 2-MeNH-4-Pym | 1-Pr-2-Et-4-(3,4-deH-Pip) |
| 1-2927 | 4-F-Ph | 2-MeNH-4-Pym | 1-Bu-2-Et-4-(3,4-deH-Pip) |
| 1-2928 | 4-F-Ph | 2-MeNH-4-Pym | 1-Phet-2-Et-4-(3,4-deH-Pip) |
| 1-2929 | 4-F-Ph | 2-MeNH-4-Pym | 2-Pr-4-(3,4-deH-Pip) |
| 1-2930 | 4-F-Ph | 2-MeNH-4-Pym | 1-Me-2-Pr-4-(3,4-deH-Pip) |
| 1-2931 | 4-F-Ph | 2-MeNH-4-Pym | 1-Et-2-Pr-4-(3,4-deH-Pip) |

| Compound No. | $R^1$ | $R^2$ | $R^3$ |
|---|---|---|---|
| 1-2932 | 4-F-Ph | 2-MeNH-4-Pym | 1,2-diPr-4-(3,4-deH-Pip) |
| 1-2933 | 4-F-Ph | 2-MeNH-4-Pym | 1-Bu-2-Pr-4-(3,4-deH-Pip) |
| 1-2934 | 4-F-Ph | 2-MeNH-4-Pym | 1-Phet-2-Pr-4-(3,4-deH-Pip) |
| 1-2935 | 4-F-Ph | 2-MeNH-4-Pym | 2-Bu-4-(3,4-deH-Pip) |
| 1-2936 | 4-F-Ph | 2-MeNH-4-Pym | 1-Me-2-Bu-4-(3,4-deH-Pip) |
| 1-2937 | 4-F-Ph | 2-MeNH-4-Pym | 1-Et-2-Bu-4-(3,4-deH-Pip) |
| 1-2938 | 4-F-Ph | 2-MeNH-4-Pym | 1-Pr-2-Bu-4-(3,4-deH-Pip) |
| 1-2939 | 4-F-Ph | 2-MeNH-4-Pym | 1,2-diBu-4-(3,4-deH-Pip) |
| 1-2940 | 4-F-Ph | 2-MeNH-4-Pym | 1-Phet-2-Bu-4-(3,4-deH-Pip) |
| 1-2941 | 4-F-Ph | 2-MeNH-4-Pym | 2-Allyl-4-(3,4-deH-Pip) |
| 1-2942 | 4-F-Ph | 2-MeNH-4-Pym | 1-Me-2-Allyl-4-(3,4-deH-Pip) |
| 1-2943 | 4-F-Ph | 2-MeNH-4-Pym | 1-Et-2-Allyl-4-(3,4-deH-Pip) |
| 1-2944 | 4-F-Ph | 2-MeNH-4-Pym | 1-Pr-2-Allyl-4-(3,4-deH-Pip) |
| 1-2945 | 4-F-Ph | 2-MeNH-4-Pym | 1-Bu-2-Allyl-4-(3,4-deH-Pip) |
| 1-2946 | 4-F-Ph | 2-MeNH-4-Pym | 1-Phet-2-Allyl-4-(3,4-deH-Pip) |
| 1-2947 | 4-F-Ph | 2-MeNH-4-Pym | 2-Bn-4-(3,4-deH-Pip) |
| 1-2948 | 4-F-Ph | 2-MeNH-4-Pym | 1-Me-2-Bn-4-(3,4-deH-Pip) |
| 1-2949 | 4-F-Ph | 2-MeNH-4-Pym | 1-Et-2-Bn-4-(3,4-deH-Pip) |
| 1-2950 | 4-F-Ph | 2-MeNH-4-Pym | 1-Pr-2-Bn-4-(3,4-deH-Pip) |
| 1-2951 | 4-F-Ph | 2-MeNH-4-Pym | 1-Bu-2-Bn-4-(3,4-deH-Pip) |
| 1-2952 | 4-F-Ph | 2-MeNH-4-Pym | 1-Phet-2-Bn-4-(3,4-deH-Pip) |
| 1-2953 | 4-F-Ph | 2-MeNH-4-Pym | 2-Phet-4-(3,4-deH-Pip) |
| 1-2954 | 4-F-Ph | 2-MeNH-4-Pym | 1-Me-2-Phet-4-(3,4-deH-Pip) |
| 1-2955 | 4-F-Ph | 2-MeNH-4-Pym | 1-Et-2-Phet-4-(3,4-deH-Pip) |
| 1-2956 | 4-F-Ph | 2-MeNH-4-Pym | 1-Pr-2-Phet-4-(3,4-deH-Pip) |
| 1-2957 | 4-F-Ph | 2-MeNH-4-Pym | 1-Bu-2-Phet-4-(3,4-deH-Pip) |
| 1-2958 | 4-F-Ph | 2-MeNH-4-Pym | 1,2-diPhet-4-(3,4-deH-Pip) |
| 1-2959 | 4-F-Ph | 2-MeNH-4-Pym | 2-Me-4-(4,5-deH-Pip) |
| 1-2960 | 4-F-Ph | 2-MeNH-4-Pym | 1,2-diMe-4-(4,5-deH-Pip) |
| 1-2961 | 4-F-Ph | 2-MeNH-4-Pym | 1-Et-2-Me-4-(4,5-deH-Pip) |

| Compound No. | $R^1$ | $R^2$ | $R^3$ |
|---|---|---|---|
| 1-2962 | 4-F-Ph | 2-MeNH-4-Pym | 1-Pr-2-Me-4-(4,5-deH-Pip) |
| 1-2963 | 4-F-Ph | 2-MeNH-4-Pym | 1-Bu-2-Me-4-(4,5-deH-Pip) |
| 1-2964 | 4-F-Ph | 2-MeNH-4-Pym | 1-Phet-2-Me-4-(4,5-deH-Pip) |
| 1-2965 | 4-F-Ph | 2-MeNH-4-Pym | 2-Et-4-(4,5-deH-Pip) |
| 1-2966 | 4-F-Ph | 2-MeNH-4-Pym | 1-Me-2-Et-4-(4,5-deH-Pip) |
| 1-2967 | 4-F-Ph | 2-MeNH-4-Pym | 1,2-diEt-4-(4,5-deH-Pip) |
| 1-2968 | 4-F-Ph | 2-MeNH-4-Pym | 1-Pr-2-Et-4-(4,5-deH-Pip) |
| 1-2969 | 4-F-Ph | 2-MeNH-4-Pym | 1-Bu-2-Et-4-(4,5-deH-Pip) |
| 1-2970 | 4-F-Ph | 2-MeNH-4-Pym | 1-Phet-2-Et-4-(4,5-deH-Pip) |
| 1-2971 | 4-F-Ph | 2-MeNH-4-Pym | 2-Pr-4-(4,5-deH-Pip) |
| 1-2972 | 4-F-Ph | 2-MeNH-4-Pym | 1-Me-2-Pr-4-(4,5-deH-Pip) |
| 1-2973 | 4-F-Ph | 2-MeNH-4-Pym | 1-Et-2-Pr-4-(4,5-deH-Pip) |
| 1-2974 | 4-F-Ph | 2-MeNH-4-Pym | 1,2-diPr-4-(4,5-deH-Pip) |
| 1-2975 | 4-F-Ph | 2-MeNH-4-Pym | 1-Bu-2-Pr-4-(4,5-deH-Pip) |
| 1-2976 | 4-F-Ph | 2-MeNH-4-Pym | 1-Phet-2-Pr-4-(4,5-deH-Pip) |
| 1-2977 | 4-F-Ph | 2-MeNH-4-Pym | 2-Bu-4-(4,5-deH-Pip) |
| 1-2978 | 4-F-Ph | 2-MeNH-4-Pym | 1-Me-2-Bu-4-(4,5-deH-Pip) |
| 1-2979 | 4-F-Ph | 2-MeNH-4-Pym | 1-Et-2-Bu-4-(4,5-deH-Pip) |
| 1-2980 | 4-F-Ph | 2-MeNH-4-Pym | 1-Pr-2-Bu-4-(4,5-deH-Pip) |
| 1-2981 | 4-F-Ph | 2-MeNH-4-Pym | 1,2-diBu-4-(4,5-deH-Pip) |
| 1-2982 | 4-F-Ph | 2-MeNH-4-Pym | 1-Phet-2-Bu-4-(4,5-deH-Pip) |
| 1-2983 | 4-F-Ph | 2-MeNH-4-Pym | 2-Allyl-4-(4,5-deH-Pip) |
| 1-2984 | 4-F-Ph | 2-MeNH-4-Pym | 1-Me-2-Allyl-4-(4,5-deH-Pip) |
| 1-2985 | 4-F-Ph | 2-MeNH-4-Pym | 1-Et-2-Allyl-4-(4,5-deH-Pip) |
| 1-2986 | 4-F-Ph | 2-MeNH-4-Pym | 1-Pr-2-Allyl-4-(4,5-deH-Pip) |
| 1-2987 | 4-F-Ph | 2-MeNH-4-Pym | 1-Bu-2-Allyl-4-(4,5-deH-Pip) |
| 1-2988 | 4-F-Ph | 2-MeNH-4-Pym | 1-Phet-2-Allyl-4-(4,5-deH-Pip) |
| 1-2989 | 4-F-Ph | 2-MeNH-4-Pym | 2-Bn-4-(4,5-deH-Pip) |
| 1-2990 | 4-F-Ph | 2-MeNH-4-Pym | 1-Me-2-Bn-4-(4,5-deH-Pip) |
| 1-2991 | 4-F-Ph | 2-MeNH-4-Pym | 1-Et-2-Bn-4-(4,5-deH-Pip) |

| Compound No. | $R^1$ | $R^2$ | $R^3$ |
|---|---|---|---|
| 1-2992 | 4-F-Ph | 2-MeNH-4-Pym | 1-Pr-2-Bn-4-(4,5-deH-Pip) |
| 1-2993 | 4-F-Ph | 2-MeNH-4-Pym | 1-Bu-2-Bn-4-(4,5-deH-Pip) |
| 1-2994 | 4-F-Ph | 2-MeNH-4-Pym | 1-Phet-2-Bn-4-(4,5-deH-Pip) |
| 1-2995 | 4-F-Ph | 2-MeNH-4-Pym | 2-Phet-4-(4,5-deH-Pip) |
| 1-2996 | 4-F-Ph | 2-MeNH-4-Pym | 1-Me-2-Phet-4-(4,5-deH-Pip) |
| 1-2997 | 4-F-Ph | 2-MeNH-4-Pym | 1-Et-2-Phet-4-(4,5-deH-Pip) |
| 1-2998 | 4-F-Ph | 2-MeNH-4-Pym | 1-Pr-2-Phet-4-(4,5-deH-Pip) |
| 1-2999 | 4-F-Ph | 2-MeNH-4-Pym | 1-Bu-2-Phet-4-(4,5-deH-Pip) |
| 1-3000 | 4-F-Ph | 2-MeNH-4-Pym | 1,2-diPhet-4-(4,5-deH-Pip) |
| 1-3005 | 4-F-Ph | 2-$NH_2$-4-Pyr | 3-(3,4-deH-Pip) |
| 1-3006 | 4-F-Ph | 2-$NH_2$-4-Pyr | 1-Me-3-(3,4-deH-Pip) |
| 1-3007 | 4-F-Ph | 2-$NH_2$-4-Pyr | 1-Et-4-(3,4-deH-Pip) |
| 1-3008 | 4-F-Ph | 2-$NH_2$-4-Pyr | 1-Pr-4-(3,4-deH-Pip) |
| 1-3010 | 4-F-Ph | 2-$NH_2$-4-Pyr | 1-iPr-4-(3,4-deH-Pip) |
| 1-3012 | 4-F-Ph | 2-$NH_2$-4-Pyr | 1-Bu-4-(3,4-deH-Pip) |
| 1-3013 | 4-F-Ph | 2-$NH_2$-4-Pyr | 1-tBu-4-(3,4-deH-Pip) |
| 1-3014 | 4-F-Ph | 2-$NH_2$-4-Pyr | 1-Pn-4-(3,4-deH-Pip) |
| 1-3015 | 4-F-Ph | 2-$NH_2$-4-Pyr | 1-Hx-4-(3,4-deH-Pip) |
| 1-3016 | 4-F-Ph | 2-$NH_2$-4-Pyr | 1-Hp-4-(3,4-deH-Pip) |
| 1-3017 | 4-F-Ph | 2-$NH_2$-4-Pyr | 1-Oc-4-(3,4-deH-Pip) |
| 1-3018 | 4-F-Ph | 2-$NH_2$-4-Pyr | 1-Nn-4-(3,4-deH-Pip) |
| 1-3019 | 4-F-Ph | 2-$NH_2$-4-Pyr | 1-cPr-4-(3,4-deH-Pip) |
| 1-3020 | 4-F-Ph | 2-$NH_2$-4-Pyr | 1-cPn-4-(3,4-deH-Pip) |

| Compound No. | $R^1$ | $R^2$ | $R^3$ |
|---|---|---|---|
| 1-3021 | 4-F-Ph | 2-$NH_2$-4-Pyr | 1-cHx-4-(3,4-deH-Pip) |
| 1-3022 | 4-F-Ph | 2-$NH_2$-4-Pyr | 1-Bn-4-(3,4-deH-Pip) |
| 1-3023 | 4-F-Ph | 2-$NH_2$-4-Pyr | 1-Phet-4-(3,4-deH-Pip) |
| 1-3024 | 4-F-Ph | 2-$NH_2$-4-Pyr | 1-(3-Ph-Pr)-4-(3,4-deH-Pip) |
| 1-3025 | 4-F-Ph | 2-$NH_2$-4-Pyr | 1-(4-Ph-Bu)-4-(3,4-deH-Pip) |
| 1-3026 | 4-F-Ph | 2-$NH_2$-4-Pyr | 1-Allyl-4-(3,4-deH-Pip) |
| 1-3027 | 4-F-Ph | 2-$NH_2$-4-Pyr | 1-Propargyl-4-(3,4-deH-Pip) |
| 1-3028 | 4-F-Ph | 2-$NH_2$-4-Pyr | 2,2,6,6-tetraMe-4-(3,4-deH-Pip) |
| 1-3029 | 4-F-Ph | 2-$NH_2$-4-Pyr | 1,2,2,6,6-pentaMe-4-(3,4-deH-Pip) |
| 1-3031 | 4-F-Ph | 2-$NH_2$-4-Pyr | 7-(1,2,3,5,6,8a-hexaH-Ind) |
| 1-3032 | 4-F-Ph | 2-$NH_2$-4-Pyr | 7-(1,2,3,5,8,8a-hexaH-Ind) |
| 1-3034 | 4-F-Ph | 2-$NH_2$-4-Pyr | 8-(1,3,4,6,7,9a-hexaH-2H-Qui) |
| 1-3035 | 4-F-Ph | 2-$NH_2$-4-Pyr | 8-(1,3,4,6,9,9a-hexaH-2H-Qui) |
| 1-3037 | 4-F-Ph | 2-$NH_2$-4-Pyr | 2,2-diMe-4-(3,4-deH-Pip) |
| 1-3038 | 4-F-Ph | 2-$NH_2$-4-Pyr | 1,2,2-triMe-4-(3,4-deH-Pip) |
| 1-3039 | 4-F-Ph | 2-$NH_2$-4-Pyr | 2,2-diMe-4-(4,5-deH-Pip) |
| 1-3040 | 4-F-Ph | 2-$NH_2$-4-Pyr | 1,2,2-triMe-4-(4,5-deH-Pip) |
| 1-3041 | 4-F-Ph | 2-$NH_2$-4-Pyr | 2,6-diMe-4-(3,4-deH-Pip) |
| 1-3042 | 4-F-Ph | 2-$NH_2$-4-Pyr | 1,2,6-triMe-4-(3,4-deH-Pip) |
| 1-3043 | 4-F-Ph | 2-$NH_2$-4-Pyr | 2-Me-4-(3,4-deH-Pip) |
| 1-3044 | 4-F-Ph | 2-$NH_2$-4-Pyr | 1,2-diMe-4-(3,4-deH-Pip) |
| 1-3045 | 4-F-Ph | 2-$NH_2$-4-Pyr | 1-Et-2-Me-4-(3,4-deH-Pip) |
| 1-3046 | 4-F-Ph | 2-$NH_2$-4-Pyr | 1-Pr-2-Me-4-(3,4-deH-Pip) |
| 1-3047 | 4-F-Ph | 2-$NH_2$-4-Pyr | 1-Bu-2-Me-4-(3,4-deH-Pip) |
| 1-3048 | 4-F-Ph | 2-$NH_2$-4-Pyr | 1-Phet-2-Me-4-(3,4-deH-Pip) |

| Compound No. | $R^1$ | $R^2$ | $R^3$ |
|---|---|---|---|
| 1-3049 | 4-F-Ph | 2-$NH_2$-4-Pyr | 2-Et-4-(3,4-deH-Pip) |
| 1-3050 | 4-F-Ph | 2-$NH_2$-4-Pyr | 1-Me-2-Et-4-(3,4-deH-Pip) |
| 1-3051 | 4-F-Ph | 2-$NH_2$-4-Pyr | 1,2-diEt-4-(3,4-deH-Pip) |
| 1-3052 | 4-F-Ph | 2-$NH_2$-4-Pyr | 1-Pr-2-Et-4-(3,4-deH-Pip) |
| 1-3053 | 4-F-Ph | 2-$NH_2$-4-Pyr | 1-Bu-2-Et-4-(3,4-deH-Pip) |
| 1-3054 | 4-F-Ph | 2-$NH_2$-4-Pyr | 1-Phet-2-Et-4-(3,4-deH-Pip) |
| 1-3055 | 4-F-Ph | 2-$NH_2$-4-Pyr | 2-Pr-4-(3,4-deH-Pip) |
| 1-3056 | 4-F-Ph | 2-$NH_2$-4-Pyr | 1-Me-2-Pr-4-(3,4-deH-Pip) |
| 1-3057 | 4-F-Ph | 2-$NH_2$-4-Pyr | 1-Et-2-Pr-4-(3,4-deH-Pip) |
| 1-3058 | 4-F-Ph | 2-$NH_2$-4-Pyr | 1,2-diPr-4-(3,4-deH-Pip) |
| 1-3059 | 4-F-Ph | 2-$NH_2$-4-Pyr | 1-Bu-2-Pr-4-(3,4-deH-Pip) |
| 1-3060 | 4-F-Ph | 2-$NH_2$-4-Pyr | 1-Phet-2-Pr-4-(3,4-deH-Pip) |
| 1-3061 | 4-F-Ph | 2-$NH_2$-4-Pyr | 2-Bu-4-(3,4-deH-Pip) |
| 1-3062 | 4-F-Ph | 2-$NH_2$-4-Pyr | 1-Me-2-Bu-4-(3,4-deH-Pip) |
| 1-3063 | 4-F-Ph | 2-$NH_2$-4-Pyr | 1-Et-2-Bu-4-(3,4-deH-Pip) |
| 1-3064 | 4-F-Ph | 2-$NH_2$-4-Pyr | 1-Pr-2-Bu-4-(3,4-deH-Pip) |
| 1-3065 | 4-F-Ph | 2-$NH_2$-4-Pyr | 1,2-diBu-4-(3,4-deH-Pip) |
| 1-3066 | 4-F-Ph | 2-$NH_2$-4-Pyr | 1-Phet-2-Bu-4-(3,4-deH-Pip) |
| 1-3067 | 4-F-Ph | 2-$NH_2$-4-Pyr | 2-Allyl-4-(3,4-deH-Pip) |
| 1-3068 | 4-F-Ph | 2-$NH_2$-4-Pyr | 1-Me-2-Allyl-4-(3,4-deH-Pip) |
| 1-3069 | 4-F-Ph | 2-$NH_2$-4-Pyr | 1-Et-2-Allyl-4-(3,4-deH-Pip) |
| 1-3070 | 4-F-Ph | 2-$NH_2$-4-Pyr | 1-Pr-2-Allyl-4-(3,4-deH-Pip) |
| 1-3071 | 4-F-Ph | 2-$NH_2$-4-Pyr | 1-Bu-2-Allyl-4-(3,4-deH-Pip) |
| 1-3072 | 4-F-Ph | 2-$NH_2$-4-Pyr | 1-Phet-2-Allyl-4-(3,4-deH-Pip) |
| 1-3073 | 4-F-Ph | 2-$NH_2$-4-Pyr | 2-Bn-4-(3,4-deH-Pip) |
| 1-3074 | 4-F-Ph | 2-$NH_2$-4-Pyr | 1-Me-2-Bn-4-(3,4-deH-Pip) |
| 1-3075 | 4-F-Ph | 2-$NH_2$-4-Pyr | 1-Et-2-Bn-4-(3,4-deH-Pip) |
| 1-3076 | 4-F-Ph | 2-$NH_2$-4-Pyr | 1-Pr-2-Bn-4-(3,4-deH-Pip) |
| 1-3077 | 4-F-Ph | 2-$NH_2$-4-Pyr | 1-Bu-2-Bn-4-(3,4-deH-Pip) |
| 1-3078 | 4-F-Ph | 2-$NH_2$-4-Pyr | 1-Phet-2-Bn-4-(3,4-deH-Pip) |

| Compound No. | $R^1$ | $R^2$ | $R^3$ |
|---|---|---|---|
| 1-3079 | 4-F-Ph | $2-NH_2-4-Pyr$ | 2-Phet-4-(3,4-deH-Pip) |
| 1-3080 | 4-F-Ph | $2-NH_2-4-Pyr$ | 1-Me-2-Phet-4-(3,4-deH-Pip) |
| 1-3081 | 4-F-Ph | $2-NH_2-4-Pyr$ | 1-Et-2-Phet-4-(3,4-deH-Pip) |
| 1-3082 | 4-F-Ph | $2-NH_2-4-Pyr$ | 1-Pr-2-Phet-4-(3,4-deH-Pip) |
| 1-3083 | 4-F-Ph | $2-NH_2-4-Pyr$ | 1-Bu-2-Phet-4-(3,4-deH-Pip) |
| 1-3084 | 4-F-Ph | $2-NH_2-4-Pyr$ | 1,2-diPhet-4-(3,4-deH-Pip) |
| 1-3085 | 4-F-Ph | $2-NH_2-4-Pyr$ | 2-Me-4-(4,5-deH-Pip) |
| 1-3086 | 4-F-Ph | $2-NH_2-4-Pyr$ | 1,2-diMe-4-(4,5-deH-Pip) |
| 1-3087 | 4-F-Ph | $2-NH_2-4-Pyr$ | 1-Et-2-Me-4-(4,5-deH-Pip) |
| 1-3088 | 4-F-Ph | $2-NH_2-4-Pyr$ | 1-Pr-2-Me-4-(4,5-deH-Pip) |
| 1-3089 | 4-F-Ph | $2-NH_2-4-Pyr$ | 1-Bu-2-Me-4-(4,5-deH-Pip) |
| 1-3090 | 4-F-Ph | $2-NH_2-4-Pyr$ | 1-Phet-2-Me-4-(4,5-deH-Pip) |
| 1-3091 | 4-F-Ph | $2-NH_2-4-Pyr$ | 2-Et-4-(4,5-deH-Pip) |
| 1-3092 | 4-F-Ph | $2-NH_2-4-Pyr$ | 1-Me-2-Et-4-(4,5-deH-Pip) |
| 1-3093 | 4-F-Ph | $2-NH_2-4-Pyr$ | 1,2-diEt-4-(4,5-deH-Pip) |
| 1-3094 | 4-F-Ph | $2-NH_2-4-Pyr$ | 1-Pr-2-Et-4-(4,5-deH-Pip) |
| 1-3095 | 4-F-Ph | $2-NH_2-4-Pyr$ | 1-Bu-2-Et-4-(4,5-deH-Pip) |
| 1-3096 | 4-F-Ph | $2-NH_2-4-Pyr$ | 1-Phet-2-Et-4-(4,5-deH-Pip) |
| 1-3097 | 4-F-Ph | $2-NH_2-4-Pyr$ | 2-Pr-4-(4,5-deH-Pip) |
| 1-3098 | 4-F-Ph | $2-NH_2-4-Pyr$ | 1-Me-2-Pr-4-(4,5-deH-Pip) |
| 1-3099 | 4-F-Ph | $2-NH_2-4-Pyr$ | 1-Et-2-Pr-4-(4,5-deH-Pip) |
| 1-3100 | 4-F-Ph | $2-NH_2-4-Pyr$ | 1,2-diPr-4-(4,5-deH-Pip) |
| 1-3101 | 4-F-Ph | $2-NH_2-4-Pyr$ | 1-Bu-2-Pr-4-(4,5-deH-Pip) |
| 1-3102 | 4-F-Ph | $2-NH_2-4-Pyr$ | 1-Phet-2-Pr-4-(4,5-deH-Pip) |
| 1-3103 | 4-F-Ph | $2-NH_2-4-Pyr$ | 2-Bu-4-(4,5-deH-Pip) |
| 1-3104 | 4-F-Ph | $2-NH_2-4-Pyr$ | 1-Me-2-Bu-4-(4,5-deH-Pip) |
| 1-3105 | 4-F-Ph | $2-NH_2-4-Pyr$ | 1-Et-2-Bu-4-(4,5-deH-Pip) |
| 1-3106 | 4-F-Ph | $2-NH_2-4-Pyr$ | 1-Pr-2-Bu-4-(4,5-deH-Pip) |
| 1-3107 | 4-F-Ph | $2-NH_2-4-Pyr$ | 1,2-diBu-4-(4,5-deH-Pip) |
| 1-3108 | 4-F-Ph | $2-NH_2-4-Pyr$ | 1-Phet-2-Bu-4-(4,5-deH-Pip) |

| Compound No. | $R^1$ | $R^2$ | $R^3$ |
|---|---|---|---|
| 1-3109 | 4-F-Ph | 2-$NH_2$-4-Pyr | 2-Allyl-4-(4,5-deH-Pip) |
| 1-3110 | 4-F-Ph | 2-$NH_2$-4-Pyr | 1-Me-2-Allyl-4-(4,5-deH-Pip) |
| 1-3111 | 4-F-Ph | 2-$NH_2$-4-Pyr | 1-Et-2-Allyl-4-(4,5-deH-Pip) |
| 1-3112 | 4-F-Ph | 2-$NH_2$-4-Pyr | 1-Pr-2-Allyl-4-(4,5-deH-Pip) |
| 1-3113 | 4-F-Ph | 2-$NH_2$-4-Pyr | 1-Bu-2-Allyl-4-(4,5-deH-Pip) |
| 1-3114 | 4-F-Ph | 2-$NH_2$-4-Pyr | 1-Phet-2-Allyl-4-(4,5-deH-Pip) |
| 1-3115 | 4-F-Ph | 2-$NH_2$-4-Pyr | 2-Bn-4-(4,5-deH-Pip) |
| 1-3116 | 4-F-Ph | 2-$NH_2$-4-Pyr | 1-Me-2-Bn-4-(4,5-deH-Pip) |
| 1-3117 | 4-F-Ph | 2-$NH_2$-4-Pyr | 1-Et-2-Bn-4-(4,5-deH-Pip) |
| 1-3118 | 4-F-Ph | 2-$NH_2$-4-Pyr | 1-Pr-2-Bn-4-(4,5-deH-Pip) |
| 1-3119 | 4-F-Ph | 2-$NH_2$-4-Pyr | 1-Bu-2-Bn-4-(4,5-deH-Pip) |
| 1-3120 | 4-F-Ph | 2-$NH_2$-4-Pyr | 1-Phet-2-Bn-4-(4,5-deH-Pip) |
| 1-3121 | 4-F-Ph | 2-$NH_2$-4-Pyr | 2-Phet-4-(4,5-deH-Pip) |
| 1-3122 | 4-F-Ph | 2-$NH_2$-4-Pyr | 1-Me-2-Phet-4-(4,5-deH-Pip) |
| 1-3123 | 4-F-Ph | 2-$NH_2$-4-Pyr | 1-Et-2-Phet-4-(4,5-deH-Pip) |
| 1-3124 | 4-F-Ph | 2-$NH_2$-4-Pyr | 1-Pr-2-Phet-4-(4,5-deH-Pip) |
| 1-3125 | 4-F-Ph | 2-$NH_2$-4-Pyr | 1-Bu-2-Phet-4-(4,5-deH-Pip) |
| 1-3126 | 4-F-Ph | 2-$NH_2$-4-Pyr | 1,2-diPhet-4-(4,5-deH-Pip) |
| 1-3131 | 4-F-Ph | 2-MeNH-4-Pyr | 3-(3,4-deH-Pip) |
| 1-3132 | 4-F-Ph | 2-MeNH-4-Pyr | 1-Me-3-(3,4-deH-Pip) |
| 1-3133 | 4-F-Ph | 2-MeNH-4-Pyr | 1-Et-4-(3,4-deH-Pip) |
| 1-3134 | 4-F-Ph | 2-MeNH-4-Pyr | 1-Pr-4-(3,4-deH-Pip) |
| 1-3136 | 4-F-Ph | 2-MeNH-4-Pyr | 1-iPr-4-(3,4-deH-Pip) |
| 1-3138 | 4-F-Ph | 2-MeNH-4-Pyr | 1-Bu-4-(3,4-deH-Pip) |

| Compound No. | $R^1$ | $R^2$ | $R^3$ |
|---|---|---|---|
| 1-3139 | 4-F-Ph | 2-MeNH-4-Pyr | 1-tBu-4-(3,4-deH-Pip) |
| 1-3140 | 4-F-Ph | 2-MeNH-4-Pyr | 1-Pn-4-(3,4-deH-Pip) |
| 1-3141 | 4-F-Ph | 2-MeNH-4-Pyr | 1-Hx-4-(3,4-deH-Pip) |
| 1-3142 | 4-F-Ph | 2-MeNH-4-Pyr | 1-Hp-4-(3,4-deH-Pip) |
| 1-3143 | 4-F-Ph | 2-MeNH-4-Pyr | 1-Oc-4-(3,4-deH-Pip) |
| 1-3144 | 4-F-Ph | 2-MeNH-4-Pyr | 1-Nn-4-(3,4-deH-Pip) |
| 1-3145 | 4-F-Ph | 2-MeNH-4-Pyr | 1-cPr-4-(3,4-deH-Pip) |
| 1-3146 | 4-F-Ph | 2-MeNH-4-Pyr | 1-cPn-4-(3,4-deH-Pip) |
| 1-3147 | 4-F-Ph | 2-MeNH-4-Pyr | 1-cHx-4-(3,4-deH-Pip) |
| 1-3148 | 4-F-Ph | 2-MeNH-4-Pyr | 1-Bn-4-(3,4-deH-Pip) |
| 1-3149 | 4-F-Ph | 2-MeNH-4-Pyr | 1-Phet-4-(3,4-deH-Pip) |
| 1-3150 | 4-F-Ph | 2-MeNH-4-Pyr | 1-(3-Ph-Pr)-4-(3,4-deH-Pip) |
| 1-3151 | 4-F-Ph | 2-MeNH-4-Pyr | 1-(4-Ph-Bu)-4-(3,4-deH-Pip) |
| 1-3152 | 4-F-Ph | 2-MeNH-4-Pyr | 1-Allyl-4-(3,4-deH-Pip) |
| 1-3153 | 4-F-Ph | 2-MeNH-4-Pyr | 1-Propargyl-4-(3,4-deH-Pip) |
| 1-3154 | 4-F-Ph | 2-MeNH-4-Pyr | 2,2,6,6-tetraMe-4-(3,4-deH-Pip) |
| 1-3155 | 4-F-Ph | 2-MeNH-4-Pyr | 1,2,2,6,6-pentaMe-4-(3,4-deH-Pip) |
| 1-3157 | 4-F-Ph | 2-MeNH-4-Pyr | 7-(1,2,3,5,6,8a-hexaH-Ind) |
| 1-3158 | 4-F-Ph | 2-MeNH-4-Pyr | 7-(1,2,3,5,8,8a-hexaH-Ind) |
| 1-3160 | 4-F-Ph | 2-MeNH-4-Pyr | 8-(1,3,4,6,7,9a-hexaH-2H-Qui) |
| 1-3161 | 4-F-Ph | 2-MeNH-4-Pyr | 8-(1,3,4,6,9,9a-hexaH-2H-Qui) |
| 1-3163 | 4-F-Ph | 2-MeNH-4-Pyr | 2,2-diMe-4-(3,4-deH-Pip) |
| 1-3164 | 4-F-Ph | 2-MeNH-4-Pyr | 1,2,2-triMe-4-(3,4-deH-Pip) |
| 1-3165 | 4-F-Ph | 2-MeNH-4-Pyr | 2,2-diMe-4-(4,5-deH-Pip) |
| 1-3166 | 4-F-Ph | 2-MeNH-4-Pyr | 1,2,2-triMe-4-(4,5-deH-Pip) |

| Compound No. | $R^1$ | $R^2$ | $R^3$ |
|---|---|---|---|
| 1-3167 | 4-F-Ph | 2-MeNH-4-Pyr | 2,6-diMe-4-(3,4-deH-Pip) |
| 1-3168 | 4-F-Ph | 2-MeNH-4-Pyr | 1,2,6-triMe-4-(3,4-deH-Pip) |
| 1-3169 | 4-F-Ph | 2-MeNH-4-Pyr | 2-Me-4-(3,4-deH-Pip) |
| 1-3170 | 4-F-Ph | 2-MeNH-4-Pyr | 1,2-diMe-4-(3,4-deH-Pip) |
| 1-3171 | 4-F-Ph | 2-MeNH-4-Pyr | 1-Et-2-Me-4-(3,4-deH-Pip) |
| 1-3172 | 4-F-Ph | 2-MeNH-4-Pyr | 1-Pr-2-Me-4-(3,4-deH-Pip) |
| 1-3173 | 4-F-Ph | 2-MeNH-4-Pyr | 1-Bu-2-Me-4-(3,4-deH-Pip) |
| 1-3174 | 4-F-Ph | 2-MeNH-4-Pyr | 1-Phet-2-Me-4-(3,4-deH-Pip) |
| 1-3175 | 4-F-Ph | 2-MeNH-4-Pyr | 2-Et-4-(3,4-deH-Pip) |
| 1-3176 | 4-F-Ph | 2-MeNH-4-Pyr | 1-Me-2-Et-4-(3,4-deH-Pip) |
| 1-3177 | 4-F-Ph | 2-MeNH-4-Pyr | 1,2-diEt-4-(3,4-deH-Pip) |
| 1-3178 | 4-F-Ph | 2-MeNH-4-Pyr | 1-Pr-2-Et-4-(3,4-deH-Pip) |
| 1-3179 | 4-F-Ph | 2-MeNH-4-Pyr | 1-Bu-2-Et-4-(3,4-deH-Pip) |
| 1-3180 | 4-F-Ph | 2-MeNH-4-Pyr | 1-Phet-2-Et-4-(3,4-deH-Pip) |
| 1-3181 | 4-F-Ph | 2-MeNH-4-Pyr | 2-Pr-4-(3,4-deH-Pip) |
| 1-3182 | 4-F-Ph | 2-MeNH-4-Pyr | 1-Me-2-Pr-4-(3,4-deH-Pip) |
| 1-3183 | 4-F-Ph | 2-MeNH-4-Pyr | 1-Et-2-Pr-4-(3,4-deH-Pip) |
| 1-3184 | 4-F-Ph | 2-MeNH-4-Pyr | 1,2-diPr-4-(3,4-deH-Pip) |
| 1-3185 | 4-F-Ph | 2-MeNH-4-Pyr | 1-Bu-2-Pr-4-(3,4-deH-Pip) |
| 1-3186 | 4-F-Ph | 2-MeNH-4-Pyr | 1-Phet-2-Pr-4-(3,4-deH-Pip) |
| 1-3187 | 4-F-Ph | 2-MeNH-4-Pyr | 2-Bu-4-(3,4-deH-Pip) |
| 1-3188 | 4-F-Ph | 2-MeNH-4-Pyr | 1-Me-2-Bu-4-(3,4-deH-Pip) |
| 1-3189 | 4-F-Ph | 2-MeNH-4-Pyr | 1-Et-2-Bu-4-(3,4-deH-Pip) |
| 1-3190 | 4-F-Ph | 2-MeNH-4-Pyr | 1-Pr-2-Bu-4-(3,4-deH-Pip) |
| 1-3191 | 4-F-Ph | 2-MeNH-4-Pyr | 1,2-diBu-4-(3,4-deH-Pip) |
| 1-3192 | 4-F-Ph | 2-MeNH-4-Pyr | 1-Phet-2-Bu-4-(3,4-deH-Pip) |
| 1-3193 | 4-F-Ph | 2-MeNH-4-Pyr | 2-Allyl-4-(3,4-deH-Pip) |
| 1-3194 | 4-F-Ph | 2-MeNH-4-Pyr | 1-Me-2-Allyl-4-(3,4-deH-Pip) |
| 1-3195 | 4-F-Ph | 2-MeNH-4-Pyr | 1-Et-2-Allyl-4-(3,4-deH-Pip) |
| 1-3196 | 4-F-Ph | 2-MeNH-4-Pyr | 1-Pr-2-Allyl-4-(3,4-deH-Pip) |

| Compound No. | $R^1$ | $R^2$ | $R^3$ |
|---|---|---|---|
| 1-3197 | 4-F-Ph | 2-MeNH-4-Pyr | 1-Bu-2-Allyl-4-(3,4-deH-Pip) |
| 1-3198 | 4-F-Ph | 2-MeNH-4-Pyr | 1-Phet-2-Allyl-4-(3,4-deH-Pip) |
| 1-3199 | 4-F-Ph | 2-MeNH-4-Pyr | 2-Bn-4-(3,4-deH-Pip) |
| 1-3200 | 4-F-Ph | 2-MeNH-4-Pyr | 1-Me-2-Bn-4-(3,4-deH-Pip) |
| 1-3201 | 4-F-Ph | 2-MeNH-4-Pyr | 1-Et-2-Bn-4-(3,4-deH-Pip) |
| 1-3202 | 4-F-Ph | 2-MeNH-4-Pyr | 1-Pr-2-Bn-4-(3,4-deH-Pip) |
| 1-3203 | 4-F-Ph | 2-MeNH-4-Pyr | 1-Bu-2-Bn-4-(3,4-deH-Pip) |
| 1-3204 | 4-F-Ph | 2-MeNH-4-Pyr | 1-Phet-2-Bn-4-(3,4-deH-Pip) |
| 1-3205 | 4-F-Ph | 2-MeNH-4-Pyr | 2-Phet-4-(3,4-deH-Pip) |
| 1-3206 | 4-F-Ph | 2-MeNH-4-Pyr | 1-Me-2-Phet-4-(3,4-deH-Pip) |
| 1-3207 | 4-F-Ph | 2-MeNH-4-Pyr | 1-Et-2-Phet-4-(3,4-deH-Pip) |
| 1-3208 | 4-F-Ph | 2-MeNH-4-Pyr | 1-Pr-2-Phet-4-(3,4-deH-Pip) |
| 1-3209 | 4-F-Ph | 2-MeNH-4-Pyr | 1-Bu-2-Phet-4-(3,4-deH-Pip) |
| 1-3210 | 4-F-Ph | 2-MeNH-4-Pyr | 1,2-diPhet-4-(3,4-deH-Pip) |
| 1-3211 | 4-F-Ph | 2-MeNH-4-Pyr | 2-Me-4-(4,5-deH-Pip) |
| 1-3212 | 4-F-Ph | 2-MeNH-4-Pyr | 1,2-diMe-4-(4,5-deH-Pip) |
| 1-3213 | 4-F-Ph | 2-MeNH-4-Pyr | 1-Et-2-Me-4-(4,5-deH-Pip) |
| 1-3214 | 4-F-Ph | 2-MeNH-4-Pyr | 1-Pr-2-Me-4-(4,5-deH-Pip) |
| 1-3215 | 4-F-Ph | 2-MeNH-4-Pyr | 1-Bu-2-Me-4-(4,5-deH-Pip) |
| 1-3216 | 4-F-Ph | 2-MeNH-4-Pyr | 1-Phet-2-Me-4-(4,5-deH-Pip) |
| 1-3217 | 4-F-Ph | 2-MeNH-4-Pyr | 2-Et-4-(4,5-deH-Pip) |
| 1-3218 | 4-F-Ph | 2-MeNH-4-Pyr | 1-Me-2-Et-4-(4,5-deH-Pip) |
| 1-3219 | 4-F-Ph | 2-MeNH-4-Pyr | 1,2-diEt-4-(4,5-deH-Pip) |
| 1-3220 | 4-F-Ph | 2-MeNH-4-Pyr | 1-Pr-2-Et-4-(4,5-deH-Pip) |
| 1-3221 | 4-F-Ph | 2-MeNH-4-Pyr | 1-Bu-2-Et-4-(4,5-deH-Pip) |
| 1-3222 | 4-F-Ph | 2-MeNH-4-Pyr | 1-Phet-2-Et-4-(4,5-deH-Pip) |
| 1-3223 | 4-F-Ph | 2-MeNH-4-Pyr | 2-Pr-4-(4,5-deH-Pip) |
| 1-3224 | 4-F-Ph | 2-MeNH-4-Pyr | 1-Me-2-Pr-4-(4,5-deH-Pip) |
| 1-3225 | 4-F-Ph | 2-MeNH-4-Pyr | 1-Et-2-Pr-4-(4,5-deH-Pip) |
| 1-3226 | 4-F-Ph | 2-MeNH-4-Pyr | 1,2-diPr-4-(4,5-deH-Pip) |

| Compound No. | $R^1$ | $R^2$ | $R^3$ |
|---|---|---|---|
| 1-3227 | 4-F-Ph | 2-MeNH-4-Pyr | 1-Bu-2-Pr-4-(4,5-deH-Pip) |
| 1-3228 | 4-F-Ph | 2-MeNH-4-Pyr | 1-Phet-2-Pr-4-(4,5-deH-Pip) |
| 1-3229 | 4-F-Ph | 2-MeNH-4-Pyr | 2-Bu-4-(4,5-deH-Pip) |
| 1-3230 | 4-F-Ph | 2-MeNH-4-Pyr | 1-Me-2-Bu-4-(4,5-deH-Pip) |
| 1-3231 | 4-F-Ph | 2-MeNH-4-Pyr | 1-Et-2-Bu-4-(4,5-deH-Pip) |
| 1-3232 | 4-F-Ph | 2-MeNH-4-Pyr | 1-Pr-2-Bu-4-(4,5-deH-Pip) |
| 1-3233 | 4-F-Ph | 2-MeNH-4-Pyr | 1,2-diBu-4-(4,5-deH-Pip) |
| 1-3234 | 4-F-Ph | 2-MeNH-4-Pyr | 1-Phet-2-Bu-4-(4,5-deH-Pip) |
| 1-3235 | 4-F-Ph | 2-MeNH-4-Pyr | 2-Allyl-4-(4,5-deH-Pip) |
| 1-3236 | 4-F-Ph | 2-MeNH-4-Pyr | 1-Me-Allyl-4-(4,5-deH-Pip) |
| 1-3237 | 4-F-Ph | 2-MeNH-4-Pyr | 1-Et-2-Allyl-4-(4,5-deH-Pip) |
| 1-3238 | 4-F-Ph | 2-MeNH-4-Pyr | 1-Pr-2-Allyl-4-(4,5-deH-Pip) |
| 1-3239 | 4-F-Ph | 2-MeNH-4-Pyr | 1-Bu-2-Allyl-4-(4,5-deH-Pip) |
| 1-3240 | 4-F-Ph | 2-MeNH-4-Pyr | 1-Phet-2-Allyl-4-(4,5-deH-Pip) |
| 1-3241 | 4-F-Ph | 2-MeNH-4-Pyr | 2-Bn-4-(4,5-deH-Pip) |
| 1-3242 | 4-F-Ph | 2-MeNH-4-Pyr | 1-Me-2-Bn-4-(4,5-deH-Pip) |
| 1-3243 | 4-F-Ph | 2-MeNH-4-Pyr | 1-Et-2-Bn-4-(4,5-deH-Pip) |
| 1-3244 | 4-F-Ph | 2-MeNH-4-Pyr | 1-Pr-2-Bn-4-(4,5-deH-Pip) |
| 1-3245 | 4-F-Ph | 2-MeNH-4-Pyr | 1-Bu-2-Bn-4-(4,5-deH-Pip) |
| 1-3246 | 4-F-Ph | 2-MeNH-4-Pyr | 1-Phet-2-Bn-4-(4,5-deH-Pip) |
| 1-3247 | 4-F-Ph | 2-MeNH-4-Pyr | 2-Phet-4-(4,5-deH-Pip) |
| 1-3248 | 4-F-Ph | 2-MeNH-4-Pyr | 1-Me-2-Phet-4-(4,5-deH-Pip) |
| 1-3249 | 4-F-Ph | 2-MeNH-4-Pyr | 1-Et-2-Phet-4-(4,5-deH-Pip) |
| 1-3250 | 4-F-Ph | 2-MeNH-4-Pyr | 1-Pr-2-Phet-4-(4,5-deH-Pip) |
| 1-3251 | 4-F-Ph | 2-MeNH-4-Pyr | 1-Bu-2-Phet-4-(4,5-deH-Pip) |
| 1-3252 | 4-F-Ph | 2-MeNH-4-Pyr | 1,2-diPhet-4-(4,5-deH-Pip) |
| 1-3253 | Ph | 4-Pyr | 7-(1,2,3,5,6,8a-hexaH-Ind) |
| 1-3254 | Ph | 4-Pyr | 7-(1,2,3,5,8,8a-hexaH-Ind) |
| 1-3255 | 3-F-Ph | 4-Pyr | 7-(1,2,3,5,6,8a-hexaH-Ind) |
| 1-3256 | 3-F-Ph | 4-Pyr | 7-(1,2,3,5,8,8a-hexaH-Ind) |

| Compound No. | $R^1$ | $R^2$ | $R^3$ |
|---|---|---|---|
| 1-3257 | 3-Cl-Ph | 4-Pyr | 7-(1,2,3,5,6,8a-hexaH-Ind) |
| 1-3258 | 3-Cl-Ph | 4-Pyr | 7-(1,2,3,5,8,8a-hexaH-Ind) |
| 1-3259 | 3,4-diF-Ph | 4-Pyr | 7-(1,2,3,5,6,8a-hexaH-Ind) |
| 1-3260 | 3,4-diF-Ph | 4-Pyr | 7-(1,2,3,5,8,8a-hexaH-Ind) |
| 1-3261 | 3-$CF_3$-Ph | 4-Pyr | 7-(1,2,3,5,6,8a-hexaH-Ind) |
| 1-3262 | 3-$CF_3$-Ph | 4-Pyr | 7-(1,2,3,5,8,8a-hexaH-Ind) |

| Compound No. | $R^1$ | $R^2$ | $R^3$ |
|---|---|---|---|
| 1-3312 | 4-F-Ph | 4-Pyr | 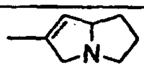 |
| 1-3313 | 4-F-Ph | 4-Pyr | 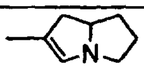 |

| Compound No. | $R^1$ | $R^2$ | $R^3$ |
|---|---|---|---|
| 1-3315 | 4-F-Ph | 4-Pyr | 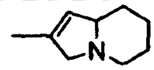 |
| 1-3316 | 4-F-Ph | 4-Pyr | 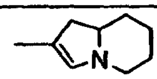 |
| 1-3318 | 4-F-Ph | 4-Pyr | 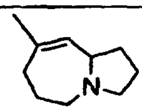 |
| 1-3319 | 4-F-Ph | 4-Pyr | 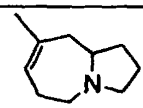 |
| 1-3321 | 4-F-Ph | 4-Pyr | 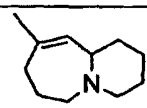 |
| 1-3322 | 4-F-Ph | 4-Pyr | 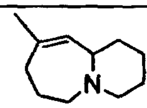 |

**Table 2**

| Compound No. | $R^1$ | $R^2$ | $R^3$ |
|---|---|---|---|
| 2-49 | Ph | 4-Pyr | 4-(3,4-deH-Pip) |
| 2-51 | Ph | 4-Pyr | 1-Me-4-(3,4-deH-Pip) |

| Compound No. | $R^1$ | $R^2$ | $R^3$ |
|---|---|---|---|
| 2-143 | 4-F-Ph | 4-Pyr | 4-(3,4-deH-Pip) |
| 2-145 | 4-F-Ph | 4-Pyr | 1-Me-4-(3,4-deH-Pip) |

| Compound No. | $R^1$ | $R^2$ | $R^3$ |
|---|---|---|---|
| 2-236 | 3-F-Ph | 4-Pyr | 4-(3,4-deH-Pip) |
| 2-238 | 3-F-Ph | 4-Pyr | 1-Me-4-(3,4-deH-Pip) |

| Compound No. | $R^1$ | $R^2$ | $R^3$ |
|---|---|---|---|
| 2-329 | 3,4-diF-Ph | 4-Pyr | 4-(3,4-deH-Pip) |
| 2-331 | 3,4-diF-Ph | 4-Pyr | 1-Me-4-(3,4-deH-Pip) |

| Compound No. | $R^1$ | $R^2$ | $R^3$ |
|---|---|---|---|
| 2-422 | 3-Cl-Ph | 4-Pyr | 4-(3,4-deH-Pip) |
| 2-424 | 3-Cl-Ph | 4-Pyr | 1-Me-4-(3,4-deH-Pip) |

| Compound No. | $R^1$ | $R^2$ | $R^3$ |
|---|---|---|---|
| 2-515 | Ph | 2-$NH_2$-4-Pym | 4-(3,4-deH-Pip) |
| 2-517 | Ph | 2-$NH_2$-4-Pym | 1-Me-4-(3,4-deH-Pip) |

| Compound No. | $R^1$ | $R^2$ | $R^3$ |
|---|---|---|---|
| 2-608 | 4-F-Ph | $2-NH_2-4-Pym$ | 4-(3,4-deH-Pip) |
| 2-610 | 4-F-Ph | $2-NH_2-4-Pym$ | 1-Me-4-(3,4-deH-Pip) |

| Compound No. | $R^1$ | $R^2$ | $R^3$ |
|---|---|---|---|
| 2-701 | 3-F-Ph | $2-NH_2-4-Pym$ | 4-(3,4-deH-Pip) |
| 2-703 | 3-F-Ph | $2-NH_2-4-Pym$ | 1-Me-4-(3,4-deH-Pip) |

| Compound No. | $R^1$ | $R^2$ | $R^3$ |
|---|---|---|---|
| 2-794 | 3,4-diF-Ph | $2-NH_2-4-Pym$ | 4-(3,4-deH-Pip) |
| 2-796 | 3,4-diF-Ph | $2-NH_2-4-Pym$ | 1-Me-4-(3,4-deH-Pip) |

| Compound No. | $R^1$ | $R^2$ | $R^3$ |
|---|---|---|---|
| 2-887 | 3-Cl-Ph | $2-NH_2-4-Pym$ | 4-(3,4-deH-Pip) |
| 2-889 | 3-Cl-Ph | $2-NH_2-4-Pym$ | 1-Me-4-(3,4-deH-Pip) |

| Compound No. | $R^1$ | $R^2$ | $R^3$ |
|---|---|---|---|
| 2-980 | Ph | 2-MeNH-4-Pym | 4-(3,4-deH-Pip) |
| 2-982 | Ph | 2-MeNH-4-Pym | 1-Me-4-(3,4-deH-Pip) |

| Compound No. | $R^1$ | $R^2$ | $R^3$ |
|---|---|---|---|
| 2-1073 | 4-F-Ph | 2-MeNH-4-Pym | 4-(3,4-deH-Pip) |
| 2-1075 | 4-F-Ph | 2-MeNH-4-Pym | 1-Me-4-(3,4-deH-Pip) |

| Compound No. | $R^1$ | $R^2$ | $R^3$ |
|---|---|---|---|
| 2-1166 | 3-F-Ph | 2-MeNH-4-Pym | 4-(3,4-deH-Pip) |
| 2-1168 | 3-F-Ph | 2-MeNH-4-Pym | 1-Me-4-(3,4-deH-Pip) |

| Compound No. | $R^1$ | $R^2$ | $R^3$ |
|---|---|---|---|
| 2-1259 | 3,4-diF-Ph | 2-MeNH-4-Pym | 4-(3,4-deH-Pip) |
| 2-1261 | 3,4-diF-Ph | 2-MeNH-4-Pym | 1-Me-4-(3,4-deH-Pip) |

| Compound No. | $R^1$ | $R^2$ | $R^3$ |
|---|---|---|---|
| 2-1352 | 3-Cl-Ph | 2-MeNH-4-Pym | 4-(3,4-deH-Pip) |
| 2-1354 | 3-Cl-Ph | 2-MeNH-4-Pym | 1-Me-4-(3,4-deH-Pip) |

| Compound No. | $R^1$ | $R^2$ | $R^3$ |
|---|---|---|---|
| 2-1413 | 3-Cl-4-F-Ph | 4-Pyr | 4-(3,4-deH-Pip) |
| 2-1415 | 3-Cl-4-F-Ph | 4-Pyr | 1-Me-4-(3,4-deH-Pip) |

| Compound No. | $R^1$ | $R^2$ | $R^3$ |
|---|---|---|---|
| 2-1448 | 3,4,5-triF-Ph | 4-Pyr | 4-(3,4-deH-Pip) |
| 2-1450 | 3,4,5-triF-Ph | 4-Pyr | 1-Me-4-(3,4-deH-Pip) |

| Compound No. | $R^1$ | $R^2$ | $R^3$ |
|---|---|---|---|
| 2-1483 | 3-$CF_3$-Ph | 4-Pyr | 4-(3,4-deH-Pip) |
| 2-1485 | 3-$CF_3$-Ph | 4-Pyr | 1-Me-4-(3,4-deH-Pip) |

| Compound No. | $R^1$ | $R^2$ | $R^3$ |
|---|---|---|---|
| 2-1518 | 3-$CHF_2O$-Ph | 4-Pyr | 4-(3,4-deH-Pip) |
| 2-1520 | 3-$CHF_2O$-Ph | 4-Pyr | 1-Me-4-(3,4-deH-Pip) |

| Compound No. | $R^1$ | $R^2$ | $R^3$ |
|---|---|---|---|
| 2-1553 | 3-Cl-4-F-Ph | 2-$NH_2$-4-Pym | 4-(3,4-deH-Pip) |
| 2-1555 | 3-Cl-4-F-Ph | 2-$NH_2$-4-Pym | 1-Me-4-(3,4-deH-Pip) |

| Compound No. | $R^1$ | $R^2$ | $R^3$ |
|---|---|---|---|
| 2-1588 | 3,4,5-triF-Ph | 2-$NH_2$-4-Pym | 4-(3,4-deH-Pip) |

| Compound No. | $R^1$ | $R^2$ | $R^3$ |
|---|---|---|---|
| 2-1590 | 3,4,5-triF-Ph | 2-$NH_2$-4-Pym | 1-Me-4-(3,4-deH-Pip) |

| Compound No. | $R^1$ | $R^2$ | $R^3$ |
|---|---|---|---|
| 2-1623 | 3-$CF_3$-Ph | 2-$NH_2$-4-Pym | 4-(3,4-deH-Pip) |
| 2-1625 | 3-$CF_3$-Ph | 2-$NH_2$-4-Pym | 1-Me-4-(3,4-deH-Pip) |

| Compound No. | $R^1$ | $R^2$ | $R^3$ |
|---|---|---|---|
| 2-1658 | 3-$CHF_2O$-Ph | 2-$NH_2$-4-Pym | 4-(3,4-deH-Pip) |
| 2-1660 | 3-$CHF_2O$-Ph | 2-$NH_2$-4-Pym | 1-Me-4-(3,4-deH-Pip) |

| Compound No. | $R^1$ | $R^2$ | $R^3$ |
|---|---|---|---|
| 2-1693 | 3-Cl-4-F-Ph | 2-MeNH-4-Pym | 4-(3,4-deH-Pip) |
| 2-1695 | 3-Cl-4-F-Ph | 2-MeNH-4-Pym | 1-Me-4-(3,4-deH-Pip) |

| Compound No. | $R^1$ | $R^2$ | $R^3$ |
|---|---|---|---|
| 2-1728 | 3,4,5-triF-Ph | 2-MeNH-4-Pym | 4-(3,4-deH-Pip) |
| 2-1730 | 3,4,5-triF-Ph | 2-MeNH-4-Pym | 1-Me-4-(3,4-deH-Pip) |

| Compound No. | $R^1$ | $R^2$ | $R^3$ |
|---|---|---|---|
| 2-1763 | 3-$CF_3$-Ph | 2-MeNH-4-Pym | 4-(3,4-deH-Pip) |
| 2-1765 | 3-$CF_3$-Ph | 2-MeNH-4-Pym | 1-Me-4-(3,4-deH-Pip) |

| Compound No. | $R^1$ | $R^2$ | $R^3$ |
|---|---|---|---|
| 2-1798 | 3-$CHF_2O$-Ph | 2-MeNH-4-Pym | 4-(3,4-deH-Pip) |

| Compound No. | $R^1$ | $R^2$ | $R^3$ |
|---|---|---|---|
| 2-1800 | 3-$CHF_2O$-Ph | 2-MeNH-4-Pym | 1-Me-4-(3,4-deH-Pip) |

| Compound No. | $R^1$ | $R^2$ | $R^3$ |
|---|---|---|---|
| 2-1833 | Ph | 2-$NH_2$-4-Pyr | 4-(3,4-deH-Pip) |
| 2-1835 | Ph | 2-$NH_2$-4-Pyr | 1-Me-4-(3,4-deH-Pip) |

| Compound No. | $R^1$ | $R^2$ | $R^3$ |
|---|---|---|---|
| 2-1868 | 4-F-Ph | 2-$NH_2$-4-Pyr | 4-(3,4-deH-Pip) |
| 2-1870 | 4-F-Ph | 2-$NH_2$-4-Pyr | 1-Me-4-(3,4-deH-Pip) |

| Compound No. | $R^1$ | $R^2$ | $R^3$ |
|---|---|---|---|
| 2-1903 | 3-F-Ph | 2-$NH_2$-4-Pyr | 4-(3,4-deH-Pip) |
| 2-1905 | 3-F-Ph | 2-$NH_2$-4-Pyr | 1-Me-4-(3,4-deH-Pip) |

| Compound No. | $R^1$ | $R^2$ | $R^3$ |
|---|---|---|---|
| 2-1938 | 3,4-diF-Ph | 2-$NH_2$-4-Pyr | 4-(3,4-deH-Pip) |
| 2-1940 | 3,4-diF-Ph | 2-$NH_2$-4-Pyr | 1-Me-4-(3,4-deH-Pip) |

| Compound No. | $R^1$ | $R^2$ | $R^3$ |
|---|---|---|---|
| 2-1973 | 3-Cl-Ph | 2-$NH_2$-4-Pyr | 4-(3,4-deH-Pip) |
| 2-1975 | 3-Cl-Ph | 2-$NH_2$-4-Pyr | 1-Me-4-(3,4-deH-Pip) |

| Compound No. | $R^1$ | $R^2$ | $R^3$ |
|---|---|---|---|
| 2-2008 | Ph | 2-MeNH-4-Pyr | 4-(3,4-deH-Pip) |

| Compound No. | $R^1$ | $R^2$ | $R^3$ |
|---|---|---|---|
| 2-2010 | Ph | 2-MeNH-4-Pyr | 1-Me-4-(3,4-deH-Pip) |

| Compound No. | $R^1$ | $R^2$ | $R^3$ |
|---|---|---|---|
| 2-2043 | 4-F-Ph | 2-MeNH-4-Pyr | 4-(3,4-deH-Pip) |
| 2-2045 | 4-F-Ph | 2-MeNH-4-Pyr | 1-Me-4-(3,4-deH-Pip) |

| Compound No. | $R^1$ | $R^2$ | $R^3$ |
|---|---|---|---|
| 2-2078 | 3-F-Ph | 2-MeNH-4-Pyr | 4-(3,4-deH-Pip) |
| 2-2080 | 3-F-Ph | 2-MeNH-4-Pyr | 1-Me-4-(3,4-deH-Pip) |

| Compound No. | $R^1$ | $R^2$ | $R^3$ |
|---|---|---|---|
| 2-2113 | 3,4-diF-Ph | 2-MeNH-4-Pyr | 4-(3,4-deH-Pip) |
| 2-2115 | 3,4-diF-Ph | 2-MeNH-4-Pyr | 1-Me-4-(3,4-deH-Pip) |

| Compound No. | $R^1$ | $R^2$ | $R^3$ |
|---|---|---|---|
| 2-2148 | 3-Cl-Ph | 2-MeNH-4-Pyr | 4-(3,4-deH-Pip) |
| 2-2150 | 3-Cl-Ph | 2-MeNH-4-Pyr | 1-Me-4-(3,4-deH-Pip) |

| Compound No. | $R^1$ | $R^2$ | $R^3$ |
|---|---|---|---|
| 2-2183 | Ph | 4-Pym | 4-(3,4-deH-Pip) |
| 2-2185 | Ph | 4-Pym | 1-Me-4-(3,4-deH-Pip) |

| Compound No. | $R^1$ | $R^2$ | $R^3$ |
|---|---|---|---|
| 2-2218 | 4-F-Ph | 4-Pym | 4-(3,4-deH-Pip) |

| Compound No. | $R^1$ | $R^2$ | $R^3$ |
|---|---|---|---|
| 2-2220 | 4-F-Ph | 4-Pym | 1-Me-4-(3,4-deH-Pip) |

| Compound No. | $R^1$ | $R^2$ | $R^3$ |
|---|---|---|---|
| 2-2253 | 3-F-Ph | 4-Pym | 4-(3,4-deH-Pip) |
| 2-2255 | 3-F-Ph | 4-Pym | 1-Me-4-(3,4-deH-Pip) |

| Compound No. | $R^1$ | $R^2$ | $R^3$ |
|---|---|---|---|
| 2-2288 | 3,4-diF-Ph | 4-Pym | 4-(3,4-deH-Pip) |
| 2-2290 | 3,4-diF-Ph | 4-Pym | 1-Me-4-(3,4-deH-Pip) |

| Compound No. | $R^1$ | $R^2$ | $R^3$ |
|---|---|---|---|
| 2-2323 | 3-Cl-Ph | 4-Pym | 4-(3,4-deH-Pip) |
| 2-2325 | 3-Cl-Ph | 4-Pym | 1-Me-4-(3,4-deH-Pip) |

| Compound No. | $R^1$ | $R^2$ | $R^3$ |
|---|---|---|---|
| 2-2358 | Ph | 2-MeO-4-Pym | 4-(3,4-deH-Pip) |
| 2-2360 | Ph | 2-MeO-4-Pym | 1-Me-4-(3,4-deH-Pip) |

| Compound No. | $R^1$ | $R^2$ | $R^3$ |
|---|---|---|---|
| 2-2393 | 4-F-Ph | 2-MeO-4-Pym | 4-(3,4-deH-Pip) |
| 2-2395 | 4-F-Ph | 2-MeO-4-Pym | 1-Me-4-(3,4-deH-Pip) |

| Compound No. | $R^1$ | $R^2$ | $R^3$ |
|---|---|---|---|
| 2-2428 | 3-F-Ph | 2-MeO-4-Pym | 4-(3,4-deH-Pip) |

| Compound No. | $R^1$ | $R^2$ | $R^3$ |
|---|---|---|---|
| 2-2430 | 3-F-Ph | 2-MeO-4-Pym | 1-Me-4-(3,4-deH-Pip) |

| Compound No. | $R^1$ | $R^2$ | $R^3$ |
|---|---|---|---|
| 2-2463 | 3,4-diF-Ph | 2-MeO-4-Pym | 4-(3,4-deH-Pip) |
| 2-2465 | 3,4-diF-Ph | 2-MeO-4-Pym | 1-Me-4-(3,4-deH-Pip) |

| Compound No. | $R^1$ | $R^2$ | $R^3$ |
|---|---|---|---|
| 2-2498 | 3-Cl-Ph | 2-MeO-4-Pym | 4-(3,4-deH-Pip) |
| 2-2500 | 3-Cl-Ph | 2-MeO-4-Pym | 1-Me-4-(3,4-deH-Pip) |

| Compound No. | $R^1$ | $R^2$ | $R^3$ |
|---|---|---|---|
| 2-2525 | 4-F-Ph | 4-Pyr | 1-Ac-4-(3,4-deH-Pip) |

| Compound No. | $R^1$ | $R^2$ | $R^3$ |
|---|---|---|---|
| 2-2557 | 4-F-Ph | 2-$NH_2$-4-Pym | 1-Ac-4-(3,4-deH-Pip) |

| Compound No. | $R^1$ | $R^2$ | $R^3$ |
|---|---|---|---|
| 2-2580 | 4-F-Ph | 2-MeNH-4-Pym | 1-Ac-4-(3,4-deH-Pip) |
| 2-2596 | 4-F-Ph | 2-BnNH-4-Pyr | 4-(3,4-deH-Pip) |
| 2-2598 | 4-F-Ph | 2-BnNH-4-Pym | 4-(3,4-deH-Pip) |
| 2-2600 | 4-F-Ph | 2-(α-Me-BnNH)-4-Pyr | 4-(3,4-deH-Pip) |
| 2-2602 | 4-F-Ph | 2-(α-Me-BnNH)-4-Pym | 4-(3,4-deH-Pip) |
| 2-2604 | 3-Cl-Ph | 2-BnNH-4-Pyr | 4-(3,4-deH-Pip) |
| 2-2606 | 3-Cl-Ph | 2-BnNH-4-Pym | 4-(3,4-deH-Pip) |

| Compound No. | $R^1$ | $R^2$ | $R^3$ |
|---|---|---|---|
| 2-2608 | 3-Cl-Ph | 2-(α-Me-BnNH)-4-Pyr | 4-(3,4-deH-Pip) |
| 2-2610 | 3-Cl-Ph | 2-(α-Me-BnNH)-4-Pym | 4-(3,4-deH-Pip) |
| 2-2612 | 3-$CF_3$-Ph | 2-BnNH-4-Pyr | 4-(3,4-deH-Pip) |
| 2-2614 | 3-$CF_3$-Ph | 2-BnNH-4-Pym | 4-(3,4-deH-Pip) |
| 2-2616 | 3-$CF_3$-Ph | 2-(α-Me-BnNH)-4-Pyr | 4-(3,4-deH-Pip) |
| 2-2618 | 3-$CF_3$-Ph | 2-(α-Me-BnNH)-4-Pym | 4-(3,4-deH-Pip) |
| 2-2627 | 4-F-Ph | 4-Pyr | 3-(3,4-deH-Pip) |
| 2-2628 | 4-F-Ph | 4-Pyr | 1-Me-3-(3,4-deH-Pip) |
| 2-2629 | 4-F-Ph | 4-Pyr | 1-Et-4-(3,4-deH-Pip) |
| 2-2630 | 4-F-Ph | 4-Pyr | 1-Pr-4-(3,4-deH-Pip) |
| 2-2632 | 4-F-Ph | 4-Pyr | 1-iPr-4-(3,4-deH-Pip) |

| Compound No. | $R^1$ | $R^2$ | $R^3$ |
|---|---|---|---|
| 2-2634 | 4-F-Ph | 4-Pyr | 1-Bu-4-(3,4-deH-Pip) |
| 2-2635 | 4-F-Ph | 4-Pyr | 1-tBu-4-(3,4-deH-Pip) |
| 2-2636 | 4-F-Ph | 4-Pyr | 1-Pn-4-(3,4-deH-Pip) |
| 2-2637 | 4-F-Ph | 4-Pyr | 1-Hx-4-(3,4-deH-Pip) |
| 2-2638 | 4-F-Ph | 4-Pyr | 1-Hp-4-(3,4-deH-Pip) |
| 2-2639 | 4-F-Ph | 4-Pyr | 1-Oc-4-(3,4-deH-Pip) |
| 2-2640 | 4-F-Ph | 4-Pyr | 1-Nn-4-(3,4-deH-Pip) |
| 2-2641 | 4-F-Ph | 4-Pyr | 1-cPr-4-(3,4-deH-Pip) |
| 2-2642 | 4-F-Ph | 4-Pyr | 1-cPn-4-(3,4-deH-Pip) |
| 2-2643 | 4-F-Ph | 4-Pyr | 1-cHx-4-(3,4-deH-Pip) |
| 2-2644 | 4-F-Ph | 4-Pyr | 1-Bn-4-(3,4-deH-Pip) |
| 2-2645 | 4-F-Ph | 4-Pyr | 1-Phet-4-(3,4-deH-Pip) |
| 2-2646 | 4-F-Ph | 4-Pyr | 1-(3-Ph-Pr)-4-(3,4-deH-Pip) |
| 2-2647 | 4-F-Ph | 4-Pyr | 1-(4-Ph-Bu)-4-(3,4-deH-Pip) |
| 2-2648 | 4-F-Ph | 4-Pyr | 1-Allyl-4-(3,4-deH-Pip) |
| 2-2649 | 4-F-Ph | 4-Pyr | 1-Propargyl-4-(3,4-deH-Pip) |
| 2-2650 | 4-F-Ph | 4-Pyr | 2,2,6,6-tetraMe-4-(3,4-deH-Pip) |
| 2-2651 | 4-F-Ph | 4-Pyr | 1,2,2,6,6-pentaMe-4-(3,4-deH-Pip) |
| 2-2653 | 4-F-Ph | 4-Pyr | 7-(1,2,3,5,6,8a-hexaH-Ind) |
| 2-2654 | 4-F-Ph | 4-Pyr | 7-(1,2,3,5,8,8a-hexaH-Ind) |
| 2-2656 | 4-F-Ph | 4-Pyr | 8-(1,3,4,6,7,9a-hexaH-2H-Qui) |
| 2-2657 | 4-F-Ph | 4-Pyr | 8-(1,3,4,6,9,9a-hexaH-2H-Qui) |
| 2-2659 | 4-F-Ph | 4-Pyr | 2,2-diMe-4-(3,4-deH-Pip) |
| 2-2660 | 4-F-Ph | 4-Pyr | 1,2,2-triMe-4-(3,4-deH-Pip) |
| 2-2661 | 4-F-Ph | 4-Pyr | 2,2-diMe-4-(4,5-deH-Pip) |

| Compound No. | $R^1$ | $R^2$ | $R^3$ |
|---|---|---|---|
| 2-2662 | 4-F-Ph | 4-Pyr | 1,2,2-triMe-4-(4,5-deH-Pip) |
| 2-2663 | 4-F-Ph | 4-Pyr | 2,6-diMe-4-(3,4-deH-Pip) |
| 2-2664 | 4-F-Ph | 4-Pyr | 1,2,6-triMe-4-(3,4-deH-Pip) |
| 2-2665 | 4-F-Ph | 4-Pyr | 2-Me-4-(3,4-deH-Pip) |
| 2-2666 | 4-F-Ph | 4-Pyr | 1,2-diMe-4-(3,4-deH-Pip) |
| 2-2667 | 4-F-Ph | 4-Pyr | 1-Et-2-Me-4-(3,4-deH-Pip) |
| 2-2668 | 4-F-Ph | 4-Pyr | 1-Pr-2-Me-4-(3,4-deH-Pip) |
| 2-2669 | 4-F-Ph | 4-Pyr | 1-Bu-2-Me-4-(3,4-deH-Pip) |
| 2-2670 | 4-F-Ph | 4-Pyr | 1-Phet-2-Me-4-(3,4-deH-Pip) |
| 2-2671 | 4-F-Ph | 4-Pyr | 2-Et-4-(3,4-deH-Pip) |
| 2-2672 | 4-F-Ph | 4-Pyr | 1-Me-2-Et-4-(3,4-deH-Pip) |
| 2-2673 | 4-F-Ph | 4-Pyr | 1,2-diEt-4-(3,4-deH-Pip) |
| 2-2674 | 4-F-Ph | 4-Pyr | 1-Pr-2-Et-4-(3,4-deH-Pip) |
| 2-2675 | 4-F-Ph | 4-Pyr | 1-Bu-2-Et-4-(3,4-deH-Pip) |
| 2-2676 | 4-F-Ph | 4-Pyr | 1-Phet-2-Et-4-(3,4-deH-Pip) |
| 2-2677 | 4-F-Ph | 4-Pyr | 2-Pr-4-(3,4-deH-Pip) |
| 2-2678 | 4-F-Ph | 4-Pyr | 1-Me-2-Pr-4-(3,4-deH-Pip) |
| 2-2679 | 4-F-Ph | 4-Pyr | 1-Et-2-Pr-4-(3,4-deH-Pip) |
| 2-2680 | 4-F-Ph | 4-Pyr | 1,2-diPr-4-(3,4-deH-Pip) |
| 2-2681 | 4-F-Ph | 4-Pyr | 1-Bu-2-Pr-4-(3,4-deH-Pip) |
| 2-2682 | 4-F-Ph | 4-Pyr | 1-Phet-2-Pr-4-(3,4-deH-Pip) |
| 2-2683 | 4-F-Ph | 4-Pyr | 2-Bu-4-(3,4-deH-Pip) |
| 2-2684 | 4-F-Ph | 4-Pyr | 1-Me-2-Bu-4-(3,4-deH-Pip) |
| 2-2685 | 4-F-Ph | 4-Pyr | 1-Et-2-Bu-4-(3,4-deH-Pip) |
| 2-2686 | 4-F-Ph | 4-Pyr | 1-Pr-2-Bu-4-(3,4-deH-Pip) |
| 2-2687 | 4-F-Ph | 4-Pyr | 1,2-diBu-4-(3,4-deH-Pip) |
| 2-2688 | 4-F-Ph | 4-Pyr | 1-Phet-2-Bu-4-(3,4-deH-Pip) |
| 2-2689 | 4-F-Ph | 4-Pyr | 2-Allyl-4-(3,4-deH-Pip) |
| 2-2690 | 4-F-Ph | 4-Pyr | 1-Me-2-Allyl-4-(3,4-deH-Pip) |
| 2-2691 | 4-F-Ph | 4-Pyr | 1-Et-2-Allyl-4-(3,4-deH-Pip) |

| Compound No. | $R^1$ | $R^2$ | $R^3$ |
|---|---|---|---|
| 2-2692 | 4-F-Ph | 4-Pyr | 1-Pr-2-Allyl-4-(3,4-deH-Pip) |
| 2-2693 | 4-F-Ph | 4-Pyr | 1-Bu-2-Allyl-4-(3,4-deH-Pip) |
| 2-2694 | 4-F-Ph | 4-Pyr | 1-Phet-2-Allyl-4-(3,4-deH-Pip) |
| 2-2695 | 4-F-Ph | 4-Pyr | 2-Bn-4-(3,4-deH-Pip) |
| 2-2696 | 4-F-Ph | 4-Pyr | 1-Me-2-Bn-4-(3,4-deH-Pip) |
| 2-2697 | 4-F-Ph | 4-Pyr | 1-Et-2-Bn-4-(3,4-deH-Pip) |
| 2-2698 | 4-F-Ph | 4-Pyr | 1-Pr-2-Bn-4-(3,4-deH-Pip) |
| 2-2699 | 4-F-Ph | 4-Pyr | 1-Bu-2-Bn-4-(3,4-deH-Pip) |
| 2-2700 | 4-F-Ph | 4-Pyr | 1-Phet-2-Bn-4-(3,4-deH-Pip) |
| 2-2701 | 4-F-Ph | 4-Pyr | 2-Phet-4-(3,4-deH-Pip) |
| 2-2702 | 4-F-Ph | 4-Pyr | 1-Me-2-Phet-4-(3,4-deH-Pip) |
| 2-2703 | 4-F-Ph | 4-Pyr | 1-Et-2-Phet-4-(3,4-deH-Pip) |
| 2-2704 | 4-F-Ph | 4-Pyr | 1-Pr-2-Phet-4-(3,4-deH-Pip) |
| 2-2705 | 4-F-Ph | 4-Pyr | 1-Bu-2-Phet-4-(3,4-deH-Pip) |
| 2-2706 | 4-F-Ph | 4-Pyr | 1,2-diPhet-4-(3,4-deH-Pip) |
| 2-2707 | 4-F-Ph | 4-Pyr | 2-Me-4-(4,5-deH-Pip) |
| 2-2708 | 4-F-Ph | 4-Pyr | 1,2-diMe-4-(4,5-deH-Pip) |
| 2-2709 | 4-F-Ph | 4-Pyr | 1-Et-2-Me-4-(4,5-deH-Pip) |
| 2-2710 | 4-F-Ph | 4-Pyr | 1-Pr-2-Me-4-(4,5-deH-Pip) |
| 2-2711 | 4-F-Ph | 4-Pyr | 1-Bu-2-Me-4-(4,5-deH-Pip) |
| 2-2712 | 4-F-Ph | 4-Pyr | 1-Phet-2-Me-4-(4,5-deH-Pip) |
| 2-2713 | 4-F-Ph | 4-Pyr | 2-Et-4-(4,5-deH-Pip) |
| 2-2714 | 4-F-Ph | 4-Pyr | 1-Me-2-Et-4-(4,5-deH-Pip) |
| 2-2715 | 4-F-Ph | 4-Pyr | 1,2-diEt-4-(4,5-deH-Pip) |
| 2-2716 | 4-F-Ph | 4-Pyr | 1-Pr-2-Et-4-(4,5-deH-Pip) |
| 2-2717 | 4-F-Ph | 4-Pyr | 1-Bu-2-Et-4-(4,5-deH-Pip) |
| 2-2718 | 4-F-Ph | 4-Pyr | 1-Phet-2-Et-4-(4,5-deH-Pip) |
| 2-2719 | 4-F-Ph | 4-Pyr | 2-Pr-4-(4,5-deH-Pip) |
| 2-2720 | 4-F-Ph | 4-Pyr | 1-Me-2-Pr-4-(4,5-deH-Pip) |
| 2-2721 | 4-F-Ph | 4-Pyr | 1-Et-2-Pr-4-(4,5-deH-Pip) |

| Compound No. | $R^1$ | $R^2$ | $R^3$ |
|---|---|---|---|
| 2-2722 | 4-F-Ph | 4-Pyr | 1,2-diPr-4-(4,5-deH-Pip) |
| 2-2723 | 4-F-Ph | 4-Pyr | 1-Bu-2-Pr-4-(4,5-deH-Pip) |
| 2-2724 | 4-F-Ph | 4-Pyr | 1-Phet-2-Pr-4-(4,5-deH-Pip) |
| 2-2725 | 4-F-Ph | 4-Pyr | 2-Bu-4-(4,5-deH-Pip) |
| 2-2726 | 4-F-Ph | 4-Pyr | 1-Me-2-Bu-4-(4,5-deH-Pip) |
| 2-2727 | 4-F-Ph | 4-Pyr | 1-Et-2-Bu-4-(4,5-deH-Pip) |
| 2-2728 | 4-F-Ph | 4-Pyr | 1-Pr-2-Bu-4-(4,5-deH-Pip) |
| 2-2729 | 4-F-Ph | 4-Pyr | 1,2-diBu-4-(4,5-deH-Pip) |
| 2-2730 | 4-F-Ph | 4-Pyr | 1-Phet-2-Bu-4-(4,5-deH-Pip) |
| 2-2731 | 4-F-Ph | 4-Pyr | 2-Allyl-4-(4,5-deH-Pip) |
| 2-2732 | 4-F-Ph | 4-Pyr | 1-Me-2-Allyl-4-(4,5-deH-Pip) |
| 2-2733 | 4-F-Ph | 4-Pyr | 1-Et-2-Allyl-4-(4,5-deH-Pip) |
| 2-2734 | 4-F-Ph | 4-Pyr | 1-Pr-2-Allyl-4-(4,5-deH-Pip) |
| 2-2735 | 4-F-Ph | 4-Pyr | 1-Bu-2-Allyl-4-(4,5-deH-Pip) |
| 2-2736 | 4-F-Ph | 4-Pyr | 1-Phet-2-Allyl-4-(4,5-deH-Pip) |
| 2-2737 | 4-F-Ph | 4-Pyr | 2-Bn-4-(4,5-deH-Pip) |
| 2-2738 | 4-F-Ph | 4-Pyr | 1-Me-2-Bn-4-(4,5-deH-Pip) |
| 2-2739 | 4-F-Ph | 4-Pyr | 1-Et-2-Bn-4-(4,5-deH-Pip) |
| 2-2740 | 4-F-Ph | 4-Pyr | 1-Pr-2-Bn-4-(4,5-deH-Pip) |
| 2-2741 | 4-F-Ph | 4-Pyr | 1-Bu-2-Bn-4-(4,5-deH-Pip) |
| 2-2742 | 4-F-Ph | 4-Pyr | 1-Phet-2-Bn-4-(4,5-deH-Pip) |
| 2-2743 | 4-F-Ph | 4-Pyr | 2-Phet-4-(4,5-deH-Pip) |
| 2-2744 | 4-F-Ph | 4-Pyr | 1-Me-2-Phet-4-(4,5-deH-Pip) |
| 2-2745 | 4-F-Ph | 4-Pyr | 1-Et-2-Phet-4-(4,5-deH-Pip) |
| 2-2746 | 4-F-Ph | 4-Pyr | 1-Pr-2-Phet-4-(4,5-deH-Pip) |
| 2-2747 | 4-F-Ph | 4-Pyr | 1-Bu-2-Phet-4-(4,5-deH-Pip) |
| 2-2748 | 4-F-Ph | 4-Pyr | 1,2-diPhet-4-(4,5-deH-Pip) |

| Compound No. | $R^1$ | $R^2$ | $R^3$ |
|---|---|---|---|
| 2-2753 | 4-F-Ph | $2-NH_2-4-Pym$ | 3-(3,4-deH-Pip) |
| 2-2754 | 4-F-Ph | $2-NH_2-4-Pym$ | 1-Me-3-(3,4-deH-Pip) |
| 2-2755 | 4-F-Ph | $2-NH_2-4-Pym$ | 1-Et-4-(3,4-deH-Pip) |
| 2-2756 | 4-F-Ph | $2-NH_2-4-Pym$ | 1-Pr-4-(3,4-deH-Pip) |
| 2-2758 | 4-F-Ph | $2-NH_2-4-Pym$ | 1-iPr-4-(3,4-deH-Pip) |
| 2-2760 | 4-F-Ph | $2-NH_2-4-Pym$ | 1-Bu-4-(3,4-deH-Pip) |
| 2-2761 | 4-F-Ph | $2-NH_2-4-Pym$ | 1-tBu-4-(3,4-deH-Pip) |
| 2-2762 | 4-F-Ph | $2-NH_2-4-Pym$ | 1-Pn-4-(3,4-deH-Pip) |
| 2-2763 | 4-F-Ph | $2-NH_2-4-Pym$ | 1-Hx-4-(3,4-deH-Pip) |
| 2-2764 | 4-F-Ph | $2-NH_2-4-Pym$ | 1-Hp-4-(3,4-deH-Pip) |
| 2-2765 | 4-F-Ph | $2-NH_2-4-Pym$ | 1-Oc-4-(3,4-deH-Pip) |
| 2-2766 | 4-F-Ph | $2-NH_2-4-Pym$ | 1-Nn-4-(3,4-deH-Pip) |
| 2-2767 | 4-F-Ph | $2-NH_2-4-Pym$ | 1-cPr-4-(3,4-deH-Pip) |
| 2-2768 | 4-F-Ph | $2-NH_2-4-Pym$ | 1-cPn-4-(3,4-deH-Pip) |
| 2-2769 | 4-F-Ph | $2-NH_2-4-Pym$ | 1-cHx-4-(3,4-deH-Pip) |
| 2-2770 | 4-F-Ph | $2-NH_2-4-Pym$ | 1-Bn-4-(3,4-deH-Pip) |
| 2-2771 | 4-F-Ph | $2-NH_2-4-Pym$ | 1-Phet-4-(3,4-deH-Pip) |
| 2-2772 | 4-F-Ph | $2-NH_2-4-Pym$ | 1-(3-Ph-Pr)-4-(3,4-deH-Pip) |
| 2-2773 | 4-F-Ph | $2-NH_2-4-Pym$ | 1-(4-Ph-Bu)-4-(3,4-deH-Pip) |
| 2-2774 | 4-F-Ph | $2-NH_2-4-Pym$ | 1-Allyl-4-(3,4-deH-Pip) |
| 2-2775 | 4-F-Ph | $2-NH_2-4-Pym$ | 1-Propargyl-4-(3,4-deH-Pip) |
| 2-2776 | 4-F-Ph | $2-NH_2-4-Pym$ | 2,2,6,6-tetraMe-4-(3,4-deH-Pip) |
| 2-2777 | 4-F-Ph | $2-NH_2-4-Pym$ | 1,2,2,6,6-pentaMe-4-(3,4-deH-Pip) |
| 2-2779 | 4-F-Ph | $2-NH_2-4-Pym$ | 7-(1,2,3,5,6,8a-hexaH-Ind) |
| 2-2780 | 4-F-Ph | $2-NH_2-4-Pym$ | 7-(1,2,3,5,8,8a-hexaH-Ind) |

| Compound No. | $R^1$ | $R^2$ | $R^3$ |
|---|---|---|---|
| 2-2782 | 4-F-Ph | $2-NH_2-4-Pym$ | 8-(1,3,4,6,7,9a-hexaH-2H-Qui) |
| 2-2783 | 4-F-Ph | $2-NH_2-4-Pym$ | 8-(1,3,4,6,9,9a-hexaH-2H-Qui) |
| 2-2785 | 4-F-Ph | $2-NH_2-4-Pym$ | 2,2-diMe-4-(3,4-deH-Pip) |
| 2-2786 | 4-F-Ph | $2-NH_2-4-Pym$ | 1,2,2-triMe-4-(3,4-deH-Pip) |
| 2-2787 | 4-F-Ph | $2-NH_2-4-Pym$ | 2,2-diMe-4-(4,5-deH-Pip) |
| 2-2788 | 4-F-Ph | $2-NH_2-4-Pym$ | 1,2,2-triMe-4-(4,5-deH-Pip) |
| 2-2789 | 4-F-Ph | $2-NH_2-4-Pym$ | 2,6-diMe-4-(3,4-deH-Pip) |
| 2-2790 | 4-F-Ph | $2-NH_2-4-Pym$ | 1,2,6-triMe-4-(3,4-deH-Pip) |
| 2-2791 | 4-F-Ph | $2-NH_2-4-Pym$ | 2-Me-4-(3,4-deH-Pip) |
| 2-2792 | 4-F-Ph | $2-NH_2-4-Pym$ | 1,2-diMe-4-(3,4-deH-Pip) |
| 2-2793 | 4-F-Ph | $2-NH_2-4-Pym$ | 1-Et-2-Me-4-(3,4-deH-Pip) |
| 2-2794 | 4-F-Ph | $2-NH_2-4-Pym$ | 1-Pr-2-Me-4-(3,4-deH-Pip) |
| 2-2795 | 4-F-Ph | $2-NH_2-4-Pym$ | 1-Bu-2-Me-4-(3,4-deH-Pip) |
| 2-2796 | 4-F-Ph | $2-NH_2-4-Pym$ | 1-Phet-2-Me-4-(3,4-deH-Pip) |
| 2-2797 | 4-F-Ph | $2-NH_2-4-Pym$ | 2-Et-4-(3,4-deH-Pip) |
| 2-2798 | 4-F-Ph | $2-NH_2-4-Pym$ | 1-Me-2-Et-4-(3,4-deH-Pip) |
| 2-2799 | 4-F-Ph | $2-NH_2-4-Pym$ | 1,2-diEt-4-(3,4-deH-Pip) |
| 2-2800 | 4-F-Ph | $2-NH_2-4-Pym$ | 1-Pr-2-Et-4-(3,4-deH-Pip) |
| 2-2801 | 4-F-Ph | $2-NH_2-4-Pym$ | 1-Bu-2-Et-4-(3,4-deH-Pip) |
| 2-2802 | 4-F-Ph | $2-NH_2-4-Pym$ | 1-Phet-2-Et-4-(3,4-deH-Pip) |
| 2-2803 | 4-F-Ph | $2-NH_2-4-Pym$ | 2-Pr-4-(3,4-deH-Pip) |
| 2-2804 | 4-F-Ph | $2-NH_2-4-Pym$ | 1-Me-2-Pr-4-(3,4-deH-Pip) |
| 2-2805 | 4-F-Ph | $2-NH_2-4-Pym$ | 1-Et-2-Pr-4-(3,4-deH-Pip) |
| 2-2806 | 4-F-Ph | $2-NH_2-4-Pym$ | 1,2-diPr-4-(3,4-deH-Pip) |
| 2-2807 | 4-F-Ph | $2-NH_2-4-Pym$ | 1-Bu-2-Pr-4-(3,4-deH-Pip) |
| 2-2808 | 4-F-Ph | $2-NH_2-4-Pym$ | 1-Phet-2-Pr-4-(3,4-deH-Pip) |
| 2-2809 | 4-F-Ph | $2-NH_2-4-Pym$ | 2-Bu-4-(3,4-deH-Pip) |
| 2-2810 | 4-F-Ph | $2-NH_2-4-Pym$ | 1-Me-2-Bu-4-(3,4-deH-Pip) |

| Compound No. | $R^1$ | $R^2$ | $R^3$ |
|---|---|---|---|
| 2-2811 | 4-F-Ph | 2-$NH_2$-4-Pym | 1-Et-2-Bu-4-(3,4-deH-Pip) |
| 2-2812 | 4-F-Ph | 2-$NH_2$-4-Pym | 1-Pr-2-Bu-4-(3,4-deH-Pip) |
| 2-2813 | 4-F-Ph | 2-$NH_2$-4-Pym | 1,2-diBu-4-(3,4-deH-Pip) |
| 2-2814 | 4-F-Ph | 2-$NH_2$-4-Pym | 1-Phet-2-Bu-4-(3,4-deH-Pip) |
| 2-2815 | 4-F-Ph | 2-$NH_2$-4-Pym | 2-Allyl-4-(3,4-deH-Pip) |
| 2-2816 | 4-F-Ph | 2-$NH_2$-4-Pym | 1-Me-2-Allyl-4-(3,4-deH-Pip) |
| 2-2817 | 4-F-Ph | 2-$NH_2$-4-Pym | 1-Et-2-Allyl-4-(3,4-deH-Pip) |
| 2-2818 | 4-F-Ph | 2-$NH_2$-4-Pym | 1-Pr-2-Allyl-4-(3,4-deH-Pip) |
| 2-2819 | 4-F-Ph | 2-$NH_2$-4-Pym | 1-Bu-2-Allyl-4-(3,4-deH-Pip) |
| 2-2820 | 4-F-Ph | 2-$NH_2$-4-Pym | 1-Phet-2-Allyl-4-(3,4-deH-Pip) |
| 2-2821 | 4-F-Ph | 2-$NH_2$-4-Pym | 2-Bn-4-(3,4-deH-Pip) |
| 2-2822 | 4-F-Ph | 2-$NH_2$-4-Pym | 1-Me-2-Bn-4-(3,4-deH-Pip) |
| 2-2823 | 4-F-Ph | 2-$NH_2$-4-Pym | 1-Et-2-Bn-4-(3,4-deH-Pip) |
| 2-2824 | 4-F-Ph | 2-$NH_2$-4-Pym | 1-Pr-2-Bn-4-(3,4-deH-Pip) |
| 2-2825 | 4-F-Ph | 2-$NH_2$-4-Pym | 1-Bu-2-Bn-4-(3,4-deH-Pip) |
| 2-2826 | 4-F-Ph | 2-$NH_2$-4-Pym | 1-Phet-2-Bn-4-(3,4-deH-Pip) |
| 2-2827 | 4-F-Ph | 2-$NH_2$-4-Pym | 2-Phet-4-(3,4-deH-Pip) |
| 2-2828 | 4-F-Ph | 2-$NH_2$-4-Pym | 1-Me-2-Phet-4-(3,4-deH-Pip) |
| 2-2829 | 4-F-Ph | 2-$NH_2$-4-Pym | 1-Et-2-Phet-4-(3,4-deH-Pip) |
| 2-2830 | 4-F-Ph | 2-$NH_2$-4-Pym | 1-Pr-2-Phet-4-(3,4-deH-Pip) |
| 2-2831 | 4-F-Ph | 2-$NH_2$-4-Pym | 1-Bu-2-Phet-4-(3,4-deH-Pip) |
| 2-2832 | 4-F-Ph | 2-$NH_2$-4-Pym | 1,2-diPhet-4-(3,4-deH-Pip) |
| 2-2833 | 4-F-Ph | 2-$NH_2$-4-Pym | 2-Me-4-(4,5-deH-Pip) |
| 2-2834 | 4-F-Ph | 2-$NH_2$-4-Pym | 1,2-diMe-4-(4,5-deH-Pip) |
| 2-2835 | 4-F-Ph | 2-$NH_2$-4-Pym | 1-Et-2-Me-4-(4,5-deH-Pip) |
| 2-2836 | 4-F-Ph | 2-$NH_2$-4-Pym | 1-Pr-2-Me-4-(4,5-deH-Pip) |
| 2-2837 | 4-F-Ph | 2-$NH_2$-4-Pym | 1-Bu-2-Me-4-(4,5-deH-Pip) |
| 2-2838 | 4-F-Ph | 2-$NH_2$-4-Pym | 1-Phet-2-Me-4-(4,5-deH-Pip) |
| 2-2839 | 4-F-Ph | 2-$NH_2$-4-Pym | 2-Et-4-(4,5-deH-Pip) |
| 2-2840 | 4-F-Ph | 2-$NH_2$-4-Pym | 1-Me-2-Et-4-(4,5-deH-Pip) |

| Compound No. | $R^1$ | $R^2$ | $R^3$ |
|---|---|---|---|
| 2-2841 | 4-F-Ph | 2-$NH_2$-4-Pym | 1,2-diEt-4-(4,5-deH-Pip) |
| 2-2842 | 4-F-Ph | 2-$NH_2$-4-Pym | 1-Pr-2-Et-4-(4,5-deH-Pip) |
| 2-2843 | 4-F-Ph | 2-$NH_2$-4-Pym | 1-Bu-2-Et-4-(4,5-deH-Pip) |
| 2-2844 | 4-F-Ph | 2-$NH_2$-4-Pym | 1-Phet-2-Et-4-(4,5-deH-Pip) |
| 2-2845 | 4-F-Ph | 2-$NH_2$-4-Pym | 2-Pr-4-(4,5-deH-Pip) |
| 2-2846 | 4-F-Ph | 2-$NH_2$-4-Pym | 1-Me-2-Pr-4-(4,5-deH-Pip) |
| 2-2847 | 4-F-Ph | 2-$NH_2$-4-Pym | 1-Et-2-Pr-4-(4,5-deH-Pip) |
| 2-2848 | 4-F-Ph | 2-$NH_2$-4-Pym | 1,2-diPr-4-(4,5-deH-Pip) |
| 2-2849 | 4-F-Ph | 2-$NH_2$-4-Pym | 1-Bu-2-Pr-4-(4,5-deH-Pip) |
| 2-2850 | 4-F-Ph | 2-$NH_2$-4-Pym | 1-Phet-2-Pr-4-(4,5-deH-Pip) |
| 2-2851 | 4-F-Ph | 2-$NH_2$-4-Pym | 2-Bu-4-(4,5-deH-Pip) |
| 2-2852 | 4-F-Ph | 2-$NH_2$-4-Pym | 1-Me-2-Bu-4-(4,5-deH-Pip) |
| 2-2853 | 4-F-Ph | 2-$NH_2$-4-Pym | 1-Et-2-Bu-4-(4,5-deH-Pip) |
| 2-2854 | 4-F-Ph | 2-$NH_2$-4-Pym | 1-Pr-2-Bu-4-(4,5-deH-Pip) |
| 2-2855 | 4-F-Ph | 2-$NH_2$-4-Pym | 1,2-diBu-4-(4,5-deH-Pip) |
| 2-2856 | 4-F-Ph | 2-$NH_2$-4-Pym | 1-Phet-2-Bu-4-(4,5-deH-Pip) |
| 2-2857 | 4-F-Ph | 2-$NH_2$-4-Pym | 2-Allyl-4-(4,5-deH-Pip) |
| 2-2858 | 4-F-Ph | 2-$NH_2$-4-Pym | 1-Me-2-Allyl-4-(4,5-deH-Pip) |
| 2-2859 | 4-F-Ph | 2-$NH_2$-4-Pym | 1-Et-2-Allyl-4-(4,5-deH-Pip) |
| 2-2860 | 4-F-Ph | 2-$NH_2$-4-Pym | 1-Pr-2-Allyl-4-(4,5-deH-Pip) |
| 2-2861 | 4-F-Ph | 2-$NH_2$-4-Pym | 1-Bu-2-Allyl-4-(4,5-deH-Pip) |
| 2-2862 | 4-F-Ph | 2-$NH_2$-4-Pym | 1-Phet-2-Allyl-4-(4,5-deH-Pip) |
| 2-2863 | 4-F-Ph | 2-$NH_2$-4-Pym | 2-Bn-4-(4,5-deH-Pip) |
| 2-2864 | 4-F-Ph | 2-$NH_2$-4-Pym | 1-Me-2-Bn-4-(4,5-deH-Pip) |
| 2-2865 | 4-F-Ph | 2-$NH_2$-4-Pym | 1-Et-2-Bn-4-(4,5-deH-Pip) |
| 2-2866 | 4-F-Ph | 2-$NH_2$-4-Pym | 1-Pr-2-Bn-4-(4,5-deH-Pip) |
| 2-2867 | 4-F-Ph | 2-$NH_2$-4-Pym | 1-Bu-2-Bn-4-(4,5-deH-Pip) |
| 2-2868 | 4-F-Ph | 2-$NH_2$-4-Pym | 1-Phet-2-Bn-4-(4,5-deH-Pip) |
| 2-2869 | 4-F-Ph | 2-$NH_2$-4-Pym | 2-Phet-4-(4,5-deH-Pip) |
| 2-2870 | 4-F-Ph | 2-$NH_2$-4-Pym | 1-Me-2-Phet-4-(4,5-deH-Pip) |

| Compound No. | $R^1$ | $R^2$ | $R^3$ |
|---|---|---|---|
| 2-2871 | 4-F-Ph | 2-$NH_2$-4-Pym | 1-Et-2-Phet-4-(4,5-deH-Pip) |
| 2-2872 | 4-F-Ph | 2-$NH_2$-4-Pym | 1-Pr-2-Phet-4-(4,5-deH-Pip) |
| 2-2873 | 4-F-Ph | 2-$NH_2$-4-Pym | 1-Bu-2-Phet-4-(4,5-deH-Pip) |
| 2-2874 | 4-F-Ph | 2-$NH_2$-4-Pym | 1,2-diPhet-4-(4,5-deH-Pip) |
| 2-2879 | 4-F-Ph | 2-MeNH-4-Pym | 3-(3,4-deH-Pip) |
| 2-2880 | 4-F-Ph | 2-MeNH-4-Pym | 1-Me-3-(3,4-deH-Pip) |
| 2-2881 | 4-F-Ph | 2-MeNH-4-Pym | 1-Et-4-(3,4-deH-Pip) |
| 2-2882 | 4-F-Ph | 2-MeNH-4-Pym | 1-Pr-4-(3,4-deH-Pip) |
| 2-2884 | 4-F-Ph | 2-MeNH-4-Pym | 1-iPr-4-(3,4-deH-Pip) |
| 2-2886 | 4-F-Ph | 2-MeNH-4-Pym | 1-Bu-4-(3,4-deH-Pip) |
| 2-2887 | 4-F-Ph | 2-MeNH-4-Pym | 1-tBu-4-(3,4-deH-Pip) |
| 2-2888 | 4-F-Ph | 2-MeNH-4-Pym | 1-Pn-4-(3,4-deH-Pip) |
| 2-2889 | 4-F-Ph | 2-MeNH-4-Pym | 1-Hx-4-(3,4-deH-Pip) |
| 2-2890 | 4-F-Ph | 2-MeNH-4-Pym | 1-Hp-4-(3,4-deH-Pip) |
| 2-2891 | 4-F-Ph | 2-MeNH-4-Pym | 1-Oc-4-(3,4-deH-Pip) |
| 2-2892 | 4-F-Ph | 2-MeNH-4-Pym | 1-Nn-4-(3,4-deH-Pip) |
| 2-2893 | 4-F-Ph | 2-MeNH-4-Pym | 1-cPr-4-(3,4-deH-Pip) |
| 2-2894 | 4-F-Ph | 2-MeNH-4-Pym | 1-cPn-4-(3,4-deH-Pip) |
| 2-2895 | 4-F-Ph | 2-MeNH-4-Pym | 1-cHx-4-(3,4-deH-Pip) |
| 2-2896 | 4-F-Ph | 2-MeNH-4-Pym | 1-Bn-4-(3,4-deH-Pip) |
| 2-2897 | 4-F-Ph | 2-MeNH-4-Pym | 1-Phet-4-(3,4-deH-Pip) |
| 2-2898 | 4-F-Ph | 2-MeNH-4-Pym | 1-(3-Ph-Pr)-4-(3,4-deH-Pip) |
| 2-2899 | 4-F-Ph | 2-MeNH-4-Pym | 1-(4-Ph-Bu)-4-(3,4-deH-Pip) |
| 2-2900 | 4-F-Ph | 2-MeNH-4-Pym | 1-Allyl-4-(3,4-deH-Pip) |

| Compound No. | $R^1$ | $R^2$ | $R^3$ |
|---|---|---|---|
| 2-2901 | 4-F-Ph | 2-MeNH-4-Pym | 1-Propargyl-4-(3,4-deH-Pip) |
| 2-2902 | 4-F-Ph | 2-MeNH-4-Pym | 2,2,6,6-tetraMe-4-(3,4-deH-Pip) |
| 2-2903 | 4-F-Ph | 2-MeNH-4-Pym | 1,2,2,6,6-pentaMe-4-(3,4-deH-Pip) |
| 2-2905 | 4-F-Ph | 2-MeNH-4-Pym | 7-(1,2,3,5,6,8a-hexaH-Ind) |
| 2-2906 | 4-F-Ph | 2-MeNH-4-Pym | 7-(1,2,3,5,8,8a-hexaH-Ind) |
| 2-2908 | 4-F-Ph | 2-MeNH-4-Pym | 8-(1,3,4,6,7,9a-hexaH-2H-Qui) |
| 2-2909 | 4-F-Ph | 2-MeNH-4-Pym | 8-(1,3,4,6,9,9a-hexaH-2H-Qui) |
| 2-2911 | 4-F-Ph | 2-MeNH-4-Pym | 2,2-diMe-4-(3,4-deH-Pip) |
| 2-2912 | 4-F-Ph | 2-MeNH-4-Pym | 1,2,2-triMe-4-(3,4-deH-Pip) |
| 2-2913 | 4-F-Ph | 2-MeNH-4-Pym | 2,2-diMe-4-(4,5-deH-Pip) |
| 2-2914 | 4-F-Ph | 2-MeNH-4-Pym | 1,2,2-triMe-4-(4,5-deH-Pip) |
| 2-2915 | 4-F-Ph | 2-MeNH-4-Pym | 2,6-diMe-4-(3,4-deH-Pip) |
| 2-2916 | 4-F-Ph | 2-MeNH-4-Pym | 1,2,6-triMe-4-(3,4-deH-Pip) |
| 2-2917 | 4-F-Ph | 2-MeNH-4-Pym | 2-Me-4-(3,4-deH-Pip) |
| 2-2918 | 4-F-Ph | 2-MeNH-4-Pym | 1,2-diMe-4-(3,4-deH-Pip) |
| 2-2919 | 4-F-Ph | 2-MeNH-4-Pym | 1-Et-2-Me-4-(3,4-deH-Pip) |
| 2-2920 | 4-F-Ph | 2-MeNH-4-Pym | 1-Pr-2-Me-4-(3,4-deH-Pip) |
| 2-2921 | 4-F-Ph | 2-MeNH-4-Pym | 1-Bu-2-Me-4-(3,4-deH-Pip) |
| 2-2922 | 4-F-Ph | 2-MeNH-4-Pym | 1-Phet-2-Me-4-(3,4-deH-Pip) |
| 2-2923 | 4-F-Ph | 2-MeNH-4-Pym | 2-Et-4-(3,4-deH-Pip) |
| 2-2924 | 4-F-Ph | 2-MeNH-4-Pym | 1-Me-2-Et-4-(3,4-deH-Pip) |
| 2-2925 | 4-F-Ph | 2-MeNH-4-Pym | 1,2-diEt-4-(3,4-deH-Pip) |
| 2-2926 | 4-F-Ph | 2-MeNH-4-Pym | 1-Pr-2-Et-4-(3,4-deH-Pip) |
| 2-2927 | 4-F-Ph | 2-MeNH-4-Pym | 1-Bu-2-Et-4-(3,4-deH-Pip) |
| 2-2928 | 4-F-Ph | 2-MeNH-4-Pym | 1-Phet-2-Et-4-(3,4-deH-Pip) |
| 2-2929 | 4-F-Ph | 2-MeNH-4-Pym | 2-Pr-4-(3,4-deH-Pip) |

| Compound No. | $R^1$ | $R^2$ | $R^3$ |
|---|---|---|---|
| 2-2930 | 4-F-Ph | 2-MeNH-4-Pym | 1-Me-2-Pr-4-(3,4-deH-Pip) |
| 2-2931 | 4-F-Ph | 2-MeNH-4-Pym | 1-Et-2-Pr-4-(3,4-deH-Pip) |
| 2-2932 | 4-F-Ph | 2-MeNH-4-Pym | 1,2-diPr-4-(3,4-deH-Pip) |
| 2-2933 | 4-F-Ph | 2-MeNH-4-Pym | 1-Bu-2-Pr-4-(3,4-deH-Pip) |
| 2-2934 | 4-F-Ph | 2-MeNH-4-Pym | 1-Phet-2-Pr-4-(3,4-deH-Pip) |
| 2-2935 | 4-F-Ph | 2-MeNH-4-Pym | 2-Bu-4-(3,4-deH-Pip) |
| 2-2936 | 4-F-Ph | 2-MeNH-4-Pym | 1-Me-2-Bu-4-(3,4-deH-Pip) |
| 2-2937 | 4-F-Ph | 2-MeNH-4-Pym | 1-Et-2-Bu-4-(3,4-deH-Pip) |
| 2-2938 | 4-F-Ph | 2-MeNH-4-Pym | 1-Pr-2-Bu-4-(3,4-deH-Pip) |
| 2-2939 | 4-F-Ph | 2-MeNH-4-Pym | 1,2-diBu-4-(3,4-deH-Pip) |
| 2-2940 | 4-F-Ph | 2-MeNH-4-Pym | 1-Phet-2-Bu-4-(3,4-deH-Pip) |
| 2-2941 | 4-F-Ph | 2-MeNH-4-Pym | 2-Allyl-4-(3,4-deH-Pip) |
| 2-2942 | 4-F-Ph | 2-MeNH-4-Pym | 1-Me-2-Allyl-4-(3,4-deH-Pip) |
| 2-2943 | 4-F-Ph | 2-MeNH-4-Pym | 1-Et-2-Allyl-4-(3,4-deH-Pip) |
| 2-2944 | 4-F-Ph | 2-MeNH-4-Pym | 1-Pr-2-Allyl-4-(3,4-deH-Pip) |
| 2-2945 | 4-F-Ph | 2-MeNH-4-Pym | 1-Bu-2-Allyl-4-(3,4-deH-Pip) |
| 2-2946 | 4-F-Ph | 2-MeNH-4-Pym | 1-Phet-2-Allyl-4-(3,4-deH-Pip) |
| 2-2947 | 4-F-Ph | 2-MeNH-4-Pym | 2-Bn-4-(3,4-deH-Pip) |
| 2-2948 | 4-F-Ph | 2-MeNH-4-Pym | 1-Me-2-Bn-4-(3,4-deH-Pip) |
| 2-2949 | 4-F-Ph | 2-MeNH-4-Pym | 1-Et-2-Bn-4-(3,4-deH-Pip) |
| 2-2950 | 4-F-Ph | 2-MeNH-4-Pym | 1-Pr-2-Bn-4-(3,4-deH-Pip) |
| 2-2951 | 4-F-Ph | 2-MeNH-4-Pym | 1-Bu-2-Bn-4-(3,4-deH-Pip) |
| 2-2952 | 4-F-Ph | 2-MeNH-4-Pym | 1-Phet-2-Bn-4-(3,4-deH-Pip) |
| 2-2953 | 4-F-Ph | 2-MeNH-4-Pym | 2-Phet-4-(3,4-deH-Pip) |
| 2-2954 | 4-F-Ph | 2-MeNH-4-Pym | 1-Me-2-Phet-4-(3,4-deH-Pip) |
| 2-2955 | 4-F-Ph | 2-MeNH-4-Pym | 1-Et-2-Phet-4-(3,4-deH-Pip) |
| 2-2956 | 4-F-Ph | 2-MeNH-4-Pym | 1-Pr-2-Phet-4-(3,4-deH-Pip) |
| 2-2957 | 4-F-Ph | 2-MeNH-4-Pym | 1-Bu-2-Phet-4-(3,4-deH-Pip) |
| 2-2958 | 4-F-Ph | 2-MeNH-4-Pym | 1,2-diPhet-4-(3,4-deH-Pip) |
| 2-2959 | 4-F-Ph | 2-MeNH-4-Pym | 2-Me-4-(4,5-deH-Pip) |

| Compound No. | $R^1$ | $R^2$ | $R^3$ |
|---|---|---|---|
| 2-2960 | 4-F-Ph | 2-MeNH-4-Pym | 1,2-diMe-4-(4,5-deH-Pip) |
| 2-2961 | 4-F-Ph | 2-MeNH-4-Pym | 1-Et-2-Me-4-(4,5-deH-Pip) |
| 2-2962 | 4-F-Ph | 2-MeNH-4-Pym | 1-Pr-2-Me-4-(4,5-deH-Pip) |
| 2-2963 | 4-F-Ph | 2-MeNH-4-Pym | 1-Bu-2-Me-4-(4,5-deH-Pip) |
| 2-2964 | 4-F-Ph | 2-MeNH-4-Pym | 1-Phet-2-Me-4-(4,5-deH-Pip) |
| 2-2965 | 4-F-Ph | 2-MeNH-4-Pym | 2-Et-4-(4,5-deH-Pip) |
| 2-2966 | 4-F-Ph | 2-MeNH-4-Pym | 1-Me-2-Et-4-(4,5-deH-Pip) |
| 2-2967 | 4-F-Ph | 2-MeNH-4-Pym | 1,2-diEt-4-(4,5-deH-Pip) |
| 2-2968 | 4-F-Ph | 2-MeNH-4-Pym | 1-Pr-2-Et-4-(4,5-deH-Pip) |
| 2-2969 | 4-F-Ph | 2-MeNH-4-Pym | 1-Bu-2-Et-4-(4,5-deH-Pip) |
| 2-2970 | 4-F-Ph | 2-MeNH-4-Pym | 1-Phet-2-Et-4-(4,5-deH-Pip) |
| 2-2971 | 4-F-Ph | 2-MeNH-4-Pym | 2-Pr-4-(4,5-deH-Pip) |
| 2-2972 | 4-F-Ph | 2-MeNH-4-Pym | 1-Me-2-Pr-4-(4,5-deH-Pip) |
| 2-2973 | 4-F-Ph | 2-MeNH-4-Pym | 1-Et-2-Pr-4-(4,5-deH-Pip) |
| 2-2974 | 4-F-Ph | 2-MeNH-4-Pym | 1,2-diPr-4-(4,5-deH-Pip) |
| 2-2975 | 4-F-Ph | 2-MeNH-4-Pym | 1-Bu-2-Pr-4-(4,5-deH-Pip) |
| 2-2976 | 4-F-Ph | 2-MeNH-4-Pym | 1-Phet-2-Pr-4-(4,5-deH-Pip) |
| 2-2977 | 4-F-Ph | 2-MeNH-4-Pym | 2-Bu-4-(4,5-deH-Pip) |
| 2-2978 | 4-F-Ph | 2-MeNH-4-Pym | 1-Me-2-Bu-4-(4,5-deH-Pip) |
| 2-2979 | 4-F-Ph | 2-MeNH-4-Pym | 1-Et-2-Bu-4-(4,5-deH-Pip) |
| 2-2980 | 4-F-Ph | 2-MeNH-4-Pym | 1-Pr-2-Bu-4-(4,5-deH-Pip) |
| 2-2981 | 4-F-Ph | 2-MeNH-4-Pym | 1,2-diBu-4-(4,5-deH-Pip) |
| 2-2982 | 4-F-Ph | 2-MeNH-4-Pym | 1-Phet-2-Bu-4-(4,5-deH-Pip) |
| 2-2983 | 4-F-Ph | 2-MeNH-4-Pym | 2-Allyl-4-(4,5-deH-Pip) |
| 2-2984 | 4-F-Ph | 2-MeNH-4-Pym | 1-Me-2-Allyl-4-(4,5-deH-Pip) |
| 2-2985 | 4-F-Ph | 2-MeNH-4-Pym | 1-Et-2-Allyl-4-(4,5-deH-Pip) |
| 2-2986 | 4-F-Ph | 2-MeNH-4-Pym | 1-Pr-2-Allyl-4-(4,5-deH-Pip) |
| 2-2987 | 4-F-Ph | 2-MeNH-4-Pym | 1-Bu-2-Allyl-4-(4,5-deH-Pip) |
| 2-2988 | 4-F-Ph | 2-MeNH-4-Pym | 1-Phet-2-Allyl-4-(4,5-deH-Pip) |
| 2-2989 | 4-F-Ph | 2-MeNH-4-Pym | 2-Bn-4-(4,5-deH-Pip) |

| Compound No. | $R^1$ | $R^2$ | $R^3$ |
|---|---|---|---|
| 2-2990 | 4-F-Ph | 2-MeNH-4-Pym | 1-Me-2-Bn-4-(4,5-deH-Pip) |
| 2-2991 | 4-F-Ph | 2-MeNH-4-Pym | 1-Et-2-Bn-4-(4,5-deH-Pip) |
| 2-2992 | 4-F-Ph | 2-MeNH-4-Pym | 1-Pr-2-Bn-4-(4,5-deH-Pip) |
| 2-2993 | 4-F-Ph | 2-MeNH-4-Pym | 1-Bu-2-Bn-4-(4,5-deH-Pip) |
| 2-2994 | 4-F-Ph | 2-MeNH-4-Pym | 1-Phet-2-Bn-4-(4,5-deH-Pip) |
| 2-2995 | 4-F-Ph | 2-MeNH-4-Pym | 2-Phet-4-(4,5-deH-Pip) |
| 2-2996 | 4-F-Ph | 2-MeNH-4-Pym | 1-Me-2-Phet-4-(4,5-deH-Pip) |
| 2-2997 | 4-F-Ph | 2-MeNH-4-Pym | 1-Et-2-Phet-4-(4,5-deH-Pip) |
| 2-2998 | 4-F-Ph | 2-MeNH-4-Pym | 1-Pr-2-Phet-4-(4,5-deH-Pip) |
| 2-2999 | 4-F-Ph | 2-MeNH-4-Pym | 1-Bu-2-Phet-4-(4,5-deH-Pip) |
| 2-3000 | 4-F-Ph | 2-MeNH-4-Pym | 1,2-diPhet-4-(4,5-deH-Pip) |
| 2-3005 | 4-F-Ph | $2-NH_2-4-Pyr$ | 3-(3,4-deH-Pip) |
| 2-3006 | 4-F-Ph | $2-NH_2-4-Pyr$ | 1-Me-3-(3,4-deH-Pip) |
| 2-3007 | 4-F-Ph | $2-NH_2-4-Pyr$ | 1-Et-4-(3,4-deH-Pip) |
| 2-3008 | 4-F-Ph | $2-NH_2-4-Pyr$ | 1-Pr-4-(3,4-deH-Pip) |
| 2-3010 | 4-F-Ph | $2-NH_2-4-Pyr$ | 1-iPr-4-(3,4-deH-Pip) |
| 2-3012 | 4-F-Ph | $2-NH_2-4-Pyr$ | 1-Bu-4-(3,4-deH-Pip) |
| 2-3013 | 4-F-Ph | $2-NH_2-4-Pyr$ | 1-tBu-4-(3,4-deH-Pip) |
| 2-3014 | 4-F-Ph | $2-NH_2-4-Pyr$ | 1-Pn-4-(3,4-deH-Pip) |
| 2-3015 | 4-F-Ph | $2-NH_2-4-Pyr$ | 1-Hx-4-(3,4-deH-Pip) |
| 2-3016 | 4-F-Ph | $2-NH_2-4-Pyr$ | 1-Hp-4-(3,4-deH-Pip) |
| 2-3017 | 4-F-Ph | $2-NH_2-4-Pyr$ | 1-Oc-4-(3,4-deH-Pip) |
| 2-3018 | 4-F-Ph | $2-NH_2-4-Pyr$ | 1-Nn-4-(3,4-deH-Pip) |
| 2-3019 | 4-F-Ph | $2-NH_2-4-Pyr$ | 1-cPr-4-(3,4-deH-Pip) |

| Compound No. | $R^1$ | $R^2$ | $R^3$ |
|---|---|---|---|
| 2-3020 | 4-F-Ph | 2-$NH_2$-4-Pyr | 1-cPn-4-(3,4-deH-Pip) |
| 2-3021 | 4-F-Ph | 2-$NH_2$-4-Pyr | 1-cHx-4-(3,4-deH-Pip) |
| 2-3022 | 4-F-Ph | 2-$NH_2$-4-Pyr | 1-Bn-4-(3,4-deH-Pip) |
| 2-3023 | 4-F-Ph | 2-$NH_2$-4-Pyr | 1-Phet-4-(3,4-deH-Pip) |
| 2-3024 | 4-F-Ph | 2-$NH_2$-4-Pyr | 1-(3-Ph-Pr)-4-(3,4-deH-Pip) |
| 2-3025 | 4-F-Ph | 2-$NH_2$-4-Pyr | 1-(4-Ph-Bu)-4-(3,4-deH-Pip) |
| 2-3026 | 4-F-Ph | 2-$NH_2$-4-Pyr | 1-Allyl-4-(3,4-deH-Pip) |
| 2-3027 | 4-F-Ph | 2-$NH_2$-4-Pyr | 1-Propargyl-4-(3,4-deH-Pip) |
| 2-3028 | 4-F-Ph | 2-$NH_2$-4-Pyr | 2,2,6,6-tetraMe-4-(3,4-deH-Pip) |
| 2-3029 | 4-F-Ph | 2-$NH_2$-4-Pyr | 1,2,2,6,6-pentaMe-4-(3,4-deH-Pip) |
| 2-3031 | 4-F-Ph | 2-$NH_2$-4-Pyr | 7-(1,2,3,5,6,8a-hexaH-Ind) |
| 2-3032 | 4-F-Ph | 2-$NH_2$-4-Pyr | 7-(1,2,3,5,8,8a-hexaH-Ind) |
| 2-3034 | 4-F-Ph | 2-$NH_2$-4-Pyr | 8-(1,3,4,6,7,9a-hexaH-2H-Qui) |
| 2-3035 | 4-F-Ph | 2-$NH_2$-4-Pyr | 8-(1,3,4,6,9,9a-hexaH-2H-Qui) |
| 2-3037 | 4-F-Ph | 2-$NH_2$-4-Pyr | 2,2-diMe-4-(3,4-deH-Pip) |
| 2-3038 | 4-F-Ph | 2-$NH_2$-4-Pyr | 1,2,2-triMe-4-(3,4-deH-Pip) |
| 2-3039 | 4-F-Ph | 2-$NH_2$-4-Pyr | 2,2-diMe-4-(4,5-deH-Pip) |
| 2-3040 | 4-F-Ph | 2-$NH_2$-4-Pyr | 1,2,2-triMe-4-(4,5-deH-Pip) |
| 2-3041 | 4-F-Ph | 2-$NH_2$-4-Pyr | 2,6-diMe-4-(3,4-deH-Pip) |
| 2-3042 | 4-F-Ph | 2-$NH_2$-4-Pyr | 1,2,6-triMe-4-(3,4-deH-Pip) |
| 2-3043 | 4-F-Ph | 2-$NH_2$-4-Pyr | 2-Me-4-(3,4-deH-Pip) |
| 2-3044 | 4-F-Ph | 2-$NH_2$-4-Pyr | 1,2-diMe-4-(3,4-deH-Pip) |
| 2-3045 | 4-F-Ph | 2-$NH_2$-4-Pyr | 1-Et-2-Me-4-(3,4-deH-Pip) |
| 2-3046 | 4-F-Ph | 2-$NH_2$-4-Pyr | 1-Pr-2-Me-4-(3,4-deH-Pip) |
| 2-3047 | 4-F-Ph | 2-$NH_2$-4-Pyr | 1-Bu-2-Me-4-(3,4-deH-Pip) |
| 2-3048 | 4-F-Ph | 2-$NH_2$-4-Pyr | 1-Phet-2-Me-4-(3,4-deH-Pip) |

| Compound No. | $R^1$ | $R^2$ | $R^3$ |
|---|---|---|---|
| 2-3049 | 4-F-Ph | $2-NH_2-4-Pyr$ | 2-Et-4-(3,4-deH-Pip) |
| 2-3050 | 4-F-Ph | $2-NH_2-4-Pyr$ | 1-Me-2-Et-4-(3,4-deH-Pip) |
| 2-3051 | 4-F-Ph | $2-NH_2-4-Pyr$ | 1,2-diEt-4-(3,4-deH-Pip) |
| 2-3052 | 4-F-Ph | $2-NH_2-4-Pyr$ | 1-Pr-2-Et-4-(3,4-deH-Pip) |
| 2-3053 | 4-F-Ph | $2-NH_2-4-Pyr$ | 1-Bu-2-Et-4-(3,4-deH-Pip) |
| 2-3054 | 4-F-Ph | $2-NH_2-4-Pyr$ | 1-Phet-2-Et-4-(3,4-deH-Pip) |
| 2-3055 | 4-F-Ph | $2-NH_2-4-Pyr$ | 2-Pr-4-(3,4-deH-Pip) |
| 2-3056 | 4-F-Ph | $2-NH_2-4-Pyr$ | 1-Me-2-Pr-4-(3,4-deH-Pip) |
| 2-3057 | 4-F-Ph | $2-NH_2-4-Pyr$ | 1-Et-2-Pr-4-(3,4-deH-Pip) |
| 2-3058 | 4-F-Ph | $2-NH_2-4-Pyr$ | 1,2-diPr-4-(3,4-deH-Pip) |
| 2-3059 | 4-F-Ph | $2-NH_2-4-Pyr$ | 1-Bu-2-Pr-4-(3,4-deH-Pip) |
| 2-3060 | 4-F-Ph | $2-NH_2-4-Pyr$ | 1-Phet-2-Pr-4-(3,4-deH-Pip) |
| 2-3061 | 4-F-Ph | $2-NH_2-4-Pyr$ | 2-Bu-4-(3,4-deH-Pip) |
| 2-3062 | 4-F-Ph | $2-NH_2-4-Pyr$ | 1-Me-2-Bu-4-(3,4-deH-Pip) |
| 2-3063 | 4-F-Ph | $2-NH_2-4-Pyr$ | 1-Et-2-Bu-4-(3,4-deH-Pip) |
| 2-3064 | 4-F-Ph | $2-NH_2-4-Pyr$ | 1-Pr-2-Bu-4-(3,4-deH-Pip) |
| 2-3065 | 4-F-Ph | $2-NH_2-4-Pyr$ | 1,2-diBu-4-(3,4-deH-Pip) |
| 2-3066 | 4-F-Ph | $2-NH_2-4-Pyr$ | 1-Phet-2-Bu-4-(3,4-deH-Pip) |
| 2-3067 | 4-F-Ph | $2-NH_2-4-Pyr$ | 2-Allyl-4-(3,4-deH-Pip) |
| 2-3068 | 4-F-Ph | $2-NH_2-4-Pyr$ | 1-Me-2-Allyl-4-(3,4-deH-Pip) |
| 2-3069 | 4-F-Ph | $2-NH_2-4-Pyr$ | 1-Et-2-Allyl-4-(3,4-deH-Pip) |
| 2-3070 | 4-F-Ph | $2-NH_2-4-Pyr$ | 1-Pr-2-Allyl-4-(3,4-deH-Pip) |
| 2-3071 | 4-F-Ph | $2-NH_2-4-Pyr$ | 1-Bu-2-Allyl-4-(3,4-deH-Pip) |
| 2-3072 | 4-F-Ph | $2-NH_2-4-Pyr$ | 1-Phet-2-Allyl-4-(3,4-deH-Pip) |
| 2-3073 | 4-F-Ph | $2-NH_2-4-Pyr$ | 2-Bn-4-(3,4-deH-Pip) |
| 2-3074 | 4-F-Ph | $2-NH_2-4-Pyr$ | 1-Me-2-Bn-4-(3,4-deH-Pip) |
| 2-3075 | 4-F-Ph | $2-NH_2-4-Pyr$ | 1-Et-2-Bn-4-(3,4-deH-Pip) |
| 2-3076 | 4-F-Ph | $2-NH_2-4-Pyr$ | 1-Pr-2-Bn-4-(3,4-deH-Pip) |
| 2-3077 | 4-F-Ph | $2-NH_2-4-Pyr$ | 1-Bu-2-Bn-4-(3,4-deH-Pip) |
| 2-3078 | 4-F-Ph | $2-NH_2-4-Pyr$ | 1-Phet-2-Bn-4-(3,4-deH-Pip) |

| Compound No. | $R^1$ | $R^2$ | $R^3$ |
|---|---|---|---|
| 2-3079 | 4-F-Ph | 2-$NH_2$-4-Pyr | 2-Phet-4-(3,4-deH-Pip) |
| 2-3080 | 4-F-Ph | 2-$NH_2$-4-Pyr | 1-Me-2-Phet-4-(3,4-deH-Pip) |
| 2-3081 | 4-F-Ph | 2-$NH_2$-4-Pyr | 1-Et-2-Phet-4-(3,4-deH-Pip) |
| 2-3082 | 4-F-Ph | 2-$NH_2$-4-Pyr | 1-Pr-2-Phet-4-(3,4-deH-Pip) |
| 2-3083 | 4-F-Ph | 2-$NH_2$-4-Pyr | 1-Bu-2-Phet-4-(3,4-deH-Pip) |
| 2-3084 | 4-F-Ph | 2-$NH_2$-4-Pyr | 1,2-diPhet-4-(3,4-deH-Pip) |
| 2-3085 | 4-F-Ph | 2-$NH_2$-4-Pyr | 2-Me-4-(4,5-deH-Pip) |
| 2-3086 | 4-F-Ph | 2-$NH_2$-4-Pyr | 1,2-diMe-4-(4,5-deH-Pip) |
| 2-3087 | 4-F-Ph | 2-$NH_2$-4-Pyr | 1-Et-2-Me-4-(4,5-deH-Pip) |
| 2-3088 | 4-F-Ph | 2-$NH_2$-4-Pyr | 1-Pr-2-Me-4-(4,5-deH-Pip) |
| 2-3089 | 4-F-Ph | 2-$NH_2$-4-Pyr | 1-Bu-2-Me-4-(4,5-deH-Pip) |
| 2-3090 | 4-F-Ph | 2-$NH_2$-4-Pyr | 1-Phet-2-Me-4-(4,5-deH-Pip) |
| 2-3091 | 4-F-Ph | 2-$NH_2$-4-Pyr | 2-Et-4-(4,5-deH-Pip) |
| 2-3092 | 4-F-Ph | 2-$NH_2$-4-Pyr | 1-Me-2-Et-4-(4,5-deH-Pip) |
| 2-3093 | 4-F-Ph | 2-$NH_2$-4-Pyr | 1,2-diEt-4-(4,5-deH-Pip) |
| 2-3094 | 4-F-Ph | 2-$NH_2$-4-Pyr | 1-Pr-2-Et-4-(4,5-deH-Pip) |
| 2-3095 | 4-F-Ph | 2-$NH_2$-4-Pyr | 1-Bu-2-Et-4-(4,5-deH-Pip) |
| 2-3096 | 4-F-Ph | 2-$NH_2$-4-Pyr | 1-Phet-2-Et-4-(4,5-deH-Pip) |
| 2-3097 | 4-F-Ph | 2-$NH_2$-4-Pyr | 2-Pr-4-(4,5-deH-Pip) |
| 2-3098 | 4-F-Ph | 2-$NH_2$-4-Pyr | 1-Me-2-Pr-4-(4,5-deH-Pip) |
| 2-3099 | 4-F-Ph | 2-$NH_2$-4-Pyr | 1-Et-2-Pr-4-(4,5-deH-Pip) |
| 2-3100 | 4-F-Ph | 2-$NH_2$-4-Pyr | 1,2-diPr-4-(4,5-deH-Pip) |
| 2-3101 | 4-F-Ph | 2-$NH_2$-4-Pyr | 1-Bu-2-Pr-4-(4,5-deH-Pip) |
| 2-3102 | 4-F-Ph | 2-$NH_2$-4-Pyr | 1-Phet-2-Pr-4-(4,5-deH-Pip) |
| 2-3103 | 4-F-Ph | 2-$NH_2$-4-Pyr | 2-Bu-4-(4,5-deH-Pip) |
| 2-3104 | 4-F-Ph | 2-$NH_2$-4-Pyr | 1-Me-2-Bu-4-(4,5-deH-Pip) |
| 2-3105 | 4-F-Ph | 2-$NH_2$-4-Pyr | 1-Et-2-Bu-4-(4,5-deH-Pip) |
| 2-3106 | 4-F-Ph | 2-$NH_2$-4-Pyr | 1-Pr-2-Bu-4-(4,5-deH-Pip) |
| 2-3107 | 4-F-Ph | 2-$NH_2$-4-Pyr | 1,2-diBu-4-(4,5-deH-Pip) |
| 2-3108 | 4-F-Ph | 2-$NH_2$-4-Pyr | 1-Phet-2-Bu-4-(4,5-deH-Pip) |

| Compound No. | $R^1$ | $R^2$ | $R^3$ |
|---|---|---|---|
| 2-3109 | 4-F-Ph | $2-NH_2-4-Pyr$ | 2-Allyl-4-(4,5-deH-Pip) |
| 2-3110 | 4-F-Ph | $2-NH_2-4-Pyr$ | 1-Me-2-Allyl-4-(4,5-deH-Pip) |
| 2-3111 | 4-F-Ph | $2-NH_2-4-Pyr$ | 1-Et-2-Allyl-4-(4,5-deH-Pip) |
| 2-3112 | 4-F-Ph | $2-NH_2-4-Pyr$ | 1-Pr-2-Allyl-4-(4,5-deH-Pip) |
| 2-3113 | 4-F-Ph | $2-NH_2-4-Pyr$ | 1-Bu-2-Allyl-4-(4,5-deH-Pip) |
| 2-3114 | 4-F-Ph | $2-NH_2-4-Pyr$ | 1-Phet-2-Allyl-4-(4,5-deH-Pip) |
| 2-3115 | 4-F-Ph | $2-NH_2-4-Pyr$ | 2-Bn-4-(4,5-deH-Pip) |
| 2-3116 | 4-F-Ph | $2-NH_2-4-Pyr$ | 1-Me-2-Bn-4-(4,5-deH-Pip) |
| 2-3117 | 4-F-Ph | $2-NH_2-4-Pyr$ | 1-Et-2-Bn-4-(4,5-deH-Pip) |
| 2-3118 | 4-F-Ph | $2-NH_2-4-Pyr$ | 1-Pr-2-Bn-4-(4,5-deH-Pip) |
| 2-3119 | 4-F-Ph | $2-NH_2-4-Pyr$ | 1-Bu-2-Bn-4-(4,5-deH-Pip) |
| 2-3120 | 4-F-Ph | $2-NH_2-4-Pyr$ | 1-Phet-2-Bn-4-(4,5-deH-Pip) |
| 2-3121 | 4-F-Ph | $2-NH_2-4-Pyr$ | 2-Phet-4-(4,5-deH-Pip) |
| 2-3122 | 4-F-Ph | $2-NH_2-4-Pyr$ | 1-Me-2-Phet-4-(4,5-deH-Pip) |
| 2-3123 | 4-F-Ph | $2-NH_2-4-Pyr$ | 1-Et-2-Phet-4-(4,5-deH-Pip) |
| 2-3124 | 4-F-Ph | $2-NH_2-4-Pyr$ | 1-Pr-2-Phet-4-(4,5-deH-Pip) |
| 2-3125 | 4-F-Ph | $2-NH_2-4-Pyr$ | 1-Bu-2-Phet-4-(4,5-deH-Pip) |
| 2-3126 | 4-F-Ph | $2-NH_2-4-Pyr$ | 1,2-diPhet-4-(4,5-deH-Pip) |
| 2-3131 | 4-F-Ph | 2-MeNH-4-Pyr | 3-(3,4-deH-Pip) |
| 2-3132 | 4-F-Ph | 2-MeNH-4-Pyr | 1-Me-3-(3,4-deH-Pip) |
| 2-3133 | 4-F-Ph | 2-MeNH-4-Pyr | 1-Et-4-(3,4-deH-Pip) |
| 2-3134 | 4-F-Ph | 2-MeNH-4-Pyr | 1-Pr-4-(3,4-deH-Pip) |
| 2-3136 | 4-F-Ph | 2-MeNH-4-Pyr | 1-iPr-4-(3,4-deH-Pip) |
| 2-3138 | 4-F-Ph | 2-MeNH-4-Pyr | 1-Bu-4-(3,4-deH-Pip) |

| Compound No. | $R^1$ | $R^2$ | $R^3$ |
|---|---|---|---|
| 2-3139 | 4-F-Ph | 2-MeNH-4-Pyr | 1-tBu-4-(3,4-deH-Pip) |
| 2-3140 | 4-F-Ph | 2-MeNH-4-Pyr | 1-Pn-4-(3,4-deH-Pip) |
| 2-3141 | 4-F-Ph | 2-MeNH-4-Pyr | 1-Hx-4-(3,4-deH-Pip) |
| 2-3142 | 4-F-Ph | 2-MeNH-4-Pyr | 1-Hp-4-(3,4-deH-Pip) |
| 2-3143 | 4-F-Ph | 2-MeNH-4-Pyr | 1-Oc-4-(3,4-deH-Pip) |
| 2-3144 | 4-F-Ph | 2-MeNH-4-Pyr | 1-Nn-4-(3,4-deH-Pip) |
| 2-3145 | 4-F-Ph | 2-MeNH-4-Pyr | 1-cPr-4-(3,4-deH-Pip) |
| 2-3146 | 4-F-Ph | 2-MeNH-4-Pyr | 1-cPn-4-(3,4-deH-Pip) |
| 2-3147 | 4-F-Ph | 2-MeNH-4-Pyr | 1-cHx-4-(3,4-deH-Pip) |
| 2-3148 | 4-F-Ph | 2-MeNH-4-Pyr | 1-Bn-4-(3,4-deH-Pip) |
| 2-3149 | 4-F-Ph | 2-MeNH-4-Pyr | 1-Phet-4-(3,4-deH-Pip) |
| 2-3150 | 4-F-Ph | 2-MeNH-4-Pyr | 1-(3-Ph-Pr)-4-(3,4-deH-Pip) |
| 2-3151 | 4-F-Ph | 2-MeNH-4-Pyr | 1-(4-Ph-Bu)-4-(3,4-deH-Pip) |
| 2-3152 | 4-F-Ph | 2-MeNH-4-Pyr | 1-Allyl-4-(3,4-deH-Pip) |
| 2-3153 | 4-F-Ph | 2-MeNH-4-Pyr | 1-Propargyl-4-(3,4-deH-Pip) |
| 2-3154 | 4-F-Ph | 2-MeNH-4-Pyr | 2,2,6,6-tetraMe-4-(3,4-deH-Pip) |
| 2-3155 | 4-F-Ph | 2-MeNH-4-Pyr | 1,2,2,6,6-pentaMe-4-(3,4-deH-Pip) |
| 2-3157 | 4-F-Ph | 2-MeNH-4-Pyr | 7-(1,2,3,5,6,8a-hexaH-Ind) |
| 2-3158 | 4-F-Ph | 2-MeNH-4-Pyr | 7-(1,2,3,5,8,8a-hexaH-Ind) |
| 2-3160 | 4-F-Ph | 2-MeNH-4-Pyr | 8-(1,3,4,6,7,9a-hexaH-2H-Qui) |
| 2-3161 | 4-F-Ph | 2-MeNH-4-Pyr | 8-(1,3,4,6,9,9a-hexaH-2H-Qui) |
| 2-3163 | 4-F-Ph | 2-MeNH-4-Pyr | 2,2-diMe-4-(3,4-deH-Pip) |
| 2-3164 | 4-F-Ph | 2-MeNH-4-Pyr | 1,2,2-triMe-4-(3,4-deH-Pip) |
| 2-3165 | 4-F-Ph | 2-MeNH-4-Pyr | 2,2-diMe-4-(4,5-deH-Pip) |
| 2-3166 | 4-F-Ph | 2-MeNH-4-Pyr | 1,2,2-triMe-4-(4,5-deH-Pip) |
| 2-3167 | 4-F-Ph | 2-MeNH-4-Pyr | 2,6-diMe-4-(3,4-deH-Pip) |

| Compound No. | $R^1$ | $R^2$ | $R^3$ |
|---|---|---|---|
| 2-3168 | 4-F-Ph | 2-MeNH-4-Pyr | 1,2,6-triMe-4-(3,4-deH-Pip) |
| 2-3169 | 4-F-Ph | 2-MeNH-4-Pyr | 2-Me-4-(3,4-deH-Pip) |
| 2-3170 | 4-F-Ph | 2-MeNH-4-Pyr | 1,2-diMe-4-(3,4-deH-Pip) |
| 2-3171 | 4-F-Ph | 2-MeNH-4-Pyr | 1-Et-2-Me-4-(3,4-deH-Pip) |
| 2-3172 | 4-F-Ph | 2-MeNH-4-Pyr | 1-Pr-2-Me-4-(3,4-deH-Pip) |
| 2-3173 | 4-F-Ph | 2-MeNH-4-Pyr | 1-Bu-2-Me-4-(3,4-deH-Pip) |
| 2-3174 | 4-F-Ph | 2-MeNH-4-Pyr | 1-Phet-2-Me-4-(3,4-deH-Pip) |
| 2-3175 | 4-F-Ph | 2-MeNH-4-Pyr | 2-Et-4-(3,4-deH-Pip) |
| 2-3176 | 4-F-Ph | 2-MeNH-4-Pyr | 1-Me-2-Et-4-(3,4-deH-Pip) |
| 2-3177 | 4-F-Ph | 2-MeNH-4-Pyr | 1,2-diEt-4-(3,4-deH-Pip) |
| 2-3178 | 4-F-Ph | 2-MeNH-4-Pyr | 1-Pr-2-Et-4-(3,4-deH-Pip) |
| 2-3179 | 4-F-Ph | 2-MeNH-4-Pyr | 1-Bu-2-Et-4-(3,4-deH-Pip) |
| 2-3180 | 4-F-Ph | 2-MeNH-4-Pyr | 1-Phet-2-Et-4-(3,4-deH-Pip) |
| 2-3181 | 4-F-Ph | 2-MeNH-4-Pyr | 2-Pr-4-(3,4-deH-Pip) |
| 2-3182 | 4-F-Ph | 2-MeNH-4-Pyr | 1-Me-2-Pr-4-(3,4-deH-Pip) |
| 2-3183 | 4-F-Ph | 2-MeNH-4-Pyr | 1-Et-2-Pr-4-(3,4-deH-Pip) |
| 2-3184 | 4-F-Ph | 2-MeNH-4-Pyr | 1,2-diPr-4-(3,4-deH-Pip) |
| 2-3185 | 4-F-Ph | 2-MeNH-4-Pyr | 1-Bu-2-Pr-4-(3,4-deH-Pip) |
| 2-3186 | 4-F-Ph | 2-MeNH-4-Pyr | 1-Phet-2-Pr-4-(3,4-deH-Pip) |
| 2-3187 | 4-F-Ph | 2-MeNH-4-Pyr | 2-Bu-4-(3,4-deH-Pip) |
| 2-3188 | 4-F-Ph | 2-MeNH-4-Pyr | 1-Me-2-Bu-4-(3,4-deH-Pip) |
| 2-3189 | 4-F-Ph | 2-MeNH-4-Pyr | 1-Et-2-Bu-4-(3,4-deH-Pip) |
| 2-3190 | 4-F-Ph | 2-MeNH-4-Pyr | 1-Pr-2-Bu-4-(3,4-deH-Pip) |
| 2-3191 | 4-F-Ph | 2-MeNH-4-Pyr | 1,2-diBu-4-(3,4-deH-Pip) |
| 2-3192 | 4-F-Ph | 2-MeNH-4-Pyr | 1-Phet-2-Bu-4-(3,4-deH-Pip) |
| 2-3193 | 4-F-Ph | 2-MeNH-4-Pyr | 2-Allyl-4-(3,4-deH-Pip) |
| 2-3194 | 4-F-Ph | 2-MeNH-4-Pyr | 1-Me-2-Allyl-4-(3,4-deH-Pip) |
| 2-3195 | 4-F-Ph | 2-MeNH-4-Pyr | 1-Et-2-Allyl-4-(3,4-deH-Pip) |
| 2-3196 | 4-F-Ph | 2-MeNH-4-Pyr | 1-Pr-2-Allyl-4-(3,4-deH-Pip) |

| Compound No. | $R^1$ | $R^2$ | $R^3$ |
|---|---|---|---|
| 2-3197 | 4-F-Ph | 2-MeNH-4-Pyr | 1-Bu-2-Allyl-4-(3,4-deH-Pip) |
| 2-3198 | 4-F-Ph | 2-MeNH-4-Pyr | 1-Phet-2-Allyl-4-(3,4-deH-Pip) |
| 2-3199 | 4-F-Ph | 2-MeNH-4-Pyr | 2-Bn-4-(3,4-deH-Pip) |
| 2-3200 | 4-F-Ph | 2-MeNH-4-Pyr | 1-Me-2-Bn-4-(3,4-deH-Pip) |
| 2-3201 | 4-F-Ph | 2-MeNH-4-Pyr | 1-Et-2-Bn-4-(3,4-deH-Pip) |
| 2-3202 | 4-F-Ph | 2-MeNH-4-Pyr | 1-Pr-2-Bn-4-(3,4-deH-Pip) |
| 2-3203 | 4-F-Ph | 2-MeNH-4-Pyr | 1-Bu-2-Bn-4-(3,4-deH-Pip) |
| 2-3204 | 4-F-Ph | 2-MeNH-4-Pyr | 1-Phet-2-Bn-4-(3,4-deH-Pip) |
| 2-3205 | 4-F-Ph | 2-MeNH-4-Pyr | 2-Phet-4-(3,4-deH-Pip) |
| 2-3206 | 4-F-Ph | 2-MeNH-4-Pyr | 1-Me-2-Phet-4-(3,4-deH-Pip) |
| 2-3207 | 4-F-Ph | 2-MeNH-4-Pyr | 1-Et-2-Phet-4-(3,4-deH-Pip) |
| 2-3208 | 4-F-Ph | 2-MeNH-4-Pyr | 1-Pr-2-Phet-4-(3,4-deH-Pip) |
| 2-3209 | 4-F-Ph | 2-MeNH-4-Pyr | 1-Bu-2-Phet-4-(3,4-deH-Pip) |
| 2-3210 | 4-F-Ph | 2-MeNH-4-Pyr | 1,2-diPhet-4-(3,4-deH-Pip) |
| 2-3211 | 4-F-Ph | 2-MeNH-4-Pyr | 2-Me-4-(4,5-deH-Pip) |
| 2-3212 | 4-F-Ph | 2-MeNH-4-Pyr | 1,2-diMe-4-(4,5-deH-Pip) |
| 2-3213 | 4-F-Ph | 2-MeNH-4-Pyr | 1-Et-2-Me-4-(4,5-deH-Pip) |
| 2-3214 | 4-F-Ph | 2-MeNH-4-Pyr | 1-Pr-2-Me-4-(4,5-deH-Pip) |
| 2-3215 | 4-F-Ph | 2-MeNH-4-Pyr | 1-Bu-2-Me-4-(4,5-deH-Pip) |
| 2-3216 | 4-F-Ph | 2-MeNH-4-Pyr | 1-Phet-2-Me-4-(4,5-deH-Pip) |
| 2-3217 | 4-F-Ph | 2-MeNH-4-Pyr | 2-Et-4-(4,5-deH-Pip) |
| 2-3218 | 4-F-Ph | 2-MeNH-4-Pyr | 1-Me-2-Et-4-(4,5-deH-Pip) |
| 2-3219 | 4-F-Ph | 2-MeNH-4-Pyr | 1,2-diEt-4-(4,5-deH-Pip) |
| 2-3220 | 4-F-Ph | 2-MeNH-4-Pyr | 1-Pr-2-Et-4-(4,5-deH-Pip) |
| 2-3221 | 4-F-Ph | 2-MeNH-4-Pyr | 1-Bu-2-Et-4-(4,5-deH-Pip) |
| 2-3222 | 4-F-Ph | 2-MeNH-4-Pyr | 1-Phet-2-Et-4-(4,5-deH-Pip) |
| 2-3223 | 4-F-Ph | 2-MeNH-4-Pyr | 2-Pr-4-(4,5-deH-Pip) |
| 2-3224 | 4-F-Ph | 2-MeNH-4-Pyr | 1-Me-2-Pr-4-(4,5-deH-Pip) |
| 2-3225 | 4-F-Ph | 2-MeNH-4-Pyr | 1-Et-2-Pr-4-(4,5-deH-Pip) |
| 2-3226 | 4-F-Ph | 2-MeNH-4-Pyr | 1,2-diPr-4-(4,5-deH-Pip) |

| Compound No. | $R^1$ | $R^2$ | $R^3$ |
|---|---|---|---|
| 2-3227 | 4-F-Ph | 2-MeNH-4-Pyr | 1-Bu-2-Pr-4-(4,5-deH-Pip) |
| 2-3228 | 4-F-Ph | 2-MeNH-4-Pyr | 1-Phet-2-Pr-4-(4,5-deH-Pip) |
| 2-3229 | 4-F-Ph | 2-MeNH-4-Pyr | 2-Bu-4-(4,5-deH-Pip) |
| 2-3230 | 4-F-Ph | 2-MeNH-4-Pyr | 1-Me-2-Bu-4-(4,5-deH-Pip) |
| 2-3231 | 4-F-Ph | 2-MeNH-4-Pyr | 1-Et-2-Bu-4-(4,5-deH-Pip) |
| 2-3232 | 4-F-Ph | 2-MeNH-4-Pyr | 1-Pr-2-Bu-4-(4,5-deH-Pip) |
| 2-3233 | 4-F-Ph | 2-MeNH-4-Pyr | 1,2-diBu-4-(4,5-deH-Pip) |
| 2-3234 | 4-F-Ph | 2-MeNH-4-Pyr | 1-Phet-2-Bu-4-(4,5-deH-Pip) |
| 2-3235 | 4-F-Ph | 2-MeNH-4-Pyr | 2-Allyl-4-(4,5-deH-Pip) |
| 2-3236 | 4-F-Ph | 2-MeNH-4-Pyr | 1-Me-2-Allyl-4-(4,5-deH-Pip) |
| 2-3237 | 4-F-Ph | 2-MeNH-4-Pyr | 1-Et-2-Allyl-4-(4,5-deH-Pip) |
| 2-3238 | 4-F-Ph | 2-MeNH-4-Pyr | 1-Pr-2-Allyl-4-(4,5-deH-Pip) |
| 2-3239 | 4-F-Ph | 2-MeNH-4-Pyr | 1-Bu-2-Allyl-4-(4,5-deH-Pip) |
| 2-3240 | 4-F-Ph | 2-MeNH-4-Pyr | 1-Phet-2-Allyl-4-(4,5-deH-Pip) |
| 2-3241 | 4-F-Ph | 2-MeNH-4-Pyr | 2-Bn-4-(4,5-deH-Pip) |
| 2-3242 | 4-F-Ph | 2-MeNH-4-Pyr | 1-Me-2-Bn-4-(4,5-deH-Pip) |
| 2-3243 | 4-F-Ph | 2-MeNH-4-Pyr | 1-Et-2-Bn-4-(4,5-deH-Pip) |
| 2-3244 | 4-F-Ph | 2-MeNH-4-Pyr | 1-Pr-2-Bn-4-(4,5-deH-Pip) |
| 2-3245 | 4-F-Ph | 2-MeNH-4-Pyr | 1-Bu-2-Bn-4-(4,5-deH-Pip) |
| 2-3246 | 4-F-Ph | 2-MeNH-4-Pyr | 1-Phet-2-Bn-4-(4,5-deH-Pip) |
| 2-3247 | 4-F-Ph | 2-MeNH-4-Pyr | 2-Phet-4-(4,5-deH-Pip) |
| 2-3248 | 4-F-Ph | 2-MeNH-4-Pyr | 1-Me-2-Phet-4-(4,5-deH-Pip) |
| 2-3249 | 4-F-Ph | 2-MeNH-4-Pyr | 1-Et-2-Phet-4-(4,5-deH-Pip) |
| 2-3250 | 4-F-Ph | 2-MeNH-4-Pyr | 1-Pr-2-Phet-4-(4,5-deH-Pip) |
| 2-3251 | 4-F-Ph | 2-MeNH-4-Pyr | 1-Bu-2-Phet-4-(4,5-deH-Pip) |
| 2-3252 | 4-F-Ph | 2-MeNH-4-Pyr | 1,2-diPhet-4-(4,5-deH-Pip) |
| 2-3253 | Ph | 4-Pyr | 7-(1,2,3,5,6,8a-hexaH-Ind) |
| 2-3254 | Ph | 4-Pyr | 7-(1,2,3,5,8,8a-hexaH-Ind) |
| 2-3255 | 3-F-Ph | 4-Pyr | 7-(1,2,3,5,6,8a-hexaH-Ind) |
| 2-3256 | 3-F-Ph | 4-Pyr | 7-(1,2,3,5,8,8a-hexaH-Ind) |

| Compound No. | $R^1$ | $R^2$ | $R^3$ |
|---|---|---|---|
| 2-3257 | 3-Cl-Ph | 4-Pyr | 7-(1,2,3,5,6,8a-hexaH-Ind) |
| 2-3258 | 3-Cl-Ph | 4-Pyr | 7-(1,2,3,5,8,8a-hexaH-Ind) |
| 2-3259 | 3,4-diF-Ph | 4-Pyr | 7-(1,2,3,5,6,8a-hexaH-Ind) |
| 2-3260 | 3,4-diF-Ph | 4-Pyr | 7-(1,2,3,5,8,8a-hexaH-Ind) |
| 2-3261 | $3-CF_3-Ph$ | 4-Pyr | 7-(1,2,3,5,6,8a-hexaH-Ind) |
| 2-3262 | $3-CF_3-Ph$ | 4-Pyr | 7-(1,2,3,5,8,8a-hexaH-Ind) |

| Compound No. | $R^1$ | $R^2$ | $R^3$ |
|---|---|---|---|
| 2-3312 | 4-F-Ph | 4-Pyr | 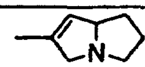 |
| 2-3313 | 4-F-Ph | 4-Pyr | 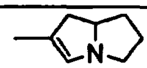 |

| Compound No. | $R^1$ | $R^2$ | $R^3$ |
|---|---|---|---|
| 2-3315 | 4-F-Ph | 4-Pyr | 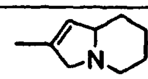 |
| 2-3316 | 4-F-Ph | 4-Pyr | 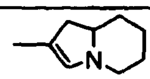 |
| 2-3318 | 4-F-Ph | 4-Pyr | 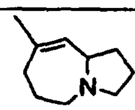 |
| 2-3319 | 4-F-Ph | 4-Pyr | 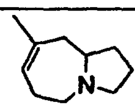 |
| 2-3321 | 4-F-Ph | 4-Pyr | 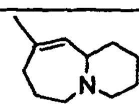 |
| 2-3322 | 4-F-Ph | 4-Pyr | 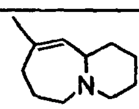 |

Of the above compounds in Tables 1 and 2, the following are preferred, that is to say Compounds Nos.: 1-49, 1-51, 1-143, 1-145, 1-236, 1-238, 1-329, 1-331, 1-422, 1-424, 1-515, 1-517, 1-608, 1-610, 1-701, 1-703, 1-794, 1-796, 1-887, 1-889, 1-980, 1-982, 1-1073, 1-1075, 1-1166, 1-1168, 1-1259, 1-1261, 1-1352, 1-1354, 1-1413, 1-1415, 1-1448, 1-1450, 1-1483,1-1485, 1-1518, 1-1520, 1-1553, 1-1555, 1-1588, 1-1590, 1-1623, 1-1625, 1-1658, 1-1660, 1-1693, 1-1695, 1-1728, 1-1?30, 1-1763, 1-1765, 1-1798, 1-1800, 1-1833, 1-1835, 1-1868, 1-1870, 1-1903, 1-1905, 1-1938, 1-1940, 1-1973, 1-1975, 1-2008, 1-2010, 1-2043, 1-2045, 1-2078, 1-2080, 1-2113, 1-2115, 1-2148, 1-2150, 1-2183, 1-2185, 1-2218, 1-2220, 1-2253, 1-2255, 1-2288, 1-2290, 1-2323, 1-2325, 1-2358, 1-2360, 1-2393, 1-2395, 1-2428, 1-2430, 1-2463, 1-2465, 1-2498, 1-2500, 1-2525, 1-2557, 1-2580, 1-2627 to 1-2630, 1-2632,1-2634 to 1-2651, 1-2653, 1-2654, 1-2656, 1-2657, 1-2659 to 1-2748, 1-2753 to 1-2756, 1-2758, 1-2760 to 1-2777, 1-2779, 1-2780, 1-2782, 1-2783,1-2785 to 1-2874, 1-2879 to 1-2882, 1-2884, 1-2886 to 1-2903, 1-2905, 1-2906, 1-2908, 1-2909, 1-2911 to 1-3000, 1-3005 to 1-3008, 1-3010, 1-3012 to 1-3029, 1-3031, 1-3032, 1-3034, 1-3035, 1-3037 to 1-3126, 1-3131 to 1-3134, 1-3136, 1-3138 to 1-3155, 1-3157, 1-3158, 1-3160, 1-3161, 1-3163 to 1-3262, 1-3312, 1-3313, 1-3315, 1-3316, 1-3318, 1-3319, 1-3321, 1-3322, 2-49, 2-51, 2-143, 2-145, 2-236, 2-238, 2-329, 2-331, 2-422, 2-424, 2-515, 2-517, 2-608, 2-610, 2-701, 2-703, 2-794, 2-796, 2-887, 2-889, 2-980, 2-982, 2-1073, 2-1075, 2-1166, 2-1168, 2-1259, 2-1261, 2-1352, 2-1354, 2-1413, 2-1415, 2-1448, 2-1450, 2-1483, 2-1485, 2-1518, 2-1520, 2-1553, 2-1555, 2-1588, 2-1590, 2-1623, 2-1625, 2-1658, 2-1660, 2-1693, 2-1695, 2-1728, 2-1730, 2-1763, 2-1765, 2-1798, 2-1800, 2-1833, 2-1835, 2-1868, 2-1870, 2-1903, 2-1905, 2-1938, 2-1940, 2-1973, 2-1975, 2-2008, 2-2010, 2-2043, 2-2045, 2-2078, 2-2080, 2-2113, 2-2115, 2-2148, 2-2150, 2-2183, 2-2185, 2-2218, 2-2220, 2-2253, 2-2255, 2-2288, 2-2290, 2-2323, 2-2325, 2-2358, 2-2360, 2-2393, 2-2395, 2-2428, 2-2430, 2-2463, 2-2465, 2-2498, 2-2500, 2-2525, 2-255.7, 2-2580, 2-2627 to 2-2630, 2-2632, 2-2634 to 2-2651, 2-2653, 2-2654, 2-2656, 2-2657, 2-2659 to 2-2748, 2-2753 to 2-2756, 2-2758, 2-2760 to 2-2777, 2-2779, 2-2780, 2-2782, 2-2783, 2-2785 to 2-2874, 2-2879 to 2-2882, 2-2884, 2-2886 to 2-2903, 2-2905, 2-2906, 2-2908, 2-2909, 2-2911 to 2-3000, 2-3005 to 2-3008, 2-3010, 2-3012 to 2-3029, 2-3031, 2-3032, 2-3034, 2-3035, 2-3037 to 2-3126, 2-3131 to 2-3134, 2-3136, 2-3138 to 2-3155, 2-3157, 2-3158, 2-3160, 2-3161, 2-3163 to 2-3262, 2-3312, 2-3313, 2-3315, 2-3316, 2-3318, 2-3319, 2-3321 and 2-3322,
more preferred are Compounds Nos.: 1-49, 1-51, 1-143, 1-145, 1-236, 1-238, 1-329, 1-331, 1-422, 1-424, 1-515, 1-517, 1-608, 1-610, 1-701, 1-703, 1-794, 1-796, 1-887, 1-889, 1-980, 1-982, 1-1073, 1-1075, 1-1166, 1-1168, 1-1259, 1-1261, 1-1352, 1-1354, 1-1413, 1-1415, 1-1448, 1-1450, 1-1483, 1-1485, 1-1518, 1-1520, 1-1553, 1-1555, 1-1588, 1-1590, 1-1623, 1-1625, 1-1658, 1-1660, 1-1693, 1-1695, 1-1728, 1-1730, 1-1763, 1-1765, 1-1798, 1-1800, 1-1833, 1-1835, 1-1868, 1-1870, 1-1903, 1-1905, 1-1938, 1-1940, 1-1973, 1-1975, 1-2008, 1-2010, 1-2043, 1-2045,. 1-2078, 1-2080, 1-2113, 1-2115, 1-2148, 1-2150, 1-2183, 1-2185, 1-2218, 1-2220, 1-2253, 1-2255, 1-2288, 1-2290, 1-2323, 1-2325, 1-2358, 1-2360, 1-2393, 1-2395, 1-2428, 1-2430, 1-2463, 1-2465, 1-2498, 1-2500, 1-2525, 1-2557, 1-2580, 1-2629 to 1-2630, 1-2632, 1-2634 to 1-2643, 1-2645, 1-2648 to 1-2651, 1-2653, 1-2654, 1-2656, 1-2657, 1-2659 to 1-2748, 1-2779, 1-2780, 1-2782, 1-2783, 1-2905, 1-2906, 1-2908, 1-2909, 1-3031, 1-3032, 1-3157, 1-3158, 1-3253, 1-3254 to 1-3262, 1-3312, 1-3313, 1-3315, 1-3316, 1-3318, 1-3319, 1-3321, 1-3322, 2-49, 2-51, 2-143, 2-145, 2-236, 2-238, 2-329, 2-331, 2-422, 2-424, 2-515, 2-517, 2-608, 2-610, 2-701, 2-703, 2-794, 2-796, 2-887, 2-889, 2-980, 2-982, 2-1073, 2-1075, 2-1166, 2-1168, 2-1259, 2-1261, 2-1352, 2-1354, 2-1413, 2-1415, 2-1448, 2-1450, 2-1483, 2-1485, 2-1518, 2-1520, 2-1553, 2-1555, 2-1588, 2-1590, 2-1623, 2-1625, 2-1658, 2-1660, 2-1693, 2-1695, 2-1728, 2-1730, 2-1763, 2-1765, 2-1798, 2-1800, 2-1833, 2-1835, 2-1868, 2-1870, 2-1903, 2-1905, 2-1938, 2-1940, 2-1973, 2-1975, 2-2008, 2-2010, 2-2043, 2-2045, 2-2078, 2-2080, 2-2113, 2-2115, 2-2148, 2-2150, 2-2183, 2-2185, 2-2218, 2-2220, 2-2253 2-2255, 2-2288, 2-2290, 2-2323, 2-2325, 2-2358, 2-2360, 2-2393, 2-2395, 2-2428, 2-2430, 2-2463, 2-2465, 2-2498, 2-2500, 2-2525, 2-2557, 2-2580. 2-3253, 2-3254 to 2-3262, 2-3312, 2-3313, 2-3315, 2-3316, 2-3318, 2-3319, 2-3321 and 2-3322;
still more preferred are Compounds Nos.: 1-49, 1-51, 1-143, 1-145, 1-236, 1-238, 1-329, 1-331, 1-422, 1-424, 1-515, 1-517, 1-608, 1-610, 1-701, 1-703, 1-794, 1-796, 1-887, 1-889, 1-980, 1-982, 1-1073, 1-1075, 1-1166, 1-1168, 1-1259, 1-1261, 1-1352, 1-1354, 1-1413, 1-1415, 1-1448, 1-1450, 1-1483, 1-1485, 1-1518, 1-1520, 1-1553, 1-1555, 1-5588, 1-1590, 1-1623, 1-1625, 1-1658, 1-1660, 1-1693, 1-1695, 1-1728, 1-1730, 1-1763, 1-1765, 1-1798, 1-1800, 1-1833, 1-1835, 1-1868, 1-1870, 1-1903, 1-1905, 1-1938, 1-1940, 1-1973, 1-1975, 1-2008, 1-2010, 1-2043, 1-2045, 1-2078, 1-2080, 1-2113, 1-2115, 1-2148, 1-2150, 1-2183,1-2185, 1-2218, 1-2220, 1-2253, 1-2255, 1-2288, 1-2290, 1-2323, 1-2325, 1-2358, 1-2360, 1-2393, 1-2395, 1-2428, 1-2430, 1-2463, 1-2465, 1-2498, 1-2500, 1-2525, 1-2557, 1-2580, 1-2629, 1-2630, 1-2632, 1-2645, 1-2650, 1-2651, 1-2653, 1-2654, 1-2656, 1-2657, 1-2665 to 1-2667, 1-2707 to 1-2709, 1-2779, 1-2780, 1-2782, 1-2783, 1-2905, 1-2906, 1-2908, 1-2909, 1-3031, 1-3032, 1-3157, 1-3158, 1-3253 to 1-3262, 1-3312, 1-3315, 1-3316, 1-3318, 1-3319, 1-3321, 1-3322, 2-49, 2-51, 2-143, 2-145, 2-236, 2-238, 2-329, 2-331, 2-422, 2-424, 2-515, 2-517, 2-608, 2-610, 2-701, 2-703, 2-794, 2-796, 2-887, 2-889, 2-980, 2-982, 2-1073, 2-1075, 2-1166, 2-1168, 2-1259, 2-1261, 2-1352, 2-1354, 2-1413, 2-1415, 2-1448, 2-1450, 2-1483, 2-1485, 2-1518, 2-1520, 2-1553, 2-1555, 2-1588, 2-1590, 2-1623, 2-1625, 2-1658, 2-1660, 2-1693, 2-1695, 2-1728, 2-1730, 2-1763, 2-1765, 2-1798, 2-1800, 2-1833, 2-1835, 2-1868, 2-1870, 2-1903, 2-1905, 2-1938, 2-1940, 2-1973, 2-1975, 2-2008, 2-2010, 2-2043, 2-2045, 2-2078, 2-2080, 2-2113, 2-2115,

2-2148, 2-2150, 2-2183, 2-2185, 2-2218, 2-2220, 2-2253, 2-2255, 2-2288, 2-2290, 2-2323, 2-2325, 2-2358, 2-2360, 2-2393, 2-2395, 2-2428, 2-2430, 2-2463, 2-2465, 2-2498, 2-2500, 2-2525, 2-2557, 2-2580, 2-3253 to 2-3262, 2-3312, 2-3313, 2-3315, 2-3316, 2-3318, 2-3319, 2-3321 and 2-3322.
The most preferred compounds are:

4-(1-acetyl-1,2,3,6-tetrahydropyridin-4-yl)-2-(4-fluorophenyl)-3-(pyridin-4-yl)-1*H*-pyrrole,
2-(4-fluorophenyl)-3-(pyridin-4-yl)-4-(1,2,3,6-tetrahydropyridin-4-yl)-1*H*-pyrrole,
2-(4-fluorophenyl)-4-(1-methyl-1,2,3,6-tetrahydropyridin-4-yl)-3-pyridin-4-yl)-1*H*-pyrrole,
2-(4-fluorophenyl)-3-(pyridin-4-yl)-4-(1,2,3,6-tetrahydro-2,2,6,6-tetramethylpyridin-4-yl)-1*H*-pyrrole,
2-(4-fluorophenyl)-4-(2-methyl-1,2,3,6-tetrahydropyridin-4-yl)-3-(pyridin-4-yl)-1*H*-pyrrole,
2-(4-fluorophenyl)-4-(6-methyl-1,2,3,6-tetrahydropyridin-4-yl)-3-(pyridin-4-yl)-1*H*-pyrrole,
4-(1-ethyl-1,2,3,6-tetrahydropyridin-4-yl)-2-(4-fluorophenyl)-3-(pyridin-4-yl)-1*H*-pyrrole,
2-(4-fluorophenyl)-4-(1-isopropyl-1,2,3,6-tetrahydropyridin-4-yl)-3-(pyridin-4-yl)-1*H*-pyrrole,
2-(4-fluorophenyl)-4-(1-propyl-1,2,3,6-tetrahydropyridin-4-yl)-3-(pyridin-4-yl)-1*H*-pyrrole,
2-(4-fluorophenyl)-4-(1-phenethyl-1,2,3,6-tetrahydropyridin-4-yl)-3-(pyridin-4-yl)-1*H*-pyrrole,
2-(4-fluorophenyl)-4-(1,2,3,5,8,8a-hexahydroindolizin-7-yl)-3-(pyridin-4-yl)-1*H*-pyrrole,
2-(4-fluorophenyl)-4-(1,2,3,5,6,8a-hexahydroindolizin-7-yl)-3-(pyridin-4-yl)-1*H*-pyrrole,
4-(1,6-dimethyl-1,2,3,6-tetrahydropyridin-4-yl)-2-(4-fluorophenyl)-3-(pyridin-4-yl)-1*H*-pyrrole,
4-(1,2-dimethyl-1,2,3,6-tetrahydropyridin-4-yl)-2-(4-fluorophenyl)-3-(pyridin-4-yl)-1*H*-pyrrole,
2-(4-fluorophenyl)-4-(1,2,2,6,6-pentamethyl-1,2,3,6-tetrahydropyridin-4-yl)-3-(pyridin-4-yl)-1*H*-pyrrole,
4-(1-ethyl-6-methyl-1,2,3,6-tetrahydropyridin-4-yl)-2-(4-fluorophenyl)-3-(pyridin-4-yl)-1*H*-pyrrole,
4-(1-ethyl-2-methyl-1,2,3,6-tetrahydropyridin-4-yl)-2-(4-fluorophenyl)-3-(pyridin-4-yl)-1*H*-pyrrole,
4-(6-allyl-1-methyl-1,2,3,6-tetrahydropyridin-4-yl)-2-(4-fluorophenyl)-3-(pyridin-4-yl)-1*H*-pyrrole,
4-(2-allyl-1-methyl-1,2,3,6-tetrahydropyridin-4-yl)-2-(4-fluorophenyl)-3-(pyridin-4-yl)-1*H*-pyrrole,
4-(6-benzyl-1-methyl-1,2,3,6-tetrahydropyridin-4-yl)-2-(4-fluorophenyl)-3-(pyridin-4-yl)-1*H*-pyrrole,
4-(2-benzyl-1-methyl-1,2,3,6-tetrahydropyridin-4-yl)-2-(4-fluorophenyl)-3-(pyridin-4-yl)-1*H*-pyrrole,
2-(4-fluorophenyl)-4-(6-methyl-1-propyl-1,2,3,6-tetrahydropyridin-4-yl)-3-(pyridin-4-yl)-1*H*-pyrrole,
2-(4-fluorophenyl)-4-(2-methyl-1-propyl-1,2,3,6-tetrahydropyridin-4-yl)-3-(pyridin-4-yl)-1*H*-pyrrole, 2-(4-fluorophenyl)-4-(1,3,4,6,7,9a-hexahydro-2*H*-quinolizin-8-yl)-3-(pyridin-4-yl)-1*H*-pyrrole,
2-(4-fluorophenyl)-4-(1,3,4,6,9,9a-hexahydro-2*H*-quinolizin-8-yl)-3-(pyridin-4-yl)-1*H*-pyrrole,
2-(4-fluorophenyl)-4-(1,2,3,5,8,8a-hexahydroindolizin-7-yl)-3-(2-methylaminopyrimidin-4-yl)-1*H*-pyrrole,
2-(4-fluorophenyl)-4-(1,2,3,5,6,8a-hexahydroindolizin-7-yl)-3-(2-methylaminopyrimidin-4-yl)-1*H*-pyrrole,
2-(3,4-difluorophenyl)-4-(1,2,3,5,8,8a-hexahydroindolizin-7-yl)-3-(pyridin-4-yl)-1*H*-pyrrole,
2-(3,4-difluorophenyl)-4-(1,2,3,5,6,8a-hexahydroindolizin-7-yl)-3-(pyridin-4-yl)-1*H*-pyrrole,
4-(1,2,3,5,8,8a-hexahydroindolizin-7-yl)-3-(pyridin-4-yl)-2-(3-trifluoromethylphenyl)-1*H*-pyrrole,
4-(1,2,3,5,6,8a-hexahydroindolizin-7-yl)-3-(pyridin-4-yl)-2-(3-trifluoromethylphenyl)-1*H*-pyrrole,
2-(3-fluorophenyl)-4-(1,2,3,5,8,8a-hexahydroindolizin-7-yl)-3-(pyridin-4-yl)-1*H*-pyrrole,
2-(3-fluorophenyl)-4-(1,2,3,5,6,8a-hexahydroindolizin-7-yl)-3-(pyridin-4-yl)-1*H*-pyrrole,
2-(3-chloraphenyl)-4-(1,2,3,5,8,8a-hexahydroindolizin-7-yl)-3-(pyridin-4-yl)-1*H*-pyrrole,
2-(3-chlorophenyl)-4-(1,2,3,5,6,8a-hexahydroindolizin-7-yl)-3-(pyridin-4-yl)-1*H*-pyrrole,
4-(1,2,3,5,8,8a-hexahydroindolizin-7-yl)-2-phenyl-3-(pyridin-4-yl)-1*H*-pyrrole, and
4-(1,2,3,5,6,8a-hexahydroindolizin-7-yl)-2-phenyl-3-(pyridin-4-yl)-1*H*-pyrrole;

and pharmacologically acceptable salts, esters or other derivatives thereof.
It should be noted that the specific combination of substituents $R^1$, $R^2$ and $R^3$ shown in the above Tables 1 and 2 can also be applied to the substituents $R^1$, $R^2$ and $R^3$ in formulae (I-2), (I-4) and (I-5) above.
The compounds of formula (I) and pharmacologically acceptable salts, esters and other derivatives thereof of the present invention can be prepared according to a variety of processes well known in the art for the preparation of compounds of this type, for example as described in the following Methods A to H, J, L and M below.

## Method A

Method A is a method to prepare compounds of formula (I-1) of the present invention.

**Reaction Scheme A**

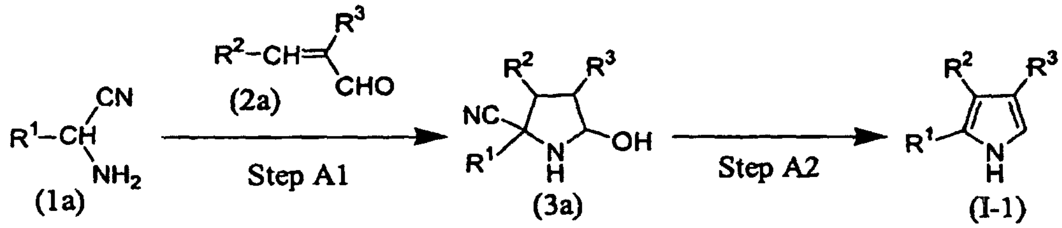


(1a) (3a) (I-1)

In the above formulae, $R^1$, $R^2$ and $R^3$ are as defined above.

**Step A1**

In this Step, a pyrrolidine compound of formula (3a) is prepared by reacting an aminonitrile compound of formula (1a) with an α,β-unsaturated aldehyde compound of formula (2a). This reaction is well known in the field of synthetic organic chemistry and can be carried out using well known techniques, for example according to the method described in EP 0733823, the contents of which are incorporated herein by reference thereto.

In more detail, this Step is carried out in the presence of a base. There is no particular restriction on the nature of the bases used, and any base commonly used in reactions of this type may equally be used here. Examples of such bases include: alkali metal hydroxides, such as lithium hydroxide, sodium hydroxide and potassium hydroxide; alkali metal hydrides, such as lithium hydride, sodium hydride and potassium hydride; alkali metal amides, such as lithium amide, sodium amide, potassium amide and lithium bis(trimethylsilyl)amide; and alkali metal alkoxides, such as lithium ethoxide, sodium methoxide, sodium ethoxide and potassium t-butoxide. Of these, we prefer the lithium amides.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: aliphatic hydrocarbons, such as hexane and heptane; aromatic hydrocarbons, such as benzene, toluene and xylene; ethers, such as diethyl ether, diisopropyl ether, tetrahydrofuran and dioxane; and alcohols, such as methanol, ethanol, propanol, isopropanol and butanol. Of these, we prefer the ethers.

The reaction can take place over a wide range of temperatures, and the precise reaction temperature is not critical to the invention. The preferred reaction temperature will depend upon such factors as the nature of the solvent, and the starting material or reagent used. However, in general, we find it convenient to carry out the reaction at a temperature of from -78°C to 100°C, more preferably from -78°C to room temperature. The time required for the reaction may also vary widely, depending on many factors, notably the reaction temperature and the nature of the reagents and solvent employed. However, provided that the reaction is effected under the preferred conditions outlined above, a period of from 10 minutes to 30 hours, more preferably from 1 hour to 20 hours, will usually suffice.

**Step A2**

In this Step, the desired pyrrole derivative of formula (I-1) of the present invention is prepared by the elimination of hydrogen cyanide and water from the compound of formula (3a) prepared in Step A1 above. These reactions are well known in the field of synthetic organic chemitry and can be carried out using well known techniques, for example according to the methods described in detail in EP 0733823.

In more detail, this may be achieved by heating the residue obtained by distilling off the solvent from the product of Step A1, or by heating the material obtained by extracting that residue, washing it with water and distilling off the solvent, preferably at a temperature not lower than 100°C, in the presence or absence of a solvent after completion of the reaction of Step A1. The reaction proceeds sufficiently in the absence of a solvent, but, when a solvent is used, the solvent is preferably inert and has a higher boiling point. Examples of suitable solvents include: toluene, xylene, dimethylformamide, dimethylacetamide, dimethyl sulfoxide, diglyme and diphenyl ether.

**Method B, Method C, Method D and Method E**

Method B, Method C, Method D and Method E are used to prepare compounds of formulae (I-2), (I-3), (I-4)

and (I-5) respectively using the processes outlined in Reaction Schemes B to E below.

**Reaction Scheme B**

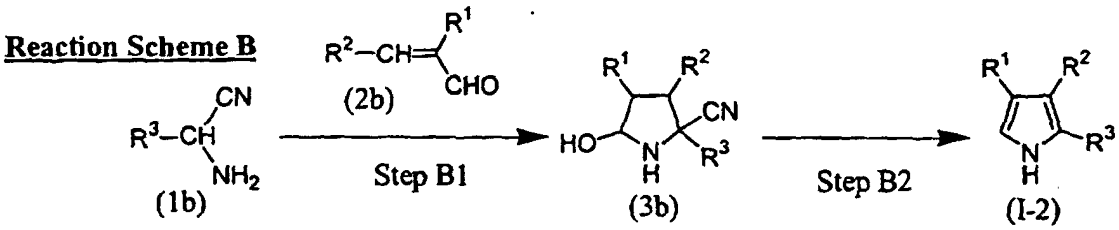

(1b) (3b) (I-2)

**Reaction Scheme C**

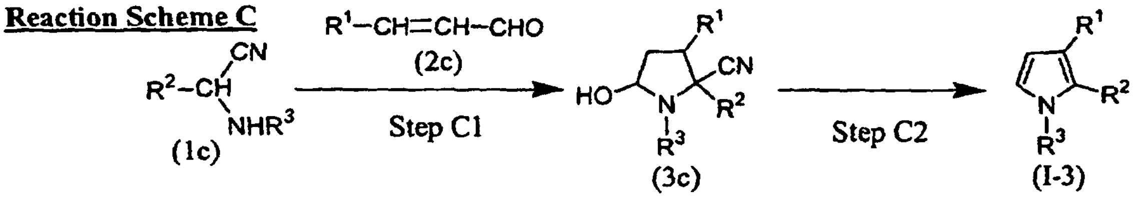

(1c) (3c) (I-3)

**Reaction Scheme D**

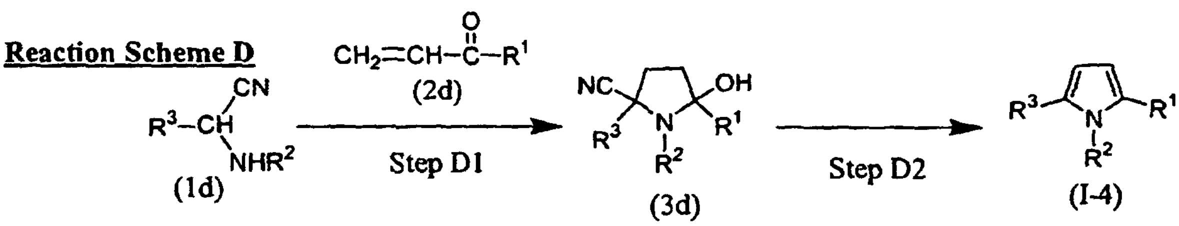

(1d) (3d) (I-4)

**Reaction Scheme E**

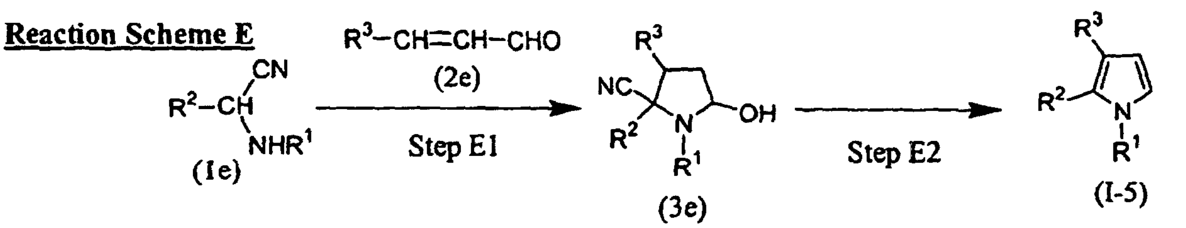

(1e) (3e) (I-5)

In the above formulae, $R^1$, $R^2$ and $R^3$ are as defined above.

In the above Reaction Schemes Step B1, Step C1, Step D1 and Step E1 are carried out in a similar manner to Step A1 above, and Step B2, Step C2, Step D2 and Step E2 are carried out similarly to Step A2 above.

**Method F**

In this method, compounds of formula (I-1) of the present invention wherein $R^3$ is an aminomethyl, aminoethyl, aminopropyl or aminopropenyl group [(I-1a), (I-1b), (I-1c) and (I-1d) respectively] are prepared as shown in Reaction Scheme F.

**Reaction Scheme F**

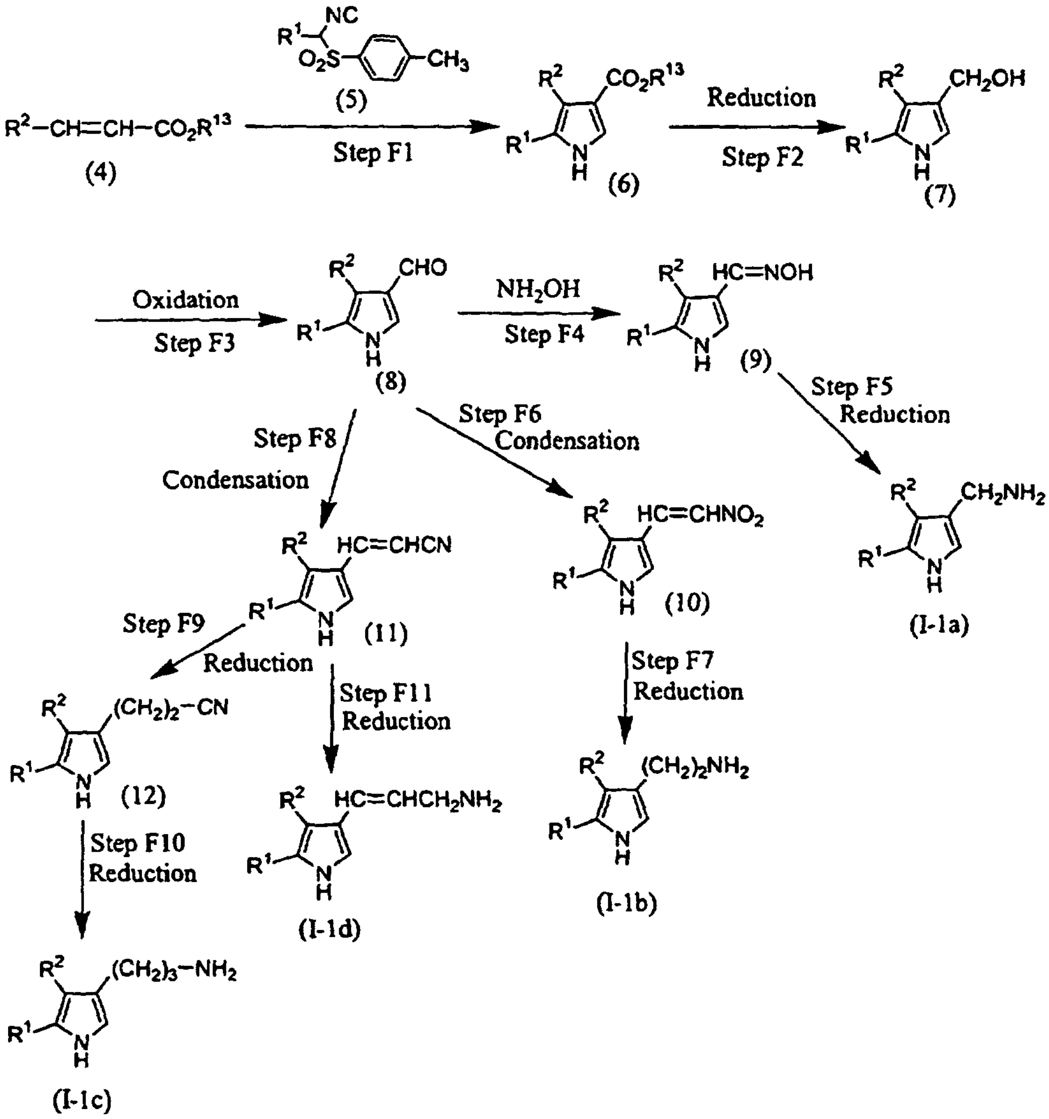


In the above formulae, $R^1$ and $R^2$ are as defined above and $R^{13}$ is selected from the group consisting of hydrogen atoms, lower alkyl groups as defined and exemplified above and aralkyl groups as defined and exemplified above.

**Step F1**

In this Step, a pyrrole carboxylic acid derivative of formula (6) is prepared by reacting an $\alpha,\beta$-unsaturated compound of formula (4) with an isonitrile compound of formula (5). Reactions of this type are well known in the field of synthetic organic chemistry and can be carried out using well known techniques, for example according to the methods described in detail in R. Di Santo et al., Synthetic Communications, 25(6), pp. 795-802 (1995), the contents of which are incorporated herein by reference thereto.

**Step F2**

In this Step, a hydroxymethyl compound of formula (7) is prepared by reducing the pyrrole carboxylic acid derivative of formula (6) prepared in Step F1 above. Reduction reactions of this type are well known in the field of synthetic organic chemistry and can be carried out using well known techniques, for example according to the method described in detail in R. F. Nystrom et al., J. Am. Chem. Soc., 71, 3245 (1945), the contents of which are incorporated

herein by reference thereto, using a reducing agent such as lithium aluminum hydride, lithium borohydride or diisobutyl aluminum hydride.

**Step F3**

In this Step, an aldehyde compound of formula (8) is prepared by oxidizing the hydroxymethyl compound of formula (7) prepared in Step F2 above. Oxidation reactions of this type are well known in the field of synthetic organic chemistry and can be carried out using well known techniques, for example according to the method described in detail in S. Bartel & F. Bohlmann, Tetrahedron Lett., 685 (1985), the contents of which are incorporated herein by reference thereto, using an oxidizing agent such as chromic acid, manganese dioxide or dimethylsulfoxide.

**Step F4**

In this Step, an oxime compound of formula (9) is prepared by reacting the aldehyde compound of formula (8) prepared in Step F3 above with hydroxyamine. Dehydration condensation reactions of this type are well known in the field of synthetic organic chemistry and can be carried out using well known techniques.

**Step F5**

In this Step, the desired compound of formula (I-1a) of the present invention is prepared by reducing the oxime compound of formula (9) prepared in Step F4 above. Reduction reactions of this type are well known in the field of synthetic organic chemistry and can be carried out using well known techniques, for example by catalytic reduction or reduction with diborane, examples of which are disclosed in detail in P. N. Rylander & D. R. Steele, Engelhald Ind. Tech. Bull., 3, 19 (1962), and J. A. Secrist, III & M. W. Logue, J. Org. Chem., 37, 335 (1972), the contents of which are incorporated herein by reference thereto,.

**Step F6**

In this Step, a nitroolefin compound of formula (10) is prepared by a dehydration condensation (aldol condensation) of the aldehyde compound of formula (8) prepared in Step F3 above and nitromethane. Dehydration condensation reactions of this type are well known in the field of synthetic organic chemistry and can be carried out using well known techniques, for example according to the method described in detail in D. E. Worall, Org. Synth., I, 413 (1941), the contents of which are incorporated herein by reference thereto.

**Step F7**

In this Step, the desired compound of formula (I-1b) of the present invention is prepared by reducing the nitroolefin compound of formula (10) prepared in Step F6 above. Reduction reactions of this type are well known in the field of synthetic organic chemistry and can be carried out using well known techniques, for example according to the method described in detail in S. I. Murahashi et al., Bull. Chem. Soc. Jpn., 63, 1252 (1990), the contents of which are incorporated herein by reference thereto.

**Step F8**

In this Step, a cyanoolefin compound of formula (11) is prepared from the aldehyde compound of formula (8) prepared in Step F3 above. This transformation can be performed, for example, via an aldol condensation reaction similar to that employed in Step F6 above, a Wittig reaction [for example according to the method described in detail in The Organic Chemistry of Phosphorous, Elsevier (1967), the contents of which are incorporated herein by reference thereto], or a Wittig-Homer reaction [for example according to the method described in detail in L. Homer et al., Chem. Ber., 95, 581 (1962), the contents of which are incorporated herein by reference thereto].

**Step F9**

In this Step, a cyanoethyl compound of formula (12) is prepared by reduction of the double bond of the cyanoolefin compound of formula (11) prepared in step F8 above. Reduction reactions of this type are well known in the field of synthetic organic chemistry and can be carried out using well known techniques, for example according to the method described in detail in S. M. Kerwin et al., J. Org. Chem., 52, 1686 (1987) and T. Hudlicky et al., J. org. Chem., 52, 4641 (1987), the contents of which are incorporated herein by reference thereto.

**Step F10**

In this Step, the desired compound of formula (I-1c) of the present invention is prepared by reducing the cyano group of the cyanoethyl compound of formula (12) prepared in Step F9 above. Reduction reactions of this type are well known in the field of synthetic organic chemistry and can be carried out using well known techniques, for example according to the methods described in detail in N. M. Yoon & H. C. Brown, J. Am. Chem. Soc., 90, 2927 (1968) and J. Meinweld et al., J. Org. Chem., 29, 2914 (1964), the contents of which are incorporated herein by reference thereto, using lithium aluminum hydride or diisobutyl aluminum hydride.

**Step F11**

In this Step, the desired compound of formula (I-1d) of the present invention is prepared by reducing the cyano group of the cyanoolefin compound of formula (11) prepared in Step F8 above. This reduction is carried out in a similar manner to that described in Step F10 above.

Derivatives of the compounds of formula (I-1a), (I-1b), (I-1c) and (I-1d) prepared above in which a nitrogen atom of an amino group present therein is substituted can be synthesized by alkylating, acylating and/or sulfonylating said amino group according to techniques well known in the field of synthetic organic chemistry.

**Method G**

In this method, compounds of formula (I-1) of the present invention wherein $R^3$ is a heterocyclyl group which may optionally be substituted with at least one group selected from Substituent group a and Substituent group δ and which is substituted with at least one group selected from Substituent group β and Substituent group γ, as defined above, a heterocyclyl group having at least one nitrogen atom which may be optionally substituted with at least one group selected from Substituent group a and Substituent group δ, as defined above, or a group of the general formula $-CH(OH)-(CH_2)_k-R^4$ (wherein $R^4$ is as defined above, and the moiety $-CH(OH)-(CH_2)_k-$ is a lower alkylene group as defined above for the group of formula X which is substituted with a hydroxy group, and k is 0 or an integer of from 1 to 5) can be prepared, as shown in Reaction Scheme G below.

## Reaction Scheme G

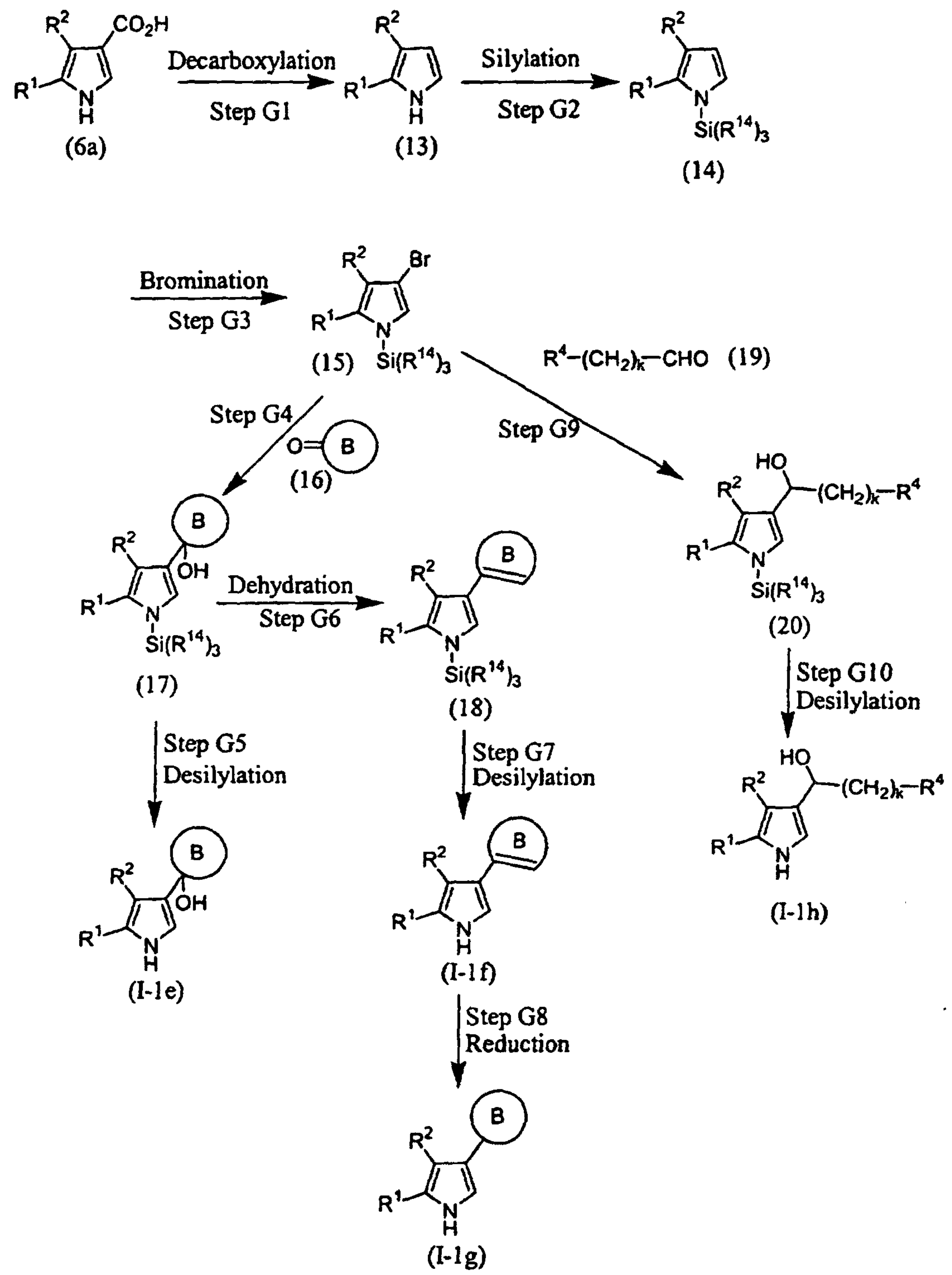


In the above formulae,

$R^1$, $R^2$, $R^4$ and k are as defined above,
the substituents $R^{14}$ may be the same or different from one another and each is selected from the group consisting of hydrogen atoms, lower alkyl groups as defined and exemplified above and aralkyl groups as defined and exemplified above, and

cyclic group B is a group of formula (II) as defined above.

**Step G1**

In this Step, a di-substituted pyrrole compound of formula (13) is prepared by decarboxylating a carboxylic acid compound of formula (6a). Decarboxylation of the compound of formula (6a) can be performed by heating under acidic, alkaline or neutral conditions according to techniques conventionally used in synthetic organic chemistry, for example according to the methods described in detail in N. Yoshida et al., Yakugaku Zasshi, 93(5), 584-598 (1973), the contents of which are incorporated herein by reference thereto.

**Steps G2 to G5**

In Step G2, a silyl compound of formula (14) is prepared by silylating the nitrogen atom at the 1-position of the di-substituted pyrrole compound of formula (13) prepared in Step G1 above.
In Step G3, a bromopyrrole compound of formula (15) is prepared by brominating the silyl compound of formula (14) prepared in Step G2 above with a brominating agent (for example, N-bromosuccinimide, etc.).
In Step G4, a hydroxyheterocyclyl compound of formula (17) is prepared by lithiating the compound of formula (15) prepared in Step G3 above and then reacting it with a heterocyclyl ketone of formula (16).
In Step G5, the desired compound of formula (I-1e) is prepared by deprotecting (desilylating) the compound of formula (17) prepared in Step G4 above with a desilylating agent [e.g. tetrabutylammonium fluoride (TBAF)].
Each of the reactions in Steps G2 to G5 is well known in the field of synthetic organic chemistry and can be carried out using well known techniques. For example, the reactions in Steps G2 to G5 can be carried out according to the method described in detail in Brian L. Bray et al., J. Org. Chem., 55, 6317-6318 (1990), the contents of which are incorporated herein by reference thereto.

**Step G6**

In this Step, an unsaturated heterocyclyl compound of formula (18) is prepared by subjecting the hydroxyheterocyclyl compound of formula (17) prepared in Step G4 above to a dehydration reaction. Dehydration reactions of this type are well known in the field of synthetic organic chemistry and can be carried out using well known techniques. For example, the dehydration reaction can typically be carried out in the presence of an acid catalyst such as sulfuric acid, a solid catalyst such as alumina or a halogenating agent such as thionyl chloride [e.g. in accordance with the methods described in detail in G. H. Coleman & H. F. Johnstone, Org. Synth., I, 183 (1941), R. L. Sawyer & D. W. Andrus, Org. Synth., III, 276 (1955) and J. S. Lomas et al., Tetrahedron Lett., 599 (1971), the contents of which are incorporated herein by reference thereto]. Alternatively, the dehydration reaction of this step can be accomplished by a reaction using a trialkylsilane, such as triethylsilane, tripropylsilane or tributylsilane, and trifluoroacetic acid, for example in accordance with the method described in Francis A. Carey & Henry S. Tremper, J. Am. Chem. Soc., 91, 2967-2972 (1969), the contents of which are incorporated herein by reference thereto.

**Step G7**

In this Step, the desired compound of formula (I-1f) of the present invention is prepared by removing the protecting group (the silyl group) from the unsaturated heterocyclyl compound of formula (18) prepared in Step G6 above in a similar manner to that performed in Step G5 above.

**Step G8**

In this Step, the desired compound of formula (I-lg) of the present invention is prepared by reducing the double bond in the cyclic group B of the compound of formula (I-1f) prepared in Step G7 above. This is carried out in a similar manner to the procedure described in Step F9 above.

**Step G9**

In this Step, a hydroxy compound of formula (20) is prepared by reaction between the bromopyrrole compound of formula (15) prepared in Step G3 above and an aldehyde compound of formula (19). This is carried out in a procedure similar to that described in Step G4 above.

Step G10

In this Step, the desired compound of formula (I-1h) of the present invention is prepared by removing the protecting group (silyl group) from the hydroxy compound of formula (20) prepared in Step G9 above. This is carried out in a similar manner to that described in Step G5 above.

Where k in the compound of formula (I-1h) above is an integer of from 1 to 5, the hydroxyalkane moiety can be converted into the corresponding alkenylene moiety by subjecting such a compound to a dehydration reaction in a similar manner to that described in Step G6 above.

Method H

Generally, the compounds of formula (I) of the present invention can be prepared by introducing the $R^3$ group into a pyrrole compound already substituted on the pyrrole ring with the $R^1$ group and $R^2$ group. Compounds of formula (I-1) can be prepared, for example, according to the Method H, as shown in Reaction Scheme H below.

Reaction Scheme H

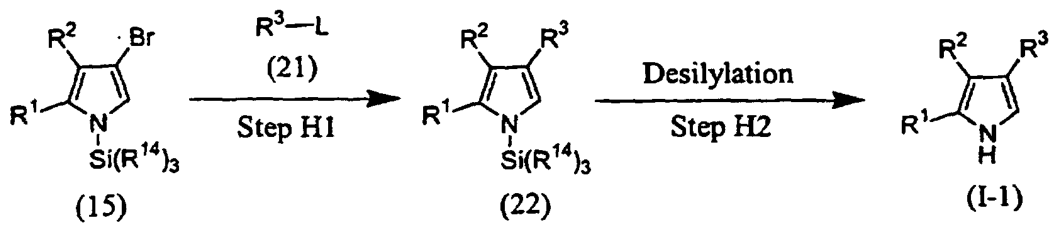


(15) (22) (I-1)

In the above formulae, $R^1$, $R^2$, $R^3$ and $R^{14}$ are as defined above, and L represents a leaving group.

The leaving group L is a group which is capable of leaving as a nucleophilic residue. Examples include halogen atoms such as fluorine, chlorine, bromine and iodine, trihalogenomethyloxy groups such as trichloromethoxy, lower alkanesulfonyloxy groups such as methanesulfonyloxy and ethanesulfonyloxy groups, lower halogeno alkane sulfonyloxy groups such as trifluoromethanesulfonyloxy and pentafluoroethanesulfonyloxy groups, and arylsulfonyloxy groups such as benzenesulfonyloxy, p-toluenesulfonyloxy and p-nitrobenzenesulfonyloxy groups. Of these, halogen atoms are preferred, and bromine atoms are particularly preferred.

Step H1

In this Step, a compound of formula (22) is prepared by lithiating the bromopyrrole compound of formula (15) prepared in Step G3 above using a procedure similar to that described in Step G4 above and then reacting the lithiated intermediate with a compound of formula (21). Substitution reactions of a lithiated pyrrole intermediate of this type are well known in the field of synthetic organic chemistry and can be carried out using well known techniques, for example according to the methods described in detail in WO 99/01449, the contents of which are incorporated herein by reference thereto.

Step H2

In this Step, the desired compound of formula (I-1) is prepared by removing the protecting group (silyl group) from the compound of formula (22) prepared in Step H1 above according to a procedure similar to that used in Step G5 above.

Method J

In this method, compounds of formula (Ib) of the present invention wherein $R^2$ is a heteroaryl group having at least one nitrogen atom and is substituted with a group of formula $-NR^cR^d$, wherein $R^c$ and $R^d$ are as defined above can be prepared, as shown in Reaction Scheme J below.

## Reaction Scheme J

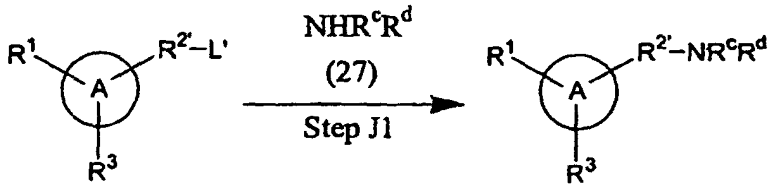


(26) (Ib)

In the above formulae, the cyclic group A, $R^1$, $R^3$, $R^c$ and $R^d$ are as defined above, $R^{2'}$ is a pyridyl or pyrimidinyl group and L' is a leaving group. Thus, the group —$R^{2'}$-L' is, for example, 2-methanesulfonylpyrimidin-4-yl, 2-methanesulfonylpyridin-4-yl, etc.

The leaving group L' is a similar group to the leaving groups defined and exemplified above in the definition of L or it is a lower alkylsulfonyl group as defined and exemplified above, such as a methanesulfonyl, ethanesulfonyl, propanesulfonyl or butanesulfonyl group. The group L' is preferably a lower alkylsulfonyl group, and more preferably a methanesulfonyl group.

### Step J1

In this Step, the desired compound of formula (Ib) of the present invention is prepared by reacting a compound of formula (26) with an amine compound of formula (27) to replace the leaving group with a group of formula -$NR^cR^d$. This step is carried out using a procedure similar to that used in Step I1 above.

The compounds which can be used as starting materials in Method A to H and Method J above, that is the compounds of formulae (1a), (1b), (1c), (1d), (1e), (2a), (2b), (2c), (2d), (2e), (4), (5), (6a), (16), (19), (21) and (27), are either known compounds themselves or are compounds which are easily obtainable by treating known compounds according to known methods (for example, according to the methods described in WO 97/5877, the contents of which are incorporated herein by reference thereto). The starting materials of formula (26) in Method J can be easily synthesized from known compounds by carrying out similar reactions to those described in Method A to Method E above.

### Method L

In this method, a 4-oxopiperidine compound of formula (35), which is a starting material falling within the scope of the compounds of formula (16) above, is prepared as shown in the following Reaction Scheme L.

**Reaction Scheme L**

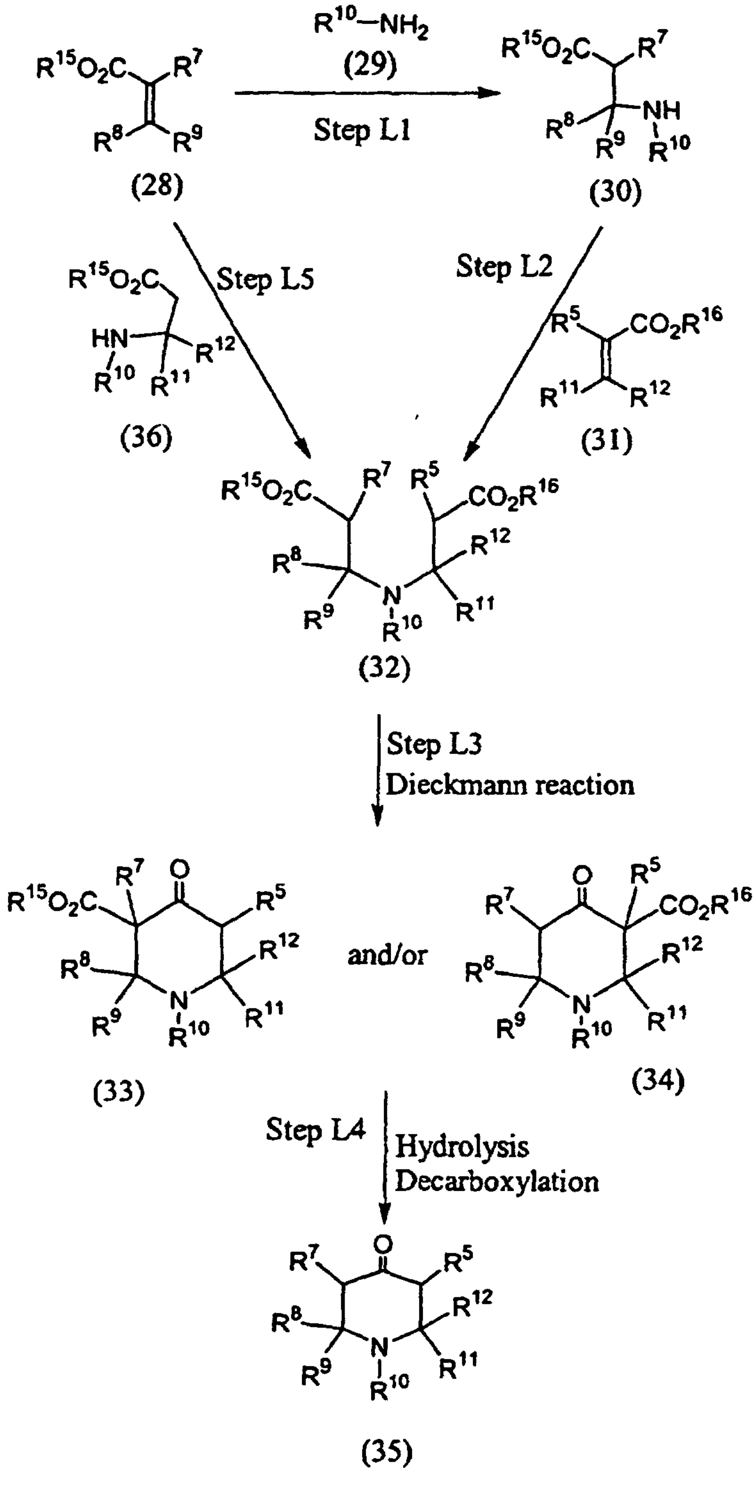


In the above formulae, $R^5$, $R^7$, $R^8$, $R^9$, $R^{10}$, $R^{11}$ and $R^{12}$ are as defined above, and $R^{15}$ and $R^{16}$ are the same or different and each is a lower alkyl group as defined above or an aralkyl group as defined above.

**Steps L1 to L5**

In step L1, an amino ester compound of formula (30) is prepared by an addition reaction between an unsaturated ester compound of formula (28) and an amine compound of formula (29).

In Step L2, a diester compound of formula (32) is obtained by a further addition reaction between an unsaturated ester compound of formula (31) and the amino ester compound of formula (30) prepared in Step L1 above.

In Step L3, the compound of formula (32) prepared in Step L2 above is subjected to Dieckmann reaction to give a keto ester compound of formula (33) and/or formula (34).

In Step L4, the compound of formula (33) and/or the compound of formula (34) prepared in Step L3 above is subjected to consecutive hydrolysis and decarboxylation reactions to prepare the desired 4-oxopiperidine compound of formula (35).

Step L5 is an alternative process for the preparation of the diester compound of formula (32) and it involves reaction of the unsaturated ester compound of formula (28) with an amino ester compound of formula (36). This is carried out in a similar manner to that described in Step L2 above. Step L5 is preferably employed where the compound of formula (32) to be prepared is one wherein $R^{10}$ and $R^{11}$ together form an alkylene group having from 1 to 6 carbon atoms which is unsubstituted or is substituted with at least one substituent selected from the group consisting of Substituent group α defined above, Substituent group β defined above, Substituent group γ defined above and Substituent group δ defined above.

Each of the reactions in Steps L1 to L5 is well known in the field of synthetic organic chemistry and can be carried out using well known techniques. For example, the reactions in Steps L1 to L5 can be carried out according to the method described in detail in U. M. Teotino, J. Org. Chem., 27, 1406 (1962), the contents of which are incorporated herein by reference thereto.

**Method M**

In this method, a compound of formula (35a), which is a 4-oxopiperidine compound of formula (35) above wherein $R^{10}$ and $R^{11}$ together form an alkylene group having from 1 to 6 carbon atoms which is unsubstituted or is substituted with at least one substituent selected from the group consisting of Substituent group α defined above, Substituent group β defined above, Substituent group γ defined above and Substituent group δ defined above and each of $R^5$, $R^7$ to $R^9$ and $R^{12}$ is a hydrogen atom can be prepared as shown in Reaction Scheme M below.

**Reaction Scheme M**

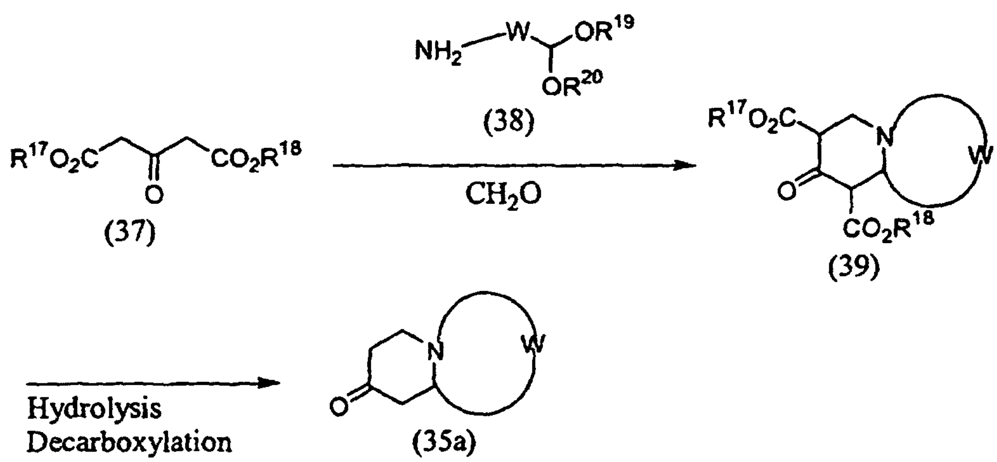

(37) (38) (39) (35a)

In the above formulae, $R^{17}$ and $R^{18}$ may be the same or different and each represents a lower alkyl group as defined and exemplified above or an aralkyl group as defined and exemplified above, $R^{19}$ and $R^{20}$ may be the same or different and each represents a lower alkyl group as defined and exemplified above or $R^{19}$ and $R^{20}$ together represent a lower alkylene group as defined and exemplified above, and W represents an alkylene group having from 1 to 6 carbon atoms which is unsubstituted or is substituted with at least one substituent selected from the group consisting of Substituent group α defined above, Substituent group β defined above, Substituent group γ defined above and Substituent group δ defined above.

The two steps of Method M can be carried out according to the methods described in detail in O. Pollet et al., Heterocycles, 43, 1391 (1996) and Anet et al., Austral. J. Scient. Res., <A3>, 635-640 (1950), the contents of which are incorporated herein by reference thereto.

After completion of each of the reactions described in the steps of Method A to Method M above, the desired

compound may be isolated from the reaction mixture in a conventional manner. For example, it can be obtained by neutralizing the reaction mixture as needed, removing insoluble matters by filtration, if any are present, adding organic solvents which are not miscible with each other, such as water and ethyl acetate, washing with water or the like, separating the organic layer containing the desired compound, drying it over anhydrous magnesium sulfate or the like and then distilling off the solvent.

If necessary, the desired compound thus obtained can be isolated and purified by using a conventional method such as recrystallization or reprecipitation or by a chromatographic method. Examples of chromatography include adsorption column chromatography using a carrier such as silica gel, alumina or magnesium-silica gel type Florisil, chromatography using a synthetic adsorbent, for example, partition column chromatography using a carrier such as Sephadex LH-20 (product of Pharmacia), Amberlite XAD-11 (product of Rohm & Haas) or Diaion HP-20 (product of Mitsubishi Chemical), ion exchange chromatography and normal-phase reverse-phase column chromatography (high-performance liquid chromatography) using a silica gel or alkylated silica gel. If necessary, two or more of these techniques can be used in combination to isolate and purify the desired compound.

The heteroaryl-substituted pyrrole derivatives of the present invention exhibit excellent inhibitory activity against the production of inflammatory cytokines. Consequently, they are effective as a medicament, particularly as an agent for the prophylaxis or treatment of diseases mediated by inflammatory cytokines. Examples of such a medicament include analgesics, anti-inflammatory drugs and virucides, and agents for the prophylaxis and treatment of chronic rheumatoid arthritis, osteoarthritis, allergic diseases, asthma, septicaemia, psoriasis, osteoporosis, autoimmune diseases (e.g. systemic lupus erythematosus, ulcerative colitis and Crohn's disease), diabetes, nephritis, hepatitis, arteriosclerosis, cancer, ischemic heart disease and Alzheimer's disease. Of these, the compunds of the present invention are particularly useful as analgesics and anti-inflammatory drugs and as agents for the prophylaxis and treatment of chronic rheumatism, osteoarthritis, allergosis, sepsis, psoriasis, osteoporosis, ulcerative colitis, diabetes, hepatitis and arteriosclerosis.

The compounds of formula (I) and pharmacologically acceptable salts, esters and other derivatives thereof according to the present invention can be administered by a number of different routes. Examples of these administration routes include oral administration in the form of tablets, capsules, granules, powders or syrups and parenteral administration in the form of injections or suppositories. Such formulations can be prepared in a known manner by using additives such as an excipients, lubricants, binders, disintegrators, stabilizers, corrigents and diluents.

Examples of suitable excipients include: organic excipients, examples of which include sugar derivatives such as lactose, sucrose, dextrose, mannitol and sorbitol, starch derivatives such as corn starch, potato starch, $\alpha$-starch, dextrin and carboxymethyl starch, cellulose derivatives such as crystalline cellulose, low-substituted hydroxypropylcellulose, hydroxypropylmethylcellulose, carboxymethylcellulose, calcium carboxymethylcellulose and sodium internally-crosslinked carboxymethylcellulose, gum arabic, dextran and pullulan; and inorganic excipients, examples of which include silicate derivatives such as soft silicic acid anhydride, synthetic aluminum silicate and magnesium aluminometasilicate, phosphates such as calcium phosphate, carbonates such as calcium carbonate, and sulfates such as calcium sulfate.

Examples of suitable lubricants include: stearic acid; metal salts of stearic acid such as calcium stearate and magnesium stearate; talc; colloidal silica; waxes such as bee gum and spermaceti; boric acid; adipic acid; sulfates such as sodium sulfate; glycol; fumaric acid; sodium benzoate; DL-leucine; sodium salts of an aliphatic acid; lauryl sulfates such as sodium lauryl sulfate and magnesium lauryl sulfate; silicic acid derivatives such as silicic anhydride and silicic acid hydrate; and starch derivatives exemplified above as examples of suitable excipients.

Examples of suitable binders include polyvinylpyrrolidone, Macrogol and compounds similar to those exemplified above as suitable excipients.

Examples of suitable disintegrators include compounds similar to those exemplified above as suitable excipients and chemically modified starch or cellulose derivatives such as sodium cross carmellose, sodium carboxymethyl starch and crosslinked polyvinylpyrrolidone.

Examples of suitable stabilizers include: paraoxybenzoate esters such as methylparaben and propylparaben; alcohols such as chlorobutanol, benzyl alcohol and phenylethyl alcohol; benzalkonium chloride; phenol derivatives such as phenol and cresol; thimerosal; dehydroacetic acid; and sorbic acid. Examples of suitable corrigents include sweeteners, acidifiers and flavors commonly employed for this purpose.

The dose of the compound of formula (I) or a pharmacologically acceptable salt, ester or other derivative thereof according to the present invention will vary depending on a variety of factors including the condition to be treated, the age of the patient and the administration route. When administered orally, it is administered to an adult in an amount of 0.1 mg (preferably 0.5 mg) a day as a lower limit and 2000 mg (preferably 500 mg) a day as an upper limit. It can be administered in from one to several portions depending on the condition of the patient. When administered intravenously, it is administered to an adult in an amount of 0.01 mg (preferably 0.05 mg) a day as a lower limit and 200 mg (preferably 50 mg) a day as an upper limit. It can be administered in from one to several portions depending on the condition of the patient.

The following synthetic examples, examples, preparative examples, formulation examples and test examples are intended to further illustrate the present invention and are not intended to limit the scope of this invention in any way.

Synthetic Example 1

4-(3-Aminopropyl)-2-(4-fluorophenyl)-3-(pyridin-4-yl)-1*H*-pyrrole

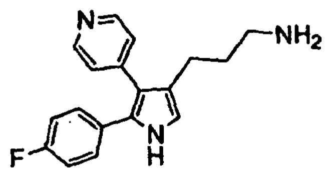

4-Ethoxycarbonyl-2-(4-fluorophenyl)-3-(pyridin-4-yl)-1*H*-pyrrole

36 ml (54.7 mmol) of a 1.53 N solution of butyllithium in hexane were added to 240 ml of tetrahydrofuran. A solution of 15.90 g (54.7 mmol) of α-(p-toluenesulfonyl)-4-fluorobenzylisonitrile in 120 ml of tetrahydrofuran was then added to the resulting solution at -45°C, followed by stirring of the resulting mixture for 10 minutes at the same temperature. At the end of this time, 25.00 g (273 mmol) of 95% lithium bromide were added, the resulting mixture was stirred for 30 minutes and then a solution of 8.73 g (49.2 mmol) of ethyl 3-(4-pyridyl)acrylate in 120 ml of tetrahydrofuran was added. The resulting mixture was stirred at the same temperature for 1 hour and then the cooling bath was removed and the mixture was stirred at room temperature for a further 1 hour. At the end of this time, 500 ml of water were added and the resulting mixture was extracted with ethyl acetate. The organic extract was washed with water and then dried over anhydrous sodium sulfate, after which it was concentrated by evaporation under reduced pressure to afford a solid. The solid was washed with diethyl ether to give 13.61 g (yield 89%) of the title compound as a pale yellow powder.
$^1$H-Nuclear magnetic resonance spectrum (500 MHz, $CDCl_3$) δ ppm:

8.84 (1H, broad singlet);
8.51 (2H, doublet, J=7 Hz);
7.58 (1H, doublet, J=3 Hz);
7.21 (2H, doublet, J=6 Hz);
7.11 (2H, doublet of doublets, J=9 Hz, 5 Hz);
6.97 (2H, triplet, J=9 Hz);
4.18 (2H, quartet, J=7 Hz);
1.20 (3H, triplet, J=7 Hz).

Example 2

2-(4-Fluorophenyl)-3-(pyridin-4-yl)-4-(3-trifluoroacetylaminopropyl)-1*H*-pyrrole (Compound No. 1-2533)

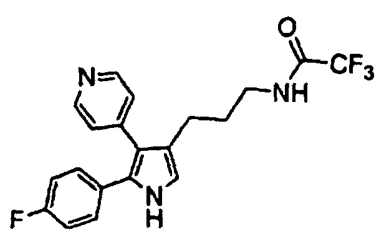

3.3 ml (23.2 mmol) of trifluoroacetic anhydride were added to a solution of 685 mg (2.32 mmol) of 4-(3-aminopropyl)-2-(4-fluorophenyl)-3-(pyridin-4-yl)-1*H*-pyrrole (which was prepared as described in Example 1 above) in 70 ml of tetrahydrofuran. The resulting mixture was stirred at room temperature for 30 minutes. At the end of this time, 200 ml of a saturated aqueous solution of sodium hydrogencarbonate were added to the reaction mixture, and then this was extracted with ethyl acetate. The organic extract was washed with water and concentrated by evaporation under reduced pressure. The resulting residue was purified by chromatography on a silica gel column using a 1:1 by volume mixture of hexane and ethyl acetate as the eluant to give 280 mg (yield 31 %) of the title compound as a white powder.
Melting point: 229 - 230°C
$^1$H-Nuclear magnetic resonance spectrum (500 MHz, $CDCl_3$) δ ppm:

10.09 (1H, broad singlet);
8.49 (2H, doublet, J=6 Hz);
7.93 (1H, broad singlet);
7.18 (2H, doublet of doublets, J=9 Hz, 5 Hz);
7.13 (2H, doublet, J=6 Hz);
6.94 (2H, triplet, J=9 Hz);
6.74 ( 1 H, doublet, J=3 Hz);
3.29 (2H, doublet of doublets, J=13 Hz, 7 Hz);
2.56 (2H, triplet, J=8 Hz);
1.74 (2H, quintet, J=8 Hz).

Synthetic Example 2

2-(4-Fluorophenyl)-3-(pyridin-4-yl)-4-(2,3,5,6-tetrafluoropyridin-4-yl)-1*H*-pyrrole (Compound No. 1-2538)

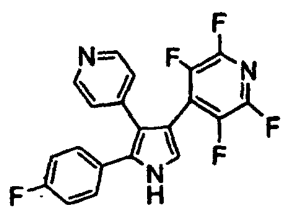

2(i) 2-(4-Fluorophenyl)-3-(pyridin-4-yl)-1*H*-pyrrole

15.00 g (48.3 mmol) of 4-ethoxycarbonyl-2-(4-fluorophenyl)-3-(pyridin-4-yl)-1*H*-pyrrole [prepared as described in Synthetic Example 1 above] were dissolved in a mixture of 90 ml of acetic acid, 30 ml of sulfuric acid and 60 ml of water and the resulting solution was stirred at 100°C for 16 hours. At the end of this time, the reaction mixture was cooled to room temperature and then made alkaline with a 10% aqueous solution of sodium hydroxide. The resulting mixture was extracted with ethyl acetate and the organic extract was washed with water, dried over anhydrous sodium sulfate and then concentrated by evaporation under reduced pressure to give 11.40 g (yield 99%) of the title compound as a pale red powder.
$^1$H-Nuclear magnetic resonance spectrum (500 MHz, $CDCl_3$) δ ppm:

9.78 (1H, broad singlet);
8.42 (2H, doublet, J=7 Hz);
7.37 (2H, doublet of doublets, J=9 Hz, 5 Hz);
7.22 (2H, doublet, J=6 Hz);
7.06 (2H, triplet, J=9 Hz);
6.90 (1H, triplet, J=3 Hz);
6.47 (1H, triplet, J=3 Hz).

2(ii) 2-(4-Fluorophenyl)-3-(pyridin-4-yl)-1-triisopropylsilyl-1*H*-pyrrole

31 ml (47.4 mmol) of a 1.57 N solution of butyllithium in hexane were added to a solution of 11.30 g (47.4 mmol) of 2-(4-fluorophenyl)-3-(pyridin-4-yl)-1*H*-pyrrole [prepared as described in step 2(i) above] in 300 ml of tetrahydrofuran at -78°C and the mixture was stirred for 10 minutes. At the end of this time, 13.4 ml (49.8 mmol) of triisopropylsilyl triflate were added to the reaction mixture at the same temperature. After removal of the cooling bath, the mixture was stirred at room temperature for 30 minutes. 200 ml of water and 300 ml of a saturated aqueous solution of sodium hydrogencarbonate were then added to the reaction mixture before extracting with ethyl acetate. The organic extract was washed with water, dried over anhydrous sodium sulfate and then concentrated by evaporation under reduced pressure to give 18.70 g (quantitative yield) of the title compound as a reddish purple oil.
$^1$H-Nuclear magnetic resonance spectrum (500 MHz, DMSO-$d_6$) δ ppm:

8.25 (2H, doublet, J=6 Hz);
7.39 (2H, doublet of doublets, J=9 Hz, 6 Hz);
7.28 (2H, triplet, J=9 Hz);
7.00 (1H, doublet, J=3 Hz);
6.91(2H, doublet, J=7 Hz);
6.71 (1H, doublet, J=3 Hz);
1.15-1.05 (3H, multiplet);
0.98 (18H, doublet, J=8 Hz).

2(iii) 4-Bromo-2-(4-fluorophenyl)-3-(pyridin-4-yl)-1-triisopropylsilyl-1*H*-pyrrole

A suspension of 8.61 g (47.4 mmol) of N-bromosuccinimide in 100 ml of tetrahydrofuran was added to a solution of 18.70 g (47.4 mmol) of 2-(4-fluorophenyl)-3-(pyridin-4-yl)-1-triisopropylsilyl-1*H*-pyrrole [prepared as described in step 2(ii) above] in 300 ml of tetrahydrofuran at -78°C, after which the resulting mixture was stirred at the same temperature for 6 hours. After removal of the cooling bath, the mixture was then stirred at room temperature for a further 1 hour. At the end of this time, 400 ml of hexane was added to the reaction mixture and the insoluble material was removed by filtration. The resulting filtrate was concentrated by evaporation under reduced pressure and the residue thus obtained was purified by chromatography on a silica gel column using a 2:1 by volume mixture of hexane and ethyl acetate as the eluant to give 9.57 g (yield 43%) of the title compound as pale yellow prismatic crystals.
$^1$H-Nuclear magnetic resonance spectrum (500 MHz, DMSO-$d_6$) δ ppm:

8.36 (2H, doublet, J=6 Hz);
7.34 (2H, doublet of doublets, J=9 Hz, 6 Hz);
7.18 (2H, triplet, J=9 Hz);
7.12 (1H, singlet);
7.04 (2H, doublet, J=6 Hz);
1.16-1.08 (3H, multiplet);
0.99 (18H, doublet, J=8 Hz).

2(iv) 2-(4-Fluorophenyl)-3-(pyridin-4-yl)-4-(2,3,5,6-tetrafluoropyridin-4-yl)-1*H*-pyrrole

1.8 ml (2.75 mmol) of 1.54 N solution of t-butyllithium in pentane were added to a solution of 650 mg (1.37 mmol) of 4-bromo-2-(4-fluorophenyl)-3-(pyridin-4-yl)-1-triisopropylsilyl-1*H*-pyrrole [prepared as described in step 2(iii) above] in 15 ml of tetrahydrofuran at -78°C and the mixture was stirred for 10 minutes. At the end of this time, 165 μl (1.51 mmol) of pentafluoropyridine were added to the reaction mixture. The cooling bath was then removed and the mixture was stirred for 30 minutes. At the end of this time, 70 ml of water and 50 ml of a saturated aqueous solution of sodium hydrogencarbonate were added to the reaction mixture, and then this was extracted with ethyl acetate. The organic extract was washed with water and then concentrated by evaporation under reduced pressure. The residue thus obtained was purified by chromatography on a silica gel column using a 4:1 by volume mixture of hexane and ethyl acetate as the eluant to give 346 mg (yield 65%) of the title compound as a pale yellow powder.
Melting point: >300°
$^1$H-Nuclear magnetic resonance spectrum (500 MHz, $CD_3OD$) δ ppm:

8.34 (2H, doublet, J=8 Hz);
7.32 (1H, singlet);
7.30 (2H, doublet of doublets, J=11 Hz, 7 Hz);

7.09 (2H, triplet, J=11 Hz);
7.06(2H, doublet, J=8 Hz).

Synthetic Example 3

1-(1-Benzylpiperidin-4-yl)-3-(4-fluorophenyl)-2-(pyridin-4-yl)-1*H*-pyrrole

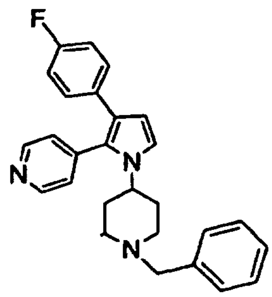

3(i) 1-Benzyl-4-(pyridin-4-yl)methyleneaminopiperidine

8.57 ml (42.01 mmol) of 4-amino-1-benzylpiperidine were added to a solution of 3.95 ml (42.01 mmol) of 4-formylpyridine in 5 ml of ethanol, and the resulting mixture was then heated under reflux for 1 hour. At the end of this time, the solvent was distilled off from the reaction mixture by evaporation under reduced pressure to afford 11.78 g (quantitative yield) of the title compound as a pale brown powder.
$^1$H-Nuclear magnetic resonance spectrum (400 MHz, $CDCl_3$) $\delta$ ppm:

8.68 (2H, doublet, J=6 Hz);
8.30 (1H, singlet);
7.60 (2H, doublet, J=6 Hz);
7.38-7.22 (5H, multiplet);
3.56 (2H, singlet);
3.37-3.27 ( 1 H, multiplet);
3.00-2.90 (2H, multiplet);
2.23-2.10 (2H, multiplet);
1.96-1.83 (2H, multiplet);
1.79-1.60 (2H, multiplet).

3(ii) (±)-α-(1-Benzylpiperidin-4-yl)amino-α-(pyridin-4-yl)acetonitrile

A mixture of 11.73 g (42.00 mmol) of 1-benzyl-4-(pyridin-4-yl)-methyleneaminopiperidine [prepared as described in step 3(i) above] and 9.22 ml (65.67 mmol) of 95% trimethylsilyl cyanide was stirred at 100°C for 3 hours and then it was cooled to room temperature. 100 ml of methanol were added to the reaction mixture and the resulting mixture was then stirred for 30 minutes. At the end of this time, the solvent was distilled off from the reaction mixture by evaporation under reduced pressure to afford 12.87 g (quantitative yield) of the title compound as a brown oil.
$^1$H-Nuclear magnetic resonance spectrum (400 MHz, $CDCl_3$) $\delta$ ppm:

8.67 (2H, doublet, J=6 Hz);
7.50 (2H, doublet, J=6 Hz);
7.37-7.22 (5H, multiplet);
4.85 (1H, singlet);
3.53 (2H, singlet);
2.98-2.77 (4H, multiplet);
2.33-1.44 (5H, multiplet).

3(iii) 1-(1-Benzylpiperidin-4-yl)-3-(4-fluorophenyl)-2-(pyridin-4-yl)-1*H*-pyrrole

14.25 g (46.51 mmol) of α-(1-benzylpiperidin-4-yl)amino-α-(pyridin-4-yl)acetonitrile [prepared as described in step 3(ii) above] and 6.98 g (46.51 mmol) of 3-(4-fluorophenyl)acrolein were dissolved in 145 ml of N,N-dimethylacetamide, 1.29 g (9.30 mmol) of potassium carbonate were added to the solution, and then this was stirred at room temperature for 5 hours. At the end of this time, water was added to the reaction mixture and then this was extracted with ethyl acetate. The organic extract was washed with water and then concentrated by evaporation under reduced pressure. 150 ml of ethylene glycol were added to the residue thus obtained and the resulting mixture was stirred at 180°C for 1 hour. After cooling to room temperature, a saturated aqueous solution of sodium hydrogencarbonate was added to the reaction mixture which was then extracted with ethyl acetate. The organic extract was washed with water and then concentrated by evaporation under reduced pressure. The residue thus obtained was purified by chromatography on a silica gel column using a 1:3 by volume mixture of hexane and ethyl acetate as the eluant to give 10.31 g (yield 49%) of the title compound as a pale brown powder.
Melting point: 132 - 139°C
$^1$H-Nuclear magnetic resonance spectrum (400 MHz, $CDCl_3$) δ ppm:

8.61 (2H, doublet, J=6 Hz);
7.37-7.23 (5H, multiplet);
7.13 (2H, doublet, J=6 Hz);
7.03 (2H, doublet of doublets, J=9 Hz, 5 Hz);
6.96 (1H, doublet, J=3 Hz);
6.87 (2H, triplet, J=9 Hz);
6.38 (1H, doublet, J=3 Hz);
3.87-3.80 (1H, multiplet);
3.50 (2H, singlet);
2.97 (2H, doublet, J=12 Hz);
2.12-1.56 (6H, multiplet).

Synthetic Example 4

3-(4-Fluorophenyl)-2-(2-methylaminopyrimidin-4-yl)-1-(piperidin-4-yl)-1*H*-pyrrole

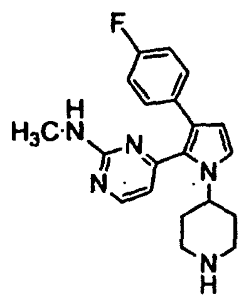

4(i) 1-(1-Benzylpiperidin-4-yl)-3-(4-fluorophenyl)-2-(2-methylthiopyrimidin-4-yl)-1*H*-pyrrole

In a similar manner to the procedures described in Synthetic Examples 3(i), 3(ii) and 3(iii) above, reactions were carried out using 4-formyl-2-methylthiopyrimidine as a starting material instead of 4-formylpyridine to give the title compound (total yield 34%) as a brown oil.
$^1$H-Nuclear magnetic resonance spectrum (400 MHz, $CDCl_3$) δ ppm:

8.21 (1H, doublet, J=5 Hz);
7.39-7.24 (5H, multiplet);
7.16 (2H, doublet of doublets, J=9 Hz, 5 Hz);
7.06 (1H, doublet, J=3 Hz);
6.96 (2H, triplet, J=9 Hz);

6.57 (1H, doublet, J=5 Hz);
6.27 (1H, doublet, J=3 Hz);
4.83-4.73 (1H, multiplet);
3.54 (2H, singlet);
3.02 (2H, doublet, J=9 Hz);
2.75 (2H, triplet, J=6 Hz);
2.46 (2H, triplet, J=6 Hz);
2.13-1.96 (2H, multiplet).

4(ii) 1-(1-benzylpiperidin-4-yl)-3-(4-fluorophenyl)-2-(2-methanesulfonylpyrimidin-4-yl)-1*H*-pyrrole

3.51 g (15.26 mmol) of 69-75 wt.% of m-chloroperbenzoic acid were added in small portions to a solution of 3.50 g (7.63 mmol) of 1-(1-benzylpiperidin-4-yl)-3-(4-fluorophenyl)-2-(2-methylthiopyrimidin-4-yl)-1*H*-pyrrole [prepared as described in step 4(i) above] in 35 ml of ethyl acetate with ice-cooling. The resulting mixture was stirred at room temperature 2 days, after which a 10% aqueous solution of sodium thiosulfate was added to the reaction mixture followed by extraction with ethyl acetate. The organic extract was washed with water and then concentrated by evaporation under reduced pressure. The residue thus obtained was purified by chromatography on a silica gel column using a 9:1 by volume mixture of dichloromethane and methanol as the eluant to give 2.48 g (yield 66%) of the title compound as a pale brown amorphous solid.
$^1$H-Nuclear magnetic resonance spectrum (400 MHz, $CDCl_3$) δ ppm:

8.35 (1H, doublet, J=5 Hz);
7.63-7.56 (2H, multiplet);
7.47-7.38 (3H, multiplet);
7.29-7.24 (1H, multiplet);
7.22 (2H, doublet of doublets, J=9 Hz, 5 Hz);
7.05 (2H, triplet, J=9 Hz);
6.90 (2H, doublet, J=5 Hz);
6.29 (1H, doublet, J=3 Hz);
5.43-5.33 (1H, multiplet);
4.46 (2H, singlet);
3.56-3.27 (4H, multiplet);
3.03-2.86 (2H, multiplet);
2.93 (3H, singlet);
2.26-2.14 (1H, multiplet);
2.10-2.01 (1H, multiplet).

4(iii) 1-(1-Benzylpiperidin-4-yl)-3-(4-fluorophenyl)-2-(2-methylaminopyrimidin-4-yl)-1*H*-pyrrole

A mixture of 1.19 g (2.43 mmol) of 1-(1-benzylpiperidin-4-yl)-3-(4-fluorophenyl)-2-(2-methanesulfonylpyrimidin-4-yl)-1*H*-pyrrole [prepared as described in Step 4(ii) above] and 13 ml of a 2M solution of methylamine in tetrahydrofuran was stirred at 100°C for 30 minutes in a sealed tube. At the end of this time, the solvent was distilled off under reduced pressure. The resulting residue was purified by chromatography on a silica gel column using a 1:2 by volume mixture of hexane and ethyl acetate as the eluant to give 260 mg (yield 24%) of the title compound as a yellow amorphous solid.
$^1$H-Nuclear magnetic resonance spectrum (500 MHz, $CDCl_3$) δ ppm:

8.03 (1H, doublet, J=5 Hz);
7.42-7.23 (5H, multiplet);
7.18 (2H, doublet of doublets, J=9 Hz, 5 Hz);
7.00 (1H, doublet, J=3 Hz);
6.94 (2H, triplet, J=9 Hz);
6.26 (1H, doublet, J=3 Hz);
6.23 (1H, doublet, J=5 Hz);
5.14-5.04 (1H, multiplet);
4.82-4.68 (1H, multiplet);
3.53 (2H, singlet);
3.10-2.95 (5H, multiplet);

2.12-1.96 (6H, multiplet).

4(iv) 3-(4-Fluorophenyl)-2-(2-methylaminopyrimidin-4-yl)-1-(piperidin-4-yl)-1*H*-pyrrole

In a similar manner to that described in Example 9(ii) above, 1-(1-benzylpiperidin-4-yl)-3-(4-fluorophenyl)-2-(2-methylaminopyrimidin-4-yl)-1*H*-pyrrole [prepared as described in step 4(iii) above] was debenzylated to give the title compound (yield 39%) as a pale yellow powder.
Melting point: 189- 191°C
$^1$H-Nuclear magnetic resonance spectrum (500 MHz, $CDCl_3$) δ ppm:

8.05 (1H, doublet, J=5 Hz);
7.19 (2H, doublet of doublets, J=9 Hz, 5 Hz);
7.00 (1H, doublet, J=3 Hz);
6.95 (2H, triplet, J=9 Hz);
6.28 (1H, doublet, J=3 Hz);
6.24 (1H, doublet, J=5 Hz);
5.14-5.04 (1H, multiplet);
4.96-4.81 (1H, multiplet);
3.24 (2H, doublet, J=12 Hz);
3.04 (3H, doublet, J=5 Hz);
2.70 (2H, doublet of quartets, J=12 Hz, 2 Hz);
2.14 (2H, doublet, J=12 Hz);
1.91 (2H, doublet of quartets, J=12 Hz, 4 Hz).

Synthetic Example 5

2-(4-Fluorophenyl)-3-(pyridin-4-yl)-4-(3-trifluoroacetylaminopropyl)-1*H*-pyrrole

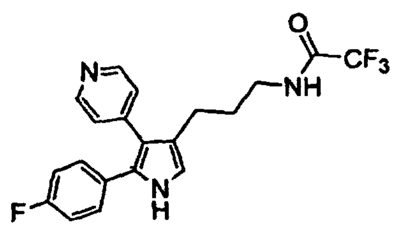

3.3 ml (23.2 mmol) of trifluoroacetic anhydride were added to a solution of 685 mg (2.32 mmol) of 4-(3-aminoprapyl)-2-(4-fluorophenyl)-3-(pyridin-4-yl)-1*H*-pyrrole (which was prepared as described in Synthetic Example 1 above) in 70 ml of tetrahydrofuran. The resulting mixture was stirred at room temperature for 30 minutes. At the end of this time, 200 ml of a saturated aqueous solution of sodium hydrogencarbonate were added to the reaction mixture, and then this was extracted with ethyl acetate. The organic extract was washed with water and concentrated by evaporation under reduced pressure. The resulting residue was purified by chromatography on a silica gel column using a 1:1 by volume mixture of hexane and ethyl acetate as the eluant to give 280 mg (yield 31%) of the title compound as a white powder.
Melting point: 229 - 230°C
$^1$H-Nuclear magnetic resonance spectrum (500 MHz, $CDCl_3$) δ ppm:

10.09 (1H, broad singlet);
8.49 (2H, doublet, J=6 Hz);
7.93 (1H, broad singlet);
7.18 (2H, doublet of doublets, J=9 Hz, 5 Hz);
7.13 (2H, doublet, J=6 Hz);
6.94 (2H, triplet, J=9 Hz);
6.74 (1H, doublet, J=3 Hz);

3.29 (2H, doublet of doublets, J=13 Hz, 7 Hz);
2.56 (2H, triplet, J=8 Hz);
1.74 (2H, quintet, J=8 Hz).

Synthetic Example 6

2-(4-Fluorophenyl)-4-(3-methylaminopropyl)-3-(pyridin-4-yl)-1*H*-pyrrole

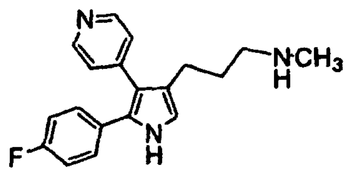

6(i) 4-[3-(t-Butoxycarbonylamino)propyl]-2-(4-fluorophenyl)-3-(pyridin-4-yl)-1*H*-pyrrole

In a similar manner to that described in Synthetic Example 5 above, a reaction was carried out using di-t-butyl dicarbonate instead of trifluoroacetic anhydride and using methanol instead of tetrahydrofuran to give the title compound (yield 68%) as a white powder.
$^1$H-Nuclear magnetic resonance spectrum (400 MHz, $CDCl_3$) δ ppm:

8.51 (2H, doublet, J=6 Hz);
8.26 (1H, broad singlet);
7.15-7.12 (4H, multiplet);
6.96 (2H, triplet, J=9 Hz);
6.75 (1H, doublet, J=3 Hz);
4.45 (1H, broad singlet);
3.12-3.09 (2H, multiplet);
2.54 (2H, triplet, J=8 Hz);
1.67-1.63 (2H, multiplet);
1.43 (9H, singlet).

6(ii) 2-(4-Fluorophenyl)-4-(3-methylaminopropyl)-3-(pyridin-4-yl)-1*H*-pyrrole

400 mg (1.01 mmol) of 4-[3-(t-butoxycarbonylamino)propyl]-2-(4-fluorophenyl)-3-(pyridin-4-yl)-1*H*-pyrrole [prepared as described in step 6(i) above] were added to a suspension of 154 mg of lithium aluminum hydride in 8 ml of tetrahydrofuran. The resulting mixture was heated under reflux for 2 hours. At the end of this time, the reaction mixture was worked up and then purified in the same manner as that described in Example 1(vi) above to give 300 mg (yield 96%) of the title compound as a white powder.
Melting point: 193 -198°C
$^1$H-Nuclear magnetic resonance spectrum (400 MHz, $CDCl_3$) δ ppm:

8.51 (2H, doublet, J=6 Hz);
8.34 (1H, broad singlet);
7.17-7.12 (4H, multiplet);
6.96 (2H, triplet, J=9 Hz);
6.74 (1H, doublet, J=3 Hz);
2.57 (2H, triplet, J=7 Hz);
2.54 (2H, triplet, J=7 Hz);
2.38 (3H, singlet);
1.69 (2H, quintet, J=7 Hz).

Example 9

4-(1-Acetyl-1,2,3,6-tetrahydropyridin-4-yl)-2-(4-fluorophenyl)-3-(pyridin-4-yl)-1*H*-pyrrole (Compound No. 1-2525)

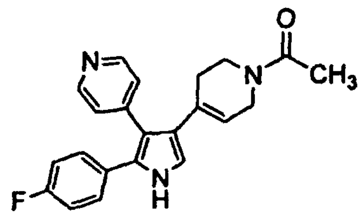

9(i) 4-(1-Benzyl-4-hydroxypiperidin-4-yl)-2-(4-fluorophenyl)-3-(pyridin-4-yl)-1-triisopropylsilyl-1*H*-pyrrole

In a similar manner to that described in Synthetic Example 2(iv) above, a reaction was carried out using 1-benzylpiperidine-4-one instead of pentafluoropyridine to afford the title compound (yield 61%) as white needle-like crystals.
$^1$H-Nuclear magnetic resonance spectrum (500 MHz, $CD_3OD$) δ ppm:

8.21 (2H, doublet, J=6 Hz);
7.35-7.15 (9H, multiplet);
6.93 (2H, triplet, J=9 Hz);
6.90 (1H, singlet);
3.49 (2H, singlet);
2.58-2.41 (4H, broad multiplet);
2.00-1.91 (2H, broad multiplet);
1.83-1.74 (2H, broad multiplet);
1.20-1.10 (3H, multiplet);
1.06 (18H, doublet, J=7 Hz).
5.51-5.47 (1H, broad multiplet);
3.72 (2H, doublet, J=3 Hz);
3.42 (2H, triplet, J=6 Hz);
2.66-2.55 (2H, broad multiplet).

Example 32

2-(4-Fluorophenyl)-4-(1-methyl-1,2,3,6-tetrahydropyridin-4-yl)-3-(pyridin-4-yl)-1*H*-pyrrole (Compound No. 1-145)

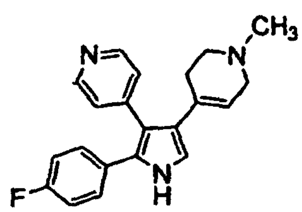

32(i) 2-(4-Fluorophenyl)-4-(4-hydroxy-1-methylpiperidin-4-yl)-3-(pyridin-4-yl)-1-triisopropylsilyl-1*H*-pyrrole

In a similar manner to that described in Synthetic Example 2(iv) above, a reaction was carried out using 1-methylpiperidine-4-one instead of pentafluoropyridine to afford the title compound (yield 47%) as a pale brown pow-

der.
$^1$H-Nuclear magnetic resonance spectrum (500 MHz, $CD_3OD$) δ ppm:

8.22 (2H, doublet, J=6 Hz);
7.27 (2H, doublet, J=6 Hz);
7.21 (2H, doublet of doublets, J=9 Hz, 5 Hz);
6.94 (2H, triplet, J=9 Hz);
6.92 (1H, singlet);
2.56-2.42 (4H, broad multiplet);
2.22 (3H, singlet);
2.00-1.91 (2H, broad multiplet);
1.84-1.78 (2H, broad multiplet);
1.20-1.11 (3H, multiplet);
1.06 (18H, doublet, J=7 Hz).

32(ii) 2-(4-Fluorophenyl)-4-(1-methyl-1,2,3,6-tetrahydropyridin-4-yl)-3-(pyridin-4-yl)-1*H*-pyrrole

In a similar manner to that described in Example 9(iii) above, using 2-(4-fluorophenyl)-4-(4-hydroxy-1-methylpiperidin-4-yl)-3-(pyridin-4-yl)-1-triisopropylsilyl-1*H*-pyrrole [prepared as described in step 32(i) above], dehydration with triethylsilane/trifluoroacetic acid and desilylation with tetrabutylammonium fluoride were carried out to give the title compound (yield 91%) as a pale yellow powder.
Melting point: 230 - 232°C (decomposition)
$^1$H-Nuclear magnetic resonance spectrum (500 MHz, DMSO-$d_6$) δ ppm:

11.36 (1H, broad singlet);
8.45 (2H, doublet, J=6 Hz);
7.19-7.14 (6H, multiplet);
6.90 (1H, doublet, J=3 Hz);
5.24-5.21 (1H, broad multiplet);
2.78-2.75 (2H, broad multiplet);
2.40 (2H, triplet, J=5 Hz);
2.18 (3H, singlet);
2.20-2.13 (2H, broad multiplet).

Example 47

2-(4-Fluorophenyl)-3-(pyridin-4-yl)-4-(1,2,3,6-tetrahydro-2,2,6,6-tetramethylpyridin-4-yl)-1*H*-pyrrole (Compound No. 1-2650)

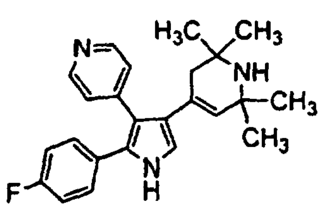

In a similar manner to the procedures described in Examples 9(i) and 9(iii) above, coupling, dehydration and desilylation reactions were carried out using 2,2,6,6-tetramethylpiperidine-4-one as a starting material instead of 1-benzylpiperidine-4-one to give the title compound (yield 50%) as a white powder.
Melting point: 258 - 259°C
$^1$H-Nuclear magnetic resonance spectrum (500 MHz, DMSO-$d_6$) δ ppm:

11.37 (1H, broad singlet);

8.42 (2H, doublet, J=6 Hz);
7.19-7.09 (6H, multiplet);
6.86 (1H, doublet, J=2 Hz);
5.04-5.02 (1H, broad multiplet);
1.93 (2H, singlet);
1.25-1.15 (1H, broad singlet);
1.04 (6H, singlet);
0.94 (6H, singlet).

Example 49

(±)-2-(4-Fluorophenyl)-4-(2-methyl-1,2,3,6-tetrahydropyridin-4-yl)-3-(pyridin-4-yl)-1*H*-pyrrole (Compound No. 1-2707)

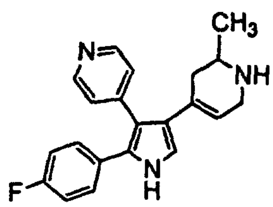

In a similar manner to the procedures described in Examples 9(i), 9(ii) and 9(iii) above, coupling, debenzylation, dehydration and desilylation reactions were carried out using (±)-1-benzyl-2-methylpiperidine-4-one as a starting material instead of 1-benzylpiperidine-4-one to afford a mixture of the title compound and the (6-methyl-1,2,3,6-tetrahydropyridin-4-yl) isomer thereof (which is the title compound of Example 50). The mixture was separated by chromatography on a silica gel column using a 25:1 by volume mixture of ethyl acetate and isopropylamine as the eluant to give the title compound (yield 3%, Rf value= 0.50) as a white powder.
Melting point: 178 - 180°C (decomposition)
$^1$H-Nuclear magnetic resonance spectrum (500 MHz, $CD_3OD$) δ ppm:

8.36 (2H, doublet, J=6 Hz);
7.24 (2H, doublet, J=6 Hz);
7.17 (2H, doublet of doublets, J=9 Hz, 6 Hz);
7.00 (2H, triplet, J=9 Hz);
6.85 (1H, singlet);
5.40-5.35 (1H, broad multiplet);
3.46-3.38 (1H, multiplet);
3.36-3.28 (1H, multiplet);
2.96-2.88 (1H, multiplet);
2.23-2.15 (1H, multiplet);
2.06-1.97 (1H, multiplet);
1.14 (3H, doublet, J=6 Hz).

Example 50

(±)-2-(4-Fluorophenyl)-4-(6-methyl-1,2,3,6-tetrahydropyridin-4-yl)-3-(pyridin-4-yl)-1*H*-pyrrole (Compound No. 1-2665)

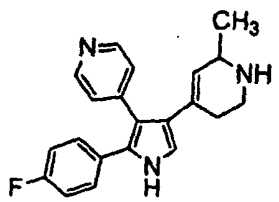

The title compound (yield 5%, Rf value = 0.45) was obtained as a white powder during the chromatography performed in Example 49 above.
Melting point: 201 - 204°C (decomposition)
$^1$H-Nuclear magnetic resonance spectrum (500 MHz, $CD_3OD$) δ ppm:

8.36 (2H, doublet, J=6 Hz);
7.24 (2H, doublet, J=6 Hz);
7.18 (2H, doublet of doublets, J=9 Hz, 6 Hz);
7.00 (2H, triplet, J=9 Hz);
6.86 (1H, singlet);
5.24-5.20 (1H, multiplet);
3.48-3.40 (1H, multiplet);
3.16-3.09 (1H, multiplet);
2.90-2.82 (1H, multiplet);
2.37-2.27 (1H, multiplet);
2.18-2.10 (1H, multiplet);
1.05 (3H, doublet, J=7 Hz).

Example 52

4-(1-Ethyl-1,2,3,6-tetrahydropyridin-4-yl)-2-(4-fluorophenyl)-3-(pyridin-4-yl)-1*H*-pyrrole (Compound No. 1-2629)

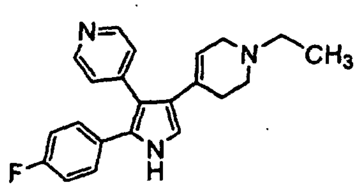

In a similar manner to the procedures described in Examples 9(i) and 9(iii) above, coupling, dehydration and desilylation reactions were carried out using 1-ethylpiperidine-4-one as a starting material instead of 1-benzylpiperidine-4-one to give the title compound (yield 55%) as a pale yellow powder.
Melting point: 234 - 236°C (decomposition)
$^1$H-Nuclear magnetic resonance spectrum (500 MHz, DMSO-$d_6$) δ ppm:

11.36 (1H, broad singlet);
8.45 (2H, doublet, J=6 Hz);
7.18-7.08 (6H, multiplet);
6.90 (1H, doublet, J=3 Hz);
5.26-5.22 (1H, broad multiplet);

2.84-2.79 (2H, broad multiplet);
2.45 (2H, triplet, J=6 Hz);
2.34 (2H, quartet, J=8 Hz);
2.18-2.12 (2H, broad multiplet);
0.99 (3H, triplet, J=8 Hz).

Example 54

2-(4-Fluorophenyl)-4-(1-isopropyl-1,2,3,6-tetrahydropyridin-4-yl)-3-(pyridin-4-yl)-1*H*-pyrrole (Compound No. 1-2632)

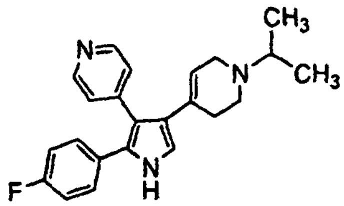

In a similar manner to the procedures described in Examples 9(i) and 9(iii) above, coupling, dehydration and desilylation reactions were carried out using 1-isopropylpiperidine-4-one as a starting material instead of 1-benzylpiperidine-4-one to give the title compound (yield 42%) as a pale yellow powder.
Melting point: 237 - 240°C (decomposition)
$^1$H-Nuclear magnetic resonance spectrum (500 MHz, DMSO-$d_6$) δ ppm:

11.35 (1H, broad singlet);
8.45 (2H, doublet, J=6 Hz);
7.18-7.08 (6H, multiplet);
6.88 (1H, doublet, J=3 Hz);
5.28-5.24 (1H, broad multiplet);
2.94-2.89 (2H, broad multiplet);
2.64 (1H, septet, J=6 Hz);
2.49 (2H, triplet, J=6 Hz);
2.15-2.09 (2H, broad multiplet);
0.96 (6H, doublet, J=6 Hz).

Example 56

2-(4-Fluorophenyl)-4-(1-propyl-1,2,3,6-tetrahydropyridin-4-yl)-3-(pyridin-4-yl)-1*H*-pyrrole (Compound No. 1-2630)

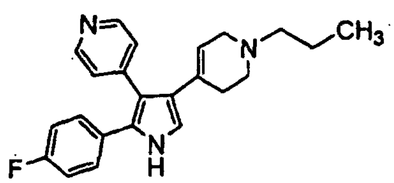

In a similar manner to the procedures described in Examples 9(i) and 9(iii) above, coupling, dehydration and desilylation reactions were carried out using 1-propylpiperidine-4-one as a starting material instead of 1-benzylpiperidine-4-one to give the title compound (yield 46%) as a white powder.
Melting point: 236 - 238°C (decomposition)
$^1$H-Nuclear magnetic resonance spectrum (500 MHz, DMSO-$d_6$) δ ppm:

11.36 (1H, broad singlet);
8.45 (2H, doublet, J=6 Hz);
7.18-7.08 (6H, multiplet);
6.90 (1H, doublet, J=3 Hz);
5.26-5.23 (1H, multiplet);
2.84-2.79 (2H, multiplet);
2.44 (2H, triplet, J=5 Hz);
2.25 (2H, triplet, J=8 Hz);
2.17-2.11 (2H, broad multiplet);
1.43 (2H, triplet of quartets, J=8 Hz, 8 Hz);
0.84 (3H, triplet, J=8 Hz).

Example 57

4-(1-Benzyl-1,2,3,6-tetrahydropyridin-4-yl)-2-(4-fluorophenyl)-3-(pyridin-4-yl)-1*H*-pyrrole (Compound No. 1-2644)

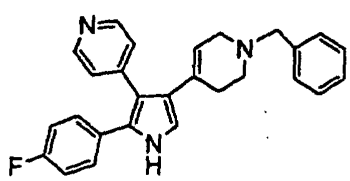

In a similar manner to that described in Example 9(iii) above, dehydration and desilylation reactions were carried out using 4-(1-benzyl-4-hydroxypiperidin-4-yl)-2-(4-fluorophenyl)-3-(pyridin-4-yl)-1-triisopropylsilyl-1*H*-pyrrole [prepared as described in Example 9(i) above] to give the title compound (yield 88%) as a pale yellow powder.
Melting point: 217 - 219°C (decomposition).
$^1$H-Nuclear magnetic resonance spectrum (500 MHz, DMSO-$d_6$) δ ppm:

11.37 (1H, broad singlet);
8.45 (2H, doublet, J=6 Hz);
7.34-7.21 (5H, multiplet);
7.18-7.08 (6H, multiplet);
6.90 (1H, doublet, J=3 Hz);
5.23-5.21 (1H, broad multiplet);
3.50 (2H, singlet);
2.86-2.81 (2H, broad multiplet)
2.48 (2H, triplet, J=6 Hz);
2.18-2.12 (2H, broad multiplet).

Example 58

2-(4-Fluorophenyl)-4-(1-phenethyl-1,2,3,6-tetrahydropyridin-4-yl)-3-(pyridin-4-yl)-1*H*-pyrrole (Compound No. 1-2645)

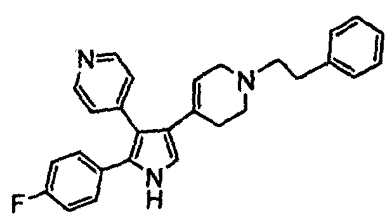

In a similar manner to the procedures described in Examples 9(i) and 9(iii) above, coupling, dehydration and desilylation reactions were carried out using 1-phenethylpiperidine-4-one as a starting material instead of 1-benzylpiperidine-4-one to give the title compound (yield 46%) as a pale brown powder.
Melting point: 219 - 221 °C (decomposition)
$^1$H-Nuclear magnetic resonance spectrum (500 MHz, DMSO-$d_6$) δ ppm:

11.37 (1H, broad singlet);
8.45 (2H, doublet, J=6 Hz);
7.29-7.09 (11H, multiplet);
6.90 (1H, doublet, J=3 Hz);
5.28-5.24 (1H, broad multiplet);
2.95-2.89 (2H, broad multiplet);
2.76-2.69 (2H, multiplet);
2.57-2.51 (4H, multiplet);
2.19-2.12 (2H, broad multiplet).

Example 59

(±)-2-(4-Fluorophenyl)-4-(1,2,3,5,8,8a-hexahydroindolizin-7-yl)-3-(pyridin-4-yl)-1*H*-pyrrole (Compound No. 1-2654)

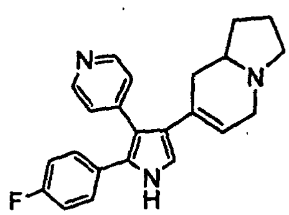

In a similar manner to the procedures described in Examples 9(i) and 9(iii) above, coupling, dehydration and desilylation reactions were carried out using (±)-1,2,3,5,6,7,8,8a-octahydroindolizine-7-one as a starting material instead of 1-benzylpiperidine-4-one to give a mixture of the title compound and the (1,2,3,5,6,8a-hexahydroindolizin-7-yl) isomer thereof (which is the compound of Example 60 below). The mixture was separated by chromatography on a silica gel column using a 10:1:1 by volume mixture of ethyl acetate, methanol and isopropylamine as the eluant to give the title compound (yield 24%, Rf value = 0.50) as a white powder.
Melting point: 220 - 222°C (decomposition)
$^1$H-Nuclear magnetic resonance spectrum (500 MHz, $CD_3OD$) δ ppm:

8.36 (2H, doublet, J=6 Hz);
7.24 (2H, doublet, J=6 Hz);
7.17 (2H, doublet of doublets, J=9 Hz, 6 Hz);
7.00 (2H, triplet, J=9 Hz);
6.85 (1H, singlet);
5.41-5.37 (1H, multiplet);
3.47-3.40 (1H, multiplet);
3.18-3.12 (1H, multiplet);
2.84-2.77 (1H, multiplet);
2.40-2.26 (2H, multiplet);
2.23-2.11 (2H, multiplet);
2.05-1.97 (1H, multiplet);
1.90-1.75 (2H, multiplet);
1.46-1.36 (1H, multiplet).

Example 60

(±)-2-(4-Fluorophenyl)-4-(1,2,3,5,6,8a-hexahydroindolizin-7-yl)-3-(pyridin-4-yl)-1*H*-pyrrole (Compound No. 1-2653)

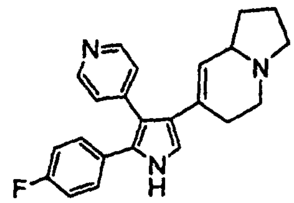

The title compound (yield 27%, Rf value = 0.40) was obtained as a pale yellow powder during the chromatography performed in Example 59 above.
Melting point: 188 - 190°C (decomposition)
$^1$H-Nuclear magnetic resonance spectrum (500 MHz, $CD_3OD$) δ ppm:

8.37 (2H, doublet, J=6 Hz);
7.23 (2H, doublet, J=6 Hz);
7.18 (2H, doublet of doublets, J=9 Hz, 5 Hz);
7.00 (2H, triplet, J=9 Hz);
6.86 (1H, singlet);
5.34-5.30 (1H, broad multiplet);
3.38-3.30 (1H, multiplet);
2.99-2.86 (2H, multiplet);
2.82-2.73 (2H, multiplet);
2.40-2.31 (1H, multiplet);
2.25-2.16 (1H, multiplet);
1.97-1.86 (2H, multiplet);
1.84-1.73 (1H, multiplet);
1.48-1.38 (1H, multiplet).

Example 61

(±)-4-(1,6-Dimethyl-1,2,3,6-tetrahydropyridin-4-yl)-2-(4-fluorophenyl)-3-(pyridin-4-yl)-1*H*-pyrrole (Compound No. 1-2666)

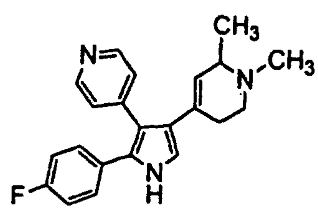

In a similar manner to the procedures described in Examples 9(i) and 9(iii) above, coupling, dehydration and desilylation reactions were carried out using (±)-1,2-dimethylpiperidine-4-one as a starting material instead of 1-benzylpiperidine-4-one to give a mixture of the title compound and the (1,2-dimethyl-1,2,3,6-tetrahydropyridin-4-yl) isomer thereof (which is the compound of Example 62 below). The mixture was separated by chromatography on a silica gel column using a 99:1 by volume mixture of ethyl acetate and isopropylamine as the eluant to give the title compound (yield 10%, Rf value = 0.40) as a pale yellow powder.
Melting point: 208 - 209°C (decomposition)

$^1$H-Nuclear magnetic resonance spectrum (500 MHz, $CDCl_3$) δ ppm:

8.47 (2H, doublet, J=6 Hz);
8.29-8.10 (1H, broad singlet);
7.17 (2H, doublet, J=6 Hz);
7.13 (2H, doublet of doublets, J=9 Hz, 5 Hz);
6.97 (2H, triplet, J=9 Hz);
6.83 (1H, doublet, J=3 Hz);
5.19 (1H, singlet);
2.90-2.83 (1H, multiplet);
2.55-2.32 (2H, multiplet);
2.37 (3H, singlet);
2.17-2.07 (2H, multiplet);
1.05 (3H, doublet, J=7 Hz).

Example 62

(±)-4-(1,2-Dimethyl-1,2,3,6-tetrahydropyridin-4-yl)-2-(4-fluorophenyl)-3-(pyridin-4-yl)-1*H*-pyrrole (Compound No. 1-2708)

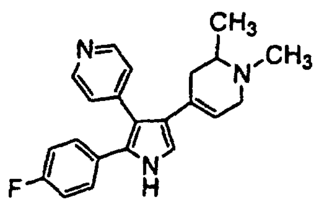

The title compound (yield 14%, Rf value = 0.30) was obtained as a pale yellow powder during the chromatography performed in Example 61 above.
Melting point: 198 - 201 °C
$^1$H-Nuclear magnetic resonance spectrum (400 MHz, $CDCl_3$) δ ppm:

8.47 (2H, doublet, J=6 Hz);
8.33 (1H, broad singlet);
7.17 (2H, doublet, J=6 Hz);
7.12 (2H, doublet of doublets, J=9 Hz, 5 Hz);
6.97 (2H, triplet, J=9 Hz);
6.82 (1H, doublet, J=3 Hz);
5.40 (1H, singlet);
3.22-3.18 (1H, multiplet);
2.95-2.91 (1H, multiplet);
2.57-2.46 (1H, multiplet);
2.34 (3H, singlet);
2.27-2.23 (1H, multiplet);
2.11-2.09 (1H, multiplet);
1.08 (3H, doublet, J=6 Hz).

Example 63

2-(4-Fluorophenyl)-4-(1,2,2,6,6-pentamethyl-1,2,3,6-tetrahydropyridin-4-yl)-3-(pyridin-4-yl)-1*H*-pyrrole(Compound No. 1-2651)

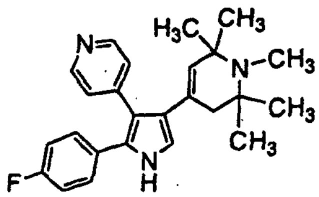

In a similar manner to the procedures described in Example 9(i) and 9(iii) above, coupling, dehydration and desilylation reactions were carried out using 1,2,2,6,6-pentamethylpiperidine-4-one as a starting material instead of 1-benzylpiperidine-4-one to give the title compound (yield 69%) as a white powder.
Melting point: 264 - 268°C (decomposition)
$^1$H-Nuclear magnetic resonance spectrum (500 MHz, DMSO-$d_6$) δ ppm:

11.38 (1H, broad singlet);
8.43 (2H, doublet, J=6 Hz);
7.20-7.09 (6H, multiplet);
6.88 (1H, doublet, J=3 Hz);
4.88 (1H, singlet);
2.18 (3H, singlet);
2.07 (2H, singlet);
1.01 (6H, singlet);
0.90 (6H, singlet).

Example 65

2-(4-Fluorophenyl)-3-(pyridin-4-yl)-4-(1,2,3,6-tetrahydropyridin-5-yl)-1*H*-pyrrole (Compound No. 1-2627)

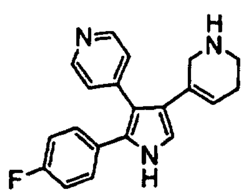

In a similar manner to the procedures described in Example 9(i), 9(ii) and 9(iii) above, coupling, debenzylation, dehydration and desilylation reactions were carried out using 1-benzylpiperidine-3-one as a starting material instead of 1-benzylpiperidine-4-one to give the title compound (yield 12%) as a pale brown powder.
Melting point: 230 - 233°C (decomposition)
$^1$H-Nuclear magnetic resonance spectrum (500 MHz, $CD_3OD$) δ ppm:

8.36 (2H, doublet, J=6 Hz);
7.24 (2H, doublet, J=6 Hz);
7.18 (2H, doublet of doublets, J=9 Hz, 6 Hz);
7.01 (2H, triplet, J=9 Hz);
6.82 (1H, singlet);

5.55-5.50 (1H, broad multiplet);
3.36-3.30 (2H, multiplet);
2.90 (2H, triplet, J=6 Hz);
2.16-2.09 (2H, broad multiplet).

Example 73

2-(4-Fluorophenyl)-3-[2-(1(S)-phenylethylamino)pyridin-4-yl]-4-(1,2,3,6-tetrahydropyridin-4-yl)-1*H*-pyrrole (Compound No. 1-2600)

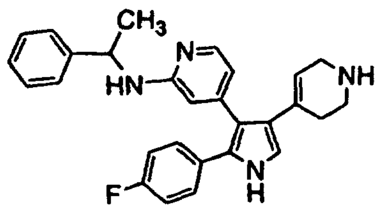

73(i) 4-Bromo-2-(4-fluorophenyl)-3-(2-fluoropyridin-4-yl)-1-triisopropylsilyl-1*H*-pyrrole

In a similar manner to the procedures described in Examples 1(i), 2(i), 2(ii) and 2(iii) above, a pyrrole ring-forming reaction, a decarboxylation reaction, a silylation reaction and a bromination reaction were carried out using ethyl 3-(2-fluoropyridin-4-yl)acrylate as a starting material instead of ethyl 3-(4-pyridyl)acrylate to give the title compound (yield 48%) as a pale yellow powder.
$^1$H-Nuclear magnetic resonance spectrum (400 MHz, DMSO-$d_6$) δ ppm:

8.05 (1H, doublet, J=5 Hz);
7.38 (2H, doublet of doublets, J=9 Hz, 5 Hz);
7.20 (2H, triplet, J=9 Hz);
7.15 (1H, singlet);
7.01-6.97 (1H, multiplet);
6.78 (1H, singlet);
1.20-1.07 (3H, multiplet);
0.99 (18H, doublet, J=7 Hz).

73(ii) 4-(1-Allyloxycarbonyl-1,2,3,6-tetrahydropyridin-4-yl)-2-(4-fluorophenyl)-3-(2-fluoropyridin-4-yl)-1*H*-pyrrole

In a similar manner to the procedures described in Examples 9(i) and 9(iii) above, coupling, dehydration and desilylation reactions were carried out using 4-bromo-2-(4-fluorophenyl)-3-(2-fluoropyridin-4-yl)-1-triisopropylsilyl-1*H*-pyrrole [prepared as described in step 73(i) above] instead of 4-bromo-2-(4-fluorophenyl)-3-(pyridin-4-yl)-1-triisopropylsilyl-1*H*-pyrrole and using 1-allyloxycarbonylpiperidine-4-one instead of 1-benzylpiperidine-4-one to give the title compound (yield 6%) as a white powder.
$^1$H-Nuclear magnetic resonance spectrum (500 MHz, $CDCl_3$) δ ppm:

8.42-8.31 (1H, broad singlet);
8.09 (1H, doublet, J=5 Hz);
7.15 (2H, doublet of doublets, J=9 Hz, 5 Hz);
7.04-6.97 (3H, multiplet);
6.82 (1H, singlet);
6.77 (1H, singlet);
6.00-5.89 (1H, multiplet);
5.63-5.39 (1H, multiplet);
5.31 (1H, doublet, J=16 Hz);
5.21 (1H, doublet, J=11 Hz);
4.62 (2H, doublet, J=5 Hz);

4.04-3.96 (2H, broad singlet);
3.63-3.52 (2H, broad singlet);
2.29-2.12 (2H, multiplet).

73(iii) 4-(1-Allyloxycarbonyl-1,2,3,6-tetrahydropyridin-4-yl)-2-(4-fluorophenyl)-3-[2-(1(S)-phenylethylamino)pyridin-4-yl]-1*H*-pyrrole

A mixture of 360 mg (0.85 mmol) of 4-(1-allyloxycarbonyl-1,2,3,6-tetrahydropyridin-4-yl)-2-(4-fluorophenyl)-3-(2-fluoropyridin-4-yl)-1*H*-pyrrole [prepared as described in step 73 (ii) above], 3.6 ml (28.3 mmol) of 1(S)-phenylethylamine and 0.36 ml of concentrated hydrochloric acid was stirred at 150°C for 10 hours. At the end of this time, a saturated aqueous solution of sodium hydrogencarbonate was added to the reaction mixture and then this was extracted with ethyl acetate. The organic extract was washed with water, dried over anhydrous magnesium sulfate and then concentrated by evaporation under reduced pressure. The residue thus obtained was purified by chromatography on a silica gel column using a 1:1 by volume mixture of hexane and ethyl acetate as the eluant to give 229 mg (yield 49%) of the title compound as a brown amorphous solid.
$^1$H-Nuclear magnetic resonance spectrum (500 MHz, $CDCl_3$) δ ppm:

8.20-8.12 (1H, broad singlet);
7.96 (1H, doublet, J=5 Hz);
7.25 (4H, singlet);
7.22-7.16 (1H, multiplet);
7.04 (2H, doublet of doublets, J=9 Hz, 5 Hz);
6.89 (2H, triplet, J=9 Hz);
6.74 (1H, singlet);
6.44 (1H, doublet, J=4 Hz);
6.10 (1H, singlet);
6.00-5.89 (1H, multiplet);
5.54-5.33 (1H, multiplet);
5.32 (1H, doublet, J=16 Hz);
5.22 (1H, doublet, J=11 Hz);
5.05 (1H, broad doublet, J=6 Hz);
4.62 (2H, doublet, J=5 Hz);
4.54-4.46 (1H, multiplet);
3.93-3.83 (2H, multiplet);
3.50-3.38 (2H, multiplet);
2.20-2.05 (2H, multiplet);
1.48 (3H, doublet, J=7 Hz).

73(iv) 2-(4-Fluorophenyl)-3-[2-(1(S)-phenylethylamino)pyridin-4-yl]-4-(1,2,3,6-tetrahydropyridin-4-yl)-1*H*-pyrrole

A mixture of 229 mg (0.44 mmol) of 4-(1-allyloxycarbonyl-1,2,3,6-tetrahydropyridin-4-yl)-2-(4-fluorophenyl)-3-[2-(1(S)-phenylethylamino)pyridin-4-yl]-1*H*-pyrrole[prepared as described in step 73(iii) above] in 2.5 ml of a 10:1 by volume mixture of dioxane and water, 55 μl (0.66 mmol) of pyrrolidine and 5 mg (0.0044 mmol) of tetrakis(triphenylphosphine)palladium (0) was stirred at 0°C for 10 minutes. At the end of this time, water was added to the reaction mixture and then this was extracted with ethyl acetate. The organic extract was washed with water and then concentrated by evaporation under reduced pressure. The residue thus obtained was purified by chromatography on a silica gel column using a 10:1:1 by volume mixture of ethyl acetate, methanol and isopropylamine as the eluant to give 41 mg (yield 21%) of the title compound as a pale brown amorphous solid.
$^1$H-Nuclear magnetic resonance spectrum (500 MHz, $CDCl_3$) δ ppm:

8.19-8.01 (1H, broad singlet);
7.96 (1H, doublet, J=5 Hz);
7.26 (4H, singlet);
7.24-7.18 (1H, multiplet);
7.06 (2H, doublet of doublets, J=9 Hz, 5 Hz);
6.89 (2H, triplet, J=9 Hz);
6.74 (1H, singlet);
6.47 (1H, doublet, J=4 Hz);

6.15 (1H, singlet);
5.47 (1H, doublet, J=3 Hz);
5.05 (1H, broad doublet, J=6 Hz);
4.60-4.52 (1H, multiplet);
3.31-3.26 (2H, multiplet);
2.94-2.84 (2H, multiplet);
2.13-2.07 (2H, multiplet);
1.48 (3H, doublet, J=7 Hz).

Example 74

4-(1-t-Butyl-1,2,3,6-tetrahydropyridin-4-yl)-2-(4-fluorophenyl)-3-(pyridin-4-yl)-1*H*-pyrrole (Compound No. 1-2635)

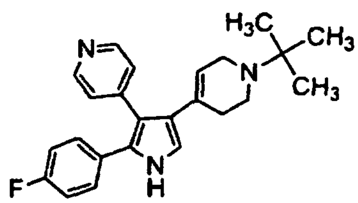

In a similar manner to the procedures described in Examples 9(i), 9(ii) and 9(iii) above, coupling, dehydration and desilylation reactions were carried out using 1-t-butylpiperidine-4-one as a starting material instead of 1-benzylpiperidine-4-one to give the title compound (yield 20%) as a pale yellow powder.
Melting point: 242 - 244°C
$^1$H-Nuclear magnetic resonance spectrum (400 MHz, $CDCl_3$-DMSO-$d_6$) $\delta$ ppm:

9.72 (1H, broad singlet);
8.46 (2H, doublet, J=6 Hz);
7.19 (2H, doublet, J=6 Hz);
7.16 (2H, doublet of doublets, J=9 Hz, 5 Hz);
6.94 (2H, triplet, J=9 Hz);
6.81 (1H, doublet, J=3 Hz);
5.46 (1H, singlet);
3.19-3.11 (2H, multiplet);
2.66-2.58 (2H, multiplet);
2.26-2.18 (2H, multiplet);
1.10 (9H, singlet).

Example 75

2-(4-Fluorophenyl)-4-(1-octyl-1,2,3,6-tetrahydropyridin-4-yl)-3-(pyridin-4-yl)-1*H*-pyrrole (Compound No. 1-2639)

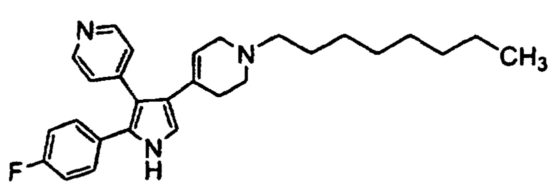

In a similar manner to the procedures described in Examples 9(i) and 9(iii) above, coupling, dehydration and desilylation reactions were carried out using 1-octylpiperidine-4-one as a starting material instead of 1-benzylpiperidine-4-one to give the title compound (yield 26%) as a white powder.
Melting point: 173 - 175°C
$^1$H-Nuclear magnetic resonance spectrum (400 MHz, $CDCl_3$) δ ppm:

8.47 (2H, doublet, J=6 Hz);
8.29 (1H, broad singlet);
7.17 (2H, doublet, J=6 Hz);
7.12 (2H, doublet of doublets, J=9 Hz, 5 Hz);
6.96 (2H, triplet, J=9 Hz);
6.82 (1H, doublet, J=3 Hz);
5.45 (1H, singlet);
2.99 (2H, broad doublet, J=3 Hz);
2.56 (2H, triplet, J=6 Hz);
2.39 (2H, triplet, J=8 Hz);
2.28-2.23 (2H, multiplet);
1.55-1.48 (2H, multiplet);
1.31-1.22 (10H, multiplet);
0.88 (3H, triplet, J=7 Hz).

Example 76

(±)-4-(1-Ethyl-6-methyl-1,2,3,6-tetrahydropyridin-4-yl)-2-(4-fluorophenyl)-3-(pyridin-4-yl)-1*H*-pyrrole (Compound No. 1-2667)

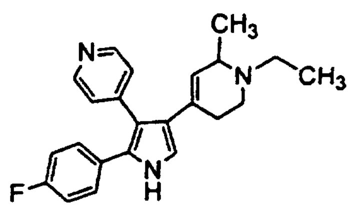

In a similar manner to the procedures described in Examples 9(i) and 9(iii) above, coupling, dehydration and desilylation reactions were carried out using (±)-1-ethyl-2-methylpiperidine-4-one as a starting material instead of 1-benzylpiperidine-4-one to afford a mixture of the title compound and the (1-ethyl-2-methyl-1,2,3,6-tetrahydropyridin-4-yl) isomer thereof (which is the compound of Example 77 below). The mixture was separated by chromatography on a silica gel column using a 50:1 by volume mixture of ethyl acetate and isopropylamine as the eluant to give the title compound (yield 33%, Rf value = 0.20) as a pale yellow powder.
Melting point: 210 - 212°C (decomposition)
$^1$H-Nuclear magnetic resonance spectrum (400 MHz, $CDCl_3$) δ ppm:

8.47 (2H, doublet, J=6 Hz);
8.31 (1H, broad singlet);
7.17 (2H, doublet, J=6 Hz);
7.13 (2H, doublet of doublets, J=9 Hz, 5 Hz);
6.97 (2H, triplet, J=9 Hz);
6.82 (1H, singlet);
5.27 (1H, singlet);
3.09-3.00 (1H, multiplet);
2.97-2.88 (1H, multiplet);
2.84-2.73 (1H, multiplet);

2.48-2.24 (3H, multiplet);
2.22-2.11 (1H, multiplet);
1.09 (3H, triplet, J=7 Hz);
1.04 (3H, doublet, J=7 Hz).

Example 77

(±)-4-(1-Ethyl-2-methyl-1,2,3,6-tetrahydropyridin-4-yl)-2-(4-fluorophenyl)-3-(pyridin-4-yl)-1*H*-pyrrole (Compound No. 1-2709)

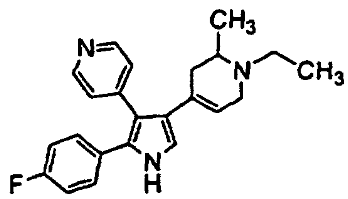

The title compound (yield 46%, Rf value = 0.15) was obtained as an orange powder during the chromatography performed in Example 76.
Melting point: 218 - 220°C (decomposition)
$^1$H-Nuclear magnetic resonance spectrum (400 MHz, $CDCl_3$) δ ppm:

8.47 (2H, doublet, J=6 Hz);
8.32 (1H, broad singlet);
7.17 (2H, doublet, J=6 Hz);
7.12 (2H, doublet of doublets, J=9 Hz, 5 Hz);
6.96 (2H, triplet, J=9 Hz);
6.82 (1H, singlet);
5.44 (1H, singlet);
3.16-2.99 (2H, multiplet);
2.90-2.79 (1H, multiplet);
2.76-2.64 (1H, multiplet);
2.51-2.32 (2H, multiplet);
2.03-1.98 (1H, multiplet);
1.08 (3H, triplet, J=7 Hz);
1.01 (3H, doublet, J=6 Hz).

Example 79

(±)-4-(6-Allyl-1-methyl-1,2,3,6-tetrahydropyridin-4-yl)-2-(4-fluorophenyl)-3-(pyridin-4-yl)-1*H*-pyrrole (Compound No. 1-2690)

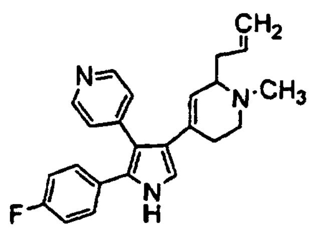

In a similar manner to the procedures described in Examples 9(i) and 9(iii) above, coupling, dehydration and desilylation reactions were carried out using (±)-2-allyl-1-methylpiperidine-4-one as a starting material instead of 1-benzylpiperidine-4-one to afford a mixture of the title compound and the (2-allyl-1-methyl-1,2,3,6-tetrahydropyridin-4-yl) isomer thereof (which is the compound of Example 80 below). The mixture was separated by chromatography on a silica gel column using a 100:1 by volume mixture of ethyl acetate and isopropylamine as the eluant to give the title compound (yield 20%, Rf value = 0.40) as a pale yellow powder.
Melting point : 218 - 220°C
$^1$H-Nuclear magnetic resonance spectrum (500 MHz, $CDCl_3$) δ ppm:

8.48 (2H, doublet, J=6 Hz);
8.39 (1H, broad singlet);
7.17 (2H, doublet, J=6 Hz);
7.11 (2H, doublet of doublets, J=9Hz, 5 Hz);
6.96 (2H, triplet, J=9 Hz);
6.83 (1H, doublet, J=3 Hz);
5.66-5.58 (1H, multiplet);
5.34 (1H, singlet);
4.96 (1H, doublet, J=10 Hz);
4.90 (1H, doublet, J=17 Hz);
2.89-2.87 (1H, multiplet);
2.71-2.67 (1H, multiplet);
2.46-2.43 (2H, multiplet);
2.37 (3H, singlet);
2.35-2.32 (1H, multiplet);
2.12-2.07 (2H, multiplet).

Example 80

(±)-4-(2-Allyl-1-methyl-1,2,3,6-tetrahydropyridin-4-yl)-2-(4-fluorophenyl)-3-(pyridin-4-yl)-1*H*-pyrrole (Compound No. 1-2732)

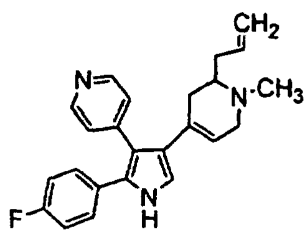

The title compound (yield 18%, Rf value = 0.35) was obtained as a pale yellow powder during the chromatography performed in Example 79 above.
Melting point : 178 - 180°C
$^1$H-Nuclear magnetic resonance spectrum (500 MHz, $CDCl_3$) δ ppm:

8.47 (2H, doublet, J=6 Hz);
8.38 (1H, broad singlet);
7.17 (2H, doublet, J=6 Hz);
7.12 (2H, doublet of doublets, J=9Hz, 5 Hz);
6.96 (2H, triplet, J=9 Hz);
6.82 (1H, doublet, J=3 Hz);
5.77-5.69 (1H, multiplet);
5.39 (1H, singlet);
5.03 (1H, doublet, J=4 Hz);

5.00 (1H, singlet);
3.19-3.15 (1H, multiplet);
2.99-2.95 (1H, multiplet);
2.57-2.41 (1H, multiplet);
2.36 (3H, singlet);
2.36-2.31 (1H, multiplet);
2.24-2.09 (3H, multiplet).

Example 81

(±)-4-(6-Benzyl-1-methyl-1,2,3,6-tetrahydropyridin-4-yl)-2-(4-fluorophenyl)-3-(pyridin-4-yl)-1*H*-pyrrole (Compound No. 1-2696)

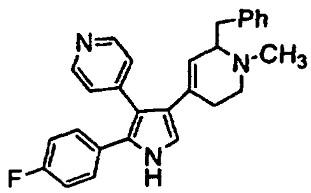

In a similar manner to the procedures described in Examples 9(i) and 9(iii) above, coupling, dehydration and desilylation reactions were carried out using (±)-2-benzyl-1-methylpiperidine-4-one as a starting material instead of 1-benzylpiperidine-4-one to afford a mixture of the title compound and the (2-benzyl-1-methyl-1,2,3,6-tetrahydropyridin-4-yl) isomer thereof (which is the compound of Example 82 below). The mixture was separated by chromatography on a silica gel column using a 100:1 by volume mixture of ethyl acetate and isopropylamine as the eluant to give the title compound (yield 11%, Rf value = 0.38) as a pale yellow powder.
Melting point : 198 - 200°C
$^1$H-Nuclear magnetic resonance spectrum (500 MHz, $CDCl_3$) δ ppm:

8.45 (2H, doublet, J=6 Hz);
8.28 (1H, broad singlet);
7.20-7.12 (3H, multiplet);
7.09 (2H, doublet, J=6 Hz);
7.10-7.07 (2H, multiplet);
6.97-6.91 (4H, multiplet);
6.76 (1H, doublet, J=3 Hz);
5.24 (1H, singlet);
3.13-3.10 (1H, multiplet);
2.94-2.89 (2H, multiplet);
2.51 (3H, singlet);
2.49-2.35 (3H, multiplet);
2.11-2.05 (1H, multiplet).

Example 82

(±)-4-(2-benzyl-1-methyl-1,2,3,6-tetrahydropyridin-4-yl)-2-(4-fluorophenyl)-3-(pyridin-4-yl)-1*H*-pyrrole (Compound No. 1-2738)

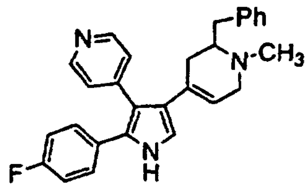

The title compound (yield 4%, Rf value = 0.33) was obtained as a pale yellow powder during the chromatography performed in Example 81 above.
Melting point: 198 - 199°C
$^1$H-Nuclear magnetic resonance spectrum (500 MHz, $CDCl_3$) δ ppm:

8.55 (1H, broad singlet);
8.43 (2H, doublet, J=6 Hz);
7.23 (2H, triplet, J=7 Hz);
7.16 (2H, triplet, J=7 Hz);
7.12 (2H, doublet, J=6 Hz);
7.10-7.05 (4H, multiplet);
6.94 (2H, triplet, J=9 Hz);
6.72 (1H, doublet, J=3 Hz);
5.40 (1H, singlet);
3.20-3.17 (1H, multiplet);
3.08-3.04 (2H, multiplet);
2.81-2.78 (1H, multiplet);
2.46 (3H, singlet);
2.43-2.38 (1H, multiplet);
2.10-2.00 (2H, multiplet).

Example 83

(-)-2-(4-Fluorophenyl)-4-(1,2,3,5,6,8a-hexahydroindolizin-7-yl)-3-(pyridin-4-yl)-1*H*-pyrrole (Compound No. 1-2653)

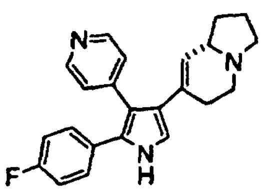

(±)-2-(4-Fluorophenyl)-4-(1,2,3,5,6,8a-hexahydroindolizin-7-yl)-3-(pyridin-4-yl)-1*H*-pyrrole (prepared as described in Example 60 above) was subjected to High Performance Liquid Chromatography using a chiral column and employing the conditions described below to give the title compound (retention time: 4.51 minutes, yield 94%) as a pale yellow powder.
Melting point: 204 - 205°C (Decomposition)
$[\alpha]^{27}_D$ -50.67° (c=0.975, methanol)
<HPLC>
Column: CHIRALPAK AD (product of Daicel Chemical Industries, Ltd.)

Eluant: n-hexane : ethanol = 80:20
Flow rate: 1.0 ml/minute
Temperature: 40°C
Detection: 254nm (UV).

Example 84

(+)-2-(4-Fluorophenyl)-4-(1,2,3,5,6,8a-hexahydroindolizin-7-yl)-3-(pyridin-4-yl)-1*H*-pyrrole (Compound No. 1-2653)

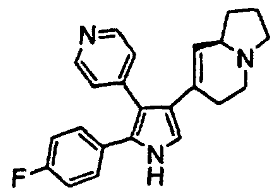

The title compound (retention time: 6.23 minutes, yield 95%) was obtained as a pale yellow powder during the High Performance Liquid Chromatography performed in Example 83 above.
Melting Point: 207-210°C (Decomposition)
$[\alpha]^{24}_D$ +46.59° (c=0.920, methanol)

Example 85

(±)-2-(4-Fluorophenyl)-4-(6-methyl-1-propyl-1,2,3,6-tetrahydropyridin-4-yl)-3-(pyridin-4-yl)-1*H*-pyrrole (Compound No. 1-2668)

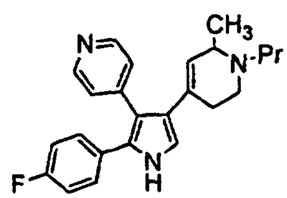

In a similar manner to the procedures described in Examples 9(i) and 9(iii) above, coupling, dehydration and desilylation reactions were carried out using (±)-2-methyl-1-propylpiperidine-4-one as a starting material instead of 1-benzylpiperidine-4-one to afford a mixture of the title compound and the (2-methyl-1-propyl-1,2,3,6-tetrahydropyridin-4-yl) isomer thereof (which is the compound of Example 86 below). The mixture was separated by chromatography on a silica gel column using a 100:1 by volume mixture of ethyl acetate and isopropylamine as the eluant to give the title compound (yield 6%, Rf value = 0.35) as a white powder.
Melting point : 214 - 215°C (decomposition)
$^1$H-Nuclear magnetic resonance spectrum (400 MHz, $CDCl_3$) δ ppm:

8.47 (2H, doublet, J=6 Hz);
8.22 ( 1 H, broad singlet);
7.17 (2H, doublet, J=6 Hz);
7.13 (2H, doublet of doublets, J=9Hz, 5 Hz);
6.97 (2H, triplet, J=9 Hz);
6.83 (1H, singlet);
5.27 (1H, singlet);
3.07-2.98 (1H, multiplet);

2.97-2.89 (1H, multiplet);
2.68-2.56 (1H, multiplet);
2.47-2.39 (1H, multiplet);
2.37-2.23 (2H, multiplet);
2.20-2.10 (1H, multiplet);
1.62-1.43 (2H, multiplet);
1.04 (3H, doublet, J=7 Hz);
0.90 (3H, triplet, J=7 Hz).

Example 86

(±)-2-(4-Fluorophenyl)-4-(2-methyl-1-propyl-1,2,3,6-tetrahydropyridin-4-yl)-3-(pyridin-4-yl)-1*H*-pyrrole (Compound No. 1-2710)

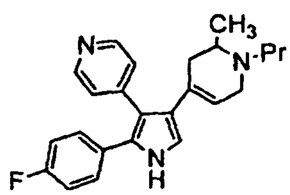

The'title compound (yield 8%, Rf value = 0.32) was obtained as a white powder during the chromatography performed in Example 85 above.
Melting point: 210 - 218°C (decomposition)
$^1$H-Nuclear magnetic resonance spectrum (400 MHz, $CDCl_3$) δ ppm:

8.47 (2H, doublet, J=6 Hz);
8.23 (1H, broad singlet);
7.17 (2H, doublet, J=6 Hz);
7.12 (2H, doublet of doublets, J=9Hz, 5 Hz);
6.96 (2H, triplet, J=9 Hz);
6.82 (1H, singlet);
5.43 (1H, singlet);
3.16-3.01 (2H, multiplet);
2.89-2.82 (1H, multiplet);
2.58-2.50 (1H, multiplet);
2.42-2.30 (2H, multiplet);
2.00-1.92 (1H, multiplet);
1.57-1.45 (2H, multiplet);
1.01 (3H, doublet, J=6 Hz);
0.90 (3H, triplet, J=7 Hz).

Example 87

(±)-2-(4-Fluorophenyl)-4-(1,3,4,6,7,9a-hexahydro-2*H*-quinolizin-8-yl)-3-(pyridin-4-yl)-1*H*-pyrrole (Compound No. 1-2656)

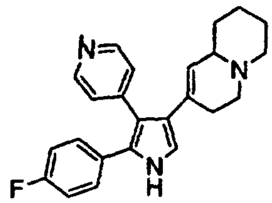

In a similar manner to the procedures described in Examples 9(i) and 9(iii) above, coupling, dehydration and desilylation reactions were carried out using (±)-octahydro-2*H*-quinolizine-2-one as a starting material instead of 1-benzylpiperidine-4-one to afford a mixture of the title compound and the (1,3,4,6,9,9a-hexahydro-2H-quinolizin-8-yl) isomer thereof (which is the compound of Example 88 below). The mixture was separated by chromatography on a silica gel column using a 100:1 by volume mixture of ethyl acetate and isopropylamine as the eluant to give the title compound (yield 6%, Rf value = 0.35) as a pale yellow powder.
Melting point : 223 - 224°C
$^1$H-Nuclear magnetic resonance spectrum (500 MHz, $CDCl_3$) δ ppm:

8.47 (2H, doublet, J=6 Hz);
8.30 (1H, broad singlet);
7.17 (2H, doublet, J=6 Hz);
7.12 (2H, doublet of doublets, J=9Hz, 5 Hz);
6.96 (2H, triplet, J=9 Hz);
6.83 (1H, doublet, J=3 Hz);
5.13 (1H, singlet);
2.88-2.81 (2H, multiplet);
2.67-2.56 (1H, multiplet);
2.45-2.43 (1H, multiplet);
2.40-2.35 (1H, multiplet);
2.17-2.07 (2H, multiplet);
1.76-1.73 (1H, multiplet);
1.69-1.64 (2H, multiplet);
1.44-1.41 (1H, multiplet);
1.34-1.18 (2H, multiplet).

Example 88

(±)-2-(4-Fluorophenyl)-4-(1,3,4,6,9,9a-hexahydro-2*H*-quinolizin-8-yl)-3-(pyridin-4-yl)-1*H*-pyrrole (Compound No. 1-2657)

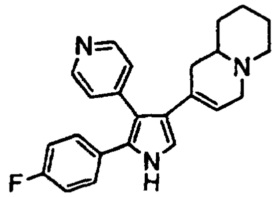

The title compound (yield 19%, Rf value = 0.30) was obtained as a pale yellow powder during the chromatography performed in Example 87 above.
Melting point : 228 - 234°C (decomposition)
$^1$H-Nuclear magnetic resonance spectrum (500 MHz, $CDCl_3$) δ ppm:

8.47 (2H, doublet, J=6 Hz);
8.28 (1H, broad singlet);
7.17 (2H, doublet, J=6 Hz);
7.12 (2H, doublet of doublets, J=9Hz, 5 Hz);
6.96 (2H, triplet, J=9 Hz);
6.81 (1H, doublet, J=3 Hz);
5.42 ( 1 H, singlet);
3.30-3.25 (1H, multiplet);
2.98-2.95 (1H, multiplet);
2.77-2.73 (1H, multiplet);
2.14-1.99 (5H, multiplet);
1.74-1.65 (3H, multiplet);
1.31-1.21 (2H, multiplet).

Example 89

(±)-2-(4-Fluorophenyl)-4-(1,2,3,5,8,8a-hexahydroindolizin-7-yl)-3-(2-methylaminopyrimidin-4-yl)-1*H*-pyrrole (Compound No. 1-2906)

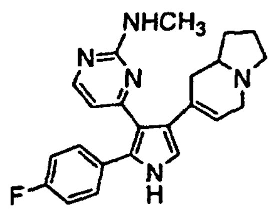

In a similar manner to the procedures described in Examples 9(i) and 9(iii) above, coupling, dehydration and desilylation reactions were carried out using 4-bromo-2-(4-fluorophenyl)-3-(2-methylaminopyrimidin-4-yl)-1-triisopropylsilyl-1*H*-pyrrole (prepared as described in Preparative Example 1 below) instead of 4-bromo-2-(4-fluorophenyl)-3-(pyridin-4-yl)-1-triisopropylsilyl-1*H*-pyrrole, and (±)-1,2,3,5,6,7,8,8a-octahydroindolizine-7-one, instead of 1-benzylpiperidine-4-one, as starting materials to afford a mixture of the title compound and the (1,2,3,5,6,8a-hexahydroindolizin-7-yl) isomer thereof (which is the compound of Example 90 below). The mixture was separated by chromatography on a silica gel column using a 200:20:1 by volume mixture of ethyl acetate, methanol and isopropylamine as the eluant to give the title compound (yield 15%, Rf value = 0.55) as a pale brown amorphous solid.
$^1$H-Nuclear magnetic resonance spectrum (400 MHz, $CD_3OD$) δ ppm:

8.08 (1H, doublet, J=5 Hz);
7.30 (2H, doublet of doublets, J=9Hz, 5 Hz);
7.02 (2H, triplet, J=9 Hz);
6.82 (1H, singlet);
6.49 (1H, doublet, J=5 Hz);
5.57-5.51 (1H, multiplet);
3.53-3.45 (1H, multiplet);
3.21-3.11 (1H, multiplet);
2.90-2.81 (1H, multiplet);
2.80 (3H, singlet);
2.47-2.28 (2H, multiplet);
2.26-2.13 (2H, multiplet);

2.07-1.96 (1H, multiplet);
1.91-1.73 (2H, multiplet);
1.49-1.37 (1H, multiplet).

Example 90

(±)-2-(4-Fluorophenyl)-4-(1,2,3,5,6,8a-hexahydroindolizin-7-yl)-3-(2-methylaminopyrimidin-4-yl)-1*H*-pyrrole (Compound No. 1-2905)

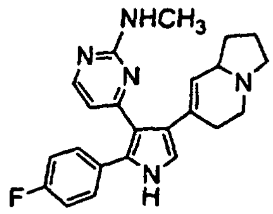

The title compound (yield 12%, Rf value = 0.50) was obtained as a pale brown amorphous solid during the chromatography performed in Example 89 above.
$^1$H-Nuclear magnetic resonance spectrum (400 MHz, $CD_3OD$) δ ppm:

8.09 (1H, doublet, J=5 Hz);
7.30 (2H, doublet of doublets, J=9Hz, 5 Hz);
7.02 (2H, triplet, J=9 Hz);
6.83 (1H, singlet);
6.46 (1H, doublet, J=5 Hz);
5.48-5.42 (1H, multiplet);
3.47-3.38 (1H, multiplet);
3.03-2.75 (4H, multiplet);
2.81 (3H, singlet);
2.46-2.36 (1H, multiplet);
2.29-2.20 (1H, multiplet);
2.03-1.87 (2H, multiplet);
1.83-1.75 (1H, multiplet);
1.54-1.43 (1H, multiplet).

Example 91

(±)-2-(3,4-Difluorophenyl)-4-(1,2,3,5,8,8a-hexahydroindolizin-7-yl)-3-(pyridin-4-yl)-1*H*-pyrrole (Compound No. 1-3260)

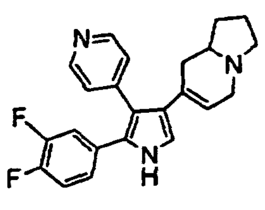

In a similar manner to the procedures described in Examples 9(i) and 9(iii) above, coupling, dehydration and

desilylation reactions were carried out using 4-bromo-2-(3,4-difluorophenyl)-3-(pyridin-4-yl)-1-triisopropylsilyl-1*H*-pyrrole (prepared as described in Preparative Example 5 below), instead of 4-bromo-2-(4-fluorophenyl)-3-(pyridin-4-yl)-1-triisopropylsilyl-1*H*-pyrrole, and (±)-1,2,3,5,6,7,8,8a-octahydroindolizine-7-one, instead of 1-benzylpiperidine-4-one, as starting materials to afford a mixture of the title compound and the (1,2,3,5,6,8a-hexahydroindolizin-7-yl) isomer thereof (which is the compound of Example 92 below). The mixture was separated by chromatography on a silica gel column using a 100:10:1 by volume mixture of ethyl acetate, methanol and isopropylamine as the eluant to give the title compound (yield 29%, Rf value = 0.66) as a pale pink powder.
Melting point : 215 - 217°C (decomposition)
$^1$H-Nuclear magnetic resonance spectrum (400 MHz, DMSO-$d_6$) δ ppm:

11.48 (1H, broad singlet);
8.48 (2H, doublet, J=6 Hz);
7.37-7.28 (1H, multiplet);
7.21-7.10 (1H, multiplet);
7.14 (2H, doublet, J=6 Hz);
6.96 (1H, doublet, J=3 Hz);
6.93-6.86 (1H, multiplet);
5.27-5.20 (1H, multiplet);
3.33-3.23 (1H, multiplet);
3.05-2.97 (1H, multiplet);
2.65-2.56 (1H, multiplet);
2.30-2.22 (1H, multiplet);
2.12-1.93 (3H, multiplet);
1.91-1.81 (1H, multiplet);
1.75-1.57 (2H, multiplet);
1.33-1.20 (1H, multiplet).

Example 92

(±)-2-(3,4-Difluorophenyl)-4-(1,2,3,5,6,8a-hexahydroindolizin-7-yl)-3-(pyridin-4-yl)-1*H*-pyrrole (Compound No. 1-3259)

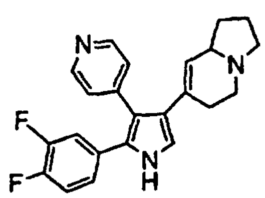

The title compound (yield 23%, Rf value = 0.31) was obtained as a pale pink powder during the chromatography performed in Example 91 above.
Melting point : 187 - 190°C (decomposition)
$^1$H-Nuclear magnetic resonance spectrum (400 MHz, DMSO-$d_6$) δ ppm:

11.48 (1H, broad singlet);
8.47 (2H, doublet, J=6 Hz);
7.39-7.29 (1H, multiplet);
7.21-7.10 (1H, multiplet);
7.14 (2H, doublet, J=6 Hz);
6.96 (1H, doublet, J=3 Hz);
6.94-6.87 (1H, multiplet);
5.18-5.13 (1H, multiplet);
3.10-3.02 (1H, multiplet);
2.89-2.82 (1H, multiplet);
2.79-2.71 (1H, multiplet);

2.63-2.45 (2H, multiplet);
2.30-2.20 (1H, multiplet);
2.06-1.97 (1H, multiplet);
1.78-1.53 (3H, multiplet);
1.22-1.10 (1H, multiplet).

Example 93

(±)-4-(1,2,3,5,8,8a-Hexahydroindolizin-7-yl)-3-(pyridin-4-yl)-2-(3-trifluoromethylphenyl)-1*H*-pyrrole (Compound No. 1-3262)

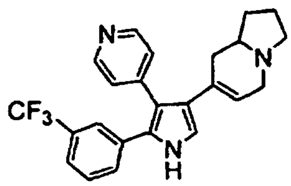

In a similar manner to the procedures described in Examples 9(i) and 9(iii) above, coupling, dehydration and desilylation reactions were carried out using 4-bromo-3-(pyridin-4-yl)-2-(3-trifluoromethylphenyl)-1-triisopropylsilyl-1*H*-pyrrole (prepared as described in Preparative Example 6 below), instead of 4-bromo-2-(4-fluorophenyl)-3-(pyridin-4-yl)-1-triisopropylsilyl-1*H*-pyrrole, and (±)-1,2,3,5,6,7,8,8a-octahydroindolizine-7-one, instead of 1-benzylpiperidine-4-one, as starting materials to afford a mixture of the title compound and the (1,2,3,5,6,8a-hexahydroindolizin-7-yl) isomer thereof (which is the compound of Example 94 below). The mixture was separated by chromatography on a silica gel column using a 100:10:1 by volume mixture of ethyl acetate, methanol and isopropylamine as the eluant to give the title compound (yield 26%, Rf value = 0.65) as a pale brown powder.
Melting point : 220 - 223°C (decomposition)
$^1$H-Nuclear magnetic resonance spectrum (400 MHz, DMSO-$d_6$) δ ppm:

11.62 (1H, broad singlet);
8.49 (2H, doublet, J=6 Hz);
7.54-7.35 (4H, multiplet);
7.16 (2H, doublet, J=6 Hz);
7.02 (1H, doublet, J=3 Hz);
5.29-5.21 (1H, multiplet);
3.35-3.24 (1H, multiplet);
3.06-2.98 (1H, multiplet);
2.65-2.56 (1H, multiplet);
2.34-2.24 (1H, multiplet);
2.13-1.94 (3H, multiplet);
1.92-1.82 (1H, multiplet);
1.75-1.58 (2H, multiplet);
1.34-1.21 (1H, multiplet).

Example 94

(±)-4-(1,2,3,5,6,8a-Hexahydroindolizin-7-yl)-3-(pyridin-4-yl)-2-(3-trifluoromethylphenyl)-1*H*-pyrrole (Compound No. 1-3261)

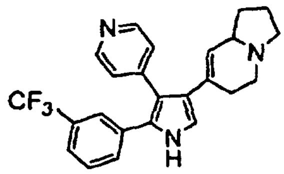

The title compound (yield 17%, Rfvalue = 0.31) was obtained as a pale brown powder during the chromatography performed in Example 93 above.
Melting point: 189 - 191°C (decomposition)
$^1$H-Nuclear magnetic resonance spectrum (400 MHz, DMSO-$d_6$) δ ppm:

11.63 (1H, broad singlet);
8.48 (2H, doublet, J=6 Hz);
7.55-7.37 (4H, multiplet);
7.15 (2H, doublet, J=6 Hz);
7.02 (1H, doublet, J=3 Hz);
5.19-5.14 (1H, multiplet);
3.12-3.03 (1H, multiplet);
2.91-2.82 (1H, multiplet);
2.80-2.71 (1H, multiplet);
2.64-2.45 (2H, multiplet);
2.33-2.21 (1H, multiplet);
2.09-1.98 (1H, multiplet);
1.78-1.53 (3H, multiplet);
1.21-1.10 (1H, multiplet).

Example 95

(±)-2-(3-Fluorophenyl)-4-(1,2,3,5,8,8a-hexahydroindolizin-7-yl)-3-(pyridin-4-yl)-1*H*-pyrrole (Compound No. 1-3256)

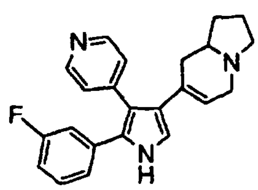

In a similar manner to the procedures described in Examples 9(i) and 9(iii) above, coupling, dehydration and desilylation reactions were carried out using 4-bromo-2-(3-fluorophenyl)-3-(pyridin-4-yl)-1-triisopropylsilyl-1*H*-pyrrole (prepared as described in Preparative Example 4 below), instead of 4-bromo-2-(4-fluorophenyl)-3-(pyridin-4-yl)-1-triisopropylsilyl-1*H*-pyrrole, and (±)-1,2,3,5,6,7,8,8a-octahydroindolizine-7-one, instead of 1-benzylpiperidine-4-one, as starting materials to afford a mixture of the title compound and the (1,2,3,5,6,8a-hexahydroindolizin-7-yl) isomer thereof (which is the compound of Example 96 below). The mixture was separated by chromatography on a silica gel column using a 30:1 by volume mixture of ethyl acetate and isopropylamine as the eluant to give the title compound (yield 17%, Rf value = 0.25) as a pale pink powder.
Melting point : 199 - 203°C (decomposition)

$^1$H-Nuclear magnetic resonance spectrum (400 MHz, $CDCl_3$) $\delta$ ppm:

8.49 (2H, doublet, J=6 Hz);
8.30 (1H, broad singlet);
7.23-7.18 (3H, multiplet);
6.96-6.82 (4H, multiplet);
5.50-5.47 (1H, multiplet);
3.53-2.96 (1H, multiplet);
3.20 (1H, triplet, J=9 Hz);
2.79 (1H, doublet, J=17 Hz);
2.32-2.08 (4H, multiplet);
2.00-1.67 (3H, multiplet);
1.48-1.33 (1H, multiplet).

Example 96

(±)-2-(3-Fluorophenyl)-4-(1,2,3,5,6,8a-hexahydroindolizin-7-yl)-3-(pyridin-4-yl)-1*H*-pyrrole (Compound No. 1-3255)

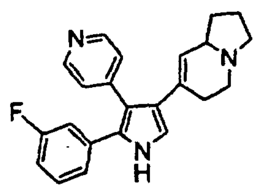

The title compound (yield 12%, Rf value = 0.10) was obtained as a pale brown powder during the chromatography performed in Example 95 above.
Melting point: 178 - 181°C (decomposition)
$^1$H-Nuclear magnetic resonance spectrum (400 MHz, $CDCl_3$) $\delta$ ppm:

8.50 (2H, doublet, J=6 Hz);
8.34 (1H, broad singlet);
7.26-7.18 (3H, multiplet);
6.95-6.84 (4H, multiplet);
5.42 (1H, singlet);
3.23-3.17 (1H, multiplet);
3.02-2.82 (2H, multiplet);
2.78-2.61 (2H, multiplet);
2.42-2.30 (1H, multiplet);
2.16-2.04 (1H, multiplet);
1.95-1.68 (3H, multiplet);
1.44-1.32 (1H, multiplet).

Example 97

(±)-2-(3-Chlorophenyl)-4-(1,2,3,5,8,8a-hexahydroindolizin-7-yl)-3-(pyridin-4-yl)-1*H*-pyrrole (Compound No. 1-3258)

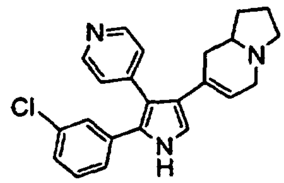

In a similar manner to the procedures described in Examples 9(i) and 9(iii) above, coupling, dehydration and desilylation reactions were carried out using 4-bromo-2-(3-chlorophenyl)-3-(pyridin-4-yl)-1-triisopropylsilyl-1*H*-pyrrole (prepared as described in Preparative Example 3 below), instead of 4-bromo-2-(4-fluorophenyl)-3-(pyridin-4-yl)-1-triisopropylsilyl-1*H*-pyrrole, and (±)-1,2,3,5,6,7,8,8a-octahydroindolizine-7-one, instead of 1-benzylpiperidine-4-one, as starting materials to afford a mixture of the title compound and the (1,2,3,5,6,8a-hexahydroindolizin-7-yl) isomer thereof (which is the compound of Example 98 below). The mixture was separated by chromatography on a silica gel column using a 30:1 by volume mixture of ethyl acetate and isopropylamine as the eluant to give the title compound (yield 18%, Rf value = 0.25) as a pale pink powder.
Melting point: 197 - 201°C (decomposition)
$^1$H-Nuclear magnetic resonance spectrum (400 MHz, $CDCl_3$) δ ppm:

8.49 (2H, doublet, J=6 Hz);
8.32 (1H, broad singlet);
7.25-7.13 (5H, multiplet);
6.96 (1H, doublet, J=7 Hz);
6.85 (1H, doublet, J=3 Hz);
5.50-5.47 (1H, multiplet);
3.54-3.45 (1H, multiplet);
3.20 (1H, triplet, J=9 Hz);
2.79 (1H, doublet, J=16 Hz);
2.31-2.07 (4H, multiplet);
2.00-1.67 (3H, multiplet);
1.48-1.33 (1H, multiplet).

Example 98

(±)-2-(3-Chlorophenyl)-4-(1,2,3,5,6,8a-hexahydoindolizin-7-yl)-3-(pyridin-4-yl)-1*H*-pyrrole (Compound No. 1-3257)

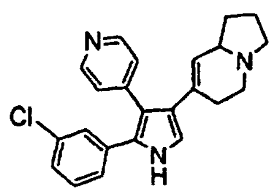

The title compound (yield 16%, Rf value = 0.10) was obtained as a pale brown powder during the chromatography performed in Example 97 above.
Melting point : 193 - 195°C (decomposition)
$^1$H-Nuclear magnetic resonance spectrum (400 MHz, $CDCl_3$) δ ppm:

8.50 (2H, doublet, J=6 Hz);

8.42 (1H, broad singlet);
7.25-7.13 (5H, multiplet);
6.96 (1H, doublet, J=7 Hz);
6.85 (1H, doublet, J=3 Hz);
5.42 (1H, singlet);
3.26-3.17 (1H, multiplet);
3.02-2.82 (2H, multiplet);
2.78-2.60 (2H, multiplet);
2.42-2.30 (1H, multiplet);
2.16-2.03 (1H, multiplet);
1.95-1.65 (3H, multiplet);
1.45-1.32 (1H, multiplet).

Example 99

(±)-4-(1,2,3,5,8,8a-Hexahydroindolizin-7-yl)-2-phenyl-3-(pyridin-4-yl)-1*H*-pyrrole (Compound No. 1-3254)

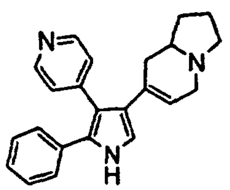

In a similar manner to the procedures described in Examples 9(i) and 9(iii) above, coupling, dehydration and desilylation reactions were carried out using 4-bromo-2-phenyl-3-(pyridin-4-yl)-1-triisopropylsilyl-1*H*-pyrrole (prepared as described in Preparative Example 2 below), instead of 4-bromo-2-(4-fluorophenyl)-3-(pyridin-4-yl)-1-triisopropylsilyl-1*H*-pyrrole, and (±)-1,2,3,5,6,7,8,8a-octahydroindolizine-7-one, instead of 1-benzylpiperidine-4-one, as starting materials to afford a mixture of the title compound and the (1,2,3,5,6,8a-hexahydroindolizin-7-yl) isomer thereof (which is the compound of Example 100 below). The mixture was separated by chromatography on a silica gel column using a 30:1 by volume mixture of ethyl acetate and isopropylamine as the eluant to give the title compound (yield 14%, Rf value = 0.24) as a pale brown powder.
Melting point : 201 - 204°C (decomposition)
$^1$H-Nuclear magnetic resonance spectrum (400 MHz, $CDCl_3$) δ ppm:

8.46 (2H, doublet, J=6 Hz);
8.28 (1H, broad singlet);
7.30-7.13 (7H, multiplet);
6.84 (1H, doublet, J=3 Hz);
5.50-5.47 (1H, multiplet);
3.54-3.45 (1H, multiplet);
3.50 (1H, triplet, J=9 Hz);
2.79 (1H, doublet, J=17 Hz);
2.32-2.07 (4H, multiplet);
2.00-1.67 (3H, multiplet);
1.48-1.33 (1H, multiplet).

Example 100

(±)-4-(1,2,3,5,6,8a-Hexahydroindolizin-7-yl)-2-phenyl-3-(pyrizin-4-yl)-1*H*-pyrrole (Compound No. 1-3253)

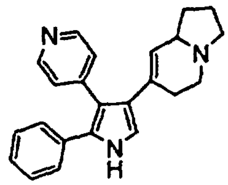

The title compound (yield 10%, Rf value = 0.11) was obtained as a pale brown powder during the chromatography performed in Example 99 above,
Melting point: 180 - 183°C (decomposition)
$^1$H-Nuclear magnetic resonance spectrum (400 MHz, $CDCl_3$) δ ppm:

8.47 (2H, doublet, J=6 Hz);
8.34 (1H, broad singlet);
7.30-7.13 (7H, multiplet);
6.84 (1H, doublet, J=3 Hz);
5.44 (1H, singlet);
3.27-3.18 (1H, multiplet);
3.02-2.82 (2H, multiplet);
2.78-2.60 (2H, multiplet);
2.43-2.30 (1H, multiplet);
2.17-2.06 (1H, multiplet);
1.95-1.65 (3H, multiplet);
1.45-1.32 (1H, multiplet).

Preparative Examples

Preparative Example 1

4-Bromo-2-(4-fluorophenyl)-3-(2-methylaminopyrimidin-4-yl)-1-triisopropylsilyl-1*H*-pyrrole

1(i) 2-(4-Fluorophenyl)-3-(2-methylthiopyrimidin-4-yl)-1*H*-pyrrole

In a similar manner to the procedures described in Synthetic Example 1(i) above (pyrrole ring forming reaction) and Synthetic Example 2(i) above (decarboxylation), reactions were carried out using ethyl 3-(2-methylthiopyrimidin-4-yl)acrylate instead of ethyl 3-(4-pyridyl)acrylate as a starting material to afford the title compound (yield 37%) as a white powder.
$^1$H-Nuclear magnetic resonance spectrum (400 MHz, $CDCl_3$) δ ppm:

8.41 (1H, broad singlet);
8.24 (1H, doublet, J=5 Hz);
7.42 (1H, doublet of doublets, J=9Hz, 5 Hz);
7.10 (2H, triplet, J=9 Hz);
6.88 (1H, triplet, J=3 Hz);
6.84 (1H, doublet, J=5 Hz);
6.81 (1H, triplet, J=3 Hz);
2.33 (3H, singlet).

1(ii) 2-(4-Fluorophenyl)-3-(2-methanesulfonylpyrimidin-4-yl)-1*H*-pyrrole

In a similar manner to that described in Synthetic Example 4(ii) above, 2-(4-fluorophenyl)-3-(2-methylthiopyrimidin-4-yl)-1*H*-pyrrole [which was prepared as described in step 1(i) above] was oxidized to afford the title compound (yield 81%) as a pale yellow powder.
$^1$H-Nuclear magnetic resonance spectrum (400 MHz, $CDCl_3$-$CD_3OD$) δ ppm:

8.58 (1H, doublet, J=6 Hz);
7.50 (2H, doublet of doublets, J=9Hz, 6 Hz);
7.47 (1H, doublet, J=6 Hz);
7.16 (2H, triplet, J=9 Hz);
6.90 (1H, doublet, J=3 Hz);
6.84 (1H, doublet, J=3 Hz);
3.05 (3H, singlet).

1(iii) 2-(4-Fluorophenyl)-3-(2-methylaminopyrimidin-4-yl)-1*H*-pyrrole

In a similar manner to that described in Synthetic Example 4(iii) above, 2-(4-fluorophenyl)-3-(2-methanesulfonylpyrimidin-4-yl)-1*H*-pyrrole [prepared as described in step 1(ii) above] was reacted with methylamine to afford the title compound (quantitative yield) as a pale brown amorphous solid.
$^1$H-Nuclear magnetic resonance spectrum (400 MHz, $CDCl_3$) δ ppm:

8.37 (1H, broad singlet);
8.08 (1H, doublet, J=5 Hz);
7.44 (2H, doublet of doublets, J=9Hz, 6 Hz);
7.08 (2H, triplet, J=9 Hz);
6.85 (1H, triplet, J=3 Hz);
6.75 (1H, triplet, J=3 Hz);
6.47 (1H, doublet, J=5 Hz);
4.90 (1H, broad quartet, J=5 Hz);
2.87 (3H, doublet, J=5 Hz).

1(iv) 2-(4-Fluorophenyl)-3-(2-methylaminopyrimidin-4-yl)-1-triisopropylsilyl-1*H*-pyrrole

In a similar manner to that described in Synthetic Example 2(ii) above, 2-(4-fluorophenyl)-3-(2-methylaminopyrimidin-4-yl)-1*H*-pyrrole [prepared as described in step 1(iii) above] was reacted with triisopropylsilyl triflate, to afford the title compound (yield 82%) as a white powder.
$^1$H-Nuclear magnetic resonance spectrum (400 MHz, $CDCl_3$) δ ppm:

7.92 (1H, doublet, J=5 Hz);
7.36 (2H, doublet of doublets, J=9Hz, 6 Hz);
7.06 (2H, triplet, J=9 Hz);
6.90 (1H, doublet, J=3 Hz);
6.89 (1H, doublet, J=3 Hz);
5.93 (1H, doublet, J=5 Hz);
4.80 (1H, broad quartet, J=5 Hz);
2.83 (3H, doublet, J=5 Hz);
1.15-0.92 (21H, multiplet).

1(v) 3-[2-(N-t-Butoxycarbonyl-N-methylamino)pyrimidin-4-yl]-2-(4-fluorophenyl)-1-triisopropylsilyl-1*H*-pyrrole

In a similar manner to that described in Synthetic Example 6(i) above, 2-(4-fluorophenyl)-3-(2-methylaminopyrimidin-4-yl)-1-trisopropylsilyl-1*H*-pyrrole [prepared as described in step 1(iv) above] was reacted with di-t-butyl dicarbonate to afford the title compound (yield 90%) as colorless oil.
$^1$H-Nuclear magnetic resonance spectrum (400 MHz, $CDCl_3$) δ ppm:

8.24 (1H, doublet, J=5 Hz);
7.36 (2H, doublet of doublets, J=9Hz, 5 Hz);
7.09 (2H, triplet, J=9 Hz);
6.95 (1H, doublet, J=3 Hz);
6.90 (1H, doublet, J=3 Hz);

6.28 (1H, doublet, J=5 Hz);
3.20 (3H, singlet);
1.51 (9H, singlet);
1.15-0.94 (21H, multiplet).

1(vi) 4-Bromo-3-[2-(N-t-butoxycarbonyl-N-methylamino)pyrimidin-4-yl]-2-(4-fluorophenyl)-1-triisopropylsilyl-1*H*-pyrrole

In a similar manner to that described in Synthetic Example 2(iii) above, 3-[2-(N-t-butoxycarbonyl-N-methylamino)pyrimidin-4-yl]-2-(4-fluorophenyl)-1-triisopropylsilyl-1*H*-pyrrole [prepared as described in step 1(v) above] was brominated to afford the title compound (yield 79%) as a pale brown amorphous solid.
$^1$H-Nuclear magnetic resonance spectrum (400 MHz, $CDCl_3$) δ ppm:

8.42 (1H, doublet, J=5 Hz);
7.28 (2H, doublet of doublets, J=9Hz, 5 Hz);
7.00 (2H, triplet, J=9 Hz);
6.93 (1H, singlet);
6.78 (1H, doublet, J=5 Hz);
3.13 (3H, singlet);
1.49 (9H, singlet);
1.15-0.93 (21H, multiplet).

1(vii) 4-Bromo-2-(4-fluorophenyl)-3-(2-methylaminopyrimidin-4-yl)-1*H*-pyrrole

472 mg (0.782 mmol) of 4-bromo-3-[2-(N-t-butoxycarbonyl-N-methylamino)pyrimidin-4-yl]-2-(4-fluorophenyl)-1-triisopropylsilyl-1*H*-pyrrole [prepared as described in step (vi) above] were dissolved in 10 of ml tetrahydrofuran, 0.98 ml (3.91 mmol) of a 4N solution of hydrogen chloride in dioxane were added to the solution, and the resulting mixture was stirred for 3 hours at 50 °C. At the end of this time, water was added to the reaction mixture and then it was neutralized with a saturated aqueous solution of sodium hydrogencarbonate. This was extracted with ethyl acetate, and the organic extract was concentrated under reduced pressure. The resulting residue was purified by chromatography on a silica gel column using a 7:3 by volume mixture of hexane and ethyl acetate as the eluant to afford 202 mg (yield 74%) of the title compound as a pale brown amorphous solid.
$^1$H-Nuclear magnetic resonance spectrum (400 MHz, $CDCl_3$) δ ppm:

8.66 (1H, broad singlet);
8.21(1H, doublet, J=5 Hz);
7.26 (2H, doublet of doublets, J=9Hz, 5 Hz);
7.01 (2H, triplet, J=9 Hz);
6.88 (1H, doublet, J=3 Hz);
6.61 (1H, doublet, J=5 Hz);
4.96 (1H, broad quartet, J=5 Hz);
2.86 (3H, doublet, J=5 Hz).

1(viii) 4-Bromo-2-(4-fluorophenyl)-3-(2-methylaminopyrimidin-4-yl)-1-triisopropylsilyl-1*H*-pyrrole

In a similar manner to the procedures described in Synthetic Example 2(ii) above, 4-bromo-2-(4-fluorophenyl)-3-(2-methylaminopyrimidin-4-yl)-1*H*-pyrrole [prepared as described in step 1(vii) above] was reacted with triisopropylsilyl triflate to afford the title compound (yield 67%) as a pale brown amorphous solid.
$^1$H-Nuclear magnetic resonance spectrum (400 MHz, $CDCl_3$) δ ppm:

8.04 (1H, doublet, J=5 Hz);
7.30 (2H, doublet of doublets, J=9Hz, 6 Hz);
6.98 (2H, triplet, J=9 Hz);
6.91 (1H, singlet);
6.32 (1H, broad singlet);
4.85 (1H, broad quartet, J=4 Hz);
2.82 (3H, doublet, J=4 Hz);
1.15-0.92 (21H, multiplet).

Preparative Example 2

4-Bromo-2-phenyl-3-(pyridin-4-yl)-1-triisopropylsilyl-1*H*-pyrrole

In a similar manner to the procedures described in Synthetic Example 1(i) above (pyrrole ring forming reaction), Synthetic Example 2(i) above (decarboxylation), Synthetic Example 2(ii) above (reaction with triisopropylslyl triflate) and Synthetic Example 2(iii) above (bromination), reactions were carried out using α-(p-toluenesulfonyl)benzylisonitrile, instead of α-(p-toluenesulfonyl)-4-fluorobenzylisonitrile, as a starting material to afford the title compound as a pale purple powder.
$^1$H-Nuclear magnetic resonance spectrum (400 MHz, $CDCl_3$) δ ppm:

8.35 (2H, doublet, J=6 Hz);
7.36-7.30 (1H, multiplet);
7.28-7.21 (4H, multiplet);
7.03 (2H, doublet, J=6 Hz);
6.94 (1H, singlet);
1.15-1.02 (21H, multiplet).

Preparative Example 3

4-Bromo-2-(3-chlomphenyl)-3-(pyridin-4-yl)-1-triisopropylsilyl-1*H*-pyrrole

In a similar manner to the procedures described in Synthetic Example 1(i) above (pyrrole ring forming reaction), Synthetic Example 2(i) above (decarboxylation), Synthetic Example 2(ii) above (reaction with triisopropysilyl triflate) and Synthetic Example 2(iii) above (bromination), reactions were carried out using α-(p-toluenesulfonyl)-3-chlorobenzylisonitrile, instead of α-(p-toluenesulfonyl)-4-fluorobenzylisonitrile, as a starting material to afford the title compound as a pale purple powder.
$^1$H-Nuclear magnetic resonance spectrum (400 MHz, $CDCl_3$) δ ppm:

8.40 (2H, doublet, J=6 Hz);
7.33-7.29 (2H, multiplet);
7.18 (1H, triplet, J=8 Hz);
7.09 (1H, doublet, J=8 Hz);
7.03 (2H, doublet, J=6 Hz);
6.95 (1H, singlet);
1.12-0.99 (21H, multiplet).

Preparative Example 4

4-Bromo-2-(3-fluorophenyl)-3-(pyridin-4-yl)-1-triisopropylsilyl-1*H*-pyrrole

In a similar manner to the procedures described in Synthetic Example 1(i) above (pyrrole ring forming reaction), Synthetic Example 2(i) above (decarboxylation), Synthetic Example 2(ii) above (reaction with triisopropysilyl triflate) and Synthetic Example 2(iii) above (bromination), reactions were carried out using α-(p-toluenesulfonyl)-3-fluorobenzylisonitrile, instead of α-(p-toluenesulfonyl)-4-fluorobenzylisonitrile, as a starting material to afford the title compound as a pale purple powder.
$^1$H-Nuclear magnetic resonance spectrum (400 MHz, $CDCl_3$) δ ppm:

8.39 (2H, doublet, J=6 Hz);
7.22 (1H, doublet of doublets, J=8Hz, 6 Hz);
7.07-7.01 (4H, multiplet);
6.98-6.94 (2H, multiplet);
1.14-0.98 (21H, multiplet).

Preparative Example 5

4-Bromo-2-(3,4-difluorophenyl)-3-(pyridin-4-yl)-1-triisopropylsilyl-1*H*-pyrrole

In a similar manner to the procedures described in Synthetic Example 1(i) above (pyrrole ring forming reaction), Synthetic Example 2(i) above (decarboxylation), Synthetic Example 2(ii) above (reaction with triisopropysilyl triflate) and Synthetic Example 2(iii) above (bromination), reactions were carried out using α-(p-toluenesulfonyl)-3,4-difluorobenzylisonitrile, instead of α-(p-toluenesulfonyl)-4-fluorobenzylisonitrile, as a starting material to afford the title compound as a pale purple powder.
$^1$H-Nuclear magnetic resonance spectrum (400 MHz, $CDCl_3$) δ ppm:

8.42 (2H, doublet, J=6 Hz);
7.12-6.93 (3H, multiplet);
7.02 (2H, doublet, J=6 Hz);
6.94 (1H, singlet);
1.18-0.94 (21H, multiplet).

Preparative Example 6

4-Bromo-2-(3-trifluoromethylphenyl)-3-(pyridin-4-yl)-1-triisopropylsilyl-1*H*-pyrrole

In a similar manner to the procedures described in Synthetic Example 1(i) above (pyrrole ring forming reaction), Synthetic Example 2(i) above (decarboxylation), Synthetic Example 2(ii) above (reaction with triisopropysilyl triflate) and Synthetic Example 2(iii) above (bromination), reactions were carried out using α-(p-toluenesulfonyl)-3-trifluoromethyl-benzylisonitrile, instead of α-(p-toluenesulfonyl)-4-fluorobenzylisonitrile, as a starting material to afford the title compound as a pale purple powder.
$^1$H-Nuclear magnetic resonance spectrum (400 MHz, $CDCl_3$) δ ppm:

8.39 (2H, doublet, J=6 Hz);
7.64-7.57 (2H, multiplet);
7.40-7.33 (2H, multiplet);
7.00 (2H, doublet, J=6 Hz);
6.98 (1H, singlet);
1.15-0.94 (21H, multiplet).

Formulation Examples

A pharmaceutical preparation containing a compound of the present invention having the above formula (I), or a pharmacologically acceptable salt, ester or other derivative thereof as its active ingredient can be produced according to, for example, the following methods.

Preparation Example 4

Tablets

5 g of the compound of Example 83, 90 g of lactose, 34 g of corn starch, 20 g of crystalline cellulose and 1 g of magnesium stearate were mixed in a blender, and the resulting mixture was then formed into tablets with a tablet machine to provide the desired tablets.

Test Examples

The biological activity of the compounds of the present invention is illustrated by the following Test Examples.

Test Example 1

Inhibition of the Production of the Cytokines IL-1β and TNFα *in vitro* in Human Whole Blood

This test was performed according to the method of Hartman, et al. [D.A. Hartman, S.J. Ochalski and R.P.

Carlson; The effects of anti-inflammatory and antiallergic drugs on cytokine release after stimulation of human whole blood by lipopolysaccharide and zymosan A: Inflamm. Res., 44, 269 (1995)].

Peripheral blood samples were collected in the presence of heparin from healthy adult volunteers. 1000 μl of whole blood were added to an Eppendorf tube to which 2 μl of a dimethyl sulfoxide solution of the test compound had been added in advance, after which 10 μl of lipopolysaccharide (E. coli 026: B6 origin, Difco) were added as a stimulant (final concentration of said lipopolysaccharide: 10 μg/ml). This was mixed well and then incubated for 6 hours at 37°C in the presence of 5% $CO_2$. At the end of the incubation, the mixture was cooled to 4°C to stop the reaction, followed immediately by centrifuging for 5 minutes at 14,000 rpm to separate and collect the supernatant plasma. The IL-1β and TNFα produced and released into the plasma were measured using a commercially available enzyme immunoassay (ELISA) kit [Cayman (IL-1β) and Genzyme (TNFα)]. The procedure was also repeated in the absence of test compound. The inhibitory effect [$IC_{50}$ (μM)] on the production of IL-1β and TNFα was determined from the amounts of the cytokines produced in the presence and absence of the test compound.

The results for the inhibition of the production *in vitro* of TNFα are shown in Table 3 below. In this Table, Compounds A and B are as follows:

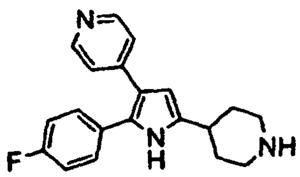

**Compound A (Compound of Example 4 of WO97/5877)**

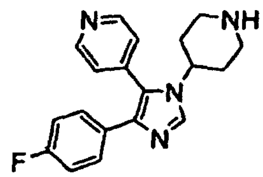

**Compound B (Compound of Example 23 of WO96/21452)**

Table 3

| Example No. | Inhibitory Effect on TNFα Production [$IC_{50}$ (μM)] |
|---|---|
| Compound A | 1.90 |
| Compound B | 1.73 |
| 58 | 0.089 |
| 60 | 0.047 |
| 83 | 0.026 |
| 85 | 0.44 |
| 86 | 0.29 |
| 87 | 0.31 |
| 92 | 0.045 |
| 94 | 0.031 |

Table 3 above demonstrates excellent inhibitory activity against the production of TNFα *in vitro* for the compounds of the present invention.

The compounds of the present invention were also found to show excellent inhibitory activity against the production of IL-1β *in vitro.*

Test Example 2

Inhibition of the Production of TNFα *in vivo*

This test was performed according to the method of Ochalski, et al. [S.J. Ochalski, D.A. Hartman, M.T. Belfast,

T.L. Walter, K.B. Glaser and R.P. Carlson; Inhibition of endotoxin-induced hypothermia and serum TNF-α levels in CD-1 mice by various pharmacological agents: Agents Actions 39, C52-C54 (1993)].

The production of TNFα was induced in mice by the intravenous injection of lipopolysaccharide (E. coli O26: B6 origin, Difco) which was prepared to a concentration of 0.045 mg/ml using physiological saline. The saline preparation of lipopolysaccharide was administered at the rate of 10 ml/l kg of body weight into the caudal vein of Balb/c mice (males, age 5-7 weeks, body weight: approx. 22 g, Japan Charles River) which had been fasted overnight starting on the day before the experiment. One hour after administration, the mice were laparotomized under ether anaesthesia and blood was collected from the abdominal vena cava. Blood collection was performed using a 1 ml volume disposable syringe equipped with a 23G needle which had been moistened with heparin on the inside wall. Following blood collection, the blood was immediately transferred to a 1.5 ml volume Eppendorf tube and centrifuged at 4°C and 14,000 rpm to separate the plasma. This plasma was then stored at -20°C until measurement of TNFα. The measurement of the amount of TNFα was performed with a commercially available enzyme immunoassay (ELISA) kit (Mouse TNFα ELISA KIT, Genzyme).

To determine the inhibitory activity of the test compounds, each test compound was suspended in a 0.5% tragacanth solution and then administered orally to the Balb/c mice at the rate of 10 ml/1 kg of body weight 30 minutes before injection of lipopolysaccharide. The level of TNFα production was then determined as described above. In the control group, 0.5% tragacanth solution was administered at the rate of 10 ml/1 kg of body weight to the test mice instead of the solutions of the test compounds. A minimum of 3 dose levels of the test compound was administered to groups of 5 test mice for each test compound. The inhibitory rate relative to the control group was calculated for each dose level. From the inhibitory rates and the dosages, $ID_{50}$ values were calculated by the least squares method.

The compounds of the present invention were found to show excellent inhibitory activity against the production of TNFα *in vivo.*

Test Example 3

Inhibition of the Production of IL-1β *in vivo*

This test was performed according to the method of Griffiths, et al. [Richard J. Griffiths, Ethan J. Stam, James T. Downs and Ivan G. Otterness; ATP Induces the Release of IL-1 from LPS-Primed Cells In Vivo: J. Immunol., 154, 2821-2828 (1995)].

The production of IL-1 was induced in mice by the intraperitoneal injection of lipopolysaccharide followed by the intraperitoneal injection of adenosine triphosphate (ATP). This was achieved by first administering a solution of lipopolysaccharide (E. coli O26: B6 origin, Difco), which had been prepared to a concentration of 0.0045 mg/ml using physiological saline, at the rate of 10 ml of said saline solution/1 kg of body weight into the peritoneal cavity of Balb/c mice (males, age 5-7 weeks, body weight: approx. 22 g, Japan Charles River) which had been fasted overnight starting on the day before the experiment. Two hours later, 0.5 ml of ATP, which had been prepared to a concentration of 6.03 mg/ml using physiological saline, were administered into the peritoneal cavity. 0.5 hours after the administration of ATP, the mice were sacrificed by suffocation using dry ice followed immediately by intraperitoneal injection of 3 ml of washing phosphate buffer solution [containing heparin (10 U/ml), p-toluenesulfonyl fluoride (0.25 mM), leupepsin (1 μg/ml), pepstatin (1 μg/ml) and EDTA (1 mM)] to wash the peritoneal cavity. A 1 ml volume disposable syringe equipped with a 21G needle was then used to recover the washing liquid. After the recovery, the washing liquid from the peritoneal cavity was immediately transferred to a 1.5 ml volume Eppendorf tube and centrifuged at 4°C and 7,500 rpm to separate the supernatant. This supernatant was then stored at -20°C until measurement of IL-1β.

The measurement of the amount of IL-1β was performed with an enzyme immunoassay (ELISA) kit (Mouse IL-1β ELISA KIT, Genzyme).

To determine the inhibitory activity of the test compounds, each test compound was suspended in a 0.5% tragacanth solution and then administered orally to the Balb/c mice at the rate of 10 ml/1 kg of body weight 30 minutes before injection of lipopolysaccharide. The level of TNFα production was then determined as described above. In the control group, 0.5% tragacanth solution was administered to the test mice at the rate of 10 ml/1 kg of body weight instead of the solutions of the test compounds. A minimum of 3 dose levels of the test compound was administered to groups of 5 test mice for each test compound. The mean inhibitory rate relative to the control group was calculated for each dose level.

In this test, the compounds of the present invention demonstrated an excellent inhibitory effect against the production of IL-1β *in vivo*.

Test Example 4

Activity in Preventing the Development of Adjuvant-Induced Arthritis *in vivo*

The test was performed according to the method described by Winder et al. (Arthritis Rheum., 12, 472-482, 1969).

Heat-killed dried Mycobacterium butyricum (Difco Laboratories, Lot 679123) was ground on an agate mortar, and was then suspended in dry-sterilised liquid paraffin (first grade, Wako Pure Chemical Industries, Ltd.) to make a 2 ml suspension. The resulting suspension was then sonicated and used as an adjuvant. Arthritis was induced by the intradermal injection of the adjuvant (100 μg of heat killed dried bacterium/0.05 ml of paraffin/paw) into the heel of the right hind paw of a Lewis rat (male, age 8 weeks, Japan Charles River). The test compounds, which had been suspended in 0.5% sodium carboxymethyl cellulose solution (CMC, Daiichi Pure Chemicals, Co., Ltd.), were administered orally once a day from the day of injection of the adjuvant (day 0) to day 20.

The volumes of the injected (right) and non-injected (left) hind paws were measured on days 3, 5, 7, 10, 13, 15, 18 and 21 using a Plethysmometer™ (Ugo Basile), the hind paws being soaked from the toe to the hairline in the bath of the Plethysmometer™. The volumes of the swollen feet (adjuvant-injected right hind foot volume - non-injected left hind foot volume) were calculated. The percent inhibition of swelling of the injected foot of the treated animals as compared to that of the control animals on day 21 was calculated as follows.

$$\text{Inhibition (\%)} = \{1\text{-(swollen foot volume of compound-treated animals)/}$$

$$\text{(swollen foot volume of control animals)}\} \times 100$$

A linear regression curve was obtained from the percent inhibition and the logarithmic value of the dosage by the least squares method. $ID_{50}$ values were calculated using this curve.

In this test, the compounds of the present invention showed excellent activity in preventing the development of adjuvant-induced arthritis.

Test Example 5

Activity in Preventing the Development of Arthritis Induced by Anti-Collagen Antibody *in vivo*

In this test, an anti-collagen antibody-induced mouse arthritis model was employed.

0.5 ml (2 mg of antibody) of an anti-collagen antibody solution (4 mg/ml, Arthritogenic mAb Cocktail: product of Immuno-Biological Laboratories Co., Ltd) were injected into the caudal vein of Balb/c mice (males, age 5-6 weeks old, Japan Charles River). Three days after injection, 0.1 ml [0.05 mg of lipopolysaccharide] of a lipopolysaccharide solution (0.5 mg/ml, Arthritogenic mAb Cocktail: product of Immuno-Biological Laboratories Co., Ltd) were administered to the mice by intraperitoneal injection.

The test compounds, which had been suspended in 0.5% tragacanth were administered orally to the test animals at the rate of 10 ml/1 kg of body weight once per day for 7 days from the day when the anti-collagen antibody was administered. To the mice of the control group, 0.5% tragacanth solution was administered at the rate of 10 ml/kg of body weight once per day for 7 days from the day when the anti-collagen antibody was administered, instead of solutions of the test compounds.

After the administration of the test compounds (or 0.5% tragacanth solution), the degree of edema in the 4 paws of each test mouse was scored according to the following basis:

0: normal (edema is not observed);
1: edema is observed in one of the five toes;
2: edema is observed in two or more of the five toes;
3: the whole of the paw is swollen.

The degree of arthritis in the test mouse was evaluated by the total of the edema scores in the 4 paws. The rate of suppression was calculated from the degrees of arthritis of the control animals and of the animals treated with the test compounds. From the rates of suppression and the dosages, $ID_{50}$ values were calculated by the least squares method.

In this test, the compounds of the present invention showed excellent activity in preventing the development of arthritis induced by anti-collagen antibody.

Test Example 6

Activity in Treating Arthritis Induced by Anti-Collagen Antibody *in vivo*

In this test, an anti-collagen antibody-induced mouse arthritis model was employed.

0.5 ml (2 mg of antibody) of an anti-collagen antibody solution (4 mg/ml, Arthritogenic mAb Cocktail: product of Immuno-Biological Laboratories Co., Ltd) were injected into the caudal vein of Balb/c mice (males, age 5-6 weeks old, Japan Charles River). Three days after injection, 0.1 ml [0.05 mg of lipopolysaccharide] of a lipopolysaccharide solution (0.5 mg/ml, Arthritogenic mAb Cocktail: product of Immuno-Biological Laboratories Co., Ltd) were administered to the mice by intraperitoneal injection.

7 days after the administration of the anti-collagen antibody solution, the degree of edema in the 4 paws of each test mouse was scored according to the basis as shown in Test Example 5 above.

Those mice in which edema in both the hind paws had been scored as "3" were selected. Test compounds, which had been suspended in 0.5% tragacanth solution, were administered orally to the selected mice at the rate of 10 ml/kg of body weight once per day for 3 days. To the mice of the control group, 0.5% tragacanth solution was administered at the rate of 10 ml/kg of body weight once per day for 3 days instead of solutions of the test compounds.

After the administration of the test compounds (or 0.5% tragacanth solution), the degree of arthritis in each test mouse was evaluated in the same manner as described in Test Example 5. The rates of treatment of arthritis induced by anti-collagen antibody were calculated from the degrees of arthritis of the control animals and of the compound-treated animals.

From the rates of treatment and the dosages, $ID_{50}$ values were calculated by the least squares method.

In this test, the compounds of the present invention showed excellent activity in treating arthritis induced by anti-collagen antibody.

As illustrated above, the compounds of the present invention exhibit excellent activity in inhibiting the production of inflammatory cytokines, particularly in inhibiting the production of IL-1β and TNFα. Furthermore, the compounds of the present invention have satisfactory oral absorptivity and a low level of toxicity. Consequently, the compounds of the present invention are useful as pharmaceuticals, suitable for the prohylaxis and treatment of both humans and animals. They can, for example, be used as an analgesic, an anti-inflammatory agent and an antiviral agent as well as an agent for use in the prophylaxis and treatment of chronic rheumatoid arthritis, degenerative arthritis, allergic diseases, asthma, septicaemia, psoriasis, osteoporosis, autoimmune diseases (e.g., systemic lupus erythematosus, ulcerative colitis and Crohn's disease), diabetes, glomerular nephritis, hepatitis and arteriosclerosis. Of these applications, the compounds of the present invention are particularly useful as an analgesic and an anti-inflammatory agent and as an agent for the prophlaxis and treatment of chronic rheumatoid arthritis, degenerative arthritis, allergic diseases, septicaemia, psoriasis, osteoporosis, ulcerative colitis, diabetes, hepatitis and arteriosclerosis.

## Claims

**1.** A compound of formula (I), or a pharmacologically acceptable salt, ester or amide thereof:

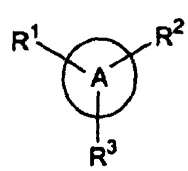

(I)

wherein:

A represents a pyrrole ring;

$R^1$ is a phenyl or naphthyl group which may optionally be substituted with at least one substituent selected from the group consisting of Substituent group α defined below, Substituent group P defined below, Substituent group γ defined below and Substituent group δ defined below;

$R^2$ is a pyridyl or pyrimidinyl group which may optionally be substituted with at least one substituent selected from the group consisting of Substituent group α defined below, Substituent group β defined below, Substituent group γ defined below and Substituent group δ defined below; and

$R^3$ is a group of the following formula (II):

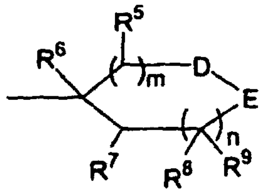

(II)

wherein

m represents 0 or 1,
n represents 1 or 2,
one of D and E represents a group of formula $>NR^{10}$ and the other represents a group of formula $>CR^{11}R^{12}$, one of $R^5$ and $R^7$ together with $R^6$ represents a single bond and the other represents a substituent selected from the group consisting of hydrogen atoms, Substituent group α defined below, Substituent group γ defined below and Substituent group δ defined below, $R^8$, $R^9$ and $R^{12}$ may be the same or different from one another and each represents a substituent selected from the group consisting of hydrogen atoms, Substituent group α defined below, Substituent group γ defined below and Substituent group δ defined below, and $R^{10}$ and $R^{11}$ may be the same or different from one another and each represents a substituent selected from the group consisting of hydrogen atoms, Substituent group α defined below, Substituent group γ defined below and Substituent group δ defined below or $R^{10}$ and $R^{11}$ together form an alkylene group having from I to 6 carbon atoms which is unsubstituted or is substituted with at least one substituent selected from the group consisting of Substituent group α defined below, Substituent group β defined below, Substituent group γ defined below and Substituent group δ defined below;

PROVIDED THAT said substituents $R^1$ and $R^3$ are bonded to the two atoms of said pyrrole ring which are adjacent to the atom of the pyrrole ring to which said substituent $R^2$ is bonded;

Substituent group α comprises hydroxyl groups, nitro groups, cyano groups, halogen atoms, lower alkoxy groups defined below, halogeno lower alkoxy groups defined below, lower alkylthio groups defined below, and halogeno lower alkylthio groups defined below;

Substituent group β comprises groups of formula $-NR^cR^d$, wherein $R^c$ and $R^d$ are the same or different from each other and each is independently selected from the group consisting of hydrogen atoms, lower alkyl groups defined below, lower alkenyl groups defined below, lower alkynyl groups defined below, aralkyl groups defined below and lower alkylsulfonyl groups, or
$R^c$ and $R^d$, together with the nitrogen atom to which $R^c$ and $R^d$ are bonded, form a heterocyclyl group defined below;

Substituent group γ comprises lower alkyl groups defined below which are substituted with a group of formula $-NR^cR^d$, wherein $R^c$ and $R^d$ are as defined above; and

Substituent group δ comprises lower alkyl groups defined below which may optionally be substituted with at least one substituent selected from Substituent group α defined above, lower alkenyl groups defined below which may optionally be substituted with at least one substituent selected from Substituent group α defined above, lower alkynyl groups defined below which may optionally be substituted with at least one substituent selected from Substituent group α defined above, aralkyl groups defined below and cycloalkyl groups defined below;

said lower alkyl groups in the definition of substituents $R^c$ and $R^d$ above, said lower alkyl groups which may optionally be substituted with at least one substituent selected from Substituent group α in the definition of Substituent group δ above and said lower alkyl moiety of the lower alkyl groups substituted with a group of

formula
- $NR^cR^d$ in the definition of Substituent group γ above are straight or branched alkyl groups having from 1 to 6 carbon atoms;

said lower alkenyl groups in the definition of substituents $R^c$ and $R^d$ above and said lower alkenyl groups which may optionally be substituted with at least one substituent selected from Substituent group α in the definition of Substituent group δ above are straight or branched alkenyl groups having from 2 to 6 carbon atoms;

said lower alkynyl groups in the definition of substituents $R^c$ and $R^d$ above and said lower alkynyl groups which may optionally be substituted with at least one substituent selected from Substituent group α in the definition of Substituent group 8 above are straight or branched alkynyl groups having from 2 to 6 carbon atoms;

said aralkyl groups in the definition of $R^c$ and $R^d$ above and in the definition of Substituent group δ above are lower alkyl groups as defined above which are substituted with at least one aryl group selected from aromatic hydrocarbon groups having from 6 to 14 carbon atoms in one or more rings, said aromatic hydrocarbon groups optionally being fused with a cycloalkyl group having from 3 to 10 carbon atoms;

said cycloalkyl groups in Substituent group δ above are cycloalkyl groups having from 3 to 7 carbon atoms;

said lower alkylsulfonyl groups in the definition of substituents $R^c$ and $R^d$ are lower alkyl groups as defined above which are bonded to a sulfonyl group;

said heterocyclyl groups formed by substitutents $R^c$ and $R^d$ together with the nitrogen atom to which they are bonded are 4- to 12- membered non-aromatic heterocyclyl groups containing one nitrogen atom and optionally further containing one further heteroatom selected from the group consisting of oxygen, sulphur and nitrogen atoms, said heterocyclyl groups optionally being fused with another cyclic group selected from the group consisting of aryl groups as defined in the definition of aralkyl groups above and heteroaryl groups selected from 5- to 7-membered aromatic heterocyclic groups containing from 1 to 3 heteroatoms selected from the group consisting of sulphur, oxygen and nitrogen atoms;

said lower alkoxy groups in the definition of Substituent group α above are lower alkyl groups as defined above which are bonded to an oxygen atom;

said halogeno lower alkoxy groups in the definition of Substituent group α above are lower alkoxy groups as defined above which are substituted with at least one halogen atom;

said lower alkylthio groups in the definition of Substituent group α above are lower alkyl groups as defined above which are bonded to a sulfur atom; and

said halogeno lower alkylthio groups in the definition of Substituent group α above are lower alkylthio groups as defined above which are substituted with at least one halogen atom.

**2.** A compound according to claim 1 or a pharmacologically acceptable salt, ester or amide thereof, wherein:

$R^1$ is a phenyl group which may optionally be substituted with at least one substituent selected from the group consisting of Substituent group $α^1$ defined below, Substituent group $β^1$ defined below, Substituent group $γ^1$ defined below and Substituent group $δ^1$ defined below;

$R^2$ is a 4-pyridyl or 4-pyrimidinyl group which may optionally be substituted with at least one substituent selected from the group consisting of Substituent group α, Substituent group β, Substituent group γ and Substituent group δ; and

$R^3$ is a group of formula (II) as defined in claim 1 wherein m is 1 and n is 1;

said Substituent group $α^1$ comprises halogen atoms, lower alkoxy groups as defined in claim 1 and halogeno lower alkoxy groups as defined in claim 1;

said Substituent group $β^1$ comprises a group of formula $-NR^cR^d$ wherein one of $R^c$ and $R^d$ is selected from

the group consisting of hydrogen atoms and lower alkyl groups as defined in claim 1 and the other is selected from the group consisting of hydrogen atoms, lower alkyl groups as defined in claim I and aralkyl groups as defined in claim 1;

said Substituent group $\gamma^1$ comprises lower alkyl groups as defined in claim 1 which are substituted with a substituent selected from the group consisting of amino groups, amino groups substituted with one or two lower alkyl groups as defined in claim 1 and amino groups substituted with an aralkyl group as defined in claim 1; and

said Substituent group $\delta^1$ comprises lower alkyl groups as defined in claim 1, halogeno lower alkyl groups comprising lower alkyl groups as defined in claim 1 which are substituted with at least one halogen atom, hydroxy lower alkyl groups comprising lower alkyl groups as defined in claim I which are substituted with at least one hydroxy group and nitro lower alkyl groups comprising lower alkyl groups as defined in claim 1 which are substituted with at least one nitro group.

**3.** A compound of formula (I) according to claim 1 or a pharmacologically acceptable salt, ester or amide thereof, wherein:

$R^1$ is a phenyl group which may optionally be substituted with at least one substituent selected from the group consisting of Substituent group $\alpha^1$ defined below, Substituent group $\beta^1$ defined below, Substituent group $\gamma^1$ defined below and Substituent group $\delta^1$ defined below;

$R^2$ is a 4-pyridyl or 4-pyrimidinyl group which may optionally be substituted with at least one substituent selected from the group consisting of Substituent group $\alpha$, Substituent group $\beta$, Substituent group $\gamma$ and Substituent group $\delta$; and

$R^3$ is a group of formula (II) as defined in claim 1 wherein E represents a group of formula $>NR^{10}$ wherein $R^{10}$ is as defined in claim 1 and D represents a group of formula $>CR^{11}R^{12}$ wherein $R^{11}$ and $R^{12}$ are as defined in claim 1;

said Substituent group $\alpha^1$ comprises halogen atoms, lower alkoxy groups as defined in claim 1 and halogeno lower alkoxy groups as defined in claim 1;

said Substituent group $\beta^1$ comprises a group of formula $-NR^cR^d$ wherein one of $R^c$ and $R^d$ is selected from the group consisting of hydrogen atoms and lower alkyl groups as defined in claim 1 and the other is selected from the group consisting of hydrogen atoms, lower alkyl groups as defined in claim 1 and aralkyl groups as defined in claim 1;

said Substituent group $\gamma^1$ comprises lower alkyl groups as defined in claim 1 which are substituted with a substituent selected from the group consisting of amino groups, amino groups substituted with one or two lower alkyl groups as defined in claim I and amino groups substituted with an aralkyl group as defined in claim 1; and

said Substituent group $\delta^1$ comprises lower alkyl groups as defined in claim 1, halogeno lower alkyl groups comprising lower alkyl groups as defined in claim 1 which are substituted with at least one halogen atom, hydroxy lower alkyl groups comprising lower alkyl groups as defined in claim 1 which are substituted with at least one hydroxy group and nitro lower alkyl groups comprising lower alkyl groups as defined in claim 1 which are substituted with at least one nitro group.

**4.** A compound of formula (I) according to claim 1 or a pharmacologically acceptable salt, ester or amide thereof, wherein:

$R^1$ is a phenyl group which may optionally be substituted with at least one substituent selected from the group consisting of halogen atoms, halogeno lower alkyl groups comprising lower alkyl groups as defined in claim I which are substituted with at least one halogen atom and halogeno lower alkoxy groups as defined in claim 1; and

$R^2$ is a 4-pyridyl or 4-pyrimidinyl group which may optionally be substituted at the 2-position thereof with a

substituent selected from the group consisting of Substituent group α, Substituent group β, Substituent group γ and Substituent group δ.

**5.** A compound of formula (I) according to claim I or a pharmacologically acceptable salt, ester or amide thereof, wherein:

$R^1$ is selected from the group consisting of phenyl, 4-fluorophenyl, 3-fluorophenyl, 3-chlorophenyl, 3,4-difluorophenyl, 3,4,5-trifluorophenyl, 3-chloro-4-fluorophenyl, 3-difluoromethoxyphenyl and 3-trifluoromethylphenyl groups;

$R^2$ is a 4-pyridyl or 4-pyrimidinyl group which may optionally be substituted at the 2-position thereof with a substituent selected from the group consisting of methoxy, amino, methylamino, benzylamino and α-methylbenzylamino groups; and

$R^3$ is a group of formula (II) as defined in claim I wherein $R^{10}$ and $R^{11}$ together form a straight chain alkylene group having 3 or 4 carbon atoms which is unsubstituted or is substituted with at least one substituent selected from the group consisting of Substituent group α, Substituent group β, Substituent group γ and Substituent group δ.

**6.** A compound of formula (I) according to claim 1 or a phartnacologically acceptable salt, ester or amide thereof, wherein:

$R^1$ is selected from the group consisting of phenyl, 4-fluorophenyl, 3-fluorophenyl, 3-chlorophenyl, 3,4-difluorophenyl, 3,4,5-trifluorophenyl, 3-chloro-4-fluorophenyl, 3-difluoromethoxyphenyl and 3-trifluoromethylphenyl groups;

$R^2$ is a 4-pyridyl or 4-pyrimidinyl group which may optionally be substituted at the 2-position thereof with a substituent selected from the group consisting of methoxy, amino, methylamino, benzylamino and α-methylbenzylamino groups; and

$R^3$ is a group of formula (II) as defined in claim I wherein each of $R^7$, $R^8$ and $R^9$ is a hydrogen atom, and $R^{10}$ and $R^{11}$ together form a straight chain alkylene group having 3 or 4 carbon atoms which is unsubstituted or is substituted with at least one substituent selected from the group consisting of Substituent group α, Substituent group β, Substituent group γ and Substituent group δ.

**7.** A compound of formula (I) according to any one of claims 1 to 6 or a pharmacologically acceptable salt, ester or amide thereof, wherein said compound of formula (I) is a compound of formula (I-1) or (I-3) below:

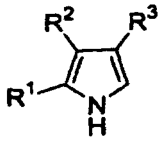

(I-1)

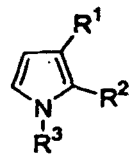

(I-3)

**8.** A compound of formula (I) according to claim 1 selected from the following group of compounds, or a pharmacologically acceptable salt, ester or amide thereof:

4-(1-acetyl-1,2,3,6-tetrahydropyridin-4-yl)-2-(4-fluorophenyl)-3-(pyridin-4-yl)-1*H*-pyrrole,
2-(4-fluorophenyl)-3-(pyridin-4-yl)-4-(1,2,3,6-tetrahydropyridin-4-yl)-1*H*-pyrrole,
2-(4-fluorophenyl)-4-(1-methyl-1,2,3,6-tetrahydropyridin-4-yl)-3-(pyridin-4-yl)-1*H*-pyrrole,
2-(4-fluorophenyl)-3-(pyridin-4-yl)-4-(1,2,3,6-tetrahydro-2,2,6,6-tetramethylpyridin-4-yl)-1*H*-pyrrole,
2-(4-fluorophenyl)-4-(2-methyl-1,2,3,6-tetrahydropyridin-4-yl)-3-(pyridin-4-yl)-1*H*-pyrrole,
2-(4-fluorophenyl)-4-(6-methyl-1,2,3,6-tetrahydropyridin-4-yl)-3-(pyridin-4-yl)-1*H*-pyrrole,
4-(1-ethyl-1,2,3,6-tetrahydropyridin-4-yl)-2-(4-fluorophenyl)-3-(pyridin-4-yl)-1*H*-pyrrole,
2-(4-fluorophenyl)-4-(1-isopropyl-1,2,3,6-tetrahydropyridin-4-yl)-3-(pyridin-4-yl)-1*H*-pyrrole,

2-(4-fluorophenyl)-4-(1-propyl-1,2,3,6-tetrahydropyridin-4-yl)-3-(pyridin-4-yl)-1*H*-pyrrole,
2-(4-fluorophenyl)-4-(1-phenethyl-1,2,3,6-tetrahydropyridin-4-yl)-3-(pyridin-4-yl)-1*H*-pyrrole,
2-(4-fluorophenyl)-4-(1,2,3,5,8,8a-hexahydroindolizin-7-yl)-3-(pyridin-4-yl)-1*H*-pyrrole,
2-(4-fluorophenyl)-4-(1,2,3,5,6,8a-hexahydroindolizin-7-yl)-3-(pyridin-4-yl)-1*H*-pyrrole,
4-(1,6-dimethyl-1,2,3,6-tetrahydropyridin-4-yl)-2-(4-fluorophenyl)-3-(pyridin-4-yl)-1*H*-pyrrole,
4-(1,2-dimethyl-1,2,3,6-tetrahydropyridin-4-yl)-2-(4-fluorophenyl)-3-(pyridin-4-yl)-1*H*-pyrrole,
2-(4-fluorophenyl)-4-(1,2,2,6,6-pentamethyl-1,2,3,6-tetrahydropyridin-4-yl)-3-(pyridin-4-yl)-1*H*-pyrrole,
4-(1-ethyl-6-methyl-1,2,3,6-tetrahydropyridin-4-yl)-2-(4-fluorophenyl)-3-(pyridin-4-yl)-1*H*-pyrrole,
4-(1-ethyl-2-methyl-1,2,3,6-tetrahydropyridin-4-yl)-2-(4-fluorophenyl)-3-(pyridin-4-yl)-1*H*-pyrrole,
4-(6-allyl-1-methyl-1,2,3,6-tetrahydropyridin-4-yl)-2-(4-fluorophenyl)-3-(pyridin-4-yl)-1*H*-pyrrole,
4-(2-allyl-1-methyl-1,2,3,6-tetrahydropyridin-4-yl)-2-(4-fluorophenyl)-3-(pyridin-4-yl)-1*H*-pyrrole,
4-(6-benzyl-1-methyl-1,2,3,6-tetrahydropyridin-4-yl)-2-(4-fluorophenyl)-3-(pyridin-4-yl)-1*H*-pyrrole,
4-(2-benzyl-1-methyl-1,2,3,6-tetrahydropyridin-4-yl)-2-(4-fluorophenyl)-3-(pyridin-4-yl)-1*H*-pyrrole,
2-(4-fluorophenyl)-4-(6-methyl-1-propyl-1,2,3,6-tetrahydropyridin-4-yl)-3-(pyridin-4-yl)-1*H*-pyrrole,
2-(4-fluorophenyl)-4-(2-methyl-1-propyl-1,2,3,6-tetrahydropyridin-4-yl)-3-(pyridin-4-yl)-1*H*-pyrrole,
2-(4-fluorophenyl)-4-(1,3,4,6,7,9a-hexahydro-2*H*-quinolizin-8-yl)-3-(pyridin-4-yl)-1*H*-pyrrole,
2-(4-fluorophenyl)-4-(1,3,4,6,9,9a-hexahydro-2*H*-quinolizin-8-yl)-3-(pyridin-4-yl)-1*H*-pyrrole,
2-(4-fluorophenyl)-4-(1,2,3,5,8,8a-hexahydroindolizin-7-yl)-3-(2-methylaminopyrimidin-4-yl)-1*H*-pyrrole,
2-(4-fluorophenyl)-4-(1,2,3,5,6,8a-hexahydroindolizin-7-yl)-3-(2-methylaminopyrimidin-4-yl)-1*H*-pyrrole,
2-(3,4-difluorophenyl)-4-(1,2,3,5,8,8a-hexahydroindolizin-7-yl)-3-(pyridin-4-yl)-1*H*-pyrrole,
2-(3,4-difluorophenyl)-4-(1,2,3,5,6,8a-hexahydroindolizin-7-yl)-3-(pyridin-4-yl)-1*H*-pyrrole,
4-(1,2,3,5,8,8a-hexahydroindonzin-7-yl)-3-(pyridin-4-yl)-2-(3-trifluoromethylphenyl)-1*H*-pyrrole,
4-(1,2,3,5,6,8a-hexahydroindolizin-7-yl)-3-(pyridin-4-yl)-2-(3-trifluoromethylphenyl)-1*H*-pyrrole,
2-(3-fluorophenyl)-4-(1,2,3,5,8,8a-hexahydroindolizin-7-yl)-3-(pyridin-4-yl)-1*H*-pyrrole,
2-(3-fluorophenyl)-4-(1,2,3,5,6,8a-hexahydroindolizin-7-yl)-3-(pyridin-4-yl)-1*H*-pyrrole,
2-(3-chlorophenyl)-4-(1,2,3,5,8,8a-hexahydroindolizin-7-yl)-3-(pyridin-4-yl)-1*H*-pyrrole,
2-(3-chlorophenyl)-4-(1,2,3,5,6,8a-hexahydroindolizin-7-yl)-3-(pyridin-4-yl)-1*H*-pyrrole,
4-(1,2,3,5,8,8a-hexahydroindolizin-7-yl)-2-phenyl-3-(pyridin-4-yl)-1*H*-pyrrole, and
4-(1,2,3,5,6,8a-hexahydroindolizin-7-yl)-2-phenyl-3-(pyridin-4-yl)-1*H*-pyrrole.

**9.** A compound of formula (I) according to claim 1 selected from the following group of compounds, or a pharmacologically acceptable salt, ester or amide thereof:

2-(4-fluorophenyl)-3-(pyridin-4-yl)-4-(1,2,3,6-tetrahydropyridin-4-yl)-1*H*-pyrrole,
2-(4-fluorophenyl)-4-(6-methyl-1,2,3,6-tetrahydropyndin-4-yl)-3-(pyridin-4-yl)-1*H*-pyrrole,
4-(1-ethyl-1,2,3,6-tetrahydropyridin-4-yl)-2-(4-fluorophenyl)-3-(pyridin-4-yl)-1*H*-pyrrole,
2-(4-fluorophenyl)-4-(1-isopropyl-1,2,3,6-tetrahydropyridin-4-yl)-3-(pyridin-4-yl)-1*H*-pyrrole,
2-(4-fluorophenyl)-4-(1-propyl-1,2,3,6-tetrahydropyridin-4-yl)-3-(pyridin-4-yl)-1*H*-pyrrole,
2-(4-fluorophenyl)-4-(1-phenethyl-1,2,3,6-tetrahydropyridin-4-yl)-3-(pyridin-4-yl)-1*H*-pyrrole,
2-(4-fluorophenyl)-4-(1,2,3,5,8,8a-hexahydroindolizin-7-yl)-3-(pyridin-4-yl)-1*H*-pyrrole,
4-(1,6-dimethyl-1,2,3,6-tetrahydropyridin-4-yl)-2-(4-fluorophenyl)-3-(pyridin-4-yl)-1*H*-pyrrole,
4-(1,2-dimethyl-1,2,3,6-tetrahydropyridin-4-yl)-2-(4-fluorophenyl)-3-(pyridin-4-yl)-1*H*-pyrrole,
4-(1-ethyl-6-methyl-1,2,3,6-tetrahydropyridin-4-yl)-2-(4-fluorophenyl)-3-(pyridin-4-yl)-1*H*-pyrrole,
4-(1-ethyl-2-methyl-1,2,3,6-tetrahydropyridin-4-yl)-2-(4-fluorophenyl)-3-(pyridin-4-yl)-1*H*-pyrrole,
2-(4-fluorophenyl)-4-(6-methyl-1-propyl-1,2,3,6-tetrahydropyridin-4-yl)-3-(pyridin-4-yl)-1*H*-pyrrole,
2-(4-fluorophenyl)-4-(2-methyl-1-propyl-1,2,3,6-tetrahydropyridin-4-yl)-3-(pyridin-4-yl)-1*H*-pyrrole,
2-(4-fluorophenyl)-4-(1,3,4,6,7,9a-hexahydro-2*H*-quinolizin-8-yl)-3-(pyridin-4-yl)-1*H*-pyrrole,
2-(3,4-difluorophenyl)-4-(1,2,3,5,6,8a-hexahydroindolizin-7-yl)-3-(pyridin-4-yl)-1*H*-pyrrole, and
4-(1,2,3,5,6,8a-hexahydroindolizin-7-yl)-3-(pyridin-4-yl)-2-(3-trifluoromethylphenyl)-1*H*-pyrrole.

**10.** A compound according to claim 1 selected from 2-(4-fluorophenyl)-4-(1-phenethyl-1,2,3,6-tetrahydropyridin-4-yl)-3-(pyridin-4-yl)-1*H*-pyrrole, and pharmacologically acceptable salts, esters and amides thereof.

**11.** A compound according to claim 1 selected from 2-(4-fluorophenyl)-4-(1,2,3,5,6,8a-hexahydroindolizin-7-yl)-3-(pyridin-4-yl)-1*H*-pyrrole, and pharmacologically acceptable salts, esters and amides thereof.

**12.** A compound according to claim 1 selected from 2-(4-fluorophenyl)-4-(6-methyl-1-propyl-1,2,3,6-tetrahydropyridin-4-yl)-3-(pyridin-4-yl)-1*H*-pyrrole, and pharmacologically acceptable salts, esters and amides thereof.

**13.** A compound according to claim 1 selected from 2-(4-fluorophenyl)-4-(2-methyl-1-propyl-1,2,3,6-tetrahydropyridin-4-yl)-3-(pyridin-4-yl)-1*H*-pyrrole, and pharmacologically acceptable salts, esters and amides thereof.

**14.** A compound according to claim 1 selected from 2-(4-fluorophenyl)-4-(1,3,4,6,7,9a-hexahydro-2*H*-quinolizin-8-yl)-3-(pyridin-4-yl)-1*H*-pyrrole, and pharmacologically acceptable salts, esters and amides thereof.

**15.** A compound according to claim 1 selected from 2-(3,4-difluorophenyl)-4-(1,2,3,5,6,8a-hexahydroindolizin-7-yl)-3-(pyridin-4-yl)-1*H*-pyrrole, and pharmacologically acceptable salts, esters and amides thereof.

**16.** A compound according to claim 1 selected from 4-(1,2,3,5,6,8a-hexahydroindolizin-7-yl)-3-(pyridin-4-yl)-2-(3-trifluoromethylphenyl)-1*H*-pyrrole, and pharmacologically acceptable salts, esters and amides thereof.

**17.** A pharmaceutical composition comprising an effective amount of a pharmacologically active compound together with a carrier or diluent therefor, wherein said pharmacologically active compound is a compound of formula (I) according to any one of claims 1 to 16 or a pharmacologically acceptable salt, ester or amide thereof.

**18.** A compound of formula (I) according to any one of claims 1 to 16 or a pharmacologically acceptable salt, ester or amide thereof for use as a medicament.

**19.** A compound of formula (I) according to any one of claims 1 to 16 or a pharmacologically acceptable salt, ester or amide thereof for use in inhibiting the production of inflammatory cytokines in a mammal.

**20.** A compound of formula (I) according to any one of claims 1 to 16 or a pharmacologically acceptable salt, ester or amide thereof for use in inhibiting bone resorption.

**21.** A compound of formula (I) according to any one of claims 1 to 16 or a pharmacologically acceptable salt, ester or amide thereof for use in the treatment or prophylaxis of inflammatory diseases.

**22.** A compound of formula (I) according to any one of claims 1 to 16 or a pharmacologically acceptable salt, ester or amide thereof for use in the treatment or prophylaxis of viral diseases.

**23.** A compound of formula (I) according to any one of claims 1 to 16 or a pharmacologically acceptable salt, ester or amide thereof for use in relieving pain or pyrexia.

**24.** A compound of formula (I) according to any one of claims 1 to 16 or a pharmacologically acceptable salt, ester or amide thereof for use in the treatment or prophylaxis of chronic rheumatoid arthritis.

**25.** A compound of formula (I) according to any one of claims 1 to 16 or a pharmacologically acceptable salt, ester or amide thereof for use in the treatment or prophylaxis of osteoarthritis.

**26.** A compound of formula (I) according to any one of claims 1 to 16 or a pharmacologically acceptable salt, ester or amide thereof for use in the treatment or prophylaxis of cancer.

**27.** A compound of formula (I) according to any one of claims 1 to 16 or a pharmacologically acceptable salt, ester or amide thereof for use in the treatment or prophylaxis of hepatitis.

**28.** A compound of formula (I) according to any one of claims 1 to 16 or a pharmacologically acceptable salt, ester or amide thereof for use in the treatment or prophylaxis of a disease selected from the group consisting of allergic diseases, septicaemia, psoriasis, osteoporosis, ulcerative colitis, diabetes, nephritis, ischemic heart disease, Alzheimer's disease and arteriosclerosis.

**29.** The use of a compound of formula (I) according to any one of claims 1 to 16 or a pharmacologically acceptable salt, ester or amide thereof in the manufacture of a medicament for the treatment or prophylaxis of diseases which can be treated or prevented by inhibiting the production of inflammatory cytokines in a mammal.

**30.** The use according to claim 29 of a compound of formula (I) according to any one of claims 1 to 16 or a pharmacologically acceptable salt, ester or amide thereof in the manufacture of a medicament for inhibiting bone resorption.

**31.** The use according to claim 29 of a compound of formula (I) according to any one of claims 1 to 16 or a pharmacologically acceptable salt, ester or amide thereof in the manufacture of a medicament for the treatment or prophylaxis of inflammatory diseases.

**32.** The use according to claim 29 of a compound of formula (I) according to any one of claims 1 to 16 or a pharmacologically acceptable salt, ester or amide thereof in the manufacture of a medicament for the treatment or prophylaxis of viral diseases.

**33.** The use according to claim 29 of a compound of formula (I) according to any one of claims 1 to 16 or a pharmacologically acceptable salt, ester or amide thereof in the manufacture of a medicament for relieving pain or pyrexia.

**34.** The use according to claim 29 of a compound of formula (I) according to any one of claims 1 to 16 or a pharmacologically acceptable salt, ester or amide thereof in the manufacture of a medicament for the treatment or prophylaxis of chronic rheumatoid arthritis.

**35.** The use according to claim 29 of a compound of formula (I) according to any one of claims 1 to 16 or a pharmacologically acceptable salt, ester or amide thereof in the manufacture of a medicament for the treatment or prophylaxis of osteoarthritis.

**36.** The use according to claim 29 of a compound of formula (I) according to any one of claims 1 to 16 or a pharmacologically acceptable salt, ester or amide thereof in the manufacture of a medicament for the treatment or prophylaxis of cancer.

**37.** The use according to claim 29 of a compound of formula (I) according to any one of claims 1 to 16 or a pharmacologically acceptable salt, ester or amide thereof in the manufacture of a medicament for the treatment or prophylaxis of hepatitis.

**38.** The use according to claim 29 of a compound of formula (I) according to any one of claims 1 to 16 or a pharmacologically acceptable salt, ester or amide thereof in the manufacture of a medicament for the treatment or prophylaxis of a disease selected from the group consisting of allergic diseases, septicaemia, psoriasis, osteoporosis, ulcerative colitis, diabetes, nephritis, ischemic heart disease, Alzheimer's disease and arteriosclerosis.

**Patentansprüche**

**1.** Verbindung der Formel (I) oder ein pharmakologisch geeignetes Salz, pharmakologisch geeigneter Ester oder pharmakologisch geeignetes Amid davon

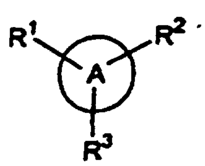

(I)

worin:

A einen Pyrrolring darstellt;

$R^1$ eine Phenyl- oder Naphthylgruppe ist, die gegebenenfalls mit mindestens einem Substituenten substituiert sein kann, der aus der Gruppe ausgewählt ist, die aus der nachstehend definierten Substituentengruppe α, der nachstehend definierten Substituentengruppe β, der nachstehend definierten Substituentengruppe γ und der nachstehend definierten Substituentengruppe δ besteht;

$R^2$ eine Pyridyl- oder Pyrimidinylgruppe ist, die gegebenenfalls mit mindestens einem Substituenten substituiert sein kann, der aus der Gruppe ausgewählt ist, die aus der nachstehend definierten Substituentengruppe α, der nachstehend definierten Substituentengruppe β, der nachstehend definierten Substituentengruppe γ und der nachstehend definierten Substituentengruppe δ besteht; und

$R^3$ eine Gruppe der folgenden Formel (II) ist:

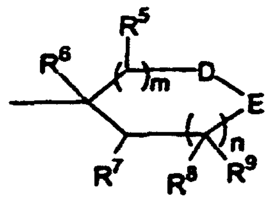

(II)

worin

m 0 oder 1 darstellt,
n 1 oder 2 darstellt,
einer der Substituenten D und E eine Gruppe der Formel >$NR^{10}$ darstellt und der andere eine Gruppe der Formel >$CR^{11}R^{12}$ darstellt,
einer der Substituenten $R^5$ und $R^7$ zusammen mit $R^6$ eine Einfachbindung darstellt und der andere einen Substituenten darstellt, der aus der Gruppe ausgewählt ist, die aus Wasserstoffatomen, der nachstehend definierten Substituentengruppe α, der nachstehend definierten Substituentengruppe γ und der nachstehend definierten Substituentengruppe δ besteht, $R^8$, $R^9$ und $R^{12}$ gleich oder unterschiedlich voneinander sein können und jeweils einen Substituenten darstellen, der aus der Gruppe ausgewählt ist, die aus Wasserstoffatomen, der nachstehend definierten Substituentengruppe α, der nachstehend definierten Substituentengruppe γ und der nachstehend definierten Substituentengruppe δ besteht, und $R^{10}$ und $R^{11}$ gleich oder unterschiedlich voneinander sein können und jeweils einen Substituenten darstellen, der aus der Gruppe ausgewählt ist, die aus Wasserstoffatomen, der nachstehend definierten Substituentengruppe α, der nachstehend definierten Substituentengruppe γ und der nachstehend definierten Substituentengruppe δ besteht, oder $R^{10}$ und $R^{11}$ zusammen eine Alkylengruppe mit 1 bis 6 Kohlenstoffatomen bilden, die unsubstituiert ist oder mit mindestens einem Substituenten substituiert ist, der aus der Gruppe ausgewählt ist, die aus der nachstehend definierten Substituentengruppe α, der nachstehend definierten Substituentengruppe β, der nachstehend definierten Substituentengruppe γ und der nachstehend definierten Substituentengruppe δ besteht;

mit der Maßgabe, dass die Substituenten $R^1$ und $R^3$ an die zwei Atome des Pyrrolrings gebunden sind, die dem Atom des Pyrrolrings, an dem der Substituent $R^2$ gebunden ist, benachbart sind;

die Substituentengruppe α umfaßt Hydroxygruppen, Nitrogruppen, Cyangruppen, Halogenatome, nachstehend definierte Niederalkoxygruppen, nachstehend definierte Halogen-Niederalkoxygruppen, nachstehend definierte Niederalkylthiogruppen und nachstehend definierte Halogen-Niederalkylthiogruppen;

die Substituentengruppe β umfaßt Gruppen der Formel $-NR^cR^d$, worin $R^c$ und $R^d$ gleich oder unterschiedlich sind und jeweils unabhängig voneinander aus der Gruppe ausgewählt sind, die aus Wasserstoffatomen, nachstehend definierten Niederalkylgruppen, nachstehend definierten Niederalkenylgruppen, nachstehend definierten Niederalkinylgruppen, nachstehend definierten Aralkylgruppen und Niederalkylsulfonylgruppen besteht, oder $R^c$ und $R^d$ zusammen mit dem Stickstoffatom, an das $R^c$ und $R^d$ gebunden sind, eine nachstehend definierte Heterocyclylgruppe bilden;

die Substituentengruppe γ umfaßt nachstehend definierte Niederalkylgruppen, die mit einer Gruppe der Formel $-NR^cR^d$ substituiert sind, worin $R^c$ und $R^d$ wie vorstehend definiert sind; und

die Substituentengruppe δ umfaßt nachstehend definierte Niederalkylgruppen, die gegebenenfalls mit mindestens einem Substituenten substituiert sein können, der aus der vorstehend definierten Substituentengruppe

$\alpha$ ausgewählt ist, nachstehend definierte Niederalkenylgruppen, die gegebenenfalls mit mindestens einem Substituenten substituiert sein können, der aus der vorstehend definierten Substituentengruppe $\alpha$ ausgewählt ist, nachstehend definierte Niederalkinylgruppen, die gegebenenfalls mit mindestens einem Substituenten substituiert sein können, der aus der vorstehend definierten Substituentengruppe $\alpha$ ausgewählt ist, nachstehend definierte Aralkylgruppen und nachstehend definierte Cycloalkylgruppen;

die Niederalkylgruppen in der vorstehenden Definition der Substituenten $R^c$ und $R^d$, die Niederalkylgruppen, die gegebenenfalls mit mindestens einem Substituenten substituiert sein können, der aus der Substituentengruppe $\alpha$ in der vorstehenden Definition der Substituentengruppe $\delta$ ausgewählt ist, und die Niederalkylreste der Niederalkylgruppen, die mit einer Gruppe der Formel $-NR^cR^d$ in der vorstehenden Definition der Substituentengruppe $\gamma$ substituiert sind, sind geradkettige oder verzweigte Alkylgruppen mit 1 bis 6 Kohlenstoffatomen;

die Niederalkenylgruppen in der vorstehenden Definition der Substituenten $R^c$ und $R^d$ und die Niederalkenylgruppen, die gegebenenfalls mit mindestens einem Substituenten, der aus der Substituentengruppe $\alpha$ in der vorstehenden Definition der Substituentengruppe $\delta$ ausgewählt ist, substituiert sein können, sind geradkettige oder verzweigte Alkenylgruppen mit 2 bis 6 Kohlenstoffatomen;

die Niederalkinylgruppen in der vorstehenden Definition der Substituenten $R^c$ und $R^d$ und die Niederalkinylgruppen, die gegebenenfalls mit mindestens einem Substituenten substituiert sein können, der aus der Substituentengruppe $\alpha$ in der vorstehenden Definition der Substituentengruppe $\delta$ ausgewählt ist, sind geradkettige oder verzweigte Alkinylgruppen mit 2 bis 6 Kohlenstoffatomen;

die Aralkylgruppen in der vorstehenden Definition von $R^c$ und $R^d$ und in der vorstehenden Definition der Substituentengruppe $\delta$ sind Niederalkylgruppen wie vorstehend definiert, die mit mindestens einer Arylgruppe substituiert sind, die unter den aromatischen Kohlenwasserstoffgruppen mit 6 bis 14 Kohlenstoffatomen in einem oder mehreren Ringen ausgewählt ist, wobei die aromatischen Kohlenwasserstoffgruppen gegebenenfalls mit einer Cycloalkylgruppe mit 3 bis 10 Kohlenstoffatomen kondensiert sind;

die Cycloalkylgruppen in der vorstehenden Substituentengruppe $\delta$ sind Cycloalkylgruppen mit 3 bis 7 Kohlenstoffatomen;

die Niederalkylsulfonylgruppen in der Definition der Substituenten $R^c$ und $R^d$ sind Niederalkylgruppen wie vorstehend definiert, die an eine Sulfonylgruppe gebunden sind;

die Heterocyclylgruppen, die von den Substituenten $R^c$ und $R^d$ zusammen mit dem Stickstoffatom, an das sie gebunden sind, gebildet werden, sind 4- bis 12-gliedrige nichtaromatische heterocyclische Gruppen, die ein Stickstoffatom und gegebenenfalls ein weiteres Heteroatom aufweisen, das aus der Gruppe ausgewählt ist, die aus Sauerstoff-, Schwefel- und Stickstoffatomen ausgewählt ist, wobei die heterocyclischen Gruppen gegebenenfalls mit einer anderen cyclischen Gruppe kondensiert sind, die aus der Gruppe ausgewählt ist, die aus Arylgruppen, die in der vorstehenden Definition von Aralkylgruppen definiert sind, und Heteroarylgruppen besteht, die unter 5- bis 7-gliedrigen aromatischen heterocyclischen Gruppen ausgewählt sind, die 1 bis 3 Heteroatome enthalten, die aus der aus Schwefel-, Sauerstoff- und Stickstoffatomen bestehenden Gruppe ausgewählt sind;

die Niederalkoxygruppen in der vorstehenden Definition der Substituentengruppe $\alpha$ sind Niederalkylgruppen wie vorstehend definiert, die an ein Sauerstoffatom gebunden sind;

die Halogen-Niederalkoxygruppen in der vorstehenden Definition der Substituentengruppe $\alpha$ sind Niederalkoxygruppen wie vorstehend definiert, die mit mindestens einem Halogenatom substituiert sind;

die Niederalkylthiogruppen in der vorstehenden Definition der Substituentengruppe $\alpha$ sind Niederalkylgruppen wie vorstehend definiert, die an ein Schwefelatom gebunden sind;

die Halogen-Niederalkylthiogruppen in der vorstehenden Definition der Substituentengruppe $\alpha$ sind Niederalkylthiogruppen wie vorstehend definiert, die mit mindestens einem Halogenatom substituiert sind.

**2.** Verbindung nach Anspruch 1 oder ein pharmakologisch geeignetes Salz, pharmakologisch geeigneter Ester oder

pharmakologisch geeignetes Amid davon, worin:

$R^1$ eine Phenylgruppe ist, die gegebenenfalls mit mindestens einem Substituenten substituiert sein kann, der aus der Gruppe ausgewählt ist, die aus der nachstehend definierten Substituentengruppe $\alpha^1$, der nachstehend definierten Substituentengruppe $\beta^1$, der nachstehend definierten Substituentengruppe $\gamma^1$ und der nachstehend definierten Substituentengruppe $\delta^1$ besteht;

$R^2$ eine 4-Pyridyl- oder 4-Pyrimidinylgruppe ist, die gegebenenfalls mit mindestens einem Substituenten substituiert sein kann, der aus der Gruppe ausgewählt ist, die aus der Substituentengruppe $\alpha$, der Substituentengruppe $\beta$, der Substituentengruppe $\gamma$ und der Substituentengruppe $\delta$ besteht;

$R^3$ eine Gruppe der in Anspruch 1 definierten Formel (II) ist, worin m 1 ist und n 1 ist;

die Substituentengruppe $\alpha^1$ Halogenatome, in Anspruch 1 definierte Niederalkoxygruppen und in Anspruch 1 definierte Halogen-Niederalkoxygruppen umfaßt;

die Substituentengruppe $\beta^1$ eine Gruppe der Formel $-NR^cR^d$ umfaßt, worin einer der Substituenten $R^c$ und $R^d$ aus der Gruppe ausgewählt ist, die aus Wasserstoffatomen und in Anspruch 1 definierten Niederalkylgruppen ausgewählt ist und der andere Substituent aus der Gruppe ausgewählt ist, die aus Wasserstoffatomen, in Anspruch 1 definierten Niederalkylgruppen und in Anspruch 1 definierten Aralkylgruppen besteht;

die Substituentengruppe $\gamma^1$ in Anspruch 1 definierte Niederalkylgruppen umfaßt, die mit einem Substituenten substituiert sind, der aus der Gruppe ausgewählt ist, die aus Aminogruppen, Aminogruppen, die mit einer oder zwei Niederalkylgruppen nach Anspruch 1 substituiert sind, und Aminogruppen besteht, die mit einer Aralkylgruppe nach Anspruch 1 substituiert sind; und

die Substituentengruppe $\delta^1$ Niederalkylgruppen nach Anspruch 1, Halogen-Niederalkylgruppen, die Niederalkylgruppen nach Anspruch 1 umfassen, die mit mindestens einem Halogenatom substituiert sind, Hydroxy-Niederalkylgruppen, die Niederalkylgruppen nach Anspruch 1 umfassen, die mit mindestens einer Hydroxygruppe substituiert sind, und Nitro-Niederalkylgruppen umfaßt, die Niederalkylgruppen nach Anspruch 1 umfassen, die mit mindestens einer Nitrogruppe substituiert sind.

**3.** Verbindung der Formel (I) nach Anspruch 1 oder ein pharmakologisch geeignetes Salz, pharmakologisch geeigneter Ester oder pharmakologisch geeignetes Amid davon, worin:

$R^1$ eine Phenylgruppe ist, die gegebenenfalls mit mindestens einem Substituenten substituiert sein kann, die aus der Gruppe ausgewählt ist, die aus der nachstehend definierten Substituentengruppe $\alpha^1$, der nachstehend definierten Substituentengruppe $\beta^1$, der nachstehend definierten Substituentengruppe $\gamma^1$ und der nachstehend definierten Substituentengruppe $\delta^1$ besteht;

$R^2$ eine 4-Pyridyl- oder 4-Pyrimidinylgruppe ist, die gegebenenfalls mit mindestens einem Substituenten substituiert sein kann, der aus der Gruppe ausgewählt ist, die aus der Substituentengruppe $\alpha$, Substituentengruppe $\beta$, Substituentengruppe $\gamma$ und Substituentengruppe $\delta$ besteht; und

$R^3$ eine Gruppe der Formel (II) nach Anspruch 1 ist, worin E eine Gruppe der Formel $>NR^{10}$ ist, worin $R^{10}$ wie in Anspruch 1 definiert ist, und D eine Gruppe der Formel $>CR^{11}R^{12}$ darstellt, worin $R^{11}$ und $R^{12}$ in Anspruch 1 definiert sind;

die Substituentengruppe $\alpha^1$ umfaßt Halogenatome, Niederalkoxygruppen nach Anspruch 1 und Halogen-Niederalkoxygruppen nach Anspruch 1;

die Substituentengruppe $\beta^1$ umfaßt eine Gruppe der Formel $-NR^cR^d$, worin einer der Substituenten $R^c$ und $R^d$ aus der Gruppe ausgewählt ist, die aus Wasserstoffatomen und Niederalkylgruppen nach Anspruch 1 besteht, und der andere Substituent aus der Gruppe ausgewählt ist, die aus Wasserstoffatomen, Niederalkylgruppen nach Anspruch 1 und Aralkylgruppen nach Anspruch 1 besteht;

die Substituentengruppe $\gamma^1$ umfaßt Niederalkylgruppen nach Anspruch 1, die mit einem Substituenten substituiert sind, der aus der Gruppe ausgewählt ist, die aus Aminogruppen, Aminogruppen, die mit einer oder zwei

Niederalkylgruppen nach Anspruch 1 substituiert sind, und Aminogruppen besteht, die mit einer Aralkylgruppe nach Anspruch 1 substituiert sind; und

die Substituentengruppe $\delta^1$ umfaßt Niederalkylgruppen nach Anspruch 1, Halogen-Niederalkylgruppen, die Niederalkylgruppen nach Anspruch 1, die mit mindestens einem Halogenatom substituiert sind, Hydroxy-Niederalkylgruppen, die Niederalkylgruppen nach Anspruch 1 umfassen, die mit mindestens einer Hydroxygruppe substituiert sind, und Nitro-Niederalkylgruppen, die Niederalkylgruppen nach Anspruch 1 umfassen, die mit mindestens einer Nitrogruppe substituiert sind.

**4.** Verbindung der Formel (I) nach Anspruch 1 oder ein pharmakologisch geeignetes Salz, pharmakologisch geeigneter Ester oder pharmakologisch geeignetes Amid davon, worin:

$R^1$ eine Phenylgruppe ist, die gegebenenfalls mit mindestens einem Substituenten substituiert sein kann, der aus der Gruppe ausgewählt ist, die aus Halogenatomen, Halogen-Niederalkylgruppen, die Niederalkylgruppen nach Anspruch 1 umfassen, die mit mindestens einem Halogenatom substituiert sind, und Halogen-Niederalkoxygruppen nach Anspruch 1 besteht;

$R^2$ eine 4-Pyridyl- oder 4-Pyrimidinylgruppe ist, die gegebenenfalls an der 2-Position mit einem Substituenten substituiert sein kann, der aus der Gruppe ausgewählt ist, die aus der Substituentengruppe $\alpha$, Substituentengruppe $\beta$, Substituentengruppe $\gamma$ und der Substituentengruppe $\delta$ besteht.

**5.** Verbindung der Formel (I) nach Anspruch 1 oder ein pharmakologisch geeignetes Salz, pharmakologisch geeigneter Ester oder pharmakologisch geeignetes Amid davon, worin:

$R^1$ aus der Gruppe ausgewählt ist, die aus Phenyl-, 4-Fluorphenyl-, 3-Fluorphenyl-, 3-Chlorphenyl-, 3,4-Difluorphenyl-, 3,4,5-Trifluorphenyl-, 3-Chlor-4-fluorphenyl-, 3-Difluormethoxyphenyl- und 3-Trifluormethylphenylgruppen besteht;

$R^2$ eine 4-Pyridyl- oder 4-Pyrimidinylgruppe ist, die gegebenenfalls an der 2-Position mit einem Substituenten substituiert sein kann, der aus der Gruppe ausgewählt ist, die aus Methoxy-, Amino-, Methylamino-, Benzylamino- und $\alpha$-Methylbenzylaminogruppen besteht; und

$R^3$ eine Gruppe der Formel (II) nach Anspruch 1 ist, worin $R^{10}$ und $R^{11}$ zusammen eine geradkettige Alkylengruppe mit 3 oder 4 Kohlenstoffatomen bilden, die unsubstituiert ist oder mit mindestens einem Substituenten substituiert ist, der aus der Gruppe ausgewählt ist, die aus der Substituentengruppe $\alpha$, Substituentengruppe $\beta$, Substituentengruppe $\gamma$ und Substituentengruppe $\delta$ besteht.

**6.** Verbindung der Formel (I) nach Anspruch 1 oder ein pharmakologisch geeignetes Salz, pharmakologisch geeigneter Ester oder pharmakologisch geeignetes Amid davon, worin:

$R^1$ aus der Gruppe ausgewählt ist, die aus Phenyl-, 4-Fluorphenyl-, 3-Fluorphenyl-, 3-Chlorphenyl-, 3,4-Difluorphenyl-, 3,4,5-Trifluorphenyl-, 3-Chlor-4-fluorphenyl-, 3-Difluormethoxyphenyl- und 3-Trifluormethylphenylgruppen besteht.

$R^2$ eine 4-Pyridyl- oder 4-Pyrimidinylgruppe ist, die gegebenenfalls an der 2-Position mit einem Substituenten substituiert sein kann, der aus der Gruppe ausgewählt ist, die aus Methoxy-, Amino-, Methylamino-, Benzylamino- und $\alpha$-Methylbenzylaminogruppen besteht; und

$R^3$ eine Gruppe der Formel (II) nach Anspruch 1 ist, worin $R^7$, $R^8$ und $R^9$ jeweils ein Wasserstoffatom sind und $R^{10}$ und $R^{11}$ zusammen eine geradkettige Alkylengruppe mit 3 oder 4 Kohlenstoffatomen bilden, die unsubstituiert ist oder mit mindestens einem Substituenten substituiert ist, der aus der Gruppe ausgewählt ist, die aus der Substituentengruppe $\alpha$, Substituentengruppe $\beta$, Substituentengruppe $\gamma$ und Substituentengruppe $\delta$ besteht.

**7.** Verbindung der Formel (I) nach einem der Ansprüche 1 bis 6 oder ein pharmakologisch geeignetes Salz, pharmakologisch geeigneter Ester oder pharmakologisch geeignetes Amid davon, worin die Verbindung der Formel (I-1) eine Verbindung der nachstehenden Formel (I-1) oder (I-3) ist:

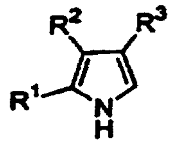
(I-1)

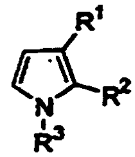
(I-3)

8. Verbindung der Formel (I) nach Anspruch 1, die aus der folgenden Gruppe von Verbindungen ausgewählt ist, oder ein pharmakologisch geeignetes Salz, pharmakologisch geeigneter Ester oder pharmakologisch geeignetes Amid davon:

4-(1-Acetyl-1,2,3,6-tetrahydropyridin-4-yl)-2-(4-fluorphenyl)-3-(pyridin-4-yl)-1H-pyrrol,
2-(4-Fluorphenyl)-3-(pyridin-4-yl)-4-(1,2,3,6-tetrahydropyridin-4-yl)-1H-pyrrol,
2-(4-Fluorphenyl)-4-(1-methyl-1,2,3,6-tetrahydropyridin-4-yl)-3-(pyridin-4-yl)-1H-pyrrol,
2-(4-Fluorphenyl)-3-(pyridin-4-yl)-4-(1,2,3,6-tetrahydro-2,2,6,6-tetramethylpyridin-4-yl)-1H-pyrrol,
2-(4-Fluorphenyl)-4-(2-methyl-1,2,3,6-tetrahydropyridin-4-yl)-3-(pyridin-4-yl)-1H-pyrrol,
2-(4-Fluorphenyl)-4-(6-methyl-1,2,3,6-tetrahydropyridin-4-yl)-3-(pyridin-4-yl)-1H-pyrrol,
4-(1-Ethyl-1,2,3,6-tetrahydropyridin-4-yl)-2-(4-fluorphenyl)-3-(pyridin-4-yl)-1H-pyrrol,
2-(4-Fluorphenyl)-4-(1-isopropyl-1,2,3,6-tetrahydropyridin-4-yl)-3-(pyridin-4-yl)-1H-pyrrol,
2-(4-Fluorphenyl)-4-(1-propyl-1,2,3,6-tetrahydropyridin-4-yl)-3-(pyridin-4-yl)-1H-pyrrol,
2-(4-Fluorphenyl)-4-(1-phenethyl-1,2,3,6-tetrahydropyridin-4-yl)-3-(pyridin-4-yl)-1H-pyrrol,
2-(4-Fluorphenyl)-4-(1,2,3,5,8,8a-hexahydroindolizin-7-yl)-3-(pyridin-4-yl)-1H-pyrrol,
2-(4-Fluorphenyl)-4-(1,2,3,5,6,8a-hexahydroindolizin-7-yl)-3-(pyridin-4-yl)-1H-pyrrol,
4-(1,6-Dimethyl-1,2,3,6-tetrahydropyridin-4-yl)-2-(4-fluorphenyl)-3-(pyridin-4-yl)-1H-pyrrol,
4-(1,2-Dimethyl-1,2,3,6-tetrahydropyridin-4-yl)-2-(4-fluorphenyl)-3-(pyridin-4-yl)-1H-pyrrol,
2-(4-Fluorphenyl)-4-(1,2,2,6,6-pentamethyl-1,2,3,6-tetrahydropyridin-4-yl)-3-(pyridin-4-yl)-1H-pyrrol,
4-(1-Ethyl-6-methyl-1,2,3,6-tetrahydropyridin-4-yl)-2-(4-fluorphenyl)-3-(pyridin-4-yl)-1H-pyrrol,
4-(1-Ethyl-2-methyl-1,2,3,6-tetrahydropyridin-4-yl)-2-(4-fluorphenyl)-3-(pyridin-4-yl)-1H-pyrrol,
4-(6-Allyl-1-methyl-1,2,3,6-tetrahydropyridin-4-yl)-2-(4-fluorphenyl)-3-(pyridin-4-yl)-1H-pyrrol,
4-(2-Allyl-1-methyl-1,2,3,6-tetrahydropyridin-4-yl)-2-(4-fluorphenyl)-3-(pyridin-4-yl)-1H-pyrrol,
4-(6-Benzyl-1-methyl-1,2,3,6-tetrahydropyridin-4-yl)-2-(4-fluorphenyl)-3-(pyridin-4-yl)-1H-pyrrol,
4-(2-Benzyl-1-methyl-1,2,3,6-tetrahydropyridin-4-yl)-2-(4-fluorphenyl)-3-(pyridin-4-yl)-1H-pyrrol,
2-(4-Fluorphenyl)-4-(6-methyl-1-propyl-1,2,3,6-tetrahydropyridin-4-yl)-3-(pyridin-4-yl)-1H-pyrrol,
2-(4-Fluorphenyl)-4-(2-methyl-1-propyl-1,2,3,6-tetrahydropyridin-4-yl)-3-(pyridin-4-yl)-1H-pyrrol,
2-(4-Fluorphenyl)-4-(1,3,4,6,7,9a-hexahydro-2H-chinolizin-8-yl)-3-(pyridin-4-yl)-1H-pyrrol,
2-(4-Fluorphenyl)-4-(1,3,4,6,9,9a-hexahydro-2H-chinolizin-8-yl)-3-(pyridin-4-yl)-1H-pyrrol,
2-(4-Fluorphenyl)-4-(1,2,3,5,8,8a-hexahydroindolizin-7-yl)-3-(2-methylaminopyrimidin-4-yl)-1H-pyrrol,
2-(4-Fluorphenyl)-4-(1,2,3,5,6,8a-hexahydroindolizin-7-yl)-3-(2-methylaminopyrimidin-4-yl)-1H-pyrrol,
2-(3,4-Difluorphenyl)-4-(1,2,3,5,8,8a-hexahydroindolizin-7-yl)-3-(pyridin-4-yl)-1H-pyrrol,
2-(3,4-Difluorphenyl)-4-(1,2,3,5,6,8a-hexahydroindolizin-7-yl)-3-(pyridin-4-yl)-1H-pyrrol,
4-(1,2,3,5,8,8a-Hexahydroindolizin-7-yl)-3-(pyridin-4-yl)-2-(3-trifluormethylphenyl)-1H-pyrrol,
4-(1,2,3,5,6,8a-Hexahydroindolizin-7-yl)-3-(pyridin-4-yl)-2-(3-trifluormethylphenyl)-1H-pyrrol,
2-(3-Fluorphenyl)-4-(1,2,3,5,8,8a-hexahydroindolizin-7-yl)-3-(pyridin-4-yl)-1H-pyrrol,
2-(3-Fluorphenyl)-4-(1,2,3,5,6,8a-hexahydroindolizin-7-yl)-3-(pyridin-4-yl)-1H-pyrrol,
2-(3-Chlorphenyl)-4-(1,2,3,5,8,8a-hexahydroindolizin-7-yl)-3-(pyridin-4-yl)-1H-pyrrol,
2-(3-Chlorphenyl)-4-(1,2,3,5,6,8a-hexahydroindolizin-7-yl)-3-(pyridin-4-yl)-1H-pyrrol,
4-(1,2,3,5,8,8a-Hexahydroindolizin-7-yl)-2-phenyl-3-(pyridin-4-yl)-1H-pyrrol, und
4-(1,2,3,5,6,8a-Hexahydroindolizin-7-yl)-2-phenyl-3-(pyridin-4-yl)-1H-pyrrol.

9. Verbindung der Formel (I) nach Anspruch 1, die aus der folgenden Gruppe von Verbindungen ausgewählt ist, oder ein pharmakologisch geeignetes Salz, pharmakologisch geeigneter Ester oder pharmakologisch geeignetes Amid davon:

2-(4-Fluorphenyl)-3-(pyridin-4-yl)-4-(1,2,3,6-tetrahydropyridin-4-yl)-1H-pyrrol,

2-(4-Fluorphenyl)-4-(6-methyl-1,2,3,6-tetrahydropyridin-4-yl)-3-(pyridin-4-yl)-1H-pyrrol,
4-(1-Ethyl-1,2,3,6-tetrahydropyridin-4-yl)-2-(4-fluorphenyl)-3-(pyridin-4-yl)-1H-pyrrol,
2-(4-Fluorphenyl)-4-(1-isopropyl-1,2,3,6-tetrahydropyridin-4-yl)-3-(pyridin-4-yl)-1H-pyrrol,
2-(4-Fluorphenyl)-4-(1-propyl-1,2,3,6-tetrahydropyridin-4-yl)-3-(pyridin-4-yl)-1H-pyrrol,
2-(4-Fluorphenyl)-4-(1-phenethyl-1,2,3,6-tetrahydropyridin-4-yl)-3-(pyridin-4-yl)1H-pyrrol,
2-(4-Fluorphenyl)-4-(1,2,3,5,6,8a-hexahydroindolizin-7-yl)-3-(pyridin-4-yl)-1H-pyrrol,
4-(1,6-Dimethyl-1,2,3,6-tetrahydropyridin-4-yl)-2-(4-fluorphenyl)-3-(pyridin-4-yl)1H-pyrrol,
4-(1,2-Dimethyl-1,2,3,6-tetrahydropyridin-4-yl)-2-(4-fluorphenyl)-3-(pyridin-4-yl)1H-pyrrol,
4-(1-Ethyl-6-methyl-1,2,3,6-tetrahydropyridin-4-yl)-2-(4-fluorphenyl)-3-(pyridin-4-yl)-1H-pyrrol,
4-(1-Ethyl-2-methyl-1,2,3,6-tetrahydropyridin-4-yl)-2-(4-fluorphenyl)-3-(pyridin-4-yl)-1H-pyrrol,
2-(4-Fluorphenyl)-4-(6-methyl-1-propyl-1,2,3,6-tetrahydropyridin-4-yl)-3-(pyridin-4-yl)-1H-pyrrol,
2-(4-Fluorphenyl)-4-(2-methyl-1-propyl-1,2,3,6-tetrahydropyridin-4-yl)-3-(pyridin-4-yl)-1H-pyrrol,
2-(4-Fluorphenyl)-4-(1,3,4,6,7,9a-hexahydro-2H-chinolizin-8-yl)-3-(pyridin-4-yl)-1H-pyrrol,
2-(3,4-Difluorphenyl)-4-(1,2,3,5,6,8a-hexahydroindolizin-7-yl)-3-(pyridin-4-yl)-1H-pyrrol, und
4-(1,2,3,5,6,8a-Hexahydroindolizin-7-yl)-3-(pyridin-4-yl)-2-(3-trifluormethylphenyl)-1H-pyrrol.

**10.** Verbindung nach Anspruch 1, ausgewählt unter 2-(4-Fluorphenyl)-4-(1-phenethyl-1,2,3,6-tetrahydropyridin-4-yl)-3-(pyridin-4-yl)-1H-pyrrol, und pharmakologisch geeigneten Salzen, Estern und Amiden davon.

**11.** Verbindung nach Anspruch 1, ausgewählt unter 2-(4-Fluorphenyl)-4-(1,2,3,5,6,8a-hexahydroindolizin-7-yl)-3-(pyridin-4-yl)-1H-pyrrol, und pharmakologisch geeigneten Salzen, Estern und Amiden davon.

**12.** Verbindung nach Anspruch 1, ausgewählt unter 2-(4-Fluorphenyl)-4-(6-methyl-1-propyl-1,2,3,6-tetrahydropyridin-4-yl)-3-(pyridin-4-yl)-1H-pyrrol, und pharmakologisch geeigneten Salzen, Estern und Amiden davon.

**13.** Verbindung nach Anspruch 1, ausgewählt unter 2-(4-Fluorphenyl)-4-(2-methyl-1-propyl-1,2,3,6-tetrahydropyridin-4-yl)-3-(pyridin-4-yl)-1H-pyrrol, und pharmakologisch geeigneten Salzen, Estern und Amiden davon.

**14.** Verbindung nach Anspruch 1, ausgewählt unter 2-(4-Fluorphenyl)-4-(1,3,4,6,7,9a-hexahydro-2H-chinolizin-8-yl)-3-(pyridin-4-yl)-1H-pyrrol, und pharmakologisch geeigneten Salzen, Estern und Amiden davon.

**15.** Verbindung nach Anspruch 1, ausgewählt unter 2-(3,4-Difluorphenyl)-4-(1,2,3,5,6,8a-hexahydroindolizin-7-yl)-3-(pyridin-4-yl)-1H-pyrrol, und pharmakologisch geeigneten Salzen, Estern und Amiden davon.

**16.** Verbindung nach Anspruch 1, ausgewählt unter 4-(1,2,3,5,6,8a-Hexahydroindolizin-7-yl)-3-(pyridin-4-yl)-2-(3-trifluormethylphenyl)-1H-pyrrol, und pharmakologisch geeigneten Salzen, Estern und Amiden davon.

**17.** Arzneimittelzusammensetzung, die eine wirksame Menge einer pharmakologisch aktiven Verbindung zusammen mit einem Träger oder Verdünnungsmittel dafür enthält, worin die pharmakologisch aktive Verbindung eine Verbindung der Formel (I) nach einem der Ansprüche 1 bis 16 oder ein pharmakologisch geeignetes Salz, pharmakologisch geeigneter Ester oder pharmakologisch geeignetes Amid davon ist.

**18.** Verbindung der Formel (I) nach einem der Ansprüche 1 bis 16 oder ein pharmakologisch geeignetes Salz, pharmakologisch geeigneter Ester oder pharmakologisch geeignetes Amid davon für die Verwendung als Arzneimittel.

**19.** Verbindung der Formel (I) nach einem der Ansprüche 1 bis 16 oder ein pharmakologisch geeignetes Salz, pharmakologisch geeigneter Ester oder pharmakologisch geeignetes Amid davon für die Verwendung zur Inhibition der Produktion von Entzündungen betreffenden Cytokinen-in einem Säuger.

**20.** Verbindung der Formel (I) nach einem der Ansprüche 1 bis 16 oder ein pharmakologisch geeignetes Salz, pharmakologisch geeigneter Ester oder pharmakologisch geeignetes Amid davon für die Verwendung zur Inhibition von Knochenresorption.

**21.** Verbindung der Formel (I) nach einem der Ansprüche 1 bis 16 oder ein pharmakologisch geeignetes Salz, pharmakologisch geeigneter Ester oder pharmakologisch geeignetes Amid davon für die Verwendung in der Behandlung oder Prophylaxe von Entzündungskrankheiten.

**22.** Verbindung der Formel (I) nach einem der Ansprüche 1 bis 16 oder ein pharmakologisch geeignetes Salz, pharmakologisch geeigneter Ester oder pharmakologisch geeignetes Amid davon für die Verwendung in der Behandlung oder Prophylaxe von Viruskrankheiten.

**23.** Verbindung der Formel (I) nach einem der Ansprüche 1 bis 16 oder ein pharmakologisch geeignetes Salz, pharmakologisch geeigneter Ester oder pharmakologisch geeignetes Amid davon für die Verwendung bei der Linderung von Schmerzen oder Fieberzuständen.

**24.** Verbindung der Formel (I) nach einem der Ansprüche 1 bis 16 oder ein pharmakologisch geeignetes Salz, pharmakologisch geeigneter Ester oder pharmakologisch geeignetes Amid davon für die Verwendung bei der Behandlung oder Prophylaxe von chronischer rheumatischer Arthritis.

**25.** Verbindung der Formel (I) nach einem der Ansprüche 1 bis 16 oder ein pharmakologisch geeignetes Salz, pharmakologisch geeigneter Ester oder pharmakologisch geeignetes Amid davon für die Verwendung bei der Behandlung oder Prophylaxe von Osteoarthritis.

**26.** Verbindung der Formel (I) nach einem der Ansprüche 1 bis 16 oder ein pharmakologisch geeignetes Salz, pharmakologisch geeigneter Ester oder pharmakologisch geeignetes Amid davon für die Verwendung bei der Behandlung oder Prophylaxe von Krebs.

**27.** Verbindung der Formel (I) nach einem der Ansprüche 1 bis 16 oder ein pharmakologisch geeignetes Salz, pharmakologisch geeigneter Ester oder pharmakologisch geeignetes Amid davon für die Verwendung bei der Behandlung oder Prophylaxe von Hepatitis.

**28.** Verbindung der Formel (I) nach einem der Ansprüche 1 bis 16 oder ein pharmakologisch geeignetes Salz, pharmakologisch geeigneter Ester oder pharmakologisch geeignetes Amid davon für die Verwendung bei der Behandlung oder Prophylaxe einer Krankheit, die aus der Gruppe ausgewählt ist, die aus allergischen Krankheiten, Sepsis, Psoriasis, Osteoporose, ulzeröse Kolitis, Diabetes, Nierenentzündung, ischämische Herzkrankheit, Alzheimer'sche Krankheit und Arteriosklerose besteht.

**29.** Verwendung einer Verbindung der Formel (I) nach einem der Ansprüche 1 bis 16 oder ein pharmakologisch geeignetes Salz, pharmakologisch geeigneter Ester oder pharmakologisch geeignetes Amid davon bei der Herstellung eines Arzneimittels für die Behandlung oder Prophylaxe von Krankheiten, die durch Inhibition der Produktion von Entzündungen betreffenden Cytokinen in einem Säuger behandelt oder verhindert werden können.

**30.** Verwendung nach Anspruch 29 einer Verbindung der Formel (I) nach einem der Ansprüche 1 bis 16 oder eines pharmakologisch geeigneten Salzes, Esters oder Amids davon bei der Herstellung eines Arzneimittels zur Inhibition von Knochenresorption.

**31.** Verwendung nach Anspruch 29 einer Verbindung der Formel (I) nach einem der Ansprüche 1 bis 16 oder eines pharmakologisch geeigneten Salzes, Esters oder Amids davon bei der Herstellung eines Arzneimittels für die Behandlung oder Prophylaxe von Entzündungskrankheiten.

**32.** Verwendung nach Anspruch 29 einer Verbindung der Formel (I) nach einem der Ansprüche 1 bis 16 oder eines pharmakologisch geeigneten Salzes, Esters oder Amids davon bei der Herstellung eines Arzneimittels für die Behandlung oder Prophylaxe von Viruserkrankungen.

**33.** Verwendung nach Anspruch 29 einer Verbindung der Formel (I) nach einem der Ansprüche 1 bis 16 oder eines pharmakologisch geeigneten Salzes, Esters oder Amids davon bei der Herstellung eines Arzneimittels zur Linderung von Schmerzen oder Fieberzuständen.

**34.** Verwendung nach Anspruch 29 einer Verbindung der Formel (I) nach einem der Ansprüche 1 bis 16 oder eines pharmakologisch geeigneten Salzes, Esters oder Amids davon bei der Herstellung eines Arzneimittels für die Behandlung oder Prophylaxe von chronischer rheumatischer Arthritis.

**35.** Verwendung nach Anspruch 29 einer Verbindung der Formel (I) nach einem der Ansprüche 1 bis 16 oder eines pharmakologisch geeigneten Salzes, Esters oder Amids davon bei der Herstellung eines Arzneimittels für die Behandlung oder Prophylaxe von Osteoarthritis.

**36.** Verwendung nach Anspruch 29 einer Verbindung der Formel (I) nach einem der Ansprüche 1 bis 16 oder eines pharmakologisch geeigneten Salzes, Esters oder Amids davon bei der Herstellung eines Arzneimittels für die Behandlung oder Prophylaxe von Krebs.

**37.** Verwendung nach Anspruch 29 einer Verbindung der Formel (I) nach einem der Ansprüche 1 bis 16 oder eines pharmakologisch geeigneten Salzes, Esters oder Amids davon bei der Herstellung eines Arzneimittels für die Behandlung oder Prophylaxe von Hepatitis.

**38.** Verwendung nach Anspruch 29 einer Verbindung der Formel (I) nach einem der Ansprüche 1 bis 16 oder eines pharmakologisch geeigneten Salzes, Esters oder Amids davon bei der Herstellung eines Arzneimittels für die Behandlung oder Prophylaxe einer Krankheit, die aus der-Gruppe ausgewählt ist , die aus allergischen Krankheiten, Sepsis, Psoriasis, Osteoporose, ulzeröser Kolitis, Diabetes, Nierenentzündung, ischämischer Herzkrankheit, Alzheimer'sche Krankheit und Arteriosklerose besteht.

**Revendications**

**1.** Composé de formule (I), ou un sel, un ester ou un amide pharmacologiquement acceptable de celui-ci :

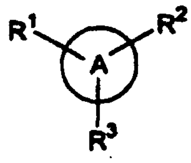

(I)

dans lequel :

A représente un cycle pyrrole ;
$R^1$ représente un groupe phényle ou naphtyle qui peut éventuellement être substitué avec au moins un substituant choisi parmi le groupe consistant en les groupes substituants α définis ci-dessous, les groupes substituants β définis ci-dessous, les groupes substituants γ définis ci-dessous et des groupes substituants δ définis ci-dessous ;
$R^2$ représente un groupe pyridyle ou pyrimidinyle qui peut être éventuellement substitué avec au moins un substituant choisi parmi le groupe consistant en les groupes substituants α définis ci-dessous, les groupes substituants β définis ci-dessous, les groupes substituants γ définis ci-dessous et les groupes substituants δ définis ci-dessous ; et
$R^3$ représente un groupe correspondant à la formule (II) suivante :

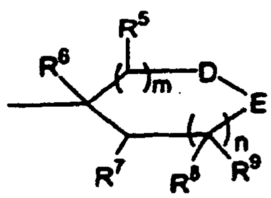

(II)

dans laquelle :

m représente 0 ou 1,
n représente 1 ou 2,
un des groupes D et E représente un groupe de formule $>NR^{10}$ et l'autre représente un groupe de formule $>CR^{11}R^{12}$,
l'un des groupes $R^5$ et $R^7$ représente ensemble avec $R^6$, une simple liaison, et l'autre représente un

substituant choisi parmi le groupe consistant en les atomes d'hydrogène, les groupes substituants α définis ci-dessous, les groupes substituants γ définis ci-dessous et les groupes substituants δ définis ci-dessous, $R^8$, $R^9$ et $R^{12}$ peuvent être identiques ou différents les uns des autres, et représentent chacun un substituant choisi parmi le groupe consistant en les atomes d'hydrogène, les groupes substituants α définis ci-dessous, les groupes substituants γ définis ci-dessous et les groupes substituants δ définis ci-dessous, et $R^{10}$ et $R^{11}$ peuvent être identiques ou différents l'un de l'autre, et représentent chacun un substituant choisi parmi le groupe consistant en les atomes d'hydrogène, les groupes substituants α définis ci-dessous, les groupes substituants γ définis ci-dessous et les groupes substituants δ définis ci-dessous, ou $R^{10}$ et $R^{11}$ forment ensemble un groupe alkylène comportant de 1 à 6 atomes de carbone qui est non substitué ou substitué avec au moins un substituant choisi parmi le groupe consistant en les groupes substituants α définis ci-dessous, les groupes substituants β définis ci-dessous, les groupes substituants γ définis ci-dessous et les groupes substituants δ définis ci-dessous ;

à condition que lesdits substituants $R^1$ et $R^3$ soient liés aux deux atomes dudit cycle pyrrole qui sont adjacents à l'atome du cycle pyrrole auquel ledit substituant $R^2$ est lié ;
les groupes substituants α comprennent les groupes hydroxy, les groupes nitro, les groupes cyano, les atomes d'halogène, les groupes alkoxy inférieurs définis ci-dessous, les groupes alkoxy inférieurs halogénés définis ci-dessous, les groupes alkylthio inférieurs définis ci-dessous, et les groupes alkylthio inférieurs halogénés définis ci-dessous ;
les groupes substituants β comprennent les groupes de formule $-NR^cR^d$ dans laquelle $R^c$ et $R^d$ sont identiques ou différents l'un de l'autre, et sont indépendamment choisis chacun parmi le groupe consistant en les atomes d'hydrogène, les groupes alkyle inférieurs définis ci-dessous, les groupes alcényle inférieurs définis ci-dessous, les groupes alcynyle inférieurs définis ci-dessous, les groupes aralkyle définis ci-dessous et les groupes alkylsulfonyle inférieurs, ou $R^c$ et $R^d$ forment ensemble avec l'atome d'azote auquel $R^c$ et $R^d$ sont liés, un groupe hétérocyclyle défini ci-dessous ;
les groupes substituants γ comprennent les groupes alkyle inférieur définis ci-dessous qui sont substitués avec un groupe de formule $-NR^cR^d$ dans laquelle $R^c$ et $R^d$ sont tels que définis ci-dessus ; et
les groupes substituants δ comprennent les groupes alkyle inférieurs définis ci-dessous qui peuvent être éventuellement substitués avec au moins un substituant choisi parmi les groupes substituants α définis ci-dessus, les groupes alcényle inférieurs définis ci-dessous qui peuvent être éventuellement substitués avec au moins un substituant choisi parmi les groupes substituants α définis ci-dessus, les groupes alcynyle inférieurs définis ci-dessus qui peuvent être éventuellement substitués avec au moins un substituant choisi parmi les groupes substituants α définis ci-dessus, les groupes aralkyle définis ci-dessous et les groupes cycloalkyle définis ci-dessous ;
lesdits groupes alkyle inférieurs dans la définition des substituants $R^c$ et $R^d$ ci-dessus, lesdits groupes alkyle inférieurs qui peuvent être éventuellement substitués avec au moins un substituant choisi parmi les groupes substituants α dans la définition des groupes substituants δ ci-dessus et ladite partie alkyle inférieure des groupes alkyle inférieurs substitués avec un groupe de formule $-NR^cR^d$ dans la définition des groupes substituants γ ci-dessus, sont des groupes alkyle linéaires ou ramifiés comportant de 1 à 6 atomes de carbone ;
lesdits groupes alcényle inférieurs dans la définition des substituants $R^c$ et $R^d$ ci-dessus, et lesdits groupes alcényle inférieurs qui peuvent être éventuellement substitués avec au moins un substituant choisi parmi les groupes substituants α dans la définition des groupes substituants δ ci-dessus, sont des groupes alcényle linéaires ou ramifiés comportant de 2 à 6 atomes de carbone ;
lesdits groupes alcynyle inférieurs dans la définition des substituants $R^c$ et $R^d$ ci-dessus, et lesdits groupes alcynyle inférieurs qui peuvent être éventuellement substitués avec au moins un substituant choisi parmi les groupes substituants α dans la définition des groupes substituants δ ci-dessus, sont des groupes alcynyle linéaires ou ramifiés comportant de 2 à 6 atomes de carbone ;
lesdits groupes aralkyle dans la définition des groupes $R^c$ et $R^d$ ci-dessus et dans la définition des groupes substituants δ ci-dessus, sont des groupes alkyle inférieurs tels que définis ci-dessus qui sont substitués avec au moins un groupe aryle choisi parmi les groupes hydrocarbonés aromatiques comportant de 6 à 14 atomes de carbone dans un ou plusieurs cycles, lesdits groupes hydrocarbonés aromatiques étant éventuellement condensés avec un groupe cycloalkyle comportant de 3 à 10 atomes de carbone ;
lesdits groupes cycloalkyle dans les groupes substituants δ ci-dessus, sont des groupes cycloalkyle comportant de 3 à 7 atomes de carbone ;
lesdits groupes alkylsulfonyle inférieurs dans la définition des substituants $R^c$ et $R^d$, sont des groupes alkyle inférieurs tels que définis ci-dessus qui sont liés à un groupe sulfonyle ;
lesdits groupes hétérocyclyle formés par les substituants $R^c$ et $R^d$ ensemble avec l'atome d'azote auquel ils sont liés, sont des groupes hétérocyclyle non aromatiques de 4 à 12 chaînons contenant un atome d'azote et

contenant en outre éventuellement un autre hétéroatome choisi parmi le groupe consistant en les atomes d'oxygène, de soufre et d'azote, lesdits groupes hétérocyclyle étant éventuellement condensés avec un autre groupe cyclique choisi parmi le groupe consistant en les groupes aryle tels que définis dans la définition des groupes aralkyle ci-dessus, et les groupes hétéroaryle choisis parmi les groupes hétérocycliques aromatiques de 5 à 7 chaînons contenant de 1 à 3 hétéroatomes choisis parmi le groupe consistant en les atomes de soufre, d'oxygène et d'azote ;
lesdits groupes alkoxy inférieurs dans la définition des groupes substituants $\alpha$ ci-dessus, sont des groupes alkyle inférieurs tels que définis ci-dessus qui sont liés à un atome d'oxygène ;
lesdits groupes alkoxy inférieurs halogénés dans la définition des groupes substituants $\alpha$ ci-dessus, sont des groupes alkoxy inférieurs tels que définis ci-dessus qui sont substitués avec au moins un atome d'halogène ;
lesdits groupes alkylthio inférieurs dans la définition des groupes substituants $\alpha$ ci-dessus, sont des groupes alkyle inférieurs tels que définis ci-dessus qui sont liés à un atome de soufre ; et
lesdits groupes alkylthio inférieurs halogénés dans la définition des groupes substituants $\alpha$ ci-dessus, sont les groupes alkylthio inférieurs tels que définis ci-dessus qui sont substitués avec au moins un atome d'halogène.

**2.** Composé selon la revendication 1, ou un sel, un ester ou un amide pharmaceutiquement acceptable de celui-ci, dans lequel :

$R^1$ représente un groupe phényle qui peut être éventuellement substitué avec au moins un substituant choisi parmi le groupe consistant en les groupes substituants $\alpha$ définis ci-dessous, les groupes substituants $\beta$ définis ci-dessous, les groupes substituants $\gamma$ définis ci-dessous et les groupes substituants $\delta$ définis ci-dessous ;
$R^2$ représente un groupe 4-pyridyle ou 4-pyrimidinyle qui peut être éventuellement substitué avec au moins un substituant choisi parmi le groupe consistant en les groupes substituants $\alpha$, les groupes substituants $\beta$, les groupes substituants $\gamma$ et les groupes substituants $\delta$ ; et
$R^3$ représente un groupe de formule (II) tel que défini dans la revendication 1 dans lequel m est égal à 1 et n est égal à 1 ;
lesdits groupes substituants $\alpha^1$ comprenant les atomes d'halogène, les groupes alkoxy inférieurs tels que définis dans la revendication 1, et les groupes alkoxy inférieurs halogénés tels que définis dans la revendication 1 ;
lesdits groupes substituants $\beta^1$ comprenant un groupe de formule $-NR^cR^d$ dans lequel l'un des groupes $R^c$ et $R^d$ est choisi parmi le groupe consistant en les atomes d'hydrogène et les groupes alkyle inférieurs tels que définis dans la revendication 1, et l'autre est choisi parmi le groupe consistant en les atomes d'hydrogène, les groupes alkyle inférieurs tels que définis dans la revendication 1 et les groupes aralkyle tels que définis dans la revendication 1 ;
lesdits groupes substituants $\gamma^1$ comprenant les groupes alkyle inférieurs tels que définis dans la revendication 1 qui sont substitués avec un substituant choisi parmi le groupe consistant en les groupes amino, les groupes amino substitués avec un ou deux groupes alkyle inférieurs tels que définis dans la revendication 1, et les groupes amino substitués avec un groupe aralkyle tel que défini dans la revendication 1 ; et
lesdits groupes substituants $\delta^1$ comprenant les groupes alkyle inférieurs tels que définis dans la revendication 1, les groupes alkyle inférieurs halogénés comprenant les groupes alkyle inférieurs tels que définis dans la revendication 1 qui sont substitués avec au moins un atome d'halogène, les groupes alkyle inférieurs hydroxylés comprenant les groupes alkyle inférieurs tels que définis dans la revendication 1 qui sont substitués avec au moins un groupe hydroxy, et les groupes alkyle inférieurs nitrés comprenant les groupes alkyle inférieurs tels que définis dans la revendication 1 qui sont substitués avec au moins un groupe nitro.

**3.** Composé de formule (I) selon la revendication 1, ou un sel, un ester ou un amide pharmacologiquement acceptable de celui-ci, dans lequel :

$R^1$ représente un groupe phényle qui peut être éventuellement substitué avec au moins un substituant choisi parmi le groupe consistant en les groupes substituants $\alpha^1$ définis ci-dessous, les groupes substituants $\beta^1$ définis ci-dessous, les groupes substituants $\gamma^1$ définis ci-dessous et les groupes substituants $\delta^1$ définis ci-dessous ;
$R^2$ représente un groupe 4-pyridyle ou 4-pyrimidinyle qui peut être éventuellement substitué avec au moins un substituant choisi parmi le groupe consistant en les groupes substituants $\alpha$, les groupes substituants $\beta$, les groupes substituants $\gamma$, et les groupes substituants $\delta$ ; et
$R^3$ représente un groupe de formule (II) tel que défini dans la revendication 1, dans lequel E représente un groupe de formule $>NR^{10}$ dans laquelle $R^{10}$ est tel que défini dans la revendication 1, et D représente un

groupe de formule >$CR^{11}R^{12}$ dans laquelle $R^{11}$ et $R^{12}$ sont tels que définis dans la revendication 1 ;
lesdits groupes substituants $\alpha^1$ comprenant les atomes d'halogène, les groupes alkoxy inférieurs tels que définis dans la revendication 1, et les groupes alkoxy inférieurs halogénés tels que définis dans la revendication 1 ;
lesdits groupes substituants $\beta^1$ comprenant un groupe de formule $-NR^cR^d$ dans laquelle l'un des groupes $R^c$ et $R^d$ est choisi parmi les groupes consistant en les atomes d'hydrogène et les groupes alkyle inférieurs tels que définis dans la revendication 1, et l'autre est choisi parmi le groupe consistant en les atomes d'hydrogène, les groupes alkyle inférieurs tels que définis dans la revendication 1, et les groupes aralkyle tels que définis dans la revendication 1 ;
lesdits groupes substituants $\gamma^1$ comprenant les groupes alkyle inférieurs tels que définis dans la revendication 1 qui sont substitués avec un substituant choisi parmi le groupe consistant en les groupes amino, les groupes amino substitués avec un ou deux groupes alkyle inférieurs tels que définis dans la revendication 1, et les groupes amino substitués avec un groupe aralkyle tel que défini dans la revendication 1 ; et
lesdits groupes substituants $\delta^1$ comprenant les groupes alkyle inférieurs tels que définis dans la revendication 1, les groupes alkyle inférieurs halogénés comprenant les groupes alkyle inférieurs tels que définis dans la revendication 1 qui sont substitués avec au moins un atome d'halogène, les groupes alkyle inférieurs hydroxylés comprenant les groupes alkyle inférieurs tels que définis dans la revendication 1 qui sont substitués avec au moins un groupe hydroxy, et les groupes alkyle inférieurs nitrés comprenant les groupes alkyle inférieurs tels que définis dans la revendication 1 qui sont substitués avec au moins un groupe nitro.

**4.** Composé de formule (I) selon la revendication 1, ou un sel, un ester ou un amide pharmacologiquement acceptable de celui-ci, dans lequel :

$R^1$ représente un groupe phényle qui peut être éventuellement substitué avec au moins un substituant choisi parmi le groupe consistant en les atomes d'halogène, les groupes alkyle inférieurs halogénés comprenant les groupes alkyle inférieurs tels que définis dans la revendication 1 qui sont substitués avec au moins un atome d'halogène, et les groupes alkoxy inférieurs halogénés tels que définis dans la revendication 1 ; et
$R^2$ représente un groupe 4-pyridyle ou 4-pyrimidinyle qui peut être éventuellement substitué à sa position 2 avec un substituant choisi parmi le groupe consistant en les groupes substituants $\alpha$, les groupes substituants $\beta$, les groupes substituants $\gamma$ et les groupes substituants $\delta$.

**5.** Composé de formule (I) selon la revendication 1, ou un sel, un ester ou un amide pharmacologiquement acceptable de celui-ci, dans lequel :

$R^1$ est choisi parmi le groupe consistant en les groupes phényle, 4-fluorophényle, 3-fluorophényle, 3-chlorophényle, 3,4-difluorophényle, 3,4,5-trifluorophényle, 3-chloro-4-fluorophényle, 3-difluorométhoxyphényle et 3-trifluorométhylphényle ;
$R^2$ représente un groupe 4-pyridyle ou 4-pyrimidinyle qui peut être éventuellement substitué à sa position 2 avec un substituant choisi parmi le groupe consistant en les groupes méthoxy, amino, méthylamino, benzylamino et $\alpha$-méthylbenzylamino ; et
$R^3$ représente un groupe de formule (II) tel que défini dans la revendication 1, dans lequel $R^{10}$ et $R^{11}$ forment ensemble un groupe alkylène à chaîne linéaire comportant 3 ou 4 atomes de carbone qui est non substitué ou substitué avec au moins un substituant choisi parmi le groupe consistant en les groupes substituants $\alpha$, les groupes substituants $\beta$, les groupes substituants $\gamma$ et les groupes substituants $\delta$.

**6.** Composé de formule (I) selon la revendication 1, ou un sel, un ester ou un amide pharmacologiquement acceptable de celui-ci, dans lequel :

$R^1$ est choisi parmi le groupe consistant en les groupes phényle, 4-fluorophényle, 3-fluorophényle, 3-chlorophényle, 3,4-difluorophényle, 3,4,5-trifluorophényle, 3-chloro-4-fluorophényle, 3-difluorométhoxyphényle et 3-trifluorométhylphényle ;
$R^2$ représente un groupe 4-pyridyle ou 4-pyrimidinyle qui peut être éventuellement substitué à sa position 2 avec un substituant choisi parmi le groupe consistant en les groupes méthoxy, amino, méthylamino, benzylamino et $\alpha$-méthylbenzylamino ; et
$R^3$ représente un groupe de formule (II) tel que défini dans la revendication 1 dans lequel chacun des groupes $R^7$, $R^8$ et $R^9$ représente un atome d'hydrogène, et $R^{10}$ et $R^{11}$ forment ensemble un groupe alkylène à chaîne linéaire comportant 3 ou 4 atomes de carbone, qui est non substitué ou substitué avec au moins un substituant choisi parmi le groupe consistant en les groupes substituants $\alpha$, les groupes substituants $\beta$, les groupes subs-

tituants γ et les groupes substituants δ.

**7.** Composé de formule (I) selon l'une quelconque des revendications 1 à 6, ou un sel, un ester ou un amide pharmacologiquement acceptable de celui-ci, dans lequel ledit composé de formule (I), est un composé de formule (I-1) ou (I-3) ci-dessous :

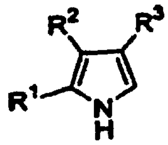
(I-1)

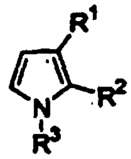
(I-3)

**8.** Composé de formule (I) selon la revendication 1, choisi parmi le groupe de composés suivant, ou un sel, un ester ou un amide pharmacologiquement acceptable de celui-ci :

le 4-(1-acétyl-1,2,3,6-tétrahydropyridin-4-yl)-2-(4-fluorophényl)-3-(pyridin-4-yl)-1*H*-pyrrole,
le 2-(4-fluorophényl)-3-(pyridin-4-yl)-4-(1,2,3,6-tétrahydropyridin-4-yl)-4-(1,2,3,6-tétrahydropyridin-4-yl)-1*H*-pyrrole,
le 2-(4-fluorophényl)-4-(1-mëthyl-1,2,3,6-tétrahydropyridin-4-yl)-3-pyridin-4-yl)-1*H*-pyrrole,
le 2-(4-fluorophényl)-3-(pyridin-4-yl)-4-(1,2,3,6-tétrahydro-2,2,6,6-tétraméthylpyridin-4-yl)-1*H*-pyrrole,
le 2-(4-fluorophényl)-4-(2-méthyl-1,2,3,6-tétrahydropyridin-4-yl)-3-pyridin-4-yl)-1*H*-pyrrole,
le 2-(4-fluorophényl)-4-(6-méthyl-1,2,3,6-tétrahydropyridin-4-yl)-3-(pyridin-4-yl)-1*H*-pyrrole,
le 4-(1-éthyl-1,2,3,6-tétrahydropyridin-4-yl)-2-(4-fluorophényl)-3-(pyridin-4-yl)-1*H*-pyrrole,
le 2-(4-fluorophényl)-4-(1-isopropyl-1,2,3,6-tétrahydropyridin-4-yl)-3-(pyridin-4-yl)-1*H*-pyrrole,
le 2-(4-fluorophényl)-4-(1-propyl-1,2,3,6-tétrahydropyridin-4-yl)-3-(pyridin-4-yl)-1*H*-pyrrole,
le 2-(9-fluorophényl)-4-(1-phénéthyl-1,2,3,6-tétrahydropyridin-4-yl)-3-(pyridin-4-yl)-1*H*-pyrrole,
le 2-(4-fluorophényl)-4-(1,2,3,5,8,8a-hexahydroindolizin-7-yl)-3-(pyridin-4-yl)-1*H*-pyrrole,
le 2-(4-fluorophényl)-4-(1,2,3,5,6,8a-hexahydroindolizin-7-yl)-3-(pyridin-4-yl)-1*H*-pyrrole,
le 4-(1,6-diméthyl-1,2,3,6-tétrahydropyridin-4-yl)-2-(4-fluorophényl)-3-(pyridin-4-yl)-1*H*-pyrrole,
le 4-(1,2-diméthyl-1,2,3,6-tétrahydropyridin-4-yl)-2-(4-fluorophényl)-3-(pyridin-4-yl)-1*H*-pyrrole,
le 2-(4-fluorophényl)-4-(1,2,2,6,6-pentaméthyl-1,2,3,6-tétrahydropyridin-4-yl)-3-(pyridin-4-yl)-1*H*-pyrrole,
le 4-(1-éthyl-6-méthyl-1,2,3,6-tétrahydropyridin-4-yl)-2-(4-fluorophényl)-3-(pyridin-4-yl)-1*H*-pyrrole,
le 4-(1-éthyl-2-méthyl-1,2,3,6-tétrahydropyridin-4-yl)-2-(4-fluorophényl)-3-(pyridin-4-yl)-1*H*-pyrrole,
le 4-(6-allyl-1-méthyl-1,2,3,6-tétrahydropyridin-4-yl)-2-(4-fluorophényl)-3-(pyridin-4-yl)-1*H*-pyrrole,
le 4-(2-allyl-1-méthyl-1,2,3,6-tétrahydropyridin-4-yl)-2-(4-fluorophényl)-3-(pyridin-4-yl)-1*H*-pyrrole,
le 4-(6-benzyl-1-méthyl-1,2,3,6-tétrahydropyridin-4-yl)-2-(4-fluorophényl)-3-(pyridin-4-yl)-1*H*-pyrrole,
le 4-(2-benzyl-1-méthyl-1,2,3,6-tétrahydropyridin-4-yl)-2-(4-fluorophényl)-3-(pyridin-4-yl)-1*H*-pyrrole,
le 2-(4-fluorophényl)-4-(6-méthyl-1-propyl-1,2,3,6-tétrahydropyridin-4-yl)-3-(pyridin-4-yl)-1*H*-pyrrole,
le 2-(4-fluorophényl)-4-(2-méthyl-1-propyl-1,2,3,6-tétrahydropyridin-4-yl)-3-(pyridin-4-yl)-1*H*-pyrrole,
le 2-(4-fluorophényl)-4-(1,3,4,6,7,9a-hexahydro-2*H*-quinolizin-8-yl)-3-(pyridin-4-yl)-1*H*-pyrrole,
le 2-(4-fluorophényl)-4-(1,3,4,6,9,9a-hexahydro-2*H*-quinolizin-8-yl)-3-(pyridin-4-yl)-1*H*-pyrrole,
le 2-(4-fluorophényl)-4-(1,2,3,5,8,8a-hexahydroindolizin-7-yl)-3-(2-méthylaminopyrimidin-4-yl)-1*H*-pyrrole,
le 2-(4-fluorophényl)-4-(1,2,3,5,6,8a-hexahydroindolizin-7-yl)-3-(2-méthylaminopyrimidin-4-yl)-1*H*-pyrrole,
le 2-(3,4-difluorophényl)-4-(1,2,3,5,8,8a-hexahydroindolizin-7-yl)-3-(pyridin-4-yl)-1*H*-pyrrole,
le 2-(3,4-difluorophényl)-4-(1,2,3,5,6,8a-hexahydroindolizin-7-yl)-3-(pyridin-4-yl)-1*H*-pyrrole,
le 4-(1,2,3,5,8,8a-hexahydroindolizin-7-yl)-3-(pyridin-4-yl)-2-(3-trifluorométhylphényl)-1*H*-pyrrole,
le 4-(1,2,3,5,6,8a-hexahydroindolizin-7-yl)-3-(pyridin-4-yl)-2-(3-trifluorométhylphényl)-1*H*-pyrrole,
le 2-(3-fluorophényl)-4-(1,2,3,5,8,8a-hexahydroindolizin-7-yl)-3-(pyridin-4-yl)-1*H*-pyrrole,
le 2-(3-fluorophényl)-4-(1,2,3,5,6,8a-hexahydroindolizin-7-yl)-3-(pyridin-4-yl)-1*H*-pyrrole,
le 2-(3-chlorophényl)-4-(1,2,3,5,8,8a-hexahydroindolizin-7-yl)-3-(pyridin-4-yl)-1*H*-pyrrole,
le 2-(3-chlorophényl)-4-(1,2,3,5,6,8a-hexahydroindolizin-7-yl)-3-(pyridin-4-yl)-1*H*-pyrrole,
le 4-(1,2,3,5,8,8a-hexahydroindolizin-7-yl)-2-phényl-3-(pyridin-4-yl)-1*H*-pyrrole, et
le 4-(1,2,3,5,6,8a-hexahydroindolizin-7-yl)-2-phényl-3-(pyridin-4-yl)-1*H*-pyrrole.

**9.** Composé de formule (I) selon la revendication 1 choisi parmi le groupe de composés suivants, ou un sel, un ester

ou un amide pharmacologiquement acceptable de celui-ci :

le 2-(4-fluorophényl)-3-(pyridin-4-yl)-4-(1,2,3,6-tétrahydropyridin-4-yl)-1*H*-pyrrole,
le 2-(4-fluorophényl)-4-(6-méthyl-1,2,3,6-tétrahydropyridin-4-yl)-3-(pyridin-4-yl)-1*H*-pyrrole,
le 4-(1-éthyl-1,2,3,6-tétrahydropyridin-4-yl)-2-(4-fluorophényl)-3-(pyridin-4-yl)-1*H*-pyrrole,
le 2-(4-fluorophényl)-4-(1-isopropyl-1,2,3,6-tétrahydropyridin-4-yl)-3-(pyridin-4-yl)-1*H*-pyrrole,
le 2-(4-fluorophényl)-4-(1-propyl-1,2,3,6-tétrahydropyridin-4-yl)-3-(pyridin-4-yl)-1*H*-pyrrole,
le 2-(4-fluorophényl)-4-(1-phénéthyl-1,2,3,6-tétrahydropyridin-4-yl)-3-(pyridin-4-yl)-1*H*-pyrrole,
le 2-(4-fluorophényl)-4-(1,2,3,5,6,8a-hexahydroindolizin-7-yl)-3-(pyridin-4-yl)-1*H*-pyrrole,
le 4-(1,6-diméthyl-1,2,3,6-tétrahydropyridin-4-yl)-2-(4-fluorophényl)-3-(pyridin-4-yl)-1*H*-pyrrole,
le 4-(1,2-diméthyl-1,2,3,6-tétrahydropyridin-4-yl)-2-(4-fluorophényl)-3-(pyridin-4-yl)-1*H*-pyrrole,
le 4-(1-éthyl-6-méthyl-1,2,3,6-tétrahydropyridin-4-yl)-2-(4-fluorophényl)-3-pyridin-4-yl)-1*H*-pyrrole,
le 4-(1-éthyl-2-méthyl-1,2,3,6-tétrahydropyridin-4-yl)-2-(4-fluorophényl)-3-(pyridin-4-yl)-1H-pyrrole,
le 2-(4-fluorophényl)-4-(6-méthyl-1-propyl-1,2,3,6-tétrahydropyridin-4-yl)-3-(pyridin-4-yl)-1*H*-pyrrole,
le 2-(4-fluorophényl)-4-(2-méthyl-1-propyl-1,2,3,6-tétrahydropyridin-4-yl)-3-(pyridin-4-yl)-1*H*-pyrrole,
le 2-(4-fluorophényl)-4-(1,3,4,6,7,9a-hexahydro-2*H*-quinolizin-8-yl)-3-(pyridin-4-yl)-1*H*-pyrrole,
le 2-(3,4-difluorophényl)-4-(1,2,3,5,6,8a-hexahydroindolizin-7-yl)-3-(pyridin-4-yl)-1*H*-pyrrole, et
le 4-(1,2,3,5,6,8a-hexahydroindolizin-7-yl)-3-(pyridin-4-yl)-2-(3-trifluorométhylphényl)-1*H*-pyrrole.

**10.** Composé selon la revendication 1 choisi parmi le 2-(4-fluorophényl)-4-(1-phénéthyl-1,2,3,6-tétrahydropyridin-4-yl)-3-(pyridin-4-yl)-1*H*-pyrrole, et les sels, les esters et les amides pharmacologiquement acceptables de celui-ci.

**11.** Composé selon la revendication 1 choisi parmi le 2-(4-fluorophényl)-4-(1,2,3,5,6,8a-hexahydroindolizin-7-yl)-3-(pyridin-4-yl)-1*H*-pyrrole, et les sels, les esters et les amides pharmacologiquement acceptables de celui-ci.

**12.** Composé selon la revendication 1 choisi parmi le 2-(4-fluorophényl)-4-(6-méthyl-1-propyl-1,2,3,6-tétrahydropyridin-4-yl)-3-(pyridin-4-yl)-1*H* pyrrole, et les sels, les esters et les amides pharmacologiquement acceptables de celui-ci.

**13.** Composé selon la revendication 1 choisi parmi le 2-(4-fluorophényl)-4-(2-méthyl-1-propyl-1,2,3,6-tétrahydropyridin-4-yl)-3-(pyridin-4-yl)-1*H*-pyrrole, et les sels, les esters et les amides pharmacologiquement acceptables de celui-ci.

**14.** Composé selon la revendication 1 choisi parmi le 2-(4-fluorophényl)-4-(1,3,4,6,7,9a-hexahydro-2*H*-quinolizin-8-yl)-3-(pyridin-4-yl)-1*H*-pyrrole, et les sels, les esters et les amides pharmacologiquement acceptables de celui-ci.

**15.** Composé selon la revendication 1 choisi parmi le 2-(3,4-difluorophényl)-4-(1,2,3,5,6,8a-hexahydroindolizin-7-yl)-3-(pyridin-4-yl)-1*H*-pyrrole, et les sels, les esters et les amides pharmacologiquement acceptables de celui-ci.

**16.** Composé selon la revendication 1 choisi parmi le 4-(1,2,3,5,6,8a-hexahydroindolizin-7-yl)-3-pyridin-4-yl)-2-(3-trifluorométhylphényl)-1*H*-pyrrole, et les sels, les esters et les amides pharmacologiquement acceptables de celui-ci.

**17.** Composition pharmaceutique comprenant une quantité efficace d'un composé pharmacologiquement actif avec un véhicule ou un diluant pour celui-ci, dans laquelle ledit composé pharmacologiquement actif est un composé de formule (I) selon l'une quelconque des revendications 1 à 16, ou un sel, un ester ou un amide pharmacologiquement acceptable de celui-ci.

**18.** Composé de formule (I) selon l'une quelconque des revendications 1 à 16, ou un sel, un ester ou un amide pharmacologiquement acceptable de celui-ci, destiné à être employé comme médicament.

**19.** Composé de formule (I) selon l'une quelconque des revendications 1 à 16, ou un sel, un ester ou un amide pharmacologiquement acceptable de celui-ci destiné à être utilisé pour inhiber la production des cytokines inflammatoires chez un mammifère.

**20.** Composé de formule (I) selon l'une quelconque des revendications 1 à 16, ou un sel, un ester ou un amide pharmacologiquement acceptable de celui-ci, destiné à être utilisé pour inhiber la résorption osseuse.

**21.** Composé de formule (I) selon l'une quelconque des revendications 1 à 16, ou un sel, un ester ou un amide pharmacologiquement acceptable de celui-ci, destiné à être utilisé pour le traitement ou la prophylaxie des maladies inflammatoires.

**22.** Composé de formule (I) selon l'une quelconque des revendications 1 à 16, ou d'un sel, d'un ester ou d'un amide pharmacologiquement acceptable de celui-ci, destiné à être utilisé pour le traitement ou la prophylaxie de maladies virales.

**23.** Composé de formule (I) selon l'une quelconque des revendications 1 à 16, ou un sel, un ester ou un amide pharmacologiquement acceptable de celui-ci, destiné à être utilisé pour atténuer la douleur ou la pyrexie.

**24.** Composé de formule (I) selon l'une quelconque des revendications 1 à 16, ou un sel, un ester ou un amide pharmacologiquement acceptable de celui-ci, destiné à être utilisé pour le traitement ou la prophylaxie de l'arthrite rhumatoïde chronique.

**25.** Composé de formule (I) selon l'une quelconque des revendications 1 à 16, ou un sel, un ester ou un amide pharmacologiquement acceptable de celui-ci, destiné à être utilisé pour le traitement ou la prophylaxie de l'ostéoarthrite.

**26.** Composé de formule (I) selon l'une quelconque des revendications 1 à 16, ou un sel, un ester ou un amide pharmacologiquement acceptable de celui-ci, destiné à être utilisé pour le traitement ou la prophylaxie d'un cancer.

**27.** Composé de formule (I) selon l'une quelconque des revendications 1 à 16, ou un sel, un ester ou un amide pharmacologiquement acceptable de celui-ci, destiné à être utilisé pour le traitement ou la prophylaxie d'une l'hépatite.

**28.** Composé de formule (I) selon l'une quelconque des revendications 1 à 16, ou un sel, un ester ou un amide pharmacologiquement acceptable de celui-ci, destiné à être utilisé pour le traitement ou la prophylaxie d'une maladie choisie parmi le groupe consistant en les maladies allergiques, une septicémie, le psoriasis, l'ostéoporose, la colite ulcéreuse, le diabète, la néphrite, une cardiopathie ischémique, la maladie d'Alzheimer et l'artériosclérose.

**29.** Utilisation d'un composé de formule (I) selon l'une quelconque des revendications 1 à 16, ou d'un sel, d'un ester ou d'un amide pharmacologiquement acceptable de celui-ci, pour la préparation d'un médicament destiné au traitement ou à la prophylaxie des maladies qui peuvent être traitées ou prévenues en inhibant la production des cytokines inflammatoires chez un mammifère.

**30.** Utilisation selon la revendication 29 d'un composé de formule (I) selon l'une quelconque des revendications 1 à 16, ou d'un sel, d'un ester ou d'un amide pharmacologiquement acceptable de celui-ci, pour la préparation d'un médicament destiné à inhiber la résorption osseuse.

**31.** Utilisation selon la revendication 29 d'un composé de formule (I) selon l'une quelconque des revendications 1 à 16, ou d'un sel, d'un ester ou d'un amide pharmacologiquement acceptable de celui-ci, pour la préparation d'un médicament destiné au traitement ou à la prophylaxie des maladies inflammatoires.

**32.** Utilisation selon la revendication 29 d'un composé de formule (I) selon l'une quelconque des revendications 1 à 16, ou d'un sel, d'un ester ou d'un amide pharmacologiquement acceptable de celui-ci, pour la préparation d'un médicament destiné au traitement ou à la prophylaxie de maladies virales.

**33.** Utilisation selon la revendication 29 d'un composé de formule (I) selon l'une quelconque des revendications 1 à 16, ou d'un sel, d'un ester ou d'un amide pharmacologiquement acceptable de celui-ci, pour la préparation d'un médicament destiné à atténuer la douleur ou la pyrexie.

**34.** Utilisation selon la revendication 29 d'un composé de formule (I) selon l'une quelconque des revendications 1 à 16, ou d'un sel, d'un ester ou d'un amide pharmacologiquement acceptable de celui-ci, pour la préparation d'un médicament destiné au traitement ou à la prophylaxie de l'arthrite rhumatoïde chronique.

**35.** Utilisation selon la revendication 29 d'un composé de formule (I) selon l'une quelconque des revendications 1 à 16, ou d'un sel, d'un ester ou d'un amide pharmacologiquement acceptable de celui-ci, pour la préparation d'un médicament destiné au traitement ou à la prophylaxie de l'ostéoarthrite.

**36.** Utilisation selon la revendication 29 d'un composé de formule (I) selon l'une quelconque des revendications 1 à

16, ou d'un sel, d'un ester ou d'un amide pharmacologiquement acceptable de celui-ci, pour la préparation d'un médicament destiné au traitement ou à la prophylaxie d'un cancer.

**37.** Utilisation selon la revendication 29 d'un composé de formule (I) selon l'une quelconque des revendications 1 à 16, ou d'un sel, d'un ester ou d'un amide pharmacologiquement acceptable de celui-ci, pour la préparation d'un médicament destiné au traitement ou à la prophylaxie d'une hépatite.

**38.** Utilisation selon la revendication 29 d'un composé de formule (I) selon l'une quelconque des revendications 1 à 16, ou d'un sel, d'un ester ou d'un amide pharmacologiquement acceptable de celui-ci, pour la préparation d'un médicament destiné au traitement ou à la prophylaxie d'une maladie choisie parmi le groupe consistant en les maladies allergiques, une septicémie, le psoriasis, l'ostéoporose, la colite ulcéreuse, le diabète, la néphrite, une cardiopathie ischémique, la maladie d'Alzheimer et l'artériosclérose.